(19)

**Europäisches Patentamt**

**European Patent Office**

**Office européen des brevets**

(11) **EP 4 721 818 A2**

(12) **EUROPEAN PATENT APPLICATION**

(43) Date of publication:
08.04.2026 Bulletin 2026/15

(21) Application number: 25222696.4

(22) Date of filing: **29.05.2020**

(51) International Patent Classification (IPC):
*A61P 25/28* (2006.01)

(52) Cooperative Patent Classification (CPC):
**A61P 25/08; A61P 25/16; A61P 25/28;**
**C07K 14/4705; C07K 14/705; C12N 15/86;**
C07K 2319/71; C07K 2319/80; C07K 2319/81;
C12N 2310/20; C12N 2740/16043;
C12N 2750/14143

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **29.05.2019 US 201962854238 P**
**05.06.2019 US 201962857727 P**
**10.04.2020 US 202063008569 P**

(62) Document number(s) of the earlier application(s) in
accordance with Art. 76 EPC:
**20815541.6 / 3 976 798**

(71) Applicant: **Encoded Therapeutics, Inc.**
**South San Francisco, CA 94080 (US)**

(72) Inventors:
• **TAGLIATELA, Stephanie**
**South San Francisco, 94080 (US)**
• **TANENHAUS, Anne**
**South San Francisco, 94080 (US)**

• **RAMAMOORTHI, Kartik**
**South San Francisco, 94080 (US)**
• **YOUNG, Andrew**
**South San Francisco, 94080 (US)**
• **OBERKOFLER, David**
**South San Francisco, 94080 (US)**

(74) Representative: **Carpmaels & Ransford LLP**
**One Southampton Row**
**London WC1B 5HA (GB)**

Remarks:
•The complete document including Reference
Table(s) and the Sequence Listing(s) can be
downloaded from the EPO website
•This application was filed on 11-12-2025 as a
divisional application to the application mentioned
under INID code 62.
•Claims filed after the date of filing of the application/
after the date of receipt of the divisional application
(Rule 68(4) EPC).

(54) **COMPOSITIONS AND METHODS FOR SELECTIVE GENE REGULATION**

(57)    Provided herein are engineered transcription factors for selective upregulation of SCNla and uses thereof for treating diseases and disorders, such as, Dravet syndrome. Also provided are microRNA binding sites and uses thereof for selective expression in parvalbumin neurons.

**EP 4 721 818 A2**

**Description**

**CROSS-REFERENCE**

This application claims the benefit of U.S. Provisional Patent Application No. 62/854,238, filed May 29, 2019; U.S. Provisional Patent Application No. 62/857,727, filed June 5, 2019; and U.S. Provisional Patent Application No. 63/008,569, filed April 10, 2020, each of which is incorporated herein by reference in its entirety.

**SEQUENCE LISTING**

The instant application contains a Sequence Listing which has been submitted electronically in ASCII format and is hereby incorporated by reference in its entirety. Said ASCII copy, created on May 28, 2020, is named 46482-724_601_SL.txt and is 418,483 bytes in size.

**BACKGROUND**

A broad range of human diseases are associated with abnormal expression of genes. In some cases, a genetic mutation in a gene causes it to be dysregulated, downregulated, or not expressed at all, resulting in haploinsufficiency. In some cases, a genetic mutation in a gene causes it to be upregulated, resulting in overexpression of the gene. Many challenges exist in treating genetic disorders or diseases. One approach is gene therapy, which involves therapeutic delivery of a nucleic acid into a patient's cells. However, various challenges associated with gene therapy remain unsolved, such as unwanted immune response elicited by gene therapy, off-target effects, limitations on cloning capacity of gene therapy vehicles (e.g., viruses), sustaining the therapeutic effect over a longer period of time, etc. The central nervous system (CNS) poses many unique challenges for the development of a therapy that addresses the underlying impairment in a gene and/or protein expression. While there are drugs that help to manage symptoms of CNS diseases/disorders, many CNS diseases/disorders, e.g, Dravet syndrome, lack specific treatments or a cure. Thus, there is a need for novel compositions and methods capable of modulating the expression of any endogenous gene to help reverse the effects of a disease or disorder, in particular, a therapy with reduced immunogenicity, reduced off-target effects, increased specificity for a target gene, and/or increased therapeutic efficacy.

**SUMMARY**

In one aspect, the application provides an expression cassette comprising a sequence encoding a non-naturally occurring transcription factor which increases expression of the SCN1A gene in a cell, wherein the non-naturally occurring transcription factor comprises a DNA

binding domain (DBD) operably linked to at least two transcription activating domains (TAD) in the following manner: TAD1-TAD2-DBD, DBD-TAD3-TAD4, or TAD1-TAD2-DBD-TAD3-TAD4. In certain embodiments, TAD1, TAD2, TAD3 and TAD4 are independently selected from the following: VP16, VP64, Viper, CITED2, CITED4, CREB3 or functional fragments thereof. In certain embodiments, TAD1 and TAD2 are the same TAD. In certain embodiments, TAD1 and TAD2 are CITED2, or a functional fragment thereof. In certain embodiments, TAD1 and TAD2 are CITED4, or a functional fragment thereof. In certain embodiments, TAD3 and TAD4 are the same TAD. In certain embodiments, TAD3 and TAD4 are CITED2, or a functional fragment thereof. In certain embodiments, TAD3 and TAD4 are CITED4, or a functional fragment thereof. In certain embodiments, TAD1, TAD2, TAD3 and TAD4 are the same TAD. In certain embodiments, TAD1, TAD2, TAD3 and TAD4 are CITED2, or a functional fragment thereof. In certain embodiments, TAD1, TAD2, TAD3 and TAD4 are CITED4, or a functional fragment thereof.

In certain embodiments, there is no linker between the at least two TAD domains.

In certain embodiments, there is a linker between the at least two TAD domains. In certain embodiments, the linker comprises or consists of GGSGGGGSG (SEQ ID NO: 177) or GGSGGGSGGGSGGGSG (SEQ ID NO: 178).

In certain embodiments, the DBD binds to a genomic region having 18-27 nucleotides.

In certain embodiments, the DBD comprises at least 80% sequence identity to its closest human counterpart. In certain embodiments, the DBD comprises at least 90% sequence identity to its closest human counterpart. In certain embodiments, the DBD and the at least two TAD each comprise at least 80% sequence identity to their closest human counterparts. In certain embodiments, the DBD and the at least two TAD each comprise at least 90% sequence identity to their closest human counterparts.

In certain embodiments, the DBD comprises a guide RNA and a nuclease inactivated Cas protein. In certain embodiments, the nuclease inactivated Cas protein is a nuclease inactivated Cas9.

In certain embodiments, the DBD comprises a zinc finger domain. In certain embodiments, the DBD comprises six to nine zinc finger domains. In certain embodiments, the DBD comprises six zinc fingers. In certain embodiments, the DBD binds to a genomic region having 18 nucleotides. In certain embodiments, the DBD comprises nine zinc fingers. In certain embodiments, the DBD binds to a genomic region having 27 nucleotides.

In certain embodiments, the DBD comprises a sequence having at least 95% sequence identity to any of SEQ ID NOs: 148-151. In certain embodiments, the DBD comprises a sequence having any one of SEQ ID NOs: 148-151.

In certain embodiments, the DBD is derived from human EGR1 or human EGR3.

In certain embodiments, the DBD comprises a sequence having at least 90% identity to any one of SEQ ID NOs: 77-98. In certain embodiments, the DBD comprises SEQ ID NOs: 77-98.

In certain embodiments, the DBD comprises a sequence having at least 90% identity to SEQ ID NO: 92. In certain embodiments, the DBD comprises SEQ ID NO: 92.

In certain embodiments, the non-naturally occurring transcription factor comprises a sequence having at least 90% identity to SEQ ID NO: 130 or 131. In certain embodiments, the non-naturally occurring transcription factor comprises SEQ ID NO: 130 or 131.

In certain embodiments, the expression cassette comprises a nucleotide sequence having at least 90% identity to any one of SEQ ID NOs: 72 or 73. In certain embodiments, the expression cassette comprises a nucleotide sequence of any one of SEQ ID NOs: 72 or 73.

In certain embodiments, the expression cassette further comprises a regulatory element that drives expression of the transcription factor at a higher level in PV neurons than in other cell types. In certain embodiments, the regulatory element comprises any one of SEQ ID NOs: 1-4. In certain embodiments, the regulatory element comprises SEQ ID NO: 2 or 3.

In certain embodiments, the expression cassette further comprises a PV selective microRNA binding site. In certain embodiments, the PV selective microRNA binding site comprises at least 90% identity to any one of SEQ ID NOs: 7, 14 or 15. In certain embodiments, the PV selective microRNA binding site comprises any one of SEQ ID NOs: 7, 14, or 15.

In certain embodiments, the expression cassette is a part of a viral vector. In certain embodiments, the viral vector is an AAV virus. In certain embodiments, the AAV virus is an AAV9 virus or a scAAV9 virus. In certain embodiments, the viral vector is a Lentivirus.

In another aspect, the application provides an expression cassette comprising a sequence encoding a non-naturally occurring transcription factor which increases expression of the SCN1A gene in a cell, wherein the non-naturally occurring transcription factor comprises a DNA binding domain operably linked to a transcription activating domain, wherein the DNA binding domain is a zinc finger protein comprising the sequence LEPGEKP - [YKCPECGKSFS X HQRTH TGEKP]n - YKCPECGKSFS X HQRTH - TGKKTS (SEQ ID NO: 147), and wherein there is no HA tag (SEQ ID NO: 303) between the DNA binding domain and the transcription activating domain. In certain embodiments, the transcription activating domain comprises a VP16, VPR or VP64 sequence, or a functional fragment thereof. In certain embodiments, the transcription activating domain comprises VP64.

In certain embodiments, the DNA binding domain binds to a genomic region having 18-27 nucleotides. In certain embodiments, the DNA binding domain is a zinc finger domain comprising SEQ ID NO: 147 wherein n = 6 to 9. In certain embodiments, the DNA binding domain is a zinc finger domain comprising SEQ ID NO: 147 wherein n = 6. In certain embodiments, the DNA binding domain binds to a genomic region having 18 nucleotides. In certain embodiments, the DNA binding domain is a zinc finger domain comprising SEQ ID NO: 147 wherein n = 9. In certain embodiments, the DNA binding domain binds to a genomic region having 27 nucleotides.

In certain embodiments, the DNA binding domain comprises a sequence having at least 95% sequence identity to any of SEQ ID NOs: 148-151. In certain embodiments, the DNA binding domain comprises a sequence having any one of SEQ ID NOs: 148-151.

In certain embodiments, the DNA binding domain comprises a sequence having at least 90% identity to any one of SEQ ID NOs: 77-91. In certain embodiments, the DNA binding domain comprises any one of SEQ ID NOs: 77-91.

In certain embodiments, the expression cassette further comprises a regulatory element that drives expression of the transcription factor at a higher level in PV neurons than in other cell types. In certain embodiments, the regulatory element comprises any one of SEQ ID NOs: 1-4. In certain embodiments, the regulatory element comprises SEQ ID NO: 2 or 3.

In certain embodiments, the non-naturally occurring transcription factor comprises a sequence having at least 90% identity to SEQ ID NO: 127. In certain embodiments, the non-naturally occurring transcription factor comprises SEQ ID NO: 127.

In certain embodiments, the expression cassette comprises a nucleotide sequence having at least 90% identity to any one of SEQ ID NOs: 93 or 71. In certain embodiments, the expression cassette comprises a nucleotide sequence of any one of SEQ ID NOs: 93 or 71.

In certain embodiments, the expression cassette further comprises a PV selective microRNA binding site. In certain embodiments, the PV selective microRNA binding site comprises at least 90% identity to any one of SEQ ID NOs: 7, 14 or 15. In certain embodiments, the PV selective microRNA binding site comprises any one of SEQ ID NOs: 7, 14, or 15.

In certain embodiments, the expression cassette is a part of a viral vector. In certain embodiments, the viral vector is an AAV virus. In certain embodiments, the AAV virus is an AAV9 virus or a scAAV9 virus. In certain embodiments, the viral vector is a Lentivirus. In another aspect, the application provides a polynucleotide comprising a PV selective microRNA binding site comprising a sequence having at least 80% sequence identity to SEQ ID NO: 14 or 15, wherein the microRNA binding site reduces expression of the transgene in excitatory neurons. In certain embodiments, the PV selective microRNA binding site comprises SEQ ID NO: 14. In certain embodiments, the PV selective microRNA binding site comprises SEQ ID NO: 15. In another aspect, the application provides an expression cassette comprising the PV selective microRNA binding site and a promoter and/or enhancer. In certain embodiments, the promoter and/or enhancer is a PV selective regulatory element that drives expression of the transgene at a higher level in parvalbumin (PV) neurons than in other cell types. In certain embodiments, the PV selective regulatory element is operably linked to a transgene.

In another aspect, the application provides an expression cassette comprising a regulatory element operably linked to a transgene and at least one microRNA binding site, wherein the regulatory element drives expression of the transgene at a higher level in parvalbumin (PV) neurons than in other cell types, and wherein the microRNA binding site reduces expression of the transgene in excitatory neurons. In certain embodiments, the expression cassette does not comprise SEQ ID NO: 67. In certain embodiments, the microRNA binding site comprises at least one binding site for MIR128 (SEQ ID NO: 9). In certain embodiments, the microRNA binding site comprises at least one binding site for MIR221 (SEQ ID NO: 11). In certain embodiments, the microRNA binding site comprises at least one binding site for MIR222 (SEQ ID NO: 13). In certain embodiments, the microRNA binding site comprises at least one binding site for MIR128 (SEQ ID NO: 9) and at least one binding site for MIR221 (SEQ ID NO: 11). In certain embodiments, the microRNA binding site comprises at least one binding site for MIR128 (SEQ ID NO: 9), at least one binding site for MIR221 (SEQ ID NO: 11), and at least one binding site for MIR222 (SEQ ID NO: 13). In certain embodiments, the microRNA binding site comprises a sequence having at least 90% identity to any one of SEQ ID NOs: 7, 14 or 15. In certain embodiments, the microRNA binding site comprises SEQ ID NO: 7, 14 or 15.

In certain embodiments, the transgene encodes a polypeptide comprising a non-naturally occurring transcription factor which increases expression of the SCN1A gene in a cell. In certain embodiments, the transcription factor binds to a genomic region having 18-27 nucleotides. In certain embodiments, the transcription factor comprises a DNA binding domain. In certain embodiments, the transcription factor comprises a DNA binding domain and a transcription activating domain.

In certain embodiments, the DNA binding domain comprises at least 80% sequence identity to its closest human counterpart. In certain embodiments, the DNA binding domain comprises at least 90% sequence identity to its closest human counterpart. In certain embodiments, the DNA binding domain and the transcription activating domain both comprise at least 80% sequence identity to their closest human counterparts. In certain embodiments, the DNA binding domain and the transcription activating domain both comprise at least 90% sequence identity to their closest human counterparts.

In certain embodiments, the DNA binding domain comprises a guide RNA and a nuclease inactivated Cas protein. In certain embodiments, the nuclease inactivated Cas protein is a nuclease inactivated Cas9.

In certain embodiments, the DNA binding domain comprises a zinc finger domain. In certain embodiments, the DNA binding domain comprises six to nine zinc finger domains. In certain embodiments, the DNA binding domain comprises six zinc fingers. In certain embodiments, the DNA binding domain binds to a genomic region having 18 nucleotides. In certain embodiments, the DNA binding domain comprises nine zinc fingers. In certain embodiments, the DNA binding domain binds to a genomic region having 27 nucleotides.

In certain embodiments, the DNA binding domain comprises a sequence having at least 95% sequence identity to any of SEQ ID NOs: 148-151. In certain embodiments, the DNA binding domain comprises a sequence having any one of SEQ ID NOs: 148-151. In certain embodiments, the DNA binding domain comprises a sequence having at least 90% identity to any one of SEQ ID NOs: 92-98. In certain embodiments, the DNA binding domain comprises any one of SEQ ID NOs: 92-98.

In certain embodiments, the DNA binding domain is a zinc finger protein comprising the sequence LEPGEKP - [YKCPECGKSFS X HQRTH TGEKP]n - YKCPECGKSFS X HQRTH - TGKKTS (SEQ ID NO: 147).

In certain embodiments, the DNA binding domain comprises a sequence having at least 90% identity to any one of SEQ ID NOs: 77-91. In certain embodiments, the DNA binding domain comprises any one of SEQ ID NOs: 77-91.

In certain embodiments, the DNA binding domain is derived from human EGR1 or human EGR3.

In certain embodiments, the transcription activating domain comprises a VP16, VPR, VP64, CITED2, CITED4, or CREB3 sequence, or a functional fragment thereof. In certain embodiments, the transcription activating domain comprises a human CITED2, CITED4, or CREB3 sequence, or a functional fragment thereof.

In certain embodiments, the regulatory element comprises a sequence having any one of SEQ ID NOs: 1-4. In certain embodiments, the regulatory element comprises a sequence having SEQ ID NO: 2 or 3.

In certain embodiments, the non-naturally occurring transcription factor comprises a sequence having at least 90% identity to any one of SEQ ID NOs: 105, 106, and 127-129. In certain embodiments, the non-naturally occurring transcription factor comprises any one of SEQ ID NOs: 105, 106, and 127-129.

In certain embodiments, the transgene comprises a nucleotide sequence having at least 90% identity to any one of SEQ ID NOs: 71, 74, 75, 76 or 184. In certain embodiments, the transgene comprises any one of SEQ ID NOs: 71, 74, 75, 76 or 184.

In certain embodiments, the expression cassette is a part of a viral vector. In certain embodiments, the viral vector is an AAV virus. In certain embodiments, AAV virus is an AAV9 virus or a scAAV9 virus. In certain embodiments, the viral vector is a Lentivirus.

In another aspect, the application provides a method for selective expression of a transgene in parvalbumin (PV) neurons of a primate comprising administering to a primate a viral vector comprising a transgene and at least one microRNA binding site, wherein the microRNA binding site reduces expression of the transgene in excitatory neurons.

In certain embodiments, the viral vector further comprises a regulatory element operably linked to the transgene, wherein the regulatory element drives expression of the transgene at a higher level in parvalbumin (PV) neurons than in other cell types.

In certain embodiments, the microRNA binding site comprises at least one binding site for MIR128 (SEQ ID NO: 9). In certain embodiments, the microRNA binding site comprises at least one binding site for MIR221 (SEQ ID NO: 11). In certain embodiments, the microRNA binding site comprises at least one binding site for MIR222 (SEQ ID NO: 13). In certain embodiments, the microRNA binding site comprises at least one binding site for MIR128 (SEQ ID NO: 9) and at least one binding site for MIR221 (SEQ ID NO: 11). In certain embodiments, the microRNA binding site comprises at least one binding site for MIR128 (SEQ ID NO: 9), at least one binding site for MIR221 (SEQ ID NO: 11), and at least one binding site for MIR222 (SEQ ID NO: 13). In certain embodiments, the microRNA binding site comprises a sequence having at least 90% identity to any one of SEQ ID NOs: 7, 14 or 15. In certain embodiments, the microRNA binding site comprises SEQ ID NO: 7, 14 or 15.

In certain embodiments, the transgene comprises a sequence encoding a non-naturally occurring transcription factor which increases expression of the SCN1A gene in a cell.

In certain embodiments, the transcription factor binds to a genomic region having 18-27 nucleotides.

In certain embodiments, the transcription factor comprises a DNA binding domain.

In certain embodiments, the transcription factor comprises a DNA binding domain and a transcription activating domain.

In certain embodiments, the DNA binding domain comprises at least 80% sequence identity to its closest human counterpart. In certain embodiments, the DNA binding domain comprises at least 90% sequence identity to its closest human counterpart. In certain embodiments, the DNA binding domain and the transcription activating domain both comprise at least 80% sequence identity to their closest human counterparts. In certain embodiments, the DNA binding domain and the transcription activating domain both comprise at least 90% sequence identity to their closest human counterparts.

In certain embodiments, the DNA binding domain comprises a guide RNA and a nuclease inactivated Cas protein. In certain embodiments, the nuclease inactivated Cas protein is a nuclease inactivated Cas9.

In certain embodiments, the DNA binding domain comprises a zinc finger domain. In certain embodiments, the DNA binding domain comprises six to nine zinc finger domains. In certain embodiments, the DNA binding domain comprises six zinc fingers. In certain embodiments, the DNA binding domain binds to a genomic region having 18 nucleotides. In certain embodiments, the DNA binding domain comprises nine zinc fingers. In certain embodiments, the DNA binding domain binds to a genomic region having 27 nucleotides.

In certain embodiments, the DNA binding domain comprises a sequence having at least 95% sequence identity to any of SEQ ID NOs: 148-151. In certain embodiments, the DNA binding domain comprises a sequence having any one of SEQ ID NOs: 148-151.

In certain embodiments, the DNA binding domain is derived from human EGR1 or human EGR3.

In certain embodiments, the DNA binding domain comprises a sequence having at least 90% identity to any one of SEQ ID NOs: 92-98. In certain embodiments, the DNA binding domain comprises any one of SEQ ID NOs: 92-98.

In certain embodiments, the DNA binding domain is a zinc finger protein comprising the sequence LEPGEKP - [YKCPECGKSFS X HQRTH TGEKP]n - YKCPECGKSFS X HQRTH - TGKKTS (SEQ ID NO: 147).

In certain embodiments, the DNA binding domain comprises a sequence having at least 90% identity to any one of SEQ ID NOs: 77-91. In certain embodiments, the DNA binding domain comprises any one of SEQ ID NOs: 77-91.

In certain embodiments, the transcription activating domain comprises a VP16, VPR, VP64, CITED2, CITED4, or CREB3 sequence, or a functional fragment thereof. In certain embodiments, the transcription activating domain comprises a human CITED2, CITED4, or CREB3 sequence, or a functional fragment thereof.

In certain embodiments, the regulatory element comprises a sequence having any one of SEQ ID NOs: 1-4. In certain embodiments, the regulatory element comprises a sequence having SEQ ID NO: 2 or 3.

In certain embodiments, the non-naturally occurring transcription factor comprises a sequence having at least 90% identity to any one of SEQ ID NOs: 105, 106, and 127-129. In certain embodiments, the non-naturally occurring transcription factor comprises any one of SEQ ID NOs: 105, 106, and 127-129.

In certain embodiments, the transgene comprises a nucleotide sequence having at least 90% identity to any one of SEQ ID NOs: 71, 74, 75, 76 or 184. In certain embodiments, the transgene comprises any one of SEQ ID NOs: 71, 74, 75, 76 or 184.

In certain embodiments, the viral vector is an AAV virus. In certain embodiments, the AAV virus is an AAV9 virus or a scAAV9 virus. In certain embodiments, the viral vector is a Lentivirus.

In certain embodiments, the primate is a human. In certain embodiments, the primate is a non-human primate. In certain embodiments, the non-human primate is an old world monkey, an orangutan, a gorilla, a chimpanzee, a marmoset, a crab-eating macaque, a rhesus macaque or a pig-tailed macaque.

In another aspect, the application provides an expression cassette comprising a sequence encoding a non-naturally occurring transcription factor which increases expression of the SCN1A gene in a cell, wherein the non-naturally occurring transcription factor comprises a sequence having at least 90% identity to SEQ ID NO: 128 or 129. In certain embodiments, the non-naturally occurring transcription factor comprises SEQ ID NO: 128 or 129.

In another aspect, the application provides a method of increasing expression of SCN1A in a cell by administering any of the expression cassettes provided herein. In certain embodiments, the cell is a neuronal cell. In certain embodiments, the neuronal cell is selected from the group consisting of unipolar, bipolar, multipolar, or pseudounipolar neurons. In certain embodiments, the cell is GABAergic neuron. In certain embodiments, the cell is a PV neuron. In certain embodiments, the cell is a non-neuronal cell. In certain embodiments, the cell is a glial cell. In certain embodiments, the glial cell is selected from the group consisting of astrocytes, oligodendrocytes, ependymal cells, Schwann cells, and satellite cells. In certain embodiments, the cell is within a subject. In certain embodiments, the subject is a mammal. In certain embodiments, the subject is a human. In certain embodiments, increasing expression of SCN1A treats a disease, disorder or symptom. In certain embodiments, the disorder is a central nervous system disorder. In certain embodiments, the disorder is epilepsy associated with SCN1A haploinsufficiency. In certain embodiments, the haploinsufficiency is the result of the subject being heterozygous for a loss of function mutation of the SCN1A gene. In certain embodiments, the disorder is epilepsy associated with an insertion, deletion, or substitution in the SCN1A gene. In certain embodiments, the disorder is epilepsy associated with a point mutation in the SCN1A gene. In certain embodiments, the disorder is Dravet Syndrome. In certain embodiments, a symptom of the central nervous system disorder is neuronal hyperactivity. In certain embodiments, treating the central nervous system disorder comprises reducing neuronal hyperactivity. In certain embodiments, a symptom of the central nervous system disorder is seizures. In certain embodiments, treating the central nervous system disorder comprises reducing the frequency of seizures. In certain embodiments, treating the central nervous system disorder comprises reducing the severity of seizures.

In another aspect, the application provides a method of increasing expression of SCN1A in the CNS by administering any one of the expression cassettes provided herein. In certain embodiments, the expression cassette is administered via unilateral intracerebroventricular (ICV) administration. In certain embodiments, the expression cassette is administered via bilateral intracerebroventricular (ICV) administration. In certain embodiments, the increased expression of SCN1A occurs in the brain. In certain embodiments, the increased expression of SCN1A occurs in the frontal cortex, parietal cortex, temporal cortex, hippocampus, medulla, and/or occipital cortex. In certain embodiments, the increased expression of SCN1A occurs in the spine. In certain embodiments, the increased expression of SCN1A occurs in the spinal cord and/or dorsal root ganglion.

## INCORPORATION BY REFERENCE

All publications, patents, and patent applications mentioned in this specification are herein incorporated by reference to the same extent as if each individual publication, patent, or patent application was specifically and individually indicated to be incorporated by reference.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative cases, in which the principles of the invention are utilized, and the accompanying drawings of which:

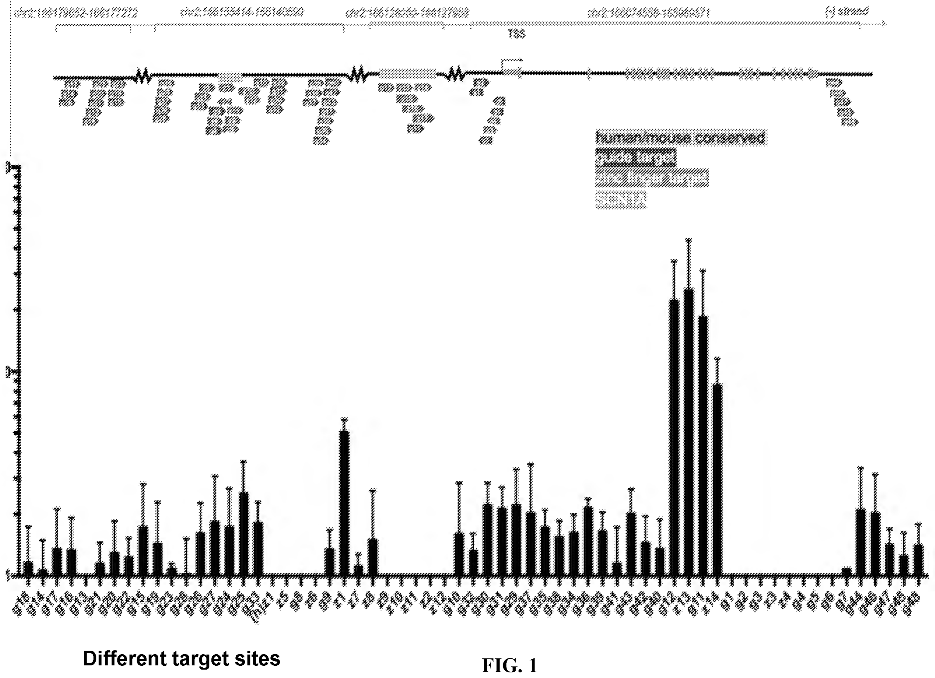

FIG. 1 illustrates upregulation of endogenous SCN1A using engineered transcription factors that bind to various regions on chromosome 2 (with reference to GRCh38.p12). Data are presented as fold change in SCN1A expression with respect to control (EGFP-KASH) condition.

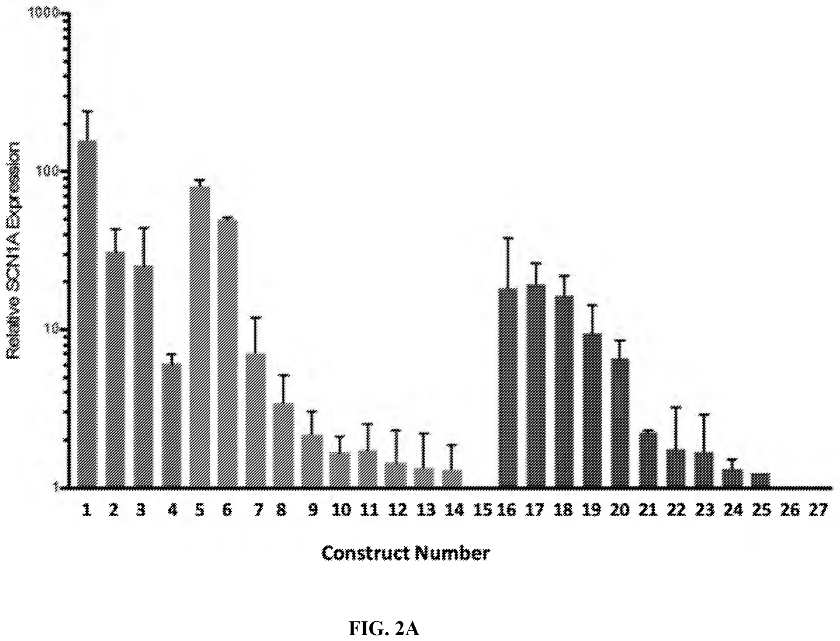

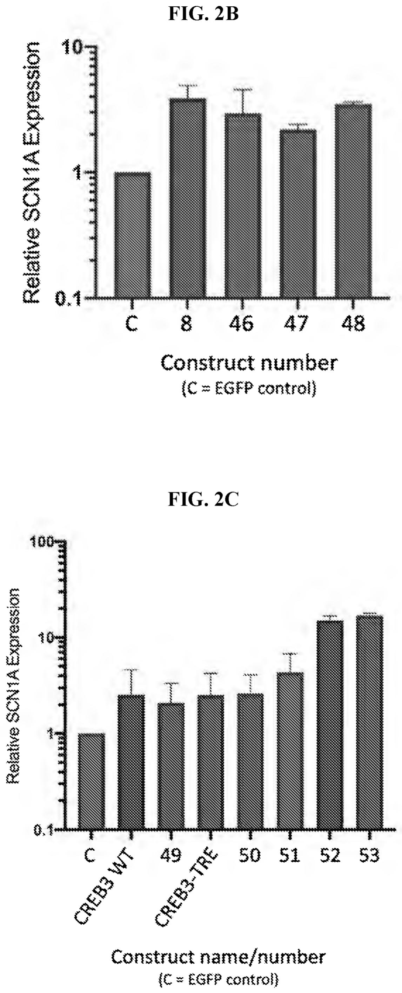

FIG. 2A, FIG. 2B, and FIG. 2C illustrate the relative expression of endogenous SCN1A in HEK293 cells using SCN1A-specific transcriptional activators (see TABLE 1). Data are presented as fold change relative to control conditions, and shown on a $Log_{10}$ scale.

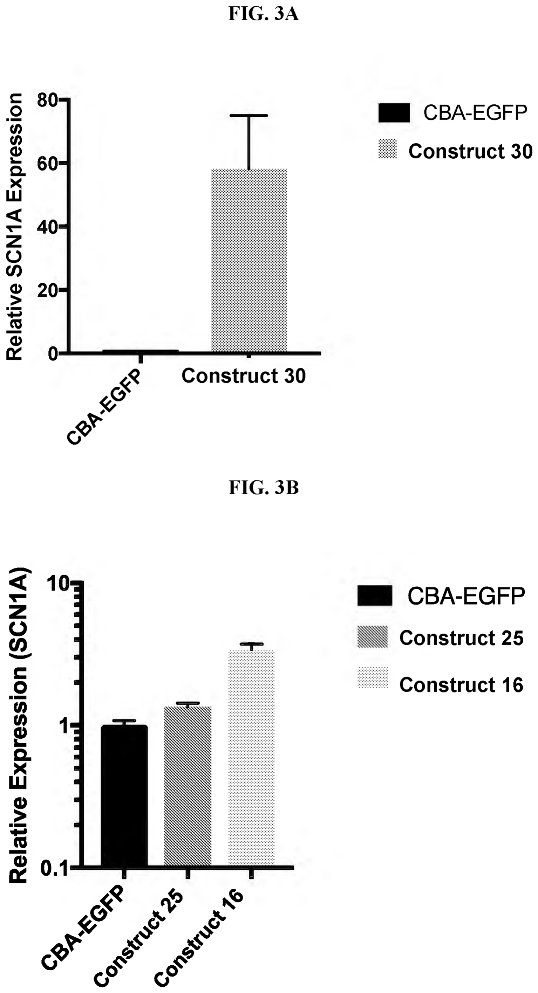

**FIG. 3A** illustrates the relative expression of endogenous SCN1A in GABA neurons using an SCN1A-specific transcriptional activator (Construct 30). Data are presented as fold change relative to control conditions (CBA-EGFP). **FIG. 3B** illustrates the relative expression of endogenous SCN1A in GABA neurons using SCN1A-specific transcriptional activators (Constructs 25 and 16). Data are presented as fold change relative to control conditions (CBA-EGFP) in $Log_{10}$.

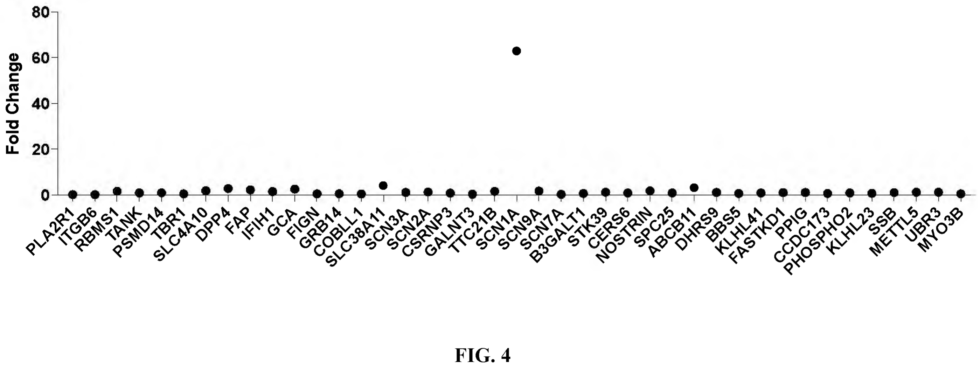

**FIG. 4** illustrates the relative expression of endogenous SCN1A and the 40 nearest neighboring genes driven by an SCN1A specific transcription factor (Construct 30). Data are presented as fold change relative to control conditions (CBA-EGFP-KASH) in $Log_{10}$.

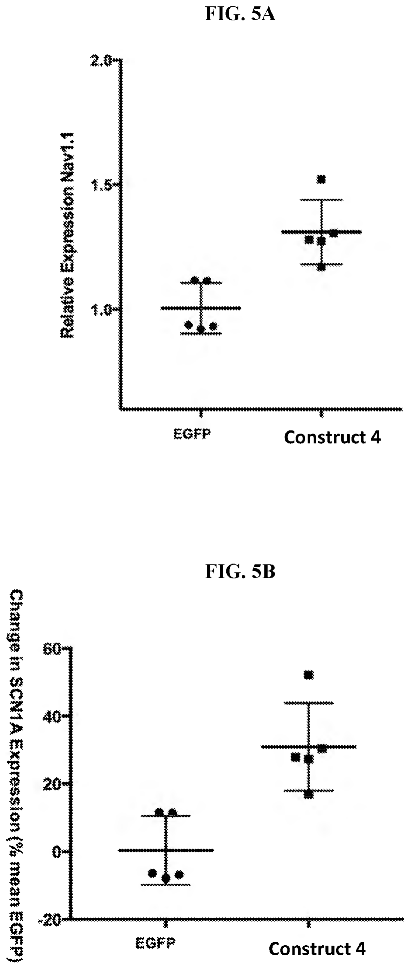

**FIG. 5A** and **FIG. 5B** illustrate expression of a SCN1A-specific transcriptional activator *in vivo* as compared to a control expression cassette which expressed eGFP. **FIG. 5A** illustrates the relative expression of SCN1A gene in mice with injected with either control eGFP or Construct 4 comprising an SCN1A transcriptional activator. **FIG. 5B** illustrates the change in SCN1A expression in terms of percentage mean eGFP. These experiments indicate transcriptional activation by Construct 4 resulted in about 20-30% upregulation of SCN1A expression.

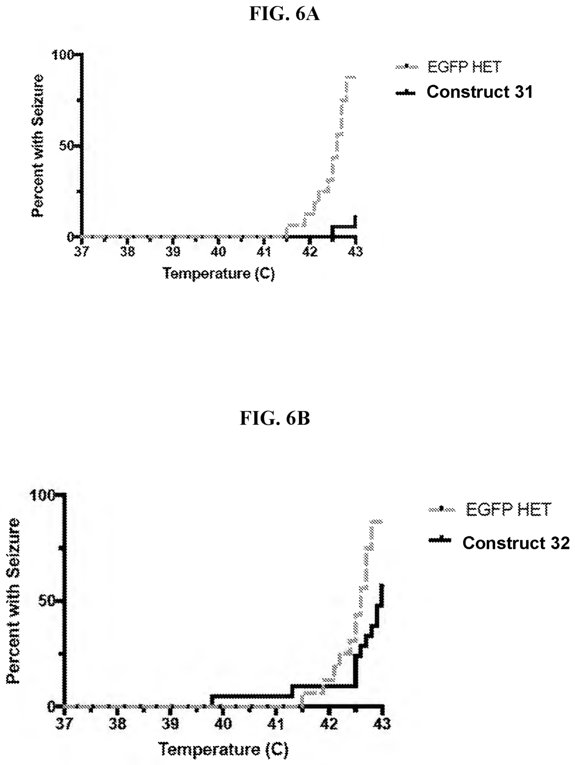

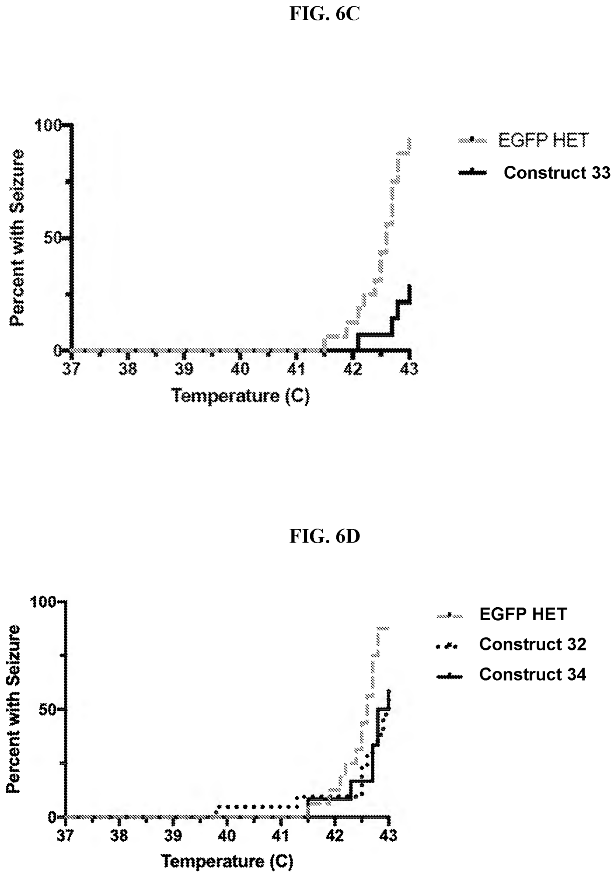

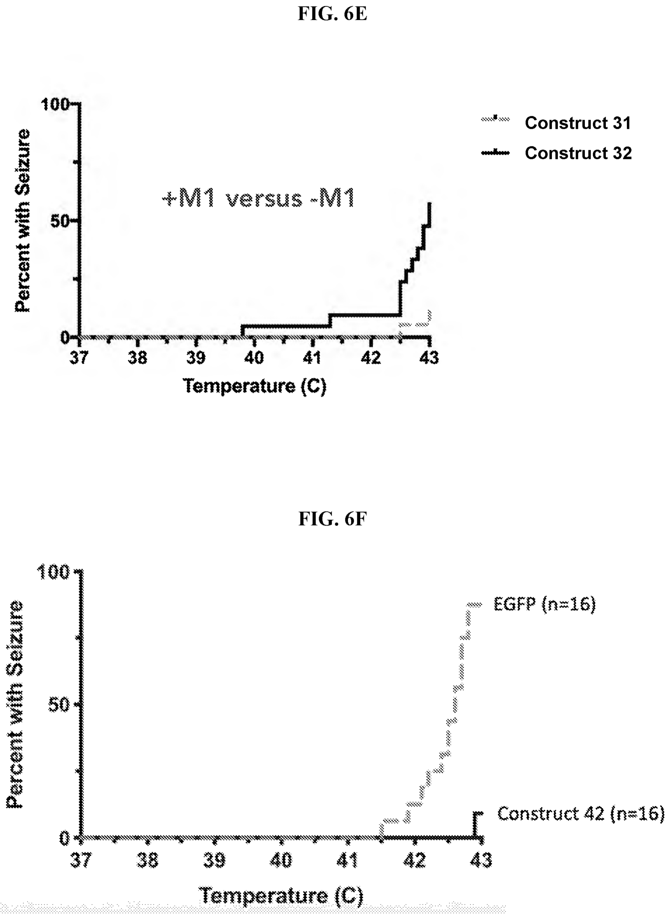

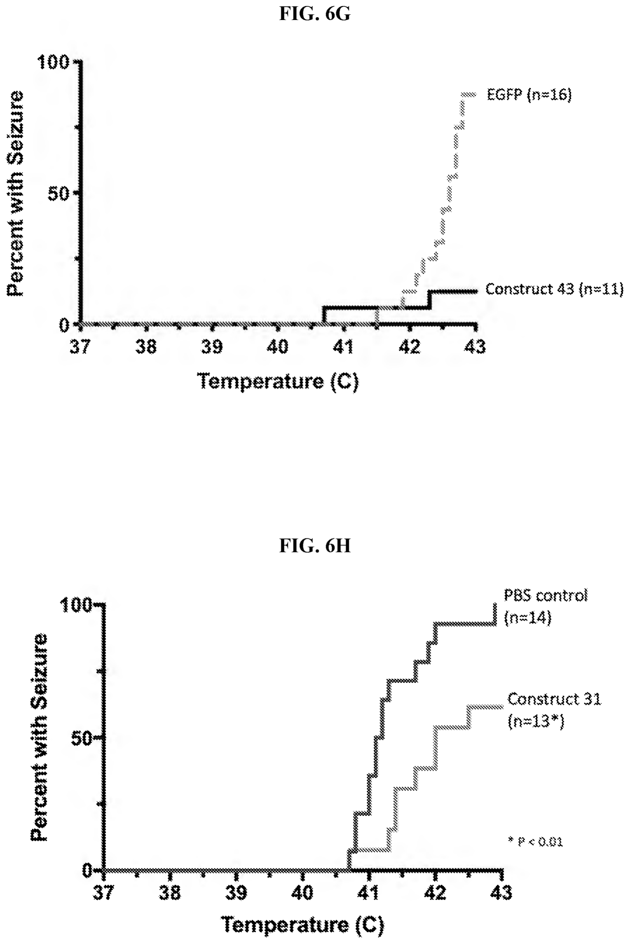

**FIG. 6A, FIG. 6B, FIG. 6C, FIG. 6D, FIG. 6E, FIG. 6F,** and **FIG. 6G** illustrate the effect on hyperthermic seizures in the Scn1a$^{tm1Kea}$ knockout mouse model of Dravet syndrome using various SCN1A specific transcription factors as compared to a control. P1 Scn1a +/- mice (heterozygous; HET) were infused with either AAV9-EGFP or an AAV9 vector expressing an SCN1A specific transcription factor (one of Constructs 31-34, 42 and 43). At P26-P28 infused mice were run through the hyperthermia induced seizure assay and the internal temperature at which they experienced a tonic-clonic seizure was recorded. **FIG. 6D** shows a direct comparison between Construct 32, which contains an HA tag located between the DBD and TAD, and Construct 34, which does not contain an HA tag. **FIG. 6E** shows a direct comparison between Construct 31, which contains the m1 microRNA binding site located between the coding region and polyA tail, and Construct 32, which does not contain the m1 microRNA binding site. **FIG. 6H** illustrates the effect on hyperthermic seizures in the Scn1a$^{RX}$ mutant mouse model of Dravet syndrome using Construct 31 (compared to PBS injected control).

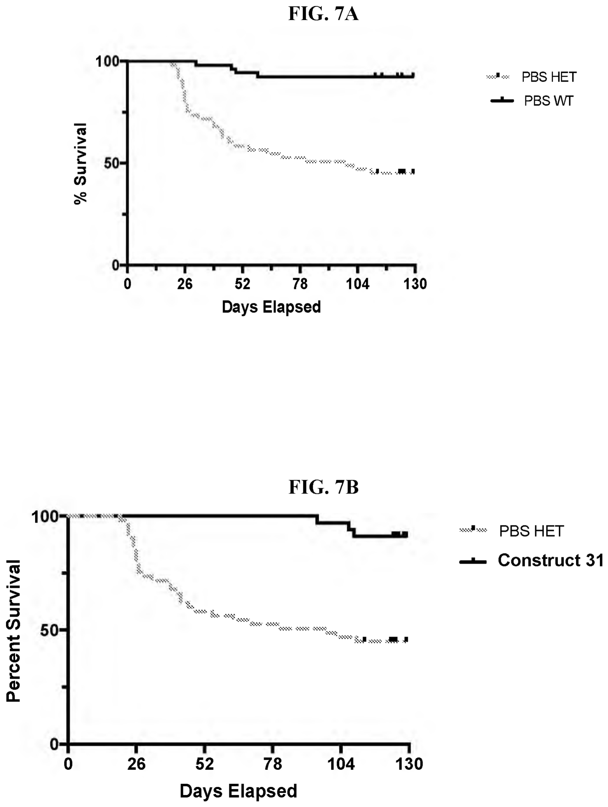

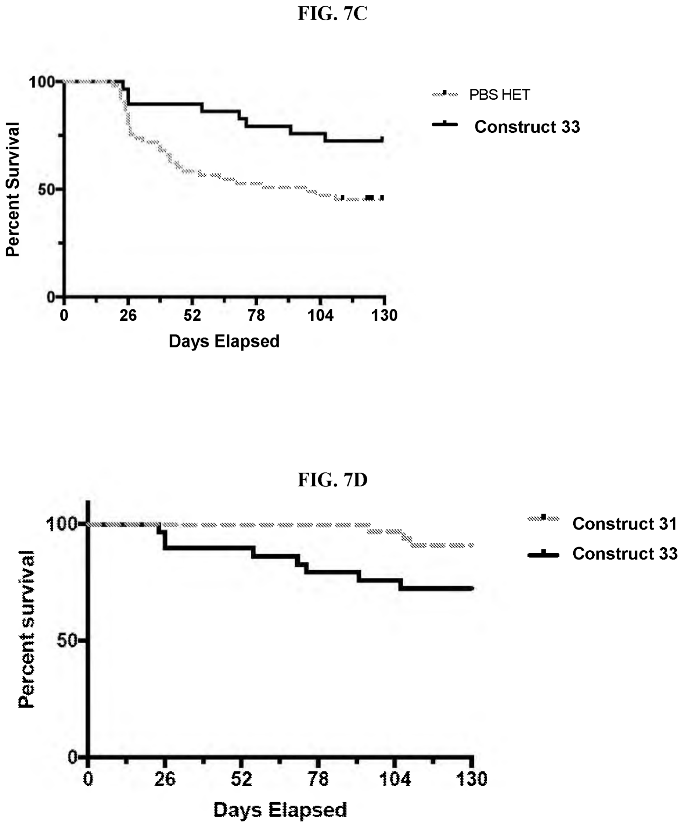

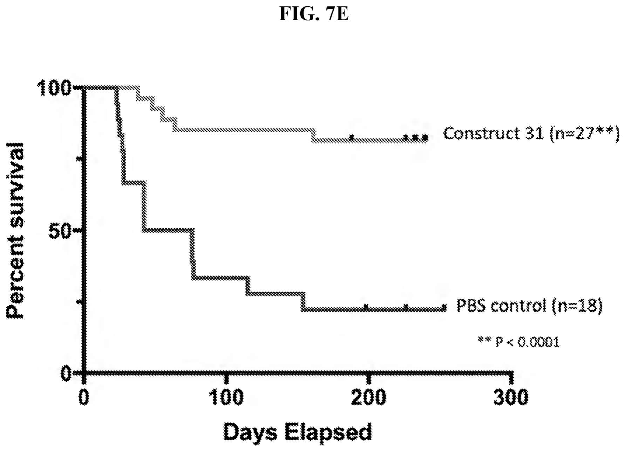

**FIG. 7A, FIG. 7B, FIG. 7C,** and **FIG. 7D** illustrate survival in the Scn1a$^{tm1Kea}$ knockout mouse model of Dravet syndrome under various conditions. **FIG. 7A** illustrates the comparison between wild-type (PBS WT) and Scn1a +/- mice (PBS HET) in a survival assay. P1 Scn1a +/(N=53) and Scn1a +/+ (N=54) mice were infused with PBS. Mice were observed in their home cage daily and in the case of any mortality, the date was recorded. There was a significant difference in survival between Scn1a +/- and Scn1a +/+ animals (P<0.0001). **FIGs. 7B-D** illustrate the effect on survival in a mouse model of Dravet syndrome for mice treated with various SCN1A specific transcription factors as compared to a control. P1 Scn1a +/- mice were infused with either PBS or an AAV9 vector expressing an SCN1A specific transcription factor (Constructs 31 or 33). Mice were observed in their home cage daily and in the case of any mortality, the date was recorded. **FIG. 7D** shows a direct comparison between Construct 31, which contains the m1 microRNA binding site located between the coding region and polyA tail, and Construct 33, which does not contain the m1 microRNA binding site. **FIG. 7E** illustrates survival in the Scn1a$^{RX}$ mutant mouse model of Dravet syndrome using Construct 31 (compared to PBS injected control).

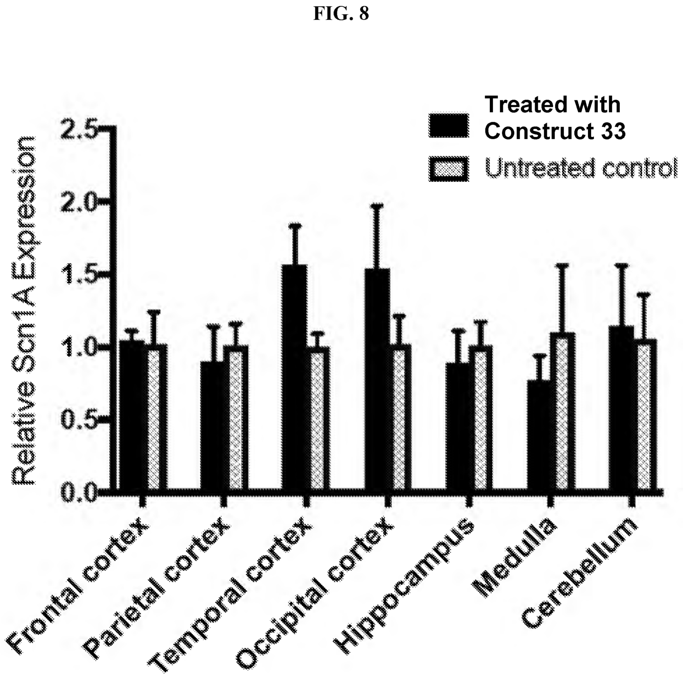

**FIG. 8** illustrates relative Scn1A mRNA expression in different brain tissues following intraparenchymal delivery of an AAV9 vector encoding an SCN1A specific transcription factor (Construct 33), administered to two cynomolgus macaques at $1.2 \times 10^{12}$ gc/animal, normalized to two untreated control animal. All animals were sacrificed 28 days after injection and Scn1A mRNA was quantified in the tissue samples by Taqman PCR. Data is reported as normalized expression of target mRNA in different tissue sections from the brain. Similar results were recorded with a different set of Scn1a gene derived primers/probe as well.

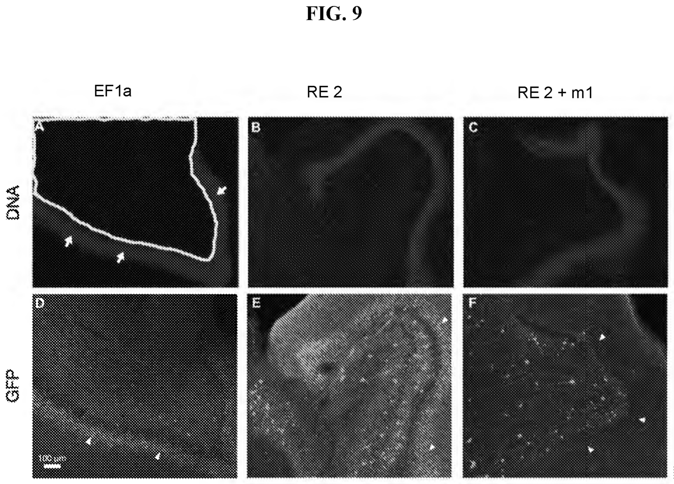

**FIGs. 9A-F** shows the pattern of expression of EGFP in marmoset hippocampus dentate gyrus region following treatment with AAV9 vectors comprising an EGFP transgene under the control of EF1a promoter, RE 2 promoter (SEQ ID NO: 2), or RE 2 promoter (SEQ ID NO: 2) with an m1 microRNA binding site (SEQ ID NO: 7) located between the EGFP coding region and polyA site. A representative region of the dentate gyrus region of the hippocampus is shown for each vector treatment. The top row shows the cell nuclei stained with DAPI and the bottom row shows the GFP positive regions stained with an anti-GFP antibody. In **FIG. 9A** (EF1a treatment) the hippocampus CA4 hylus region is outlined in yellow and the arrows point to the dentate cell granule cell body layer (DG). **FIG. 9B** and **FIG. 9C** are centered on the same region. The CA4 region, which is a mixture of excitatory and inhibitory interneurons, is highlighted as it was the only region of significant expression in the RE 2 + m1 condition. With EF1a and RE 2 driven transgene expression, GFP expression was more widespread and included other regions of the hippocampus. The DG cell layer is thought to contain primarily excitatory neurons. GFP expression driven by EF1a and RE 2 is visible in the DG cell layer **(FIG. 9D** and **FIG. 9E)** yet is not present in RE 2 + m1 treated animals **(FIG. 9F)** (white arrowheads).

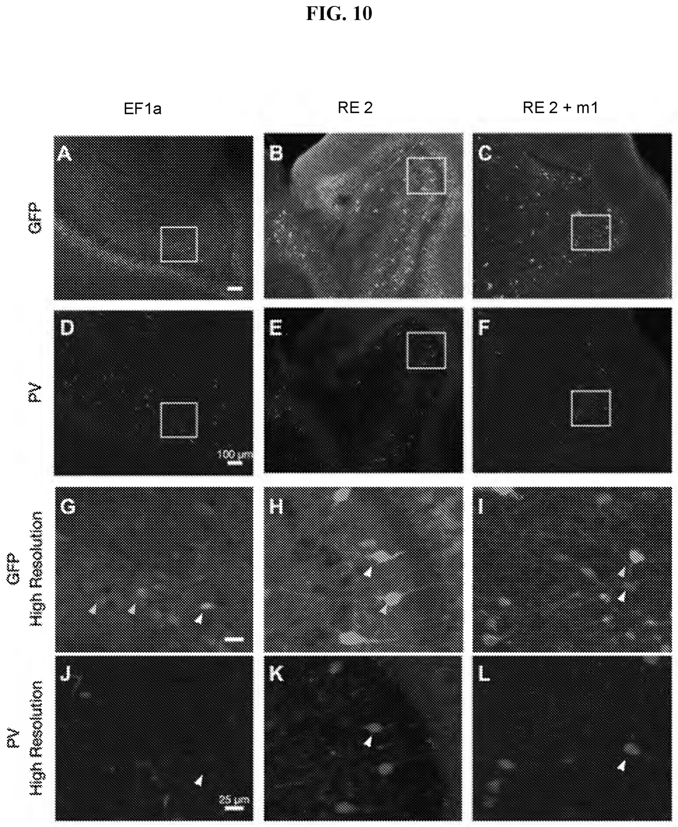

**FIGs. 10A-L** shows that the pattern of expression of EGFP in marmoset hippocampus dentate gyrus region following treatment with AAV9 vectors comprising an EGFP transgene under the control of EF 1a promoter, RE 2 promoter (SEQ ID NO: 2), or RE 2 promoter (SEQ ID NO: 2) with an m1 microRNA binding site (SEQ ID NO: 7) located between the EGFP coding region and polyA site is primarily localized to parvalbumin (PV) positive cells in the RE 2 and RE 2 + m1 treated animals. A representative region of the dentate gyrus region of the hippocampus is shown for each vector treatment. The top row shows the GFP positive regions and the next row down shows the same regions stained with the inhibitory interneuron marker for PV. The boxed region in **FIGs. 10A-F** is shown at a higher magnification in **FIGs. 10G-L.** GFP expression driven by RE 2 and RE 2 + m1 is primarily co-localized with the inhibitory interneuron marker PV **(FIGs. 10H** and **10K, 10I** and **10L** white arrowheads), whereas in EG-EF1a GFP expression is not as readily localized to PV positive cells **(FIGs. 10G** and **10J** white arrowheads). In addition, the GFP positive cells have distinctly interneuron morphology of highly branching cells with a pyramidal cell body in RE 2 and RE 2 + m1 treated animals **(FIGs. 10H** and **10I** yellow arrowheads) as compared to a less distinct cell body morphology in the EF1a treated animals **(FIG. 10G** yellow arrowheads).

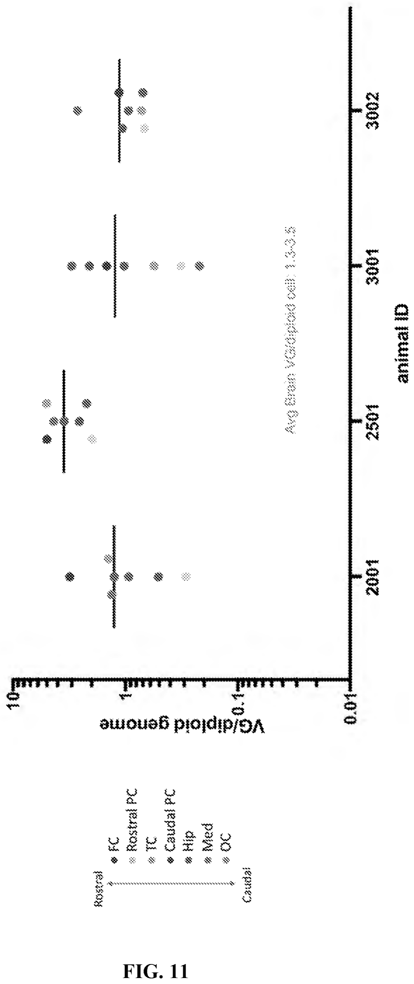

**FIG. 11** shows the VG/diploid genome in frontal cortex (FC), Rostral parietal cortex (Rostral PC), temporal cortex (TC), Caudal parietal cortex (Caudal PC), hippocampus (Hip), medulla (Med), and occipital cortex (OC) tissue samples for animals treated with AAV9-RE$^{GABA}$-eTF$^{SCN1A}$ administered at 4.8E+13 or 8E+13 vg/animal via unilateral intracerebroventricular (ICV) administration (Example 10 and Example 11). Each data point represents the VG/diploid genome for the tissue sample and the horizontal bars represent the average VG/diploid genome for all tissue samples for each animal.

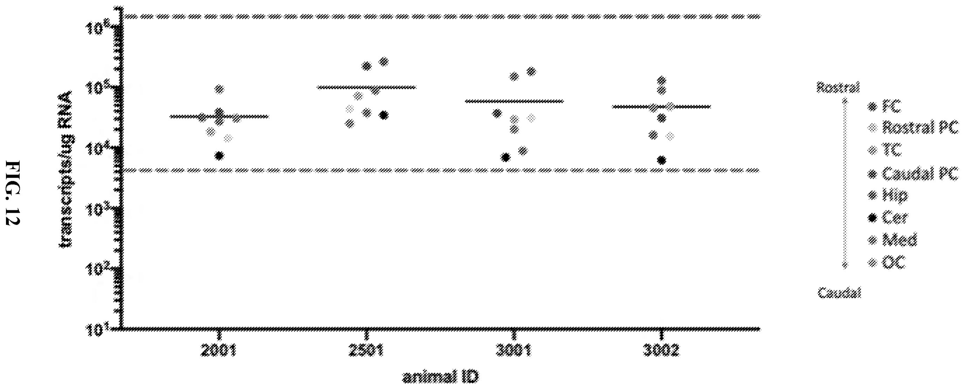

**FIG. 12** shows the transcripts/$\mu$g RNA in frontal cortex (FC), Rostral parietal cortex (Rostral PC), temporal cortex (TC), Caudal parietal cortex (Caudal PC), hippocampus (Hip), medulla (Med), and occipital cortex (OC) tissue samples for animals treated with AAV9-RE$^{GABA}$-eTF$^{SCN1A}$ administered at 4.8E+13 or 8E+13 vg/animal via unilateral intracerebroventricular (ICV) administration (Example 10 and Example 11). Each data point represents the VG/diploid genome for the tissue sample and the horizontal bars represent the average VG/diploid genome for all tissue samples for each animal. Average transcripts for *ARFGAP2* were 1.85E+6/$\mu$g RNA, and are indicated by the dashed upper boundary line. The detection limit is indicated by the dashed lower boundary line.

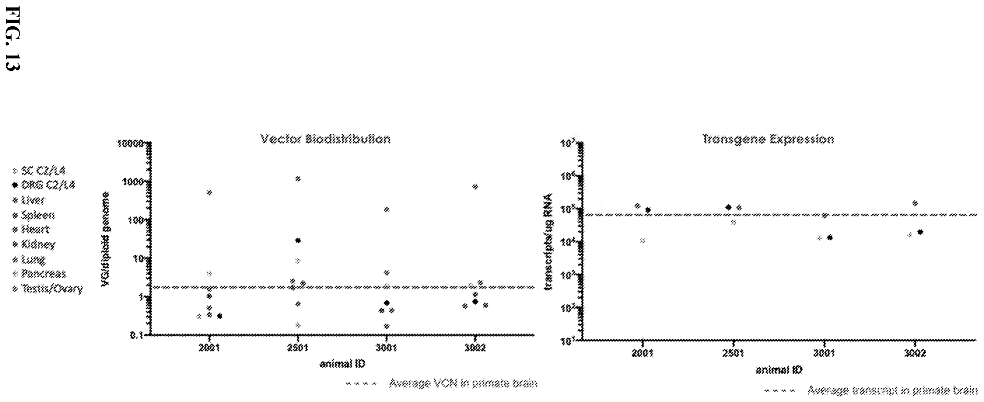

**FIG. 13** shows vector biodistribution (VG/diploid genome) and transgene expression (transcripts/$\mu$g RNA) in peripheral tissue samples outside of the brain. The peripheral tissue samples shown are spinal cord C2/L4 (SC C2/L4), dorsal root ganglion C2/L4 (DRG C2/L4), liver, spleen, heart, kidney, lung, pancreas, and testis/ovary. Average VCN (vector biodistribution) and transcript (transgene expression) in the primate brain is indicated by a dashed line.

## DETAILED DESCRIPTION OF THE DISCLOSURE

Provided herein are engineered transcription factors, or eTFs, that are non-naturally occurring and have been designed to bind to a genomic target site and modulate expression of an endogenous gene of interest. Such eTFs may be designed to either upregulate or downregulate expression (RNA and/or protein expression) of a gene of interest. Also provided herein are microRNA binding sites that may be incorporated into a viral vector and provide selective expression of a transgene in parvalbumin (PV) neurons.

In one aspect, the application provides eTFs that are capable of upregulating expression of the sodium voltage gated channel alpha subunit 1 (SCN1A) gene and increasing expression of its corresponding protein product Nav1.1 and methods of use thereof for treating diseases or disorders associated with a deficiency in Nav1.1, such as, for example, Dravet syndrome.

In another aspect, the application provides microRNA binding sites that reduce expression of a mRNA containing the microRNA binding site in excitatory neurons thereby leading to selective expression of the gene in GABAergic or parvalbumin (PV) neurons and methods of use thereof for selective expression of a gene of interest in PV neurons.

### Definitions

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" means within an acceptable error range for the particular value as determined by one of ordinary skill in the art, which will depend in part on how the value is measured or determined, i.e., the limitations of the measurement system. For example, "about" can mean within one or more than one standard deviation, per the practice in the art. Alternatively, "about" can mean a range of up to 20%, up to 15%, up to 10%, up to 5%, or up to 1% of a given value.

The terms "determining", "measuring", "evaluating", "assessing", "assaying", "analyzing", and their grammatical equivalents can be used interchangeably herein to refer to any form of measurement, and include determining if an element is present or not (for example, detection). These terms can include both quantitative and/or qualitative determinations. Assessing may be relative or absolute.

The term "expression" refers to the process by which a nucleic acid sequence or a polynucleotide is transcribed from a DNA template (such as into mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

As used herein, "operably linked", "operable linkage", "operatively linked", or grammatical equivalents thereof refer to juxtaposition of genetic elements, e.g., a promoter, an enhancer, a polyadenylation sequence, etc., wherein the elements are in a relationship permitting them to operate in the expected manner. For instance, a regulatory element, which can comprise promoter and/or enhancer sequences, is operatively linked to a coding region if the regulatory element helps initiate transcription of the coding sequence. There may be intervening residues between the regulatory element and coding region so long as this functional relationship is maintained.

A "vector" as used herein refers to a macromolecule or association of macromolecules that comprises or associates with a polynucleotide and which can be used to mediate delivery of the polynucleotide to a cell. Examples of vectors include plasmids, viral vectors, liposomes, and other gene delivery vehicles. The vector generally comprises genetic elements, e.g., regulatory elements, operatively linked to a gene to facilitate expression of the gene in a target.

As used herein, "an expression cassette" and "a nucleic acid cassette" are used interchangeably to refer to a combination of nucleic acid sequences or elements that are expressed together or are operably linked for expression. In some cases, an expression cassette refers to the combination of regulatory elements and a gene or genes to which they are operably linked for expression.

The term "AAV" is an abbreviation for adeno-associated virus, and may be used to refer to the virus itself or a derivative thereof. The term covers all serotypes, subtypes, and both naturally occurring and recombinant forms, except where required otherwise. The abbreviation "rAAV" refers to recombinant adeno-associated virus, also referred to as a recombinant AAV vector (or "rAAV vector"). The term "AAV" includes AAV1, AAV2, AAV3, AAV4, AAV5, AAV6, AAV7, AAV8, AAV9, AAV10, AAV11, AAV12, rh10, and hybrids thereof, avian AAV, bovine AAV, canine AAV, equine AAV, primate AAV, non-primate AAV, and ovine AAV. The genomic sequences of various serotypes of AAV, as well as the sequences of the native terminal repeats (TRs), Rep proteins, and capsid subunits are known in the art. Such sequences may be found in the literature or in public databases such as GenBank. An "rAAV vector" as used herein refers to an AAV vector comprising a polynucleotide sequence not of AAV origin (i.e., a polynucleotide heterologous to AAV), typically a sequence of interest for the genetic transformation of a cell. In general, the heterologous polynucleotide is flanked by at least one, and generally by two, AAV inverted terminal repeat sequences (ITRs). An rAAV vector may either be single-stranded (ssAAV) or self-complementary (scAAV). An "AAV virus" or "AAV viral particle" refers to a viral particle composed of at least one AAV capsid protein and an encapsidated polynucleotide rAAV vector. If the particle comprises a heterologous polynucleotide (i.e., a polynucleotide other than a wild-type AAV genome such as a transgene to be delivered to a mammalian cell), it is typically referred to as an "rAAV vector particle" or simply an "rAAV particle". Thus, production of rAAV particle necessarily includes production of rAAV vector, as such a vector is contained within an rAAV particle.

As used herein, the terms "treat", "treatment", "therapy" and the like refer to alleviating, delaying or slowing the progression, prophylaxis, attenuation, reducing the effects or symptoms, preventing onset, inhibiting, or ameliorating the onset of the diseases or disorders. The methods of the present disclosure may be used with any mammal. Exemplary mammals include, but are not limited to rats, cats, dogs, horses, cows, sheep, pigs, and more preferably humans. A therapeutic benefit includes eradication or amelioration of the underlying disorder being treated. Also, a therapeutic benefit is achieved with the eradication or amelioration of one or more of the physiological symptoms associated with the underlying disorder such that an improvement is observed in the subject, notwithstanding that the subject may still be afflicted with the underlying disorder. In some cases, for prophylactic benefit, a therapeutic may be administered to a subject at risk of developing a particular disease, or to a subject reporting one or more of the physiological symptoms of a disease, even though a diagnosis of this disease may not have been made. The methods of the present disclosure may be used with any mammal. In some cases, the treatment can result in a decrease or cessation of symptoms (e.g., a reduction in the frequency, duration and/or severity of seizures). A prophylactic effect includes delaying or eliminating the appearance of a disease or condition, delaying or eliminating the onset of symptoms of a disease or condition, slowing, halting, or reversing the progression of a disease or condition, or any combination thereof.

The term "effective amount" or "therapeutically effective amount" refers to that amount of a composition described herein that is sufficient to affect the intended application, including but not limited to disease treatment, as defined below. The therapeutically effective amount may vary depending upon the intended treatment application (*in vivo*), or the subject and disease condition being treated, e.g., the weight and age of the subject, the severity of the disease condition, the manner of administration and the like, which can readily be determined by one of ordinary skill in the art. The term also applies to a dose that will induce a particular response in a target cell. The specific dose will vary depending on the particular composition chosen, the dosing regimen to be followed, whether it is administered in combination with other compounds, timing of administration, the tissue to which it is administered, and the physical delivery system in which it is carried.

A "fragment" of a nucleotide or peptide sequence refers to a sequence that is shorter than a reference or "full-length" sequence.

A "variant" of a molecule refers to allelic variations of such sequences, that is, a sequence substantially similar in structure and biological activity to either the entire molecule, or to a fragment thereof.

A "functional fragment" of a DNA or protein sequence refers to a fragment that retains a biological activity (either functional or structural) that is substantially similar to a biological activity of the full-length DNA or protein sequence. A biological activity of a DNA sequence can be its ability to influence expression in a manner known to be attributed to the full-length sequence.

The terms "subject" and "individual" are used interchangeably herein to refer to a vertebrate, preferably a mammal, more preferably a human. The methods described herein can be useful in human therapeutics, veterinary applications, and/or preclinical studies in animal models of a disease or condition.

The term *"in vivo"* refers to an event that takes place in a subject's body.

The term *"in vitro"* refers to an event that takes places outside of a subject's body. For example, an *in vitro* assay encompasses any assay run outside of a subject. *In vitro* assays encompass cell-based assays in which cells alive or dead are employed. *In vitro* assays also encompass a cell-free assay in which no intact cells are employed.

In general, "sequence identity" or "sequence homology", which can be used interchangeably, refer to an exact nucleotide-to-nucleotide or amino acid-to-amino acid correspondence of two polynucleotides or polypeptide sequences, respectively. Typically, techniques for determining sequence identity include comparing two nucleotide or amino acid sequences and the determining their percent identity. Sequence comparisons, such as for the purpose of assessing identities, may be performed by any suitable alignment algorithm, including but not limited to the Needleman-Wunsch algorithm (see, e.g., the EMBOSS Needle aligner available at www.ebi.ac.uk/Tools/psa/emboss_needle/, optionally with default settings), the BLAST algorithm (see, e.g., the BLAST alignment tool available at blast.ncbi.nlm.nih.gov/Blast.cgi, optionally with default settings), and the Smith-Waterman algorithm (see, e.g., the EMBOSS Water aligner available at www.ebi.ac.uk/Tools/psa/emboss_water/, optionally with default settings). Optimal alignment may be assessed using any suitable parameters of a chosen algorithm, including default parameters. The "percent identity", also referred to as "percent homology", between two sequences may be calculated as the number of exact matches between two optimally aligned sequences divided by the length of the reference sequence and multiplied by 100. Percent identity may also be determined, for example, by comparing sequence information using the advanced BLAST computer program, including version 2.2.9, available from the National Institutes of Health. The BLAST program is based on the alignment method of Karlin and Altschul, Proc. Natl. Acad. Sci. USA 87:2264-2268 (1990) and as discussed in Altschul, et al., J. Mol. Biol. 215:403-410 (1990); Karlin and Altschul, Proc. Natl. Acad. Sci. USA 90:5873-5877 (1993); and Altschul et al., Nucleic Acids Res. 25:3389-3402 (1997). Briefly, the BLAST program defines identity as the number of identical aligned symbols (i.e., nucleotides or amino acids), divided by the total number of symbols in the shorter of the two sequences. The program may be used to determine percent identity over the entire length of the sequences being compared. Default parameters are provided to optimize searches with short query sequences, for example, with the blastp program. The program also allows use of an SEG filter to mask-off segments of the query sequences as determined by the SEG program of Wootton and Federhen, Computers and Chemistry 17: 149-163 (1993). High sequence identity generally includes ranges of sequence identity of approximately 80% to 100% and integer values there between.

As used herein, "engineered" with reference to a protein refers to a non-naturally occurring protein, including, but not limited to, a protein that is derived from a naturally occurring protein, or where a naturally occurring protein has been modified or reprogrammed to have a certain property.

As used herein, "synthetic" and "artificial" are used interchangeably to refer to a protein or a domain thereof that has low sequence identity (e.g., less than 50% sequence identity) to a naturally occurring human protein. For example, VPR and VP64 domains are synthetic transactivation domains.

As used herein, an "engineered transcription factor" or "eTF" refers to as a non-naturally occurring DNA binding protein or a non-naturally occurring transcription modulator that has been modified or reprogrammed to bind to a specific target binding site and/or to include a modified or replaced transcription effector domain.

As used herein, a "DNA binding domain" can be used to refer to one or more DNA binding motifs, such as a zinc finger or a basic helix-loop-helix (bHLH) motif, individually or collectively as part of a DNA binding protein.

The terms "transcription activation domain", "transcriptional activation domain", "transactivation domain", "trans-activating domain" and "TAD" are used interchangeably herein and refer to a domain of a protein which in conjunction with a DNA binding domain can activate transcription from a promoter by contacting transcriptional machinery (e.g., general transcription factors and/or RNA polymerase) either directly or through other proteins known as co-activators.

The terms "transcriptional repressor domain", "transcription repressor domain" and "TRD" are used interchangeably herein and refer to a domain of a protein which in conjunction with a DNA binding domain can repress transcription from a promoter by contacting transcriptional machinery (e.g., general transcription factors and/or RNA polymerase) either directly or through other proteins known as co-repressors.

The term "GRCh38.p12" refers to Genome Reference Consortium Human Build 38 patch release 12 (GRCh38.p12) having GenBank Assembly Accession No. GCA_000001405.27 and dated 2017/12/21.

Unless otherwise indicated, all terms used herein have the same meaning as they would to one skilled in the art and the practice of the present invention will employ, conventional techniques of molecular biology, microbiology, and recombinant DNA technology, which are within the knowledge of those of skill of the art.

## Engineered Transcription Factors (eTFs) that Upregulate SCN1A

In one aspect, the application provides eTFs that are capable of upregulating expression of the sodium voltage gated channel alpha subunit 1 (SCN1A) gene and increasing expression of its corresponding protein product Nav1.1. The SCN1A gene belongs to a family of genes that code for subunits used for assembling sodium channels. These channels, which transport positively charged sodium ions into cells, play a key role in a cell's ability to generate and transmit electrical signals. The SCN1A gene encodes one part (the alpha subunit) of a sodium channel called Nav1.1. These channels are primarily found in the brain, where they control the flow of sodium ions into cells. Nav1.1 channels are involved in transmitting signals from one nerve cell (or neuron) to another. Several mutations in the SCN1A gene have been found to cause genetic epilepsy with febrile seizures plus (GEFS+), which is a spectrum of seizure disorders of varying severity. These conditions include simple febrile (fever-associated) seizures, which start in infancy and usually stop by age 5, and febrile seizures plus (FS+). FS+ involves febrile and other types of seizures, including those not related to fevers (afebrile seizures), that continue beyond childhood. The GEFS+ spectrum also includes other conditions, such as Dravet syndrome (also known as severe myoclonic epilepsy of infancy or SMEI), that cause more serious seizures that last longer and may be difficult to control. These recurrent seizures (epilepsy) can worsen over time and are often accompanied by a decline in brain function. Many other mutations have been associated with familial hemiplegic migraine, a form of migraine headache that runs in families and at least one mutation has been associated with the effectiveness of certain anti-seizure medications. Thus, an eTF provided herein that increases expression of SCN1A can be used to treat a variety of disease or disorders associated with mutations in the Nav1.1 channel.

Transcription factors (TFs) are proteins that bind specific sequences in the genome and control the expression of genes. The engineered transcription factors or eTFs provided herein that upregulate SCN1A are non-naturally occurring proteins that comprise a DNA binding domain (DBD) and at least one domain that is a transcriptional modulator, e.g., either a transcriptional activation domain (TAD) or a transcriptional repressor domain (TRD). In one embodiment, an eTF that upregulates SCN1A may comprise a DBD and a TAD (e.g., TAD-DBD or DBD-TAD), wherein the DBD and TAD may be derived from the same protein or from different proteins. In another embodiment, an eTF that upregulates SCN1A may comprise a DBD and two TADs, wherein the DBD and TADs are derived from the same protein, the DBD is derived from a first protein and both TADs are derived from a second protein, the DBD and one TAD are derived from a first protein and the second TAD is derived from a second protein, or the DBD is derived from a first protein, one TAD is derived from a second protein, and the second TAD is derived from a third protein (e.g., TAD1-DBD-TAD1, TAD1-DBD-TAD2, TAD 1-TAD 1-DBD, TAD1-TAD2-DBD, DBD-TAD1-TAD1, or DBD-TAD1-TAD2). In another embodiment, an eTF that upregulates SCN1A may comprise a DBD and three TADs, wherein the DBD and TADs are derived from the same protein, the DBD is derived from a first protein and the TADs are derived from one or more different proteins, or wherein the DBD and all of the TADs are all derived from different proteins e.g., TADx-TADx-TADx-DBD, TADx-TADx-DBD-TADx, TADx-DBD-TADx-TADx, or DBD-TADx-TADx-TADx, wherein each X is independently selected and may be the same or different from one or all of the other TADs. Examples include, for example, TAD1-TAD1-DBD-TADI1, TAD1-TAD1-DBD-TAD2, TAD1-TAD2-DBD-TAD1, TAD1-TAD2-DBD-TAD2, TAD1-TAD2-DBD-TAD3, TAD1-DBD-TAD1-TAD1, TAD1- DBD-TAD2-TAD2, TAD1-DBD-TAD1-TAD2, TAD2-DBD-TAD1-TAD2, TAD1-DBD-TAD2-TAD3, TAD1-TAD1-TAD1-DBD, TAD1-TAD2-TAD2-DBD, TAD1-TAD2-TAD2-DBD, TAD1-TAD2-TAD3-DBD, DBD-TAD1-TAD1-TAD1, DBD-TAD1-TAD1-TAD2, DBD-TAD1-TAD2-TAD2, or DBD-TAD1-TAD2-TAD3, etc. In another embodiment, an eTF that upregulates SCN1A may comprise a DBD and four TADs, wherein the DBD and TADs are derived from the same protein, the DBD is derived from a first protein and the TADs are derived from one or more different proteins, or wherein the DBD and all of the TADs are all derived from different proteins e.g., TADx-TADx-TADx-TADx-DBD, TADx-TADx-TADx-DBD-TADx, TADx-TADx-DBD-TADx-TADx, TADx- DBD-TADx-TADx-TADx or DBD-TADx-TADx-TADx-TADx, wherein each X is independently selected and may be the same or different from one or all of the other TADs. Examples include, for examples, TAD1-TAD1-DBD-TAD1-TAD1, TAD1-TAD1-DBD-TAD2-TAD2, TAD1-TAD2-DBD-TAD1-TAD2, TAD1-TAD2-DBD-TAD2-TAD1, TAD1-TAD2-DBD-TAD1-TAD3, TAD1-TAD3-DBD-TAD1-TAD2, TAD1-TAD2-DBD-TAD3-TAD4, TAD1-TAD1-TAD1-DBD-TAD2, TAD1-TAD2-TAD3-DBD-TAD4, TAD1-DBD-TAD1-TAD1-TAD2, TAD1-DBD-TAD2-TAD3-TAD4, TAD1-DBD-TAD1-TAD2-TAD3, TAD2-DBD-TAD1-TAD2-TAD3, TAD1-DBD-TAD2-TAD3-TAD4, TAD1-TAD1-TAD1-TAD1-DBD, TAD1-TAD2-TAD2-TAD3-DBD, TAD1-TAD2-TAD3-TAD4-DBD, DBD-TAD1-TAD1-TAD1-TAD1, DBD-TAD1-TAD1-TAD2-TAD2, DBD-TAD1-TAD2-TAD3-TAD4, or DBD-TAD1-TAD2-TAD3-TAD3, etc. In one embodiment, an eTF that upregulates SCN1A comprises a DBD and two TADs that are located at the same terminus of the DBD (e.g., N-terminus or C-terminus) wherein the DBD is derived from a first protein and both TADs are derived from a second protein, or the DBD is derived from a first protein, one TAD is derived from a second protein, and the second TAD is derived from a third protein (e.g., TAD1-TAD1-DBD, TAD1-TAD2-DBD, DBD-TAD1-TAD1, or DBD-TAD1-TAD2). In certain embodiments, the DBD may be a synthetic construct that contains domains from multiple proteins.

In certain embodiments, a DBD and a TAD and/or two TADs may be directly conjugated, e.g. with no intervening amino acid sequence, a DBD and a TAD and/or two TADs may be conjugated using a peptide linker, or combinations thereof. In certain embodiments, a DBD is conjugated to a TAD and/or one TAD is conjugated to a second TAD via a linker having 1, 2, 3, 4, 5, 6, 7, 8, 9, 10, 11, 12, 13, 14, 15, 16, 17, 18, 19, 20, 25, 30, 35, 40, 50, 60, 70, 75, 80, 90, or 100 amino acids, or from 1-5, 1-10, 1-20, 1-30, 1-40, 1-50, 1-75, 1-100, 5-10, 5-20, 5-30, 5-40, 5-50, 5-75, 5-100, 10-20, 10-30, 10-40, 10-50, 10-75, 10-100, 20-30, 20-40, 20-50, 20-75, or 20-100 amino acids. In some cases, the DBD and the TAD and/or two TADs are conjugated via naturally occurring intervening residues found in the naturally occurring proteins from which the domains are derived. In other embodiments, the DBD and TAD and/or two TADs are conjugated via a synthetic or exogenous linker sequence. Suitable linkers can be flexible, cleavable, non-cleavable, hydrophilic and/or hydrophobic. In certain embodiments, a DBD and a TAD and/or two TADs may be fused together via a linker comprising a plurality of glycine and/or serine residues. Examples of glycine/serine peptide linkers include [GS]n, [GGGS]n (SEQ ID NO: 179), [GGGGS]n (SEQ ID NO: 180), [GGSG]n (SEQ ID NO: 181), wherein n is an integer equal to or greater than 1. In certain embodiments, a linker useful for conjugating a DBD and a TAD and/or two TADs is GGSGGGSG (SEQ ID NO: 177). In certain embodiments, a linker useful for conjugating a DBD and a TAD and/or two TADs is GGSGGGSGGGSGGGSG (SEQ ID NO: 178). In certain embodiments, when a DBD is conjugated to two TADs, the first and second TADs may be conjugated to the DBD with the same or different linkers, or one TAD may be conjugated to the DBD with a linker and the other TAD is directly conjugated to the DBD (e.g., without an intervening linker sequence), or both TADs may be directly conjugated to the DBD (e.g., without intervening linker sequences). In certain embodiments, when a DBD is conjugated to two TADs on the same terminus (e.g., N-terminus or C-terminus), the linker connecting the two TADs may be the same or different from the linker connecting the TADs to the DBD, or the TADs may be conjugated to each other with a linker but the TADs are directly conjugated to the DBD (e.g., without an intervening linker sequence), or the TADs may be directly conjugated to each other (e.g., without intervening linker sequences) but the TADs are conjugated to the DBD with a linker. In certain embodiments, the eTFs provided herein that upregulate SCN1A do not comprise one or more HA tag(s) (e.g., SEQ ID NO: 171) located between the DBD and the one or more TADs.

The eTFs provided herein that upregulate SCN1A have different properties than naturally occurring transcription factors. In certain embodiments, an eTF that upregulates SCN1A comprises a DBD derived from a naturally occurring protein that has been modified such that the DBD binds to a different target site as compared to the naturally occurring protein from which it was derived and the eTF comprising such modified DBD modulates expression from a different gene (e.g., SCN1A) as compared to the naturally occurring protein from which the DBD was derived (e.g., a gene other than SCN1A). In other embodiments, an eTF provided herein that upregulates SCN1A comprises a TAD derived from a naturally occurring protein that has been modified such that the eTF comprising such modified TAD modulates expression from a different gene (e.g., SCN1A) as compared to the naturally occurring protein from which the TAD was derived (e.g., a gene other than SCN1A), and/or the eTF comprising such modified TAD differently modulates expression of SCN1A (e.g., upregulates vs. downregulates) as compared to the naturally occurring protein from which the TAD was derived. In certain embodiments, an eTF provided herein that upregulates SCN1A comprises a DBD derived from a naturally occurring protein and a TAD derived from a naturally occurring protein (either the same or different proteins), wherein both the DBD and TAD have been modified. In such embodiments, the DBD may bind to a different target site as compared to the naturally occurring protein from which it was derived, the eTF comprising such modified DBD and TAD modulates expression from a different gene (e.g., SCN1A) as compared to the naturally occurring proteins from which the domains were derived (e.g., gene(s) other than SCN1A), and/or the eTF comprising such modified DBD and TAD differently modulates expression of SCN1A (e.g., upregulates vs. downregulates) as compared to the naturally occurring proteins from which the DBD and TAD domains were derived.

### DNA Binding Domains (DBDs)

The eTFs provided herein that upregulate SCN1A may comprise any suitable DBD that binds to a target site of interest (e.g., a target site that results in upregulation of SCN1A when bound by an eTF provided herein). In certain embodiments, the DBD may be a synthetically designed DBD. In other embodiments, the DBD may be derived from a naturally occurring protein. DBD families include basic helix-loop-helix (bHLH) (e.g., c-Myc), basic-leucine zipper (e.g., C/EBP), helix-turn-helix (e.g., Oct-1), and zinc fingers (e.g., EGR1 or EGR3). These families exhibit a wide range of DNA binding specificities and gene targets. As contemplated herein, any one of the known human transcription factor proteins can serve as a protein platform for engineering and/or reprogramming a DBD to recognize a specific target site resulting in modulation of expression of an endogenous SCN1A gene. In exemplary embodiments, a DBD provided herein comprises a zinc finger domain, a TALEN binding domain, or a gRNA/Cas complex.

The DBD provided herein may be designed to recognize any target site that results in upregulation of SCN1A. In exemplary embodiments, a DBD is designed to recognize a genomic location and upregulate expression of an endogenous SCN1A gene when bound by an eTF. Binding sites capable of modulating expression of an endogenous SCN1A gene when bound by an eTF provided herein may be located anywhere in the genome that results in modulation of gene expression of SCN1A. In various embodiments, the binding site may be located on a different chromosome from SCN1A, on the same chromosome as SCN1A, upstream of the transcriptional start site (TSS) of the SCN1A gene, downstream of the TSS of the SCN1A gene, proximal to the TSS of the SCN1A gene, distal to the SCN1A gene, within the coding region of the SCN1A gene, within an intron of the SCN1A gene, downstream of the polyA tail of the SCN1A gene, within a promoter sequence that regulates the SCN1A gene, or within an enhancer sequence that regulates the SCN1A gene.

The DBD may be designed to bind to a target binding site of any length so long as it provides specific recognition of the target binding site sequence by the DBD, e.g., with minimal or no off target binding. In certain embodiments, the target binding site may modulate expression of SCN1A when bound by an eTF at a level that is at least 2-fold, 5-fold, 10-fold, 20-fold, 50-fold, 75-fold, 100-fold, 150-fold, 200-fold, 250-fold, 500-fold, or greater as compared to all other genes. In certain embodiments, the target binding site may modulate expression of SCN1A when bound by an eTF at a level that is at least 2-fold, 5-fold, 10-fold, 20-fold, 50-fold, 75-fold, 100-fold, 150-fold, 200-fold, 250-fold, 500-fold, or greater as compared to the 40 nearest neighbor genes (e.g., the 40 genes located closest on the chromosome, either upstream or downstream, of the coding sequence of SCN1A). In certain embodiments, the target binding site may be at least 5 bp, 10 bp, 15 bp, 20 bp, 25 bp, 30 bp, 35 bp, 40 bp, 45 bp or 50 bp, or more. The specific length of the binding site will be informed by the type of DBD in the eTF. In general, the longer the length of the binding site, the greater the specificity for binding and modulation of gene expression (e.g., longer binding sites have fewer off target effects). In certain embodiments, an eTF having a DBD recognizing a longer target binding site has fewer off-target effects associated with non-specific binding (such as, for example, modulation of expression of an off-target gene or gene other than SCN1A) relative to the off-target effects observed with an eTF having a DBD that binds to a shorter target site. In some cases, the reduction in off-target binding is at least 1.2, 1.3, 1.4, 1.5, 2, 3, 4, 5, 6, 7, 8, 9, or 10 fold lower as compared to a comparable eTF having a DBD that recognizes a shorter target binding site.

In certain embodiments, a DBD provided herein can be modified to have increased binding affinity such that it binds to a target binding site for a longer period of time such that a TAD conjugated to the DBD is able to recruit more transcription factors and/or recruit such transcription factor for a longer period of time to exert a greater effect on the expression level of the endogenous SCN1A gene. In certain embodiments, a DBD may be modified to increase its specific binding (or on-target binding) to a desired target site and/or modified to decrease its non-specific or off-target binding.

In various embodiments, binding between a DBD or eTF and a target binding site may be determined using various methods. In certain embodiments, specific binding between a DBD or eTF and a target binding site may be determined using a mobility shift assay, DNase protection assay, or any other *in vitro* method known in the art for assaying protein-DNA binding. In other embodiments, specific binding between an eTF and a target binding site may be determined using a functional assay, e.g., by measuring expression (RNA or protein) of a gene (e.g., SCN1A) when the target binding site is bound by the eTF. For example, a target binding site may be positioned upstream of a reporter gene (such as, for example, eGFP) or the SCN1A gene on a vector contained in a cell or integrated into the genome of the cell, wherein the cell expresses the eTF. Alternatively, a vector expressing the eTF may be introduced into a cell type that naturally contains the SCN1A gene. Greater levels of expression of the reporter gene (or SCN1A) in the presence of the eTF as compared to a control (e.g., no eTF or an eTF that recognizes a different target site) indicate that the DBD of the eTF binds to the target site. Suitable *in vitro* (e.g., non cell based) transcriptional and translational systems may also be used in a similar manner. In certain embodiments, an eTF that binds to a target site may have at least 2-fold, 3-fold, 5-fold, 10-fold, 15-fold, 20-fold, 30-fold, 50-fold, 75-fold, 100-fold, 150-fold, or greater expression of the reporter gene or SCN1A as compared to a control (e.g., no eTF or an eTF that recognizes a different target site).

In certain embodiments, an eTF disclosed herein that upregulates SCN1A recognizes a target binding site that is at least 9bp, 12bp, 15bp, 18bp, 21bp, 24bp, 27bp, 30bp, 33bp, or 36bp in size; more than 9bp, 12bp, 15bp, 18bp, 21bp, 24bp, 27bp, or 30bp; or from 9-33bp, 9-30bp, 9-27bp, 9-24bp, 9-21bp, 9-18bp, 9-15bp, 9-12bp, 12-33bp, 12-30bp, 12-27bp, 12-24bp, 12-21bp, 12-18bp, 12-15bp, 15-33bp, 15-30bp, 15-27bp, 15-24bp, 15-21bp, 15-18bp, 18-33bp, 18-30bp, 18-27bp, 18-24bp, 18-21bp, 21-33bp, 21-30bp, 21-27bp, 21-24bp, 24-33bp, 24-30bp, 24-27bp, 27-33bp, 27-30bp, or 30-33bp. In exemplary embodiments, an eTF disclosed herein that upregulates SCN1A recognizes a target binding site that is 18-27bp, 18bp, or 27 bp.

In certain embodiments, an eTF disclosed herein that upregulates SCN1A recognizes a target binding site that is located on chromosome 2. In certain embodiments, an eTF disclosed herein that upregulates SCN1A recognizes a target binding site that is located on chromosome 2 within 110 kb, 100 kb, 90 kb, 80 kb, 70 kb, 60 kb, 50 kb, 40 kb, 30 kb, 20 kb, 10 kb, 5 kb, 4 kb, 3 kb, 2 kb, or 1 kb upstream or downstream of the TSS of SCN1A. In certain embodiments, an eTF disclosed herein that upregulates SCN1A recognizes a target binding site that is located on chromosome 2 within 110 kb upstream of the TSS of SCN1A. In certain embodiments, an eTF disclosed herein that upregulates SCN1A recognizes a target binding site that is located on chromosome 2 within 110 kb downstream of the TSS of SCN1A. In exemplary embodiments, such target binding sites are 18-27bp, 18bp, or 27 bp.

In certain embodiments, an eTF disclosed herein that upregulates SCN1A recognizes a target binding site that is located on chromosome 2 within positions 166179652-165989571, within positions 166128050-166127958, within positions 166155414-166140590, within positions 166179652-1661777272, or within positions 1659990246-165989592 (all with reference to GRCh38.p12). In exemplary embodiments, such target binding sites are 18-27bp, 18bp, or 27 bp.

In certain embodiments, an eTF disclosed herein that upregulates SCN1A recognizes a target binding site that is (i) 18-27bp, 18bp, or 27 bp, (ii) overlaps with a position on chromosome 2 selected from 166178880, 166177369, 166177362, 166177299, 166177299, 166155393, 166155264, 166149373, 166149176, 166149165, 166149118, 166148953, 166148565, 166142396, 166142391, 166142344, 166142239, 166141162, 166140928, 166140590, 165990076, 165989684, 165989571, 166155255, 166155099, 166148843, 166148361, 166142219, 166141090, 165990246, 165990193, 166149168, 166127991, 166128002, 166128037, or 166128025 (all with reference to GRCh38.p12), and (iii) is capable of producing at least a 1.2 fold increase in expression of SCN1A when bound by an eTF disclosed herein.

In certain embodiments, an eTF disclosed herein that upregulates SCN1A (i) binds to a target site comprising or consisting of any of SEQ ID NOs: 18, 25, 30, 31, or 35-66, and (ii) is capable of producing at least a 1.2 fold increase in expression of SCN1A when bound by an eTF disclosed herein.

In certain embodiments, an eTF disclosed herein that upregulates SCN1A recognizes a target binding site that is (i) 18-27bp, 18bp, or 27 bp, (ii) overlaps with a position on chromosome 2 selected from 166155255, 166155099, 166148843, 166148361, 166142219, 166141090, 165990246, 165990193, 166149168, 166127991, 166128002, 166128037, or 166128025 (all with reference to GRCh38.p12), and (iii) is capable of producing at least a 2 fold increase in expression of SCN1A when bound by an eTF disclosed herein.

In certain embodiments, an eTF disclosed herein that upregulates SCN1A (i) binds to a target site comprising or consisting of any of SEQ ID NOs: 18, 30, 31, 37, 38, 45, 47, 48, 49, 55, 61, 62, or 64, and (ii) is capable of producing at least a 2 fold increase in expression of SCN1A when bound by an eTF disclosed herein.

In certain embodiments, an eTF disclosed herein that upregulates SCN1A recognizes a target binding site that is (i) 18-27bp, 18bp, or 27 bp, and (ii) overlaps with a position on chromosome 2 selected from 166149168, 166127991, 166128002, 166128037 or 166128025 (all with reference to GRCh38.p12), and (iii) is capable of producing at least a 5 fold increase in expression of SCN1A when bound by an eTF disclosed herein.

In certain embodiments, an eTF disclosed herein that upregulates SCN1A (i) binds to a target site comprising or consisting of any of SEQ ID NOs: 18, 30, 31, 37, or 38, and (ii) is capable of producing at least a 5 fold increase in expression of SCN1A when bound by an eTF disclosed herein.

In certain embodiments, an eTF disclosed herein that upregulates SCN1A recognizes a target binding site that is (i) 18-27bp, 18bp, or 27 bp, (ii) overlaps with a position on chromosome 2 selected from 166128002, 166128037, or 166128025 (all with reference to GRCh38.p12), and (iii) is capable of producing at least a 15 fold increase in expression of SCN1A when bound by an eTF disclosed herein.

In certain embodiments, an eTF disclosed herein that upregulates SCN1A (i) binds to a target site comprising or consisting of any of SEQ ID NOs: 30, 37, or 38, and (ii) is capable of producing at least a 15 fold increase in expression of SCN1A when bound by an eTF disclosed herein.

In certain embodiments, an eTF disclosed herein that upregulates SCN1A recognizes a target binding site that is (i) 18-27bp, 18bp, or 27 bp, (ii) overlaps with a position on chromosome 2 selected from 166128037 or 166128025 (all with reference to GRCh38.p12), and (iii) is capable of producing at least a 20 fold increase in expression of SCN1A when bound by an eTF disclosed herein.

In certain embodiments, an eTF disclosed herein that upregulates SCN1A (i) binds to a target site comprising or consisting of any of SEQ ID NOs: 30 or 38, and (ii) is capable of producing at least a 20 fold increase in expression of SCN1A when bound by an eTF disclosed herein.

In certain embodiments, an eTF disclosed herein that upregulates SCN1A recognizes a target binding site that is (i) 18-27bp, 18bp, or 27 bp, (ii) overlaps with a position on chromosome 2 at position 166128025, and (iii) is capable of producing at least a 25 fold increase in expression of SCN1A when bound by an eTF disclosed herein.

In certain embodiments, an eTF disclosed herein that upregulates SCN1A (i) binds to a target site comprising or consisting of SEQ ID NO: 30, and (ii) is capable of producing at least a 25 fold increase in expression of SCN1A when bound by an eTF disclosed herein.

In certain embodiments, an eTF disclosed herein that upregulates SCN1A recognizes a target binding site that is (i) 18-27bp, 18bp, or 27 bp, and (ii) binds to a genomic region that is within at least 1 kb, 750 bp, 500 bp, 400 bp, 300 bp, 200 bp, 100 bp, or 50 bp of a genomic location having a sequence of any one of SEQ ID NOs: 18, 25, 30, 31, or 35-66. In certain embodiments, the target binding site is capable of producing at least a 1.2 fold, 2 fold, 5 fold, 15 fold, 20 fold, or 25 fold increase in expression of SCN1A when bound by an eTF disclosed herein.

In certain embodiments, an eTF disclosed herein that upregulates SCN1A recognizes a target binding site that is (i) 18-27bp, 18bp, or 27 bp, and (ii) binds to a genomic region that is at least partially overlapping with a genomic location having a sequence of any one of SEQ ID NOs: 18, 25, 30, 31, or 35-66. In certain embodiments, the target binding site is capable of producing at least a 1.2 fold, 2 fold, 5 fold, 15 fold, 20 fold, or 25 fold increase in expression of SCN1A when bound by an eTF disclosed herein.

In certain embodiments, an eTF disclosed herein that upregulates SCN1A recognizes a target binding site having any one of the following sequences: SEQ ID NOs: 18, 25, 30, 31, or 35-66. In certain embodiments, the target binding site is capable of producing at least a 1.2 fold, 2 fold, 5 fold, 15 fold, 20 fold, or 25 fold increase in expression of SCN1A when bound by an eTF disclosed herein.

In certain embodiments, an eTF disclosed herein that upregulates SCN1A results in at least 1.5 fold, 2 fold, 3 fold, 4 fold, 5 fold, 6 fold, 7 fold, 8 fold, 9 fold, 10 fold, 15 fold, 20 fold, 25 fold, 50 fold, 100 fold, or greater, or at least a 50%, 60%, 70%, 75%, 80%, 90%, 100%, 125%, 150%, 200%, 250%, 300%, 400%, or 500% or greater upregulation of SCN1A expression (SCN1A RNA and/or Nav1.1 protein) in a cell or *in vivo* as compared to a control (e.g., no eTF or an eTF that does not recognize the target site). In various embodiments, upregulation of SCN1A expression can be detected using PCR methods, Western blot, or immunoassays.

In certain embodiments, an eTF disclosed herein that upregulates SCN1A binds to a target site that is capable of increasing SCN1A expression by at least 1.2 fold, 1.3 fold, 1.4 fold, 1.5 fold, 1.6 fold, 1.7 fold, 1.8 fold, 1.9 fold, 2 fold, 3 fold, 4 fold, 5 fold, 8 fold, 10 fold, 12 fold, 15 fold, 18 fold, 20 fold, 25 fold, 30 fold, 40 fold, 50 fold, 75 fold, 100 fold, or greater or by at least 20%, 30%, 40%, 50% , 60%, 70%, 75%, 80%, 90%, 100%, 125%, 150%, 200%, 250%, 300%, 400%, or 500% or greater relative to a control in a transcriptional activation assay. An exemplary SCN1A transcriptional activation assay is provided herein in Example 3. Briefly, HEK293 are transfected with a plasmid carrying an eTF or a control eGFP reporter construct. 48h following transfection, cells are collected, RNA is isolated, and reverse transcribed and the resulting cDNA samples are analyzed by qPCR (for example, using primers having SEQ ID NOs: 185 and 186) to quantify levels of endogenous SCN1A transcript. GAPDH may be used as a reference gene to determine relative levels of SCN1A expression.

In certain embodiments, an eTF disclosed herein that upregulates SCN1A has minimal off target effects, e.g., off-target effects associated with non-specific binding such as, for example, modulation of expression of an off-target gene or gene other than SCN1A. In one embodiment, an eTF disclosed herein that upregulates SCN1A specifically upregulates SCN1A as compared to a control by at least 5 fold, 10 fold, 15 fold, 20 fold, 25 fold, 30 fold, 40 fold, or 50 fold greater than the expression produced by the eTF for one or more off target genes as compared to a control. In an exemplary embodiment, an eTF disclosed herein that upregulates SCN1A specifically upregulates transcription from the SCN1A gene as compared to a control by at least 15 fold greater than the transcription of the 40 nearest neighbor genes (e.g., the 40 nearest genes located to the coding sequence of SCN1A on chromosome 2) produced by the eTF relative to a control, e.g., PLA2R1, ITGB6, RBMS1, TANK, PSMD14, TBR1, SLC4A10, DPP4, FAP, IFIH1, GCA, FIGN, GRB14, COBLL1, SLC38A11, SCN3A, SCN2A, CSRNP3, GALNT3, TTC21B, SCN9A, SCN7A, B3GALT1, STK39, CERS6, NOSTRIN, SPC25, ABCB11, DHRS9, BBS5, KLHL41, FASTKD1, PPIG, CCDC173, PHOSPHO2, KLHL23, SSB, METTL5, UBR3, and MYO3B (see **TABLE 14).** In various embodiments, upregulation of transcription from the SCN1A gene can be detected using PCR methods.

In certain embodiments, an eTF disclosed herein that upregulates SCN1A is capable of reducing the frequency of seizures in a hyperthermic seizure (HTS) assay in the Scn1a*tm1kea* mouse model of Dravet syndrome. In certain embodiments, an eTF disclosed herein is able to reduce the frequency of seizures at 42.6° C in an HTS assay by at least 1.2 fold, 1.3 fold, 1.4 fold, 1.5 fold, 1.6 fold, 1.7 fold, 1.8 fold, 1.9 fold, 2.0 fold, or more or by at least 20%, 30% 40%, 50%, 55%, 60%, 65%, 70%, 75%, 80%, 85%, 90%, 95%, 100% or more as compared to a control (e.g., PBS treated or treatment with an AAV vector comprising a sequence encoding eGFP). In certain embodiments, an eTF disclosed herein is able to reduce the frequency of seizures at 42.6° C in an HTS assay so that at least 60%, 62%, 65%, 70%, 75%, 76%, 80%, 85%, 86%, 87%, 88%, 89%, 90%, 91%, 92%, 93%, 94%, 95%, 96%, 97%, 98% or 99% of the mice run in the assay are seizure free at 42.6° C. An exemplary HTS assay is described herein in Example 6. Briefly, litters of pups produced from male Scn1a +/- mice crossed with female C57Bl/6J mice may be dosed with an AAV9 vector encoding an eTF that upregulates SCN1A as provided herein or a control vector encoding eGFP via bilateral ICV at P1. Mice may be dosed with ~1.0E10-5.0E12 gc/mouse. The HTS assay is performed in P26-P28 SCN1A heterozygous mice and SCN1A wild-type mice in a mixed 129Stac X C57BL/6 background by increasing the body temperature of the mice (under controlled conditions and with body temperature monitoring) by ~0.5° C every 2 minutes until the onset of the first tonic-clonic seizure accompanied by loss of posture or until a body temperature of 43° C is reached. A mouse is considered to be seizure free if no seizure with loss of posture is detected over the full course of the experiment.

In certain embodiments, an eTF disclosed herein that upregulates SCN1A is capable of increasing the survival of a mouse that is heterozygous for SCN1A, e.g., an Scn1a*tm1kea* mouse line. In certain embodiments, an eTF disclosed herein is able to increase the survival rate of SCN1A heterozygous mice at P100 by at least 1.2 fold, 1.3 fold, 1.4 fold, 1.5 fold, 1.6 fold, 1.7 fold, 1.8 fold, 1.9 fold, 2.0 fold, or more or by at least 20%, 30% 40%, 50%, 55%, 60%, 65%, 70%, 75%, 80%, 85%, 90%, 95%, 100% or more as compared to a control (e.g., PBS treated or treatment with an AAV vector comprising a sequence encoding eGFP). In certain embodiments, an eTF disclosed herein is able to increase the survival rate of SCN1A heterozygous mice at P100 so that at least 65%, 70%, 75%, 80%, 85%, 86%, 87%, 88%, 89%, 90%, 91%, 92%, 93%, 94%, 95%, 96%, 97%, 98%, 99% or 100% of the mice run in the assay are still alive at P100. An exemplary survival assay is described herein in Example 7. Briefly, litters of pups produced from male Scn1a +/- mice crossed with female C57Bl/6J mice may be dosed with AAV9 vector via bilateral ICV at P1. Mice may be dosed with ~1.0E10-5.0E12 gc/mouse. The number of mice that have survived to P100 is determined.

In certain embodiments, an eTF provided herein that upregulates SCN1A may comprise a DBD from a zinc finger protein, derived from a zinc finger protein, or that is a nuclease is inactivated zinc finger protein. A zinc finger is a small protein structural motif that is characterized by the coordination of one or more zinc ions ($Zn^{2+}$) in order to stabilize the fold. Zinc finger (Znf) domains are relatively small protein motifs that contain multiple finger-like protrusions that make tandem contacts with a DNA target site. The modular nature of the zinc finger motif allows for a large number of combinations of DNA sequences to be bound with high degree of affinity and specificity, and is therefore ideally suited for engineering protein that can be targeted to and bind specific DNA sequences. Many engineered zinc finger arrays are based on the zinc finger domain of the murine transcription factor Zif268. Zif268 has three individual zinc finger motifs that collectively bind a 9 bp sequence with high affinity. A wide variety of zinc fingers proteins have been identified and are characterized into different types based on structure as further described herein. Any such zinc finger protein is useful in connection with the DBDs described herein.

Various methods for designing zinc finger proteins are available. For example, methods for designing zinc finger proteins to bind to a target DNA sequence of interest are described, see e.g., Liu Q, et al., Design of polydactyl zinc-finger proteins for unique addressing within complex genomes, Proc Natl Acad Sci USA. 94 (11): 5525-30 (1997); Wright DA et al., Standardized reagents and protocols for engineering zinc finger nucleases by modular assembly, Nat Protoc . Nat Protoc. 2006;1(3):1637-52; and CA Gersbach and T Gaj, Synthetic Zinc Finger Proteins: The Advent of Targeted Gene Regulation and Genome Modification Technologies, Am Chem Soc 47: 2309-2318 (2014). In addition, various web based tools for designing zinc finger proteins to bind to a DNA target sequence of interest are publicly available, see e.g., the Zinc Finger Nuclease Design Software Tools and Genome Engineering Data Analysis website from OmicX available on the world wide web at omictools.com/zfns-category; and the Zinc Finger Tools design website from Scripps available on the world wide web at scripps.edu/barbas/zfdesign/zfdesignhome.php. In addition, various commercially available services for designing zinc finger proteins to bind to a DNA target sequence of interest are available, see e.g., the commercially available services or kits offered by Creative Biolabs (world wide web at creative-biolabs.com/Design-and-Synthesis-of-Artificial-Zinc-Finger-Proteins.html), the Zinc Finger Consortium Modular Assembly Kit available from Addgene (world wide web at addgene.org/kits/zfc-modular-assembly/), or the CompoZr Custom ZFN Service from Sigma Aldrich (world wide web at sigmaaldrich.com/life-science/zinc-finger-nuclease-technology/custom-zfn.html).

In certain embodiments, the eTFs provided herein that upregulate SCN1A comprise a DBD comprising one or more zinc fingers or is derived from a DBD of a zinc finger protein. In some cases, the DBD comprises multiple zinc fingers, wherein each zinc finger is linked to another zinc finger or another domain either at its N-terminus or C-terminus, or both via an amino acid linker. In some cases, a DBD provided herein comprises one or more zinc fingers from one or more of the zinc finger types described in **TABLE 9.** In some cases, a DBD provided herein comprises a plurality of zinc finger structures or motifs, or a plurality of zinc fingers having one or more of SEQ ID NOs: 152-167, or any combination thereof. In certain embodiments, a DBD comprises X-[ZF-X]n and/or [X-ZF]n-X, wherein ZF is a zinc finger domain having any one of the motifs listed in **TABLE 9** (e.g., any one of SEQ ID NOs: 136-146), X is an amino acid linker comprising 1-50 amino acids, and n is an integer from 1-15, e.g., 1, 2, 3, 4, 5, 6, 7, 8, 9, 10, 11, 12, 13, 14, or 15, wherein each ZF can independently have the same sequence or a different sequence from the other ZF sequences in the DBD, and wherein each linker X can independently have the same sequence or a different sequence from the other X sequences in the DBD. Each zinc finger can be linked to another sequence, zinc finger, or domain at its C-terminus, N-terminus, or both. In a DBD, each linker X can be identical in sequence, length, and/or property (e.g., flexibility or charge), or be different in sequence, length, and/or property. In some cases, two or more linkers may be identical, while other linkers are different. In exemplary embodiments, the linker may be obtained or derived from the sequences connecting the zinc fingers found in one or more naturally occurring zinc finger proteins provided in **TABLE 9.** In other embodiments, suitable linker sequences, include, for example, linkers of 5 or more amino acids in length. See, also, U.S. Pat. Nos. 6,479,626; 6,903,185; and 7,153,949 for exemplary linker sequences of 6 or more amino acids in length, each of which is incorporated herein in their entireties. The DBD proteins provided herein may include any combination of suitable linkers between the individual zinc fingers of the protein. The DBD proteins described herein may include any combination of suitable linkers between the individual zinc fingers of the protein.

In certain embodiments, the eTFs provided herein that upregulate SCN1A comprise a DBD comprising one or more classic zinc fingers. A classical C2H2 zinc-finger has two cysteines in one chain and two histidine residues in another chain, coordinated by a zinc ion. A classical zinc-finger domain has two β-sheets and one α-helix, wherein the α-helix interacts with a DNA molecule and forms the basis of the DBD binding to a target site and may be referred to as the "recognition helix". In exemplary embodiments, the recognition helix of a zinc fingers comprises at least one amino acid substitution at position -1, 2, 3 or 6 thereby changing the binding specificity of the zinc finger domain. In other embodiments, an DBD provided herein comprises one or more non-classical zinc-fingers, e.g., C2-H2, C2-CH, and C2-C2.

In another embodiment, an eTF provided herein that upregulates SCN1A comprises a DBD comprising a zinc finger motif having the following structure: LEPGEKP - [YKCPECGKSFS X HQRTH TGEKP]n - YKCPECGKSFS X HQRTH - TGKKTS (SEQ ID NO: 147), wherein n is an integer from 1-15, e.g., 1, 2, 3, 4, 5, 6, 7, 8, 9, 10, 11, 12, 13, 14, or 15, and each X independently is a recognition sequence (e.g., a recognition helix) capable of binding to 3 bp of the target sequence. In exemplary embodiments, n is 3, 6 or 9. In a particularly preferred embodiment, n is 6. In various embodiments, each X may independently have the same amino acid sequence or a different amino acid sequence as compared to other X sequences in the DBD. In an exemplary embodiment, each X is a sequence comprising 7 amino acids that has been designed to interact with 3 bp of the target binding site of interest using the Zinger Finger Design Tool from Scripps located on world wide web at scripps.edu/barbas/zfdesign/zfdesignhome.php.

Since each zinc finger within a DBD recognizes 3 bp, the number of zinc fingers included in the DBD informs the length of the binding site recognized by the DBD, e.g., a DBD with 1 zinc finger will recognize a target binding site having 3 bp, a DBD with 2 zinc fingers will recognize a target binding site having 6 bp, a DBD with 3 zinc fingers will recognize a target binding site having 9 bp, a DBD with 4 zinc fingers will recognize a target binding site having 12 bp, a DBD with 5 zinc fingers will recognize a target binding site having 15 bp, a DBD with 6 zinc fingers will recognize a target binding site having 18 bp, a DBD with 9 zinc fingers will recognize a target binding site having 27 bp, etc. In general, DBD that recognize longer target binding sites will exhibit greater binding specificity (e.g., less off target or non-specific binding).

In other embodiments, an eTF provided herein that upregulates SCN1A comprises a DBD that is derived from a naturally occurring zinc finger protein by making one or more amino acid substitutions in one or more of the recognition helices of the zinc finger domains so as to change the binding specificity of the DBD (e.g., changing the target site recognized by the DBD). DBD provided herein may be derived from any naturally occurring zinc finger protein. In various embodiments, such DBD may be derived from a zinc finger protein of any species, e.g., a mouse, rat, human, etc. In an exemplary embodiment, a DBD provided herein is derived from a human zinc finger protein. In certain embodiments, a DBD provided herein is derived from a naturally occurring protein listed in **TABLE 9.** In an exemplary embodiment, a DBD protein provided herein is derived from a human EGR zinc finger protein, e.g., EGR1, EGR2, EGR3, or EGR4.

In certain embodiments, an eTF provided herein that upregulates SCN1A comprises a DBD that is derived from a naturally occurring protein by modifying the DBD to increase the number of zinc finger domains in the DBD protein by repeating one or more zinc fingers within the DBD of the naturally occurring protein. In certain embodiments, such modifications include duplication, triplication, quadruplication, or further multiplication of the zinc fingers within the DBD of the naturally occurring protein. In some cases, one zinc finger from a DBD of a human protein is multiplied, e.g., 2, 3, 4, 5, 6, 7, 8, 9, 10, 11, 12, or more copies of the same zinc finger motif is repeated in the DBD of the eTF. In some cases, a set of zinc fingers from a DBD of a naturally occurring protein is multiplied, e.g., a set of 3 zinc fingers from a DBD of a naturally occurring protein is duplicated to yield an eTF having a DBD with 6 zinc fingers, is triplicated to yield a DBD of an eTF with 9 zinc fingers, or is quadruplicated to yield a DBD of an eTF with 12 zinc fingers, etc. In some cases, a set of zinc fingers from a DBD of a naturally occurring protein is partially replicated to form a DBD of an eTF having a greater number of zinc fingers, e.g., a DBD of an eTF comprises four zinc fingers wherein the zinc fingers represent one copy of the first zinc finger, one copy of the second zinc finger, and two copies of a third zinc finger from a naturally occurring protein for a total of four zinc fingers in the DBD of the eTF. Such DBD are then further modified by making one or more amino acid substitutions in one or more of the recognition helices of the zinc finger domains so as to change the binding specificity of the DBD (e.g., changing the target site recognized by the DBD). In exemplary embodiments, the DBD is derived from a naturally occurring human protein, such as a human EGR zinc finger protein, e.g., EGR1, EGR2, EGR3, or EGR4.

Human EGR1 and EGR3 are characterized by a three-finger C2H2 zinc finger DBD. The generic binding rules for zinc fingers provide that all three fingers interact with its cognate DNA sequence with similar geometry, using the same amino acids in the alpha helix of each zinc finger to determine the specificity or recognition of the target binding site sequence. Such binding rules allow one to modify the DBD of EGR1 or EGR3 to engineer a DBD that recognizes a desired target binding site. In some cases, the 7-amino acid DNA recognition helix in a zinc finger motif of EGR1 or EGR3 is modified according to published zinc finger design rules. In certain embodiments, each zinc finger in the three-finger DBD of EGR1 or EGR3 is modified, e.g., by altering the sequence of one or more recognition helices and/or by increasing the number of zinc fingers in the DBD. In certain embodiments, EGR1 or EGR3 is reprogrammed to recognize a target binding site of at least 9, 12, 15, 18, 21, 24, 27, 30, 33, 36 or more base pairs at a desired target site. In certain embodiments, such DBD derived from ERG1 or EGR3 comprises at least 3, 4, 5, 6, 7, 8, 9, 10, 11, 12, or more zinc fingers. In exemplary embodiment, one or more of the zinc fingers in the DBD comprises at least one amino acid substitution at position -1, 2, 3 or 6 of the recognition helix.

In various embodiments, an eTF that upregulates SCN1A comprising a DBD derived from EGR1 or EGR3 has a DNA binding specificity that is different from the binding specificity of naturally occurring EGR1 or EGR3, e.g., the DBD recognizes a target binding site having a sequence different from the sequence of the binding site recognized by unmodified EGR1 or EGR3: (GCG(T/G)GGGCG) (SEQ ID NO: 182).

In other embodiments, an eTF provided herein that upregulates SCN1A comprises a DBD that is a gRNA/Cas complex. CRISPR (clustered regularly interspaced short palindromic repeats)/Cas9 is a genome editing tools that allows for site-specific genomic targeting. The type II CRISPR/Cas system is a prokaryotic adaptive immune response system that uses noncoding RNAs to guide the Cas9 nuclease to induce site-specific DNA cleavage. The CRISPR/Cas9 system has been harnessed to create a simple, RNA-programmable method to mediate genome editing in mammalian cells. A single guide RNA (sgRNA) may be generated to direct the Cas9 nuclease to a specific genomic location that is then bound by the gRNA/Cas9 complex. A gRNA may be designed to bind to a target site of interest using various methods and tools. For example, methods for designing gRNAs to bind to a target DNA sequence of interest are described in Aach, et al. Flexible algorithm for identifying specific Cas9 targets in genomes. BioRxiv, Cold Spring Harbor Labs. doi: http://dx.doi.org/10.1101/005074 (2014); Bae, et al. Cas-OFFinder: a fast and versatile algorithm that searches for potential off-target sites of Cas9 RNA-guided endonucleases. Bioinformatics. 30(10):1473-1475 (2014); Doench, J. G. et al. Optimized sgRNA design to maximize activity and minimize off-target effects of CRISPR-Cas9. Nat Biotech 34, 184-191 (2016); Gratz, et al. Highly specific and efficient CRISPR/Cas9-catalyzed homology-directed repair in Drosophila. Genetics. 196(4):961-971 (2014); Heigwer, et al. E-CRISP: fast CRISPR target site identification. Nat Methods. 11(2): 122-123 (2014); Ma, et al. A guide RNA sequence design platform for the CRISPR/Cas9 system for model organism genomes. Biomed Res Int. doi:http://doi.org/10.1155/2013/270805 (2013); Montague, et al. CHOPCHOP: a CRISPR/Cas9 and TALEN web tool for genome editing. Nucleic Acids Res. 42(W1):W401-W407 (2014); Liu, et al. CRISPR-ERA: a comprehensive design tool for CRISPR-mediated gene editing, repression and activation. Bioinformatics. 31(22):3676-3678 (2015); Ran, et al. In vivo genome editing using Staphylococcus aureus Cas9. Nature. 520(7546):186-191 (2015); Wu, et al. Target specificity of the CRISPR-Cas9 system. Quant Biol. 2(2):59-70 (2015); Xiao, et al. CasOT: a genome-wide Cas9/gRNA off-target searching tool. Bioinformatics. 30(8):1180-1182 (2014); Zetsche, et al. Cpf1 is a single RNA-guided endonuclease of a Class 2 CRISPR-Cas System. Cell. 163(3):759-771 (2015). In addition, various web based tools for designing gRNAs to bind to a DNA target sequence of interest are publicly available, see e.g., the CRISPR gRNA Design tool available from AUTM on world wide web at atum.bio/eCommerce/-cas9/input?multipleContacts=false; the CRISPRa/i gRNA design tool available from the Broad Institute on the world wide web at portals.broadinstitute.org/gpp/public/analysis-tools/sgrna-design-crisprai; the E-CRISP design tool available from DKFZ German Cancer Research Center available on the world wide web at e-crisp.org/E-CRISP/; and the Knockout Guide Design tool available from Synthego on the world wide web at design.synthego.com/#/. In addition, various commercially available services for designing gRNAs to bind to a DNA target sequence of interest are available, see e.g., the commercially available services offered by IDT (world wide web at idtdna.com/site/order/designtool/index/-CRISPR_SEQUENCE), ThermoFisher (world wide web at thermofisher.com/order/custom-oligo/crispr), and GenScript (world wide web at genscript.com/gRNA-design-tool.html).

In exemplary embodiments, a DBD that is a gRNA/Cas complex comprises a nuclease deactivated Cas protein or dCas, such as for example, a dCas9, such as nuclease deactivated *Staphylococcus aureus* (dSaCas9) or nuclease deactivated *Streptococcus pyogenes* Cas9 (dSpCas9). The gRNA is provided as a sequence comprising a targeting region, which targets the gRNA/Cas complex to a desired target site, and scaffold region, that facilitates the interaction with the Cas protein. Any suitable gRNA scaffold may be used in connection with the gRNAs provided herein. In an exemplary embodiment, the gRNA is a single gRNA or sgRNA and comprises the following scaffold sequence: 5'-GTTTTAGTAC TCTGGAAACAGAATCTACTAAAACAAGGCAAAATGCCGTGTTTATCT CGTCAACTTGTTGGCGAGA-3' (SEQ ID NO: 183). The targeting region of the guide RNA is attached to the 5' end of the scaffold sequence to form the complete sgRNA. In certain embodiments, a gRNA and dCas protein may be expressed from the same expression cassette. In certain embodiments, a U6 promoter is used to express the gRNA. In other embodiments, a gRNA may be expressed in a cell that has been engineered to stably express the dCas-TAD protein, e.g., either by stably integrating the dCas into the genome or on a plasmid that is stably maintained extrachromosomally.

In other embodiments, an eTF provided herein that upregulates SCN1A may comprise a DBD from a TALEN, derived from a TALEN, or that is a nuclease inactivated TALEN. Transcription activator-like effector nucleases (TALEN) are restriction enzymes that contain a DBD and a nuclease domain that can be engineered to cut specific sequences of DNA. TALENs are created by conjugating a TAL effector DNA binding domain to a DNA cleavage domain (e.g., a nuclease). Transcription activator-like effectors (TALEs) can be engineered to bind to a desired target DNA sequence thereby directing the nuclease domain to a specific location.

TAL effectors are bacterial proteins from *Xanthomonas* bacteria. The DNA binding domain contains a repeated highly conserved 33-34 amino acid sequence with divergent 12th and 13th amino acids. These two positions, referred to as the Repeat Variable Diresidue (RVD), are highly variable and show a strong correlation with specific nucleotide recognition. This straightforward relationship between amino acid sequence and DNA recognition allows the engineering of DBDs that specifically target a desired sequence by selecting a combination of repeat segments containing the appropriate RVDs.

Various methods for designing TALEs are available. For example, methods for designing TALEs to bind to a target DNA sequence of interest are described in T. Cermak et al., Nucleic Acids Research. 39 (12): e82 (2011); F. Zhang F et al., Nature Biotechnology. 29 (2): 149-53 (2011); R. Morbitzer et al., Nucleic Acids Research. 39 (13): 5790-9 (2011); T. Li et al., Nucleic Acids Research. 39 (14): 6315-25 (2011); R. Geissler et al., PLOS One. 6(5): e19509 (2011); and E. Weber et al., PLOS One. 6 (5): e19722 (2011). In addition, various web based tools for designing TALEs to bind to a DNA target sequence of interest are publicly available, see e.g., the E-Talen available on the world wide web at e-talen.org/E-TALEN/TAL and the Effector Nucleotide Targeter 2.0 tool available on the world wide web at talent.cac.cornell.edu/node/add/single-tale. In addition, various commercially available services for designing TALEs to bind to a DNA target sequence of interest are available, see e.g., the commercially available services offered by OmicX (world wide web at omictools.com/), Addgene (world wide web at addgene.org/talen/guide/), or ThermoFisher (world wide web at thermofisher.com/us/en/home/life-science/genome-editing/geneart-tals/tal-design-tool.html). In addition, the publicly available software program (DNAWorks) may be used to design oligonucleotides suitable for assembly of TALEs, see e.g., D. Hoover D Methods in Molecular Biology. 852: 215-23 (2012).

### Transcriptional Modulation Domains

The eTFs provided herein that upregulate SCN1A may comprise any suitable domain that is capable of recruiting one or more protein factors that can modulate transcription (e.g., RNA polymerase II, CBP/p300, CREB or KRAB) or the level of gene expression from a gene of interest when the eTF is bound to a target site via the DBD (e.g., a zinc finger DBD, gRNA/Cas DBD, or TALE DBD). In certain embodiments, such a domain recruits protein factors that increase the level of transcription or gene expression of a gene of interest and is a transcriptional activation domain (TAD). In other embodiments, such a domain recruits protein factors that decrease the level of transcription or gene expression from a gene of interest and is a transcriptional repressor domain (TRD). In certain embodiments, the transcriptional modulation domain (TAD or TRD) may be a synthetically designed domain. In other embodiments, the transcriptional modulation domain (TAD or TRD) may be derived from a naturally occurring protein, e.g., a transcription factor, a transcriptional co-activator, a transcriptional co-repressor, or a silencer protein. In various embodiments, the transcriptional modulation domain (TAD or TRD) may be derived from a protein of any species, e.g., a mouse, rat, monkey, virus, or human.

In one exemplary embodiment, a TAD suitable for use in the eTFs provided herein that upregulate SNC1A is derived from a viral protein. Exemplary TADs derived from viral proteins include, for example, a TAD domain of VP64 (SEQ ID NO: 133), VPR (SEQ ID NO: 132), VP16, VP128, p65, p300, or any functional fragment or variant thereof, or a sequence having at least 80%, 85%, 90%, 91%, 92%, 93%, 94%, 95%, 96%, 97%, 98%, or 99% sequence identity thereto.

In another exemplary embodiment, a TAD suitable for use in the eTFs provided herein that upregulate SCN1A is derived from a human protein. Exemplary TADs derived from human proteins include, for example, a TAD domain of CBP/p300-interacting transactivator 2 (CITED2) (SEQ ID NO: 134), CBP/p300-interacting transactivator 4 (CITED4) (SEQ ID NO: 135), EGR1 (SEQ ID NO: 176), CREB3 (SEQ ID NO: 224), or EGR3 (SEQ ID NO: 175), or any functional fragment or variant thereof, or a sequence having at least 80%, 85%, 90%, 91%, 92%, 93%, 94%, 95%, 96%, 97%, 98%, or 99% sequence identity thereto.

In certain embodiments, an eTF that upregulates SCN1A comprises a zinc finger DBD that is conjugated to a transcriptional activation domain or TAD. In various embodiments, the zinc finger DBD may be conjugated to a TAD from a viral protein, such as VP64 or VPR, or a TAD from a human protein, such as CITED2, CITED4, or CREB3. In certain embodiments, a zinc finger DBD derived from a human protein, e.g., EGR1 or EGR3, is conjugated to a TAD derived from a human protein, e.g., CITED2, CITED4, or CREB3. In certain embodiments, a zinc finger DBD derived from a human protein, e.g., EGR1 or EGR3, is conjugated to a VP64 or VPR TAD. In certain embodiments, a synthetic zinc finger DBD or zinc finger DBD having less than 75% sequence identity to a human protein, e.g., EGR1 or EGR3, is conjugated to a TAD derived from a human protein, e.g., CITED2, CITED4, or CREB3. In certain embodiments, a synthetic zinc finger DBD or zinc finger DBD having less than 75% sequence identity to a human protein, e.g., EGR1 or EGR3, is conjugated to a VP64 or VPR TAD.

In certain embodiments, a dCas protein is conjugated to a TAD. In various embodiments, the dCas9 may be conjugated to a TAD from a viral protein, such as VP64 or VPR, or a TAD from a human protein, such as CITED2, CITED4, or CREB3. In exemplary embodiments, a dCas9 is conjugated to a VP64 or VPR TAD.

In certain embodiments, a TALE protein is conjugated to a TAD. In various embodiments, the TALE may be conjugated to a TAD from a viral protein, such as VP64 or VPR, or a TAD from a human protein, such as CITED2, CITED4, or CREB3. In exemplary embodiments, a TALE is conjugated to a VP64 or VPR TAD.

### eTFs That Upregulate SCN1A and Are Highly Homologous to Human Proteins

In certain embodiments, an eTF disclosed herein that upregulates SCN1A has a high percent identity to one or more human proteins (as further described below). In certain embodiments, such eTFs have at least 75%, 80%, 85%, 86%, 87%, 88%, 89%, 90%, 91%, 92%, 93%, 94%, 95%, 96%, 97%, 98% or 99% overall sequence identity to one or more human proteins. In certain embodiments, such eTFs exhibit reduced immunogenicity as compared to an eTF having a lower overall percent sequence identity to one or more human proteins. In various embodiments, a reduction in immunogenicity can be measured using an elispot assay, an immunoassay, or an in silico method. In certain embodiments, such eTFs may comprise a DBD derived from human EGR1 or EGR3 and a TAD derived from human EGR1, EGR3, CITED2, CITED4, or CREB3. Such eTFs have little to no immunogenicity when administered to a subject or have reduced immunogenicity as compared to eTFs having lower percent identity to human protein sequences.

In certain embodiments, an eTF provided herein that upregulates SNC1A has at least 75%, 76%, 77%, 78%, 79%, 80%, 81%, 82%, 83%, 84%, 85%, 86%,87%, 88%, 89%, 90%, 91%, 92%, 93%, 94%, 95%, 96%, 97%, 98%, or 99% sequence identity to one or more human proteins. When an eTF provided herein that upregulates SCN1A comprises a DBD and a TAD derived from the same protein, the percent identity to a human protein may be determined by calculating the total number of amino acid residues in the eTF that match the human protein from which it was derived (e.g., EGR1 or EGR3), divided by the total number of amino acid residues in the eTF. When an eTF provided that upregulates SCN1A comprises a DBD from one human protein and a TAD derived from a different human protein, the percent identity to human may be determined by separately calculating the percent identity to human of each domain and summing the two together, e.g., (i) calculating the total number of amino acid residues in the DBD that match the human protein from which it was derived (e.g., EGR1 or EGR3), divided by the total number of amino acid residues in the eTF; (ii) calculating the total number of amino acid residues in the TAD that match the human protein from which it was derived (e.g., CITED2, CITED4, or CREB3), divided by the total number of amino acid residues in the eTF; and (iii) summing the total of (i) and (ii). In such an embodiment, the domains are divided as follows: the first domain runs from the N-terminus of the eTF through the start of the coding sequence for the second domain, and the second domain runs from the start of the coding sequence for the second domain through the C-terminus of the eTF (e.g., for an eTF having the configuration NLS-DBD-linker-NLS-TAD, the first domain would be NLS-DBD-linker and the second domain would be NLS-TAD). When an eTF provided herein that upregulates SNC1A comprises a DBD from one human protein and two TADs derived from one or more different human protein, the percent identity to human may be determined by separately calculating the percent identity to human of each domain and summing all the three together, e.g., (i) calculating the total number of amino acid residues in the DBD that match the human protein from which it was derived (e.g., EGR1 or EGR3), divided by the total number of amino acid residues in the eTF; (ii) calculating the total number of amino acid residues in the first TAD that match the human protein from which it was derived (e.g., CITED2, CITED4, or CREB3), divided by the total number of amino acid residues in the eTF; (iii) calculating the total number of amino acid residues in the second TAD that match the human protein from which it was derived (e.g., CITED2, CITED4, or CREB3), divided by the total number of amino acid residues in the eTF; and (iv) summing the total of (i), (ii) and (iii). In such an embodiment, the domains are divided as follows: the first domain runs from the N-terminus of the eTF through the start of the coding sequence for the second domain, the second domain runs from the start of the coding sequence for the second domain through the start of the coding sequence for the third domain, and the third domain runs from the start of the coding sequence for the third domain through the C-terminus of the eTF (e.g., for an eTF having the configuration NLS-TAD1-linker-NLS-DBD-linker-NLS-TAD2, the first domain would be NLS-TAD1-linker, the second domain would be NLS-DBD-linker, and the third domain would be NLS-TAD2). The percent identity to one or more human proteins as described in this section may be determined using the percent identity output obtained using the standard protein BLAST tool available from the NCBI (e.g., the blastp suite alignment tool, using the blastp (protein -> protein) algorithm with default parameters) available on the world wide web from the NCBI website at blast.ncbi.nlm.nih.gov/.

In certain embodiments, an eTF provided herein that upregulates SCN1A has the benefit of eliciting little, minimal, or no adverse immune response in a human subject due to a high degree of sequence identity to naturally occurring human proteins. In certain embodiments, an eTF provided herein that upregulates SCN1A elicits reduced immunogenicity, e.g., at least a 0.5, 1, 1.5, 2, 2.5, 3, 3.5, 4, 4.5, 5, 10, 15, 20, 25, 30, 35, 40, 45, or 50 fold or greater fold reduction in immunogenicity as compared to the immunogenicity observed with an eTF comprising a lower percent identity to one or more human proteins, e.g., an eTF comprising less than 50%, 55%, 65%, or 70% sequence identity to one or more human proteins. In some cases, reduction in immunogenicity can be measured using an elispot assay, an immunoassay, or an in silico method. A gene therapy having a low or minimal immunogenicity has several advantages, including improved patient tolerance, decreased dosage needed to achieve a therapeutic effect, prolonged therapeutic effects after one administration, ability to be administered multiple times or in multiple doses as needed, sustained therapeutic efficacy over a longer period of time per administration, increased safety, and/or increased effectiveness of a gene therapy.

In certain embodiments, the eTFs provided herein that upregulate SCN1A and have a high percent sequence identity to one or more human proteins comprise a DBD and a TAD derived from one or more naturally occurring human proteins. In certain embodiments, such eTFs may comprise a DBD derived from any naturally occurring human protein comprising a DBD. In exemplary embodiments, an eTF provided herein that upregulates SCN1A and has a high percent sequence identity to one or more human proteins comprises a DBD derived from a naturally occurring zinc finger protein, such as, for example, any one of Constructs 5-27, 36-41, or 44-53 listed in **TABLE 1.** In certain embodiments, an eTF provided herein that upregulates SCN1A and has a high percent sequence identity to one or more human proteins comprises a DBD derived from a human EGR protein, such as EGR1, EGR2, EGR3, or EGR4. In exemplary embodiments, an eTF provided herein that upregulates SCN1A and has a high percent sequence identity to one or more human proteins comprises a DBD derived from a human EGR1 or EGR3. In various embodiments, an eTF provided herein that upregulates SCN1A and has a high percent sequence identity to one or more human proteins comprises a DBD derived from a human zinc finger protein wherein minimal amino acid changes (e.g., 1, 2, 3, 4, 5, 6, 7, or 1-2, 1-3, 1-4, 1-5, 1-6, 1-7, 2-3, 2-4, 2-5, 2-6, 2-7, 3-4, 3-5, 36, or 3-7 amino acid changes) have been made in one or more zinc finger domains of the DBD to alter the binding specificity of the DBD to recognize a target binding site of interest. Such sequence modifications are preferably made in the recognition helices of the zinc finger domains of the DBD, while the rest of the human zinc finger DBD or protein (including the TAD) remains unmodified so as to preserve as much sequence identity to the naturally occurring human protein as possible.

In certain embodiments, the eTFs provided herein that upregulate SCN1A and have a high percent sequence identity to one or more human proteins comprises one or more transcriptional modulation domains (e.g., a TAD) derived from a human protein conjugated to a DBD derived from a human protein. In various embodiments, the transcriptional modulation domain may be derived from any naturally occurring human protein having a domain capable of recruiting one or more protein factors that can modulate transcription (e.g., RNA polymerase II, a co-activator protein, or a co-repressor protein) or the level of gene expression from a gene of interest when the eTF is bound to a target site via the DBD. In exemplary embodiments, the TAD is derived from a human EGR protein, such as for example, human EGR1, EGR2, EGR3 or EGR4, or a human cited protein, such as for example, a human CITED2 or CITED4 protein. In an exemplary embodiment, an eTF provided herein that upregulates SCN1A and has a high percent sequence identity to one or more human proteins comprises a TAD from a human EGR1 or EGR3 protein. In another exemplary embodiment, an eTF provided herein that upregulates SCN1A and has a high percent sequence identity to one or more human proteins comprises a TAD from a human CITED2 or CITED4 protein.

In one embodiment, an eTF provided herein that upregulates SCN1A and having a high percent sequence identity to one or more human proteins may comprise a human DBD (hDBD) and a human TAD (hTAD) (e.g., hTAD-hDBD or hDBD-hTAD), wherein the hDBD and hTAD may be derived from the same human protein or from human different proteins. In another embodiment, an eTF provided herein having a high percent sequence identity to one or more human proteins may comprise a hDBD and two hTADs, wherein the hDBD and hTADs are derived from the same human protein, the hDBD is derived from a first human protein and both hTADs are derived from a second human protein, the hDBD and one hTAD are derived from a first human protein and the second hTAD is derived from a second human protein, or the hDBD is derived from a first human protein, one hTAD is derived from a second human protein, and the second hTAD is derived from a third human protein (e.g., hTAD1-hDBD-hTAD1, hTAD1-hDBD-hTAD2, hTAD1-hTAD1-hDBD, hTAD1-hTAD2-hDBD, hDBD-hTAD1-hTAD1, or hDBD-hTAD1-hTAD2).

In exemplary embodiments, an eTF provided herein having a high percent sequence identity to one or more human proteins comprises any one of the following configurations: (i) a hDBD and a hTAD both derived from human EGR1; (ii) a hDBD and a hTAD both derived from human EGR3; (iii) a hDBD derived from human EGR1 and a hTAD derived from CITED2 (e.g., hEGR1 DBD-hCITED2 TAD or hCITED2 TAD-hEGR1 DBD); (iv) a hDBD derived from human EGR1 and a hTAD derived from human CITED4 (e.g., hEGR1 DBD-hCITED4 TAD or hCITED4 TAD-hEGR1 DBD); (v) a hDBD derived from human EGR3 and a hTAD derived from CITED2 (e.g., hEGR3 DBD-hCITED2 TAD or hCITED2 TAD-hEGR3 DBD); (vi) a hDBD derived from human EGR3 and a hTAD derived from human CITED4 (e.g., hEGR3 DBD-hCITED4 TAD or hCITED4 TAD-hEGR3 DBD); (vii) a hDBD derived from human EGR1 and two hTADs derived from CITED2 (e.g., hCITED2 TAD-hEGR1 DBD-hCITED2 TAD, hCITED2 TAD-hCITED2 TAD-hEGR1 DBD, or hEGR1 DBD-hCITED2 TAD-hCITED2 TAD); (viii) a hDBD derived from human EGR1 and two hTADs derived from human CITED4 (e.g., hCITED4 TAD-hEGR1 DBD-hCITED4 TAD, hCITED4 TAD-hCITED4 TAD-hEGR1 DBD, or hEGR1 DBD-hCITED4 TAD-hCITED4 TAD); (ix) a hDBD derived from human EGR3 and two hTADs derived from human CITED2 (e.g., hCITED2 TAD-hEGR3 DBD-hCITED2 TAD, hCITED2 TAD-hCITED2 TAD-hEGR3 DBD, or hEGR3 DBD-hCITED2 TAD-hCITED2 TAD); (x) a hDBD derived from human EGR3 and two hTADs derived from human CITED4 (e.g., hCITED4 TAD-hEGR3 DBD-hCITED4 TAD, hCITED4 TAD-hCITED4 TAD-hEGR3 DBD, or hEGR3 DBD-hCITED4 TAD-hCITED4 TAD); (xi) a hDBD derived from human EGR1, a first hTAD derived from human CITED2, a second hTAD derived from human CITED4 (e.g., hCITED2 TAD-hEGR1 DBD-hCITED4 TAD, hCITED4 TAD-hEGR1 DBD-hCITED2 TAD, hCITED2 TAD-hCITED4 TAD-hEGR1 DBD, hCITED4 TAD-hCITED2 TAD-hEGR1 DBD, hEGR1 DBD-hCITED4 TAD- hCITED2 TAD, or hEGR1 DBD-hCITED2 TAD- hCITED4 TAD); or (xii) a hDBD derived from human EGR3, a first hTAD derived from human CITED2, a second hTAD derived from human CITED4 (e.g., hCITED2 TAD-hEGR3 DBD-hCITED4 TAD, hCITED4 TAD-hEGR3 DBD-hCITED2 TAD, hCITED2 TAD-hCITED4 TAD-hEGR3 DBD, hCITED4 TAD-hCITED2 TAD-hEGR3 DBD, hEGR3 DBD-hCITED4 TAD-hCITED2 TAD, or hEGR3 DBD-hCITED2 TAD-hCITED4 TAD).

In certain embodiments, an eTF provided herein that upregulates SCN1A and has a high percent sequence identity to one or more human proteins comprises any one of: (i) a sequence comprising any one of SEQ ID NOs: 103-124, 128-131, 205, 207, 209, 213, 217, 219, 221, or 223; (ii) a sequence comprising any one of SEQ ID NOs: 92-98; (iii) a sequence comprising at least 80%, 85%, 86%, 87%, 88%, 89%, 90%, 91%, 92%, 93%, 94%, 95%, 96%, 97%, 98% or 99% sequence identity to any of the sequences of (i) or (ii); or (iv) a functional fragment or variant of any of the sequences of (i), (ii) or (iii). In exemplary embodiments, such eTFs are capable of upregulating SCN1A expression by at least at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control. In exemplary embodiments, such eTFs have at least at least 75%, 80%, 85%, 86%, 87%, 88%, 89%, 90%, 91%, 92%, 93%, 94%, 95%, 96%, 97%, 98% or 99% overall sequence identity to one or more human proteins. In certain embodiments, such eTFs exhibits reduced immunogenicity as compared to an eTF having a lower overall percent sequence identity to one or more human proteins. In various embodiments, a reduction in immunogenicity can be measured using an elispot assay, an immunoassay, or an in silico method.

In certain embodiments, an eTF provided herein that upregulates SCN1A and has a high percent sequence identity to one or more human proteins may additional comprise one or more amino acid sequences or domains in addition to the DBD and TAD domains, such as a nuclear localization signal or a linker, etc. In addition, a polynucleotide encoding an eTF provided herein having a high percent sequence identity to one or more human proteins may additional comprise one or more nucleic acid sequences in addition to the coding sequence for the eTF such as a promoter, enhancer, polyA tail, etc. In such embodiments, one or more of the additional amino acid sequences and/or nucleic acid sequences are preferably human sequences, derived from human sequences, or have at least 75%, 80%, 85%, 86%, 87%, 88%, 89%, 90%, 91%, 92%, 93%, 94%, 95%, 96%, 97%, 98% or 99% sequence identity to a human protein.

### Exemplary SCN1A eTFs

In certain embodiments, an eTF disclosed herein that upregulates SCN1A comprises a DNA binding domain having one or more zinc finger domains comprising a recognition helix comprising any one of SEQ ID NOs: 152-167. In certain embodiments, an eTF disclosed herein that upregulates SCN1A comprises a DNA binding domain having at least one, two, three, four, five, six, seven, eight, nine, ten, eleven or twelve zinc finger domains, wherein each zinger finger domain independently comprises a recognition helix comprising any one of SEQ ID NOs: 152-167. In certain embodiments, an eTF disclosed herein that upregulates SCN1A comprises a DNA binding domain having six zinc finger domains, wherein each zinger finger domain independently comprises a recognition helix comprising any one of SEQ ID NOs: 152-167. In certain embodiments, an eTF disclosed herein that upregulates SCN1A comprises a DNA binding domain having nine zinc finger domains, wherein each zinger finger domain independently comprises a recognition helix comprising any one of SEQ ID NOs: 152-167. In exemplary embodiments, such eTFs comprise a DNA binding domain having SEQ ID NO: 147, wherein each X is independently selected from any one of SEQ ID NOs: 152-167, and n is 6 or 9.

In certain embodiments, an eTF disclosed herein that upregulates SCN1A comprises a DNA binding domain having any one of: (i) a sequence comprising RSDNLVR x REDNLHT x RSDELVR x QSGNLTE x TSGHLVR x QNSTLTE (SEQ ID NO: 148), wherein x can be a linker of 1-50 amino acids, (ii) a sequence having at least 80%, 85%, 90%, 95%, 96%, 97%, 98%, or 99% sequence identity to SEQ ID NO: 148, or (ii) a functional fragment of (i) or (ii). In certain embodiments, such an eTF further comprises one or more TADs selected from VP64, VPR, CITED2, CITED4, or CREB3. In one embodiment, such an eTF comprises a VPR TAD domain conjugated to the C-terminus of the DBD. In certain embodiments, such an eTF comprises a CITED2 TAD conjugated to the N-terminus, the C-terminus, or the N-terminus and C-terminus of the DBD. In certain embodiments, such an eTF comprises a CITED4 TAD conjugated to the N-terminus, the C-terminus, or the N-terminus and C-terminus of the DBD. In certain embodiments, such an eTF comprises two CITED4 TADs conjugated to the N-terminus or the C-terminus of the DBD. In certain embodiments, such an eTF is capable of binding to a target site having SEQ ID NO: 18 and upregulating expression of SCN1A by at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control.

In certain embodiments, an eTF disclosed herein that upregulates SCN1A comprises a DNA binding domain having any one of: (i) a sequence comprising RSDNLVR x HRTTLTN x REDNLHT x TSHSLTE x QSSSLVR x REDNLHT (SEQ ID NO: 149), wherein x can be a linker of 1-50 amino acids, (ii) a sequence having at least 80%, 85%, 90%, 95%, 96%, 97%, 98%, or 99% sequence identity to SEQ ID NO: 149, or (ii) a functional fragment of (i) or (ii). In certain embodiments, such an eTF further comprises one or more TADs selected from VP64, VPR, CITED2, CITED4, or CREB3. In one embodiment, such an eTF comprises a VPR TAD domain conjugated to the C-terminus of the DBD. In certain embodiments, such an eTF comprises a CITED2 TAD conjugated to the N-terminus, the C-terminus, or the N-terminus and C-terminus of the DBD. In certain embodiments, such an eTF comprises a CITED4 TAD conjugated to the N-terminus, the C-terminus, or the N-terminus and C-terminus of the DBD. In certain embodiments, such an eTF comprises two CITED4 TADs conjugated to the N-terminus or the C-terminus of the DBD. In certain embodiments, such an eTF is capable of binding to a target site having SEQ ID NO: 30 and upregulating expression of SCN1A by at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control.

In certain embodiments, an eTF disclosed herein that upregulates SCN1A comprises a DNA binding domain having any one of: (i) a sequence comprising RRDELNV x RSDHLTN x RSDDLVR x RSDNLVR x HRTTLTN x REDNLHT x TSHSLTE x QSSSLVR x REDNLHT (SEQ ID NO: 151), (ii) a sequence having at least 80%, 85%, 90%, 95%, 96%, 97%, 98%, or 99% sequence identity to SEQ ID NO: 151, or (ii) a functional fragment of (i) or (ii). In certain embodiments, such an eTF further comprises one or more TADs selected from VP64, VPR, CITED2, CITED4, or CREB3. In one embodiment, such an eTF comprises a VPR TAD domain conjugated to the C-terminus of the DBD. In certain embodiments, such an eTF comprises a CITED2 TAD conjugated to the N-terminus, the C-terminus, or the N-terminus and C-terminus of the DBD. In certain embodiments, such an eTF comprises a CITED4 TAD conjugated to the N-terminus, the C-terminus, or the N-terminus and C-terminus of the DBD. In certain embodiments, such an eTF comprises two CITED4 TADs conjugated to the N-terminus or the C-terminus of the DBD. In certain embodiments, such an eTF is capable of binding to a target site having SEQ ID NO: 32 and upregulating expression of SCN1A by at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control.

In certain embodiments, an eTF disclosed herein that upregulates SCN1A comprises a DNA binding domain having any one of: (i) a sequence comprising DPGALVR x RSDNLVR x QSGDLRR x THLDLIR x TSGNLVR x RSDNLVR (SEQ ID NO: 150), (ii) a sequence having at least 89%, 85%, 90%, 95%, 96%, 97%, 98%, or 99% sequence identity to SEQ ID NO: 150, or (ii) a functional fragment of (i) or (ii). In certain embodiments, such an eTF further comprises one or more TADs selected from VP64, VPR, CITED2, CITED4, or CREB3. In one embodiment, such an eTF comprises a VPR TAD domain conjugated to the C-terminus of the DBD. In certain embodiments, such an eTF comprises a CITED2 TAD conjugated to the N-terminus, the C-terminus, or the N-terminus and C-terminus of the DBD. In certain embodiments, such an eTF comprises a CITED4 TAD conjugated to the N-terminus, the C-terminus, or the N-terminus and C-terminus of the DBD. In certain embodiments, such an eTF comprises two CITED4 TADs conjugated to the N-terminus or the C-terminus of the DBD. In certain embodiments, such an eTF is capable of binding to a target site having SEQ ID NO: 31 and upregulating expression of SCN1A by at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control.

In certain embodiments, an eTF disclosed herein that upregulates SCN1A comprises any one of: (i) a sequence comprising any one of SEQ ID NOs: 99-131, 205, 207, 209, 213, 217, 219, 221, or 223; (ii) a sequence comprising any one of SEQ ID NOs: 77-98; (iii) a sequence comprising at least 80%, 85%, 86%, 87%, 88%, 89%, 90%, 91%, 92%, 93%, 94%, 95%, 96%, 97%, 98% or 99% sequence identity to any of the sequences of (i) or (ii); or (iv) a functional fragment or variant of any of the sequences of (i), (ii) or (iii). In exemplary embodiments, such eTFs are capable of upregulating SCN1A expression by at least at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control.

In certain embodiments, an eTF disclosed herein that upregulates SCN1A comprises any one of: (i) a sequence comprising any one of SEQ ID NOs: 99-102 or 125-127; (ii) a sequence comprising any one of SEQ ID NOs: 77-91; (iii) a sequence comprising at least 80%, 85%, 86%, 87%, 88%, 89%, 90%, 91%, 92%, 93%, 94%, 95%, 96%, 97%, 98% or 99% sequence identity to any of the sequences of (i) or (ii); or (iv) a functional fragment or variant of any of the sequences of (i), (ii) or (iii). In exemplary embodiments, such eTFs are capable of upregulating SCN1A expression by at least at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control.

In certain embodiments, an eTF disclosed herein that upregulates SCN1A comprises any one of: (i) a sequence comprising any one of SEQ ID NOs: 103-124, 128-131, 205, 207, 209, 213, 217, 219, 221, or 223; (ii) a sequence comprising any one of SEQ ID NOs: 92-98; (iii) a sequence comprising at least 80%, 85%, 86%, 87%, 88%, 89%, 90%, 91%, 92%, 93%, 94%, 95%, 96%, 97%, 98% or 99% sequence identity to any of the sequences of (i) or (ii); or (iv) a functional fragment or variant of any of the sequences of (i), (ii) or (iii). In exemplary embodiments, such eTFs are capable of upregulating SCN1A expression by at least at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control. In exemplary embodiments, such eTFs have at least at least 75%, 80%, 85%, 86%, 87%, 88%, 89%, 90%, 91%, 92%, 93%, 94%, 95%, 96%, 97%, 98% or 99% overall sequence identity to one or more human proteins. In certain embodiments, such eTFs exhibits reduced immunogenicity as compared to an eTF having a lower overall percent sequence identity to one or more human proteins. In various embodiments, a reduction in immunogenicity can be measured using an elispot assay, an immunoassay, or an in silico method.

In certain embodiments, an eTF disclosed herein that upregulates SCN1A comprises any one of: (i) a sequence comprising SEQ ID NO: 127; (ii) a sequence comprising at least 80%, 85%, 86%, 87%, 88%, 89%, 90%, 91%, 92%, 93%, 94%, 95%, 96%, 97%, 98% or 99% sequence identity to SEQ ID NO: 127; or (iii) a functional fragment or variant of any of the sequences of (i) or (ii). In exemplary embodiments, such eTFs comprise SEQ ID NO: 77 and bind to a target site having SEQ ID NO: 18. In exemplary embodiments, such eTFs are capable of upregulating SCN1A expression by at least at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control.

In certain embodiments, an eTF disclosed herein that upregulates SCN1A comprises any one of: (i) a sequence comprising SEQ ID NO: 128; (ii) a sequence comprising at least 80%, 85%, 86%, 87%, 88%, 89%, 90%, 91%, 92%, 93%, 94%, 95%, 96%, 97%, 98% or 99% sequence identity to SEQ ID NO: 128; or (iii) a functional fragment or variant of any of the sequences of (i) or (ii). In exemplary embodiments, such eTFs comprise SEQ ID NO: 92 and bind to a target site having SEQ ID NO: 18. In exemplary embodiments, such eTFs are capable of upregulating SCN1A expression by at least at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control. In exemplary embodiments, such eTFs have at least at least 75%, 80%, 85%, 86%, 87%, 88%, 89%, 90%, 91%, 92%, 93%, 94%, 95%, 96%, 97%, 98% or 99% overall sequence identity to one or more human proteins. In certain embodiments, such eTFs exhibits reduced immunogenicity as compared to an eTF having a lower overall percent sequence identity to one or more human proteins. In various embodiments, a reduction in immunogenicity can be measured using an elispot assay, an immunoassay, or an in silico method.

In certain embodiments, an eTF disclosed herein that upregulates SCN1A comprises any one of: (i) a sequence comprising SEQ ID NO: 129; (ii) a sequence comprising at least 80%, 85%, 86%, 87%, 88%, 89%, 90%, 91%, 92%, 93%, 94%, 95%, 96%, 97%, 98% or 99% sequence identity to SEQ ID NO: 129; or (iii) a functional fragment or variant of any of the sequences of (i) or (ii). In exemplary embodiments, such eTFs comprise SEQ ID NO: 92 and bind to a target site having SEQ ID NO: 18. In exemplary embodiments, such eTFs are capable of upregulating SCN1A expression by at least at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control. In exemplary embodiments, such eTFs have at least at least 75%, 80%, 85%, 86%, 87%, 88%, 89%, 90%, 91%, 92%, 93%, 94%, 95%, 96%, 97%, 98% or 99% overall sequence identity to one or more human proteins. In certain embodiments, such eTFs exhibits reduced immunogenicity as compared to an eTF having a lower overall percent sequence identity to one or more human proteins. In various embodiments, a reduction in immunogenicity can be measured using an elispot assay, an immunoassay, or an in silico method.

In certain embodiments, an eTF disclosed herein that upregulates SCN1A comprises any one of: (i) a sequence comprising SEQ ID NO: 130; (ii) a sequence comprising at least 80%, 85%, 86%, 87%, 88%, 89%, 90%, 91%, 92%, 93%, 94%, 95%, 96%, 97%, 98% or 99% sequence identity to SEQ ID NO: 130; or (iii) a functional fragment or variant of any of the sequences of (i) or (ii). In exemplary embodiments, such eTFs comprise SEQ ID NO: 92 and bind to a target site having SEQ ID NO: 18. In exemplary embodiments, such eTFs are capable of upregulating SCN1A expression by at least at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control. In exemplary embodiments, such eTFs have at least at least 75%, 80%, 85%, 86%, 87%, 88%, 89%, 90%, 91%, 92%, 93%, 94%, 95%, 96%, 97%, 98% or 99% overall sequence identity to one or more human proteins. In certain embodiments, such eTFs exhibits reduced immunogenicity as compared to an eTF having a lower overall percent sequence identity to one or more human proteins. In various embodiments, a reduction in immunogenicity can be measured using an elispot assay, an immunoassay, or an in silico method.

In certain embodiments, an eTF disclosed herein that upregulates SCN1A comprises any one of: (i) a sequence comprising SEQ ID NO: 131; (ii) a sequence comprising at least 80%, 85%, 86%, 87%, 88%, 89%, 90%, 91%, 92%, 93%, 94%, 95%, 96%, 97%, 98% or 99% sequence identity to SEQ ID NO: 131; or (iii) a functional fragment or variant of any of the sequences of (i) or (ii). In exemplary embodiments, such eTFs comprise SEQ ID NO: 92 and bind to a target site having SEQ ID NO: 18. In exemplary embodiments, such eTFs are capable of upregulating SCN1A expression by at least at least 2 fold, 5 fold, 10 fold, 15 fold, 20 fold, 25 fold, 30 fold, 40 fold, 50 fold, or greater as compared to a control, or by at least 20%, 30%, 40%, 50% , 60%, 70%, 75%, 80%, 90%, 100%, 125%, 150%, 200%, 250%, 300%, 400%, or 500% or greater as compared to a control. In exemplary embodiments, such eTFs have at least at least 75%, 80%, 85%, 86%, 87%, 88%, 89%, 90%, 91%, 92%, 93%, 94%, 95%, 96%, 97%, 98% or 99% overall sequence identity to one or more human proteins. In certain embodiments, such eTFs exhibits reduced immunogenicity as compared to an eTF having a lower overall percent sequence identity to one or more human proteins. In various embodiments, a reduction in immunogenicity can be measured using an elispot assay, an immunoassay, or an in silico method.

In certain embodiments, an eTF disclosed herein that upregulates SCN1A comprises a DBD comprising a gRNA/Cas complex, wherein the gRNA comprises a targeting sequence comprising any one of SEQ ID NOs: 35-66. The target sequence of the gRNA is attached to the 5' end of a scaffold sequence having the sequence: 5'-GTTTTAGTAC TCTGGAAACAGAATCTACTAAAACAAGGCAAAATGCCGTGTTTATCT CGTCAACTTGTTGGCGAGA-3' (SEQ ID NO: 183). In exemplary embodiments, the Cas protein is a nuclease deactivated Cas9 protein. In certain embodiments, such an eTF further comprises one or more TADs conjugated to the Cas protein, wherein the TAD is selected from VP64, VPR, CITED2, CITED4, or CREB3. In one embodiment, such an eTF comprises a VPR TAD domain conjugated to the C-terminus of the Cas protein. In certain embodiments, such an eTF comprises a CITED2 TAD conjugated to the N-terminus, the C-terminus, or the N-terminus and C-terminus of the Cas protein. In certain embodiments, such an eTF comprises a CITED4 TAD conjugated to the N-terminus, the C-terminus, or the N-terminus and C-terminus of the Cas protein. In exemplary embodiments, such eTFs are capable of upregulating SCN1A expression by at least at least 2 fold, 5 fold, 10 fold, 15 fold, 20 fold, 25 fold, 30 fold, 40 fold, 50 fold, or greater as compared to a control.

**Polynucleotides**

In another aspect, the application provides polynucleotides encoding any of the eTFs that upregulate SNC1A disclosed herein. In another aspect the application provides polynucleotides comprising a PV selective microRNA binding site. In certain embodiments, the application provides polynucleotides comprising a PV selective regulatory element operably linked to a transgene and a PV selective microRNA binding site. In certain embodiments, the application provides polynucleotides comprising a sequence encoding an eTF that upregulates SCN1A as disclosed herein and a PV selective microRNA binding site. In certain embodiments, the application provides a PV selective regulatory element operably linked to a transgene encoding an eTF that upregulates SCN1A and a PV selective regulatory element.

***Polynucleotides Encoding eTFs that Upregulate SCN1A***

In certain embodiments, the application provides a polynucleotide comprising any one of the following: (i) a nucleic acid sequence encoding an eTF that upregulates SCN1A comprising any one of SEQ ID NOs: 77-131, 205, 207, 209, 213, 217, 219, 221, or 223, or a variant or a functional fragment thereof; (ii) a nucleic acid encoding a functional fragment of an eTF that upregulates SCN1A having any one of SEQ ID NOs: 77-131, 205, 207, 209, 213, 217, 219, 221, or 223; or (iii) a nucleic acid encoding an eTF that upregulates SCN1A having at least 70%, 75%, 80%, 85%, 86%, 87%, 88%, 89%, 90%, 91%, 92%, 93%, 94%, 95%, 96%, 97%, 98%, 99% or greater sequence identity to an eTF that upregulates SCN1A having any one of SEQ ID NOs: 77-131, 205, 207, 209, 213, 217, 219, 221, or 223, or a variant or a functional fragment thereof.

In certain embodiments, the application provides a polynucleotide comprising any one of the following: (i) a nucleic acid sequence encoding a DBD comprising any one of SEQ ID NOs: 92-98, or a variant or functional fragment thereof; (ii) a nucleic acid encoding a functional fragment of a DBD having any one of SEQ ID NOs: 92-98; or (iii) a nucleic acid encoding a DBD having at least 70%, 75%, 80%, 85%, 86%, 87%, 88%, 89%, 90%, 91%, 92%, 93%, 94%, 95%, 96%, 97%, 98%, 99% or greater sequence identity to a DBD having any one of SEQ ID NOs: 92-98, or a variant or functional fragment thereof, wherein the DBD is capable of binding to a target site bound by any one of SEQ ID NOs: 92-98.

In certain embodiments, the application provides a polynucleotide encoding an eTF that upregulates endogenous SCN1A, wherein the polynucleotide comprises any one of the following: (i) a nucleic acid sequence encoding an eTF comprising any one of SEQ ID NOs: 103-124, 128-131, 205, 207, 209, 213, 217, 219, 221, or 223; (ii) a nucleic acid encoding a functional fragment of an eTF having any one of SEQ ID NOs: 103-124, 128-131, 205, 207, 209, 213, 217, 219, 221, or 223; or (iii) a nucleic acid encoding an eTF having at least 70%, 75%, 80%, 85%, 86%, 87%, 88%, 89%, 90%, 91%, 92%, 93%, 94%, 95%, 96%, 97%, 98%, 99% or greater sequence identity to an eTF having any one of SEQ ID NOs: 103-124, 128-131, 205, 207, 209, 213, 217, 219, 221, or 223, wherein the eTF is capable of upregulating SCN1A.

In certain embodiments, the application provides a polynucleotide encoding a DBD that binds to a genomic target site capable of upregulating endogenous SCN1A when bound by an eTF disclosed herein, wherein the polynucleotide comprises any one of the following: (i) a nucleic acid sequence encoding a DBD comprising any one of SEQ ID NOs: 77-98; (ii) a nucleic acid encoding a functional fragment of a DBD having any one of SEQ ID NOs: 77-98; or (iii) a nucleic acid encoding an eTF having at least 70%, 75%, 80%, 85%, 86%, 87%, 88%, 89%, 90%, 91%, 92%, 93%, 94%, 95%, 96%, 97%, 98%, 99% or greater sequence identity to a DBD having any one of SEQ ID NOs: 77-98, wherein the DBD is capable of binding to a target site bound by any one of SEQ ID NOs: 77-98.

In certain embodiments, the application provides a polynucleotide encoding a DBD that binds to a genomic target site capable of upregulating endogenous SCN1A when bound by an eTF disclosed herein, wherein the polynucleotide comprises any one of the following: (i) a nucleic acid sequence encoding a DBD comprising any one of SEQ ID NOs: 148-151; (ii) a nucleic acid encoding a functional fragment of a DBD having any one of SEQ ID NOs: 148-151; or (iii) a nucleic acid encoding an eTF having at least 70%, 75%, 80%, 85%, 86%, 87%, 88%, 89%, 90%, 91%, 92%, 93%, 94%, 95%, 96%, 97%, 98%, 99% or greater sequence identity to a DBD having any one of SEQ ID NOs: 148-151, wherein the DBD is capable of binding to a target site bound by any one of SEQ ID NOs: 92-98.

In certain embodiments, the application provides a polynucleotide encoding an eTF capable of regulating endogenous SCN1A, wherein the polynucleotide comprises any one of the following: (i) a nucleic acid sequence having any of SEQ ID NOs: 70-76 or 184; (ii) a nucleic acid having a functional fragment of any one of the sequences of (i); or (iii) a nucleic acid having at least 70%, 75%, 80%, 85%, 86%, 87%, 88%, 89%, 90%, 91%, 92%, 93%, 94%, 95%, 96%, 97%, 98%, 99% or greater sequence identity to any one of the sequences of (i) or (ii), wherein the polynucleotide encodes an eTF that is capable of upregulating SCN1A.

In certain embodiments, the application provides a polynucleotide encoding an eTF capable of regulating endogenous SCN1A, wherein the polynucleotide comprises any one of the following: (i) a nucleic acid sequence having SEQ ID NO: 70; (ii) a nucleic acid having a functional fragment of SEQ ID NO: 70; or (iii) a nucleic acid having at least 70%, 75%, 80%, 85%, 86%, 87%, 88%, 89%, 90%, 91%, 92%, 93%, 94%, 95%, 96%, 97%, 98%, 99% or greater sequence identity to any one of the sequences of (i) or (ii). In exemplary embodiments, such polynucleotides encode an eTF having SEQ ID NO: 127, or a functional fragment or variant thereof that is capable of upregulating SCN1A.

In certain embodiments, the application provides a polynucleotide encoding an eTF capable of regulating endogenous SCN1A, wherein the polynucleotide comprises any one of the following: (i) a nucleic acid sequence having SEQ ID NO: 71; (ii) a nucleic acid having a functional fragment of SEQ ID NO: 71; or (iii) a nucleic acid having at least 70%, 75%, 80%, 85%, 86%, 87%, 88%, 89%, 90%, 91%, 92%, 93%, 94%, 95%, 96%, 97%, 98%, 99% or greater sequence identity to any one of the sequences of (i) or (ii). In exemplary embodiments, such polynucleotides encode an eTF having SEQ ID NO: 127, or a functional fragment or variant thereof that is capable of upregulating SCN1A.

In certain embodiments, the application provides a polynucleotide encoding an eTF capable of regulating endogenous SCN1A, wherein the polynucleotide comprises any one of the following: (i) a nucleic acid sequence having SEQ ID NO: 72; (ii) a nucleic acid having a functional fragment of SEQ ID NO: 72; or (iii) a nucleic acid having at least 70%, 75%, 80%, 85%, 86%, 87%, 88%, 89%, 90%, 91%, 92%, 93%, 94%, 95%, 96%, 97%, 98%, 99% or greater sequence identity to any one of the sequences of (i) or (ii). In exemplary embodiments, such polynucleotides encode an eTF having SEQ ID NO: 130, or a functional fragment or variant thereof that is capable of upregulating SCN1A.

In certain embodiments, the application provides a polynucleotide encoding an eTF capable of regulating endogenous SCN1A, wherein the polynucleotide comprises any one of the following: (i) a nucleic acid sequence having SEQ ID NO: 73; (ii) a nucleic acid sequence having a functional fragment of SEQ ID NO: 73; or (iii) a nucleic acid having at least 70%, 75%, 80%, 85%, 86%, 87%, 88%, 89%, 90%, 91%, 92%, 93%, 94%, 95%, 96%, 97%, 98%, 99% or greater sequence identity to any one of the sequences of (i) or (ii). In exemplary embodiments, such polynucleotides encode an eTF having SEQ ID NO: 131, or a functional fragment or variant thereof that is capable of upregulating SCN1A.

In certain embodiments, the application provides a polynucleotide encoding an eTF capable of regulating endogenous SCN1A, wherein the polynucleotide comprises any one of the following: (i) a nucleic acid sequence having SEQ ID NO: 74; (ii) a nucleic acid sequence having a functional fragment of SEQ ID NO: 74; or (iii) a nucleic acid having at least 70%, 75%, 80%, 85%, 86%, 87%, 88%, 89%, 90%, 91%, 92%, 93%, 94%, 95%, 96%, 97%, 98%, 99% or greater sequence identity to any one of the sequences of (i) or (ii). In exemplary embodiments, such polynucleotides encode an eTF having SEQ ID NO: 127, or a functional fragment or variant thereof that is capable of upregulating SCN1A.

In certain embodiments, the application provides a polynucleotide encoding an eTF capable of regulating endogenous SCN1A, wherein the polynucleotide comprises any one of the following: (i) a nucleic acid sequence having SEQ ID NO: 75; (ii) a nucleic acid sequence having a functional fragment of SEQ ID NO: 75; or (iii) a nucleic acid having at least 70%, 75%, 80%, 85%, 86%, 87%, 88%, 89%, 90%, 91%, 92%, 93%, 94%, 95%, 96%, 97%, 98%, 99% or greater sequence identity to any one of the sequences of (i) or (ii). In exemplary embodiments, such polynucleotides encode an eTF having SEQ ID NO: 127, or a functional fragment or variant thereof that is capable of upregulating SCN1A.

In certain embodiments, the application provides a polynucleotide encoding an eTF capable of regulating endogenous SCN1A, wherein the polynucleotide comprises any one of the following: (i) a nucleic acid sequence having SEQ ID NO: 76; (ii) a nucleic acid sequence having a functional fragment of SEQ ID NO: 76; or (iii) a nucleic acid having at least 70%, 75%, 80%, 85%, 86%, 87%, 88%, 89%, 90%, 91%, 92%, 93%, 94%, 95%, 96%, 97%, 98%, 99% or greater sequence identity to any one of the sequences of (i) or (ii). In exemplary embodiments, such polynucleotides encode an eTF having SEQ ID NO: 106, or a functional fragment or variant thereof that is capable of upregulating SCN1A.

In certain embodiments, the application provides a polynucleotide encoding an eTF capable of regulating endogenous SCN1A, wherein the polynucleotide comprises any one of the following: (i) a nucleic acid sequence having SEQ ID NO: 184; (ii) a nucleic acid sequence having a functional fragment of SEQ ID NO: 184; or (iii) a nucleic acid having at least 70%, 75%, 80%, 85%, 86%, 87%, 88%, 89%, 90%, 91%, 92%, 93%, 94%, 95%, 96%, 97%, 98%, 99% or greater sequence identity to any one of the sequences of (i) or (ii). In exemplary embodiments, such polynucleotides encode an eTF having SEQ ID NO: 106, or a functional fragment or variant thereof that is capable of upregulating SCN1A.

## Polynucleotides Comprising MicroRNA Binding Sites for Selective Expression in PV Neurons

In another aspect, the application provides polynucleotides comprising microRNA binding sites that lead to selective expression of a gene of interest in parvalbumin (PV) neurons. MicroRNAs or miRNAs are small non-coding RNAs (~20 nucleotides) that regulate gene expression post-transcriptionally by hybridizing to complementary recognition sites within an mRNA molecule and lead to inhibition of gene expression by promoting degradation of the mRNA transcript or by repressing translation of the protein encoded by the mRNA. The microRNA binding sites provided herein inhibit expression of a gene of interest in excitatory neurons thereby promoting selective expression of a gene of interest in PV neurons (e.g., PV selective microRNA binding sites). In certain embodiments, excitatory neurons are neurons that express one or more of STAC, Slc17a7, Car12, Syt17, ITPKA, Col6a1, CamKII, Sv2b, INHBA, and/or DKK3. In an exemplary embodiment, excitatory neurons are neurons that express CamKII.

In certain embodiments, the application provides polynucleotides comprising one or more microRNA binding sites for one or more microRNAs that promote PV selective expression, e.g., promote degradation of an mRNA comprising the microRNA binding site in excitatory neurons. Exemplary microRNAs that promote PV selective expression include, for example, miR-128, miR-221 and miR-222. In certain embodiments, the application provides polynucleotides comprising at least 1, 2, 3, 4, 5, 6, 7, 8, 9, 10 or more PV selective microRNA binding sites. In one embodiment, the application provides polynucleotides comprising at least 1, 2, 3, 4, 5, 6, 7, 8, 9, 10 or more miR-128 binding sites (SEQ ID NO: 9). In one embodiment, the application provides polynucleotides comprising at least 1, 2, 3, 4, 5, 6, 7, 8, 9, 10 or more miR-221 binding sites (SEQ ID NO: 11). In one embodiment, the application provides polynucleotides comprising at least 1, 2, 3, 4, 5, 6, 7, 8, 9, 10 or more miR-222 binding sites (SEQ ID NO: 13). In one embodiment, the application provides polynucleotides comprising at least 1 miR-128 binding site, at least one miR-221 binding site, and at least one miR-222 binding site. In one embodiment, the application provides polynucleotides comprising at least one miR-128 binding site and at least one miR-222 binding site. In one embodiment, the application provides polynucleotides comprising at least one miR-221 binding site and at least one miR-222 binding site. In an exemplary embodiment, the application provides polynucleotides comprising at least one miR-128 binding site (SEQ ID NO: 9) and at least one miR-221 binding site (SEQ ID NO: 11). In one embodiment, the application provides polynucleotides comprising at least 2 miR-128 binding sites (SEQ ID NO: 9) and at least 2 miR-221 binding sites (SEQ ID NO: 11). In one embodiment, the application provides polynucleotides comprising at least 3 miR-128 binding sites (SEQ ID NO: 9) and at least 3 miR-221 binding sites (SEQ ID NO: 11). In one embodiment, the application provides polynucleotides comprising at least 4 miR-128 binding sites (SEQ ID NO: 9) and at least 4 miR-221 binding sites (SEQ ID NO: 11). In one embodiment, the application provides polynucleotides comprising at least 5 miR-128 binding sites (SEQ ID NO: 9) and at least 5 miR-221 binding sites (SEQ ID NO: 11). In such embodiments, the binding sites may be arranged in any order. For example, for a construct containing 2 miR-128 binding sites and 2 miR-221 binding sites, the binding sites may be arranged in any of the following configurations: miR-128 - miR-128 - miR-221 - miR221, miR-128 - miR-221 - miR-128 - miR-221, miR-128 - miR221 - miR221 - miR-128, miR-221 - miR128 - miR-221 - miR128, miR-221 - miR128 - miR-128 - miR221, or miR221 - miR221- miR128 - miR128. In an exemplary embodiment, the polynucleotides provided herein comprise a sequence having 4 miR-128 binding sites (SEQ ID NO: 9) followed by four miR-221 binding sites (SEQ ID NO: 11), e.g., miR-128 - miR-128 - miR128 - miR-128 - miR221 - miR-221 - miR-221 - miR221. In another exemplary embodiment, the polynucleotides provided herein comprise a sequence having 1 miR-221 sequence (SEQ ID NO: 11), 1 miR-222 sequence (SEQ ID NO: 13) and 1 miR-128 binding site (SEQ ID NO: 9), e.g., miR-221 - miR222 - miR128. In another exemplary embodiment, the polynucleotides provided herein comprise a sequence having 2 miR-221 sequences (SEQ ID NO: 11), 2 miR-222 sequences (SEQ ID NO: 13) and 2 miR-128 binding site (SEQ ID NO: 9) arranged in the following order: miR-221 - miR222 - miR128 - miR-221 - miR222 - miR128.

In polynucleotides having more than one microRNA binding site, the binding sites may be directly adjacent to one another in the polynucleotide sequence (e.g., no linker or intervening sequence between the binding sites) or may be separated from one another by at least 1, 2, 3, 4, 5, 6, 7, 8, 9, 10, 11, 12, 13, 14, 15, 16, 17, 18, 19, 20 or more nucleotides, or from 1-20, 1-15, 1-10, 1-9, 1-8, 1-7, 1-6, 1-5, 1-4, 1-3, or 1-2 nucleotides. In exemplary embodiments, the microRNA binding sites are separated by about 5 nucleotides or by 5 nucleotides. In exemplary embodiments, the sequences separating the microRNA binding sites (as well as the junctions formed between microRNA binding sites, or junctions formed between microRNA binding sites and the sequences separating the microRNA binding sites) are not complementary to any other microRNAs, or any other neuronal microRNAs.

In certain embodiments, the polynucleotides provided herein comprise a microRNA binding site having at least 70%, 75%, 80%, 85%, 90%, 91%, 92%, 93%, 94%, 95%, 96%, 97%, 98%, 99% or 100% identity to SEQ ID NO: 7. In an exemplary embodiment, the polynucleotides provided herein comprise a microRNA binding site comprising SEQ ID NO: 7.

In certain embodiments, the polynucleotides provided herein comprise a microRNA binding site having at least 70%, 75%, 80%, 85%, 90%, 91%, 92%, 93%, 94%, 95%, 96%, 97%, 98%, 99% or 100% identity to SEQ ID NO: 14. In an exemplary embodiment, the polynucleotides provided herein comprise a microRNA binding site comprising SEQ ID NO: 14.

In certain embodiments, the polynucleotides provided herein comprise a microRNA binding site having at least 70%, 75%, 80%, 85%, 90%, 91%, 92%, 93%, 94%, 95%, 96%, 97%, 98%, 99% or 100% identity to SEQ ID NO: 15. In an exemplary embodiment, the polynucleotides provided herein comprise a microRNA binding site comprising SEQ ID NO: 15.

In certain embodiments, the microRNA binding sites provided herein are located within the 3' untranslated region of an mRNA transcript, e.g., following the translation termination codon (i.e., TAA, TGA or TAG) and before the polyA tail. The microRNA binding site may be located directly adjacent to the translation termination codon or may be separated from the translation termination codon by at least 1, 2, 3, 4, 5, 6, 7, 8, 9, 10, 11, 12, 13, 14, 15, 16, 17, 18, 19, 20 or more nucleotides, or from 1-20, 1-15, 1-10, 1-9, 1-8, 1-7, 1-6, 1-5, 1-4, 1-3, or 1-2 nucleotides and/or may be located adjacent to the polyA tail or may be separated from the polyA tail by at least 1, 2, 3, 4, 5, 6, 7, 8, 9, 10, 11, 12, 13, 14, 15, 16, 17, 18, 19, 20 or more nucleotides, or from 1-20, 1-15, 1-10, 1-9, 1-8, 1-7, 1-6, 1-5, 1-4, 1-3, or 1-2 nucleotides.

In certain embodiments, a microRNA binding site provided herein results in selective gene expression in a PV cell as compared to off target cell types. In some cases, off target cell types include, but are not limited to, excitatory neurons, non-PV CNS cell-types, and non-neuronal CNS cell types. In certain embodiments, PV selective microRNA binding sites result in selective gene expression in PV neurons over at least one, two, three, four, five, or more non-PV CNS cell types. In some instances, a non-PV CNS cell is an excitatory neuron, a dopaminergic neuron, an astrocyte, a microglia, a motor neuron, a vascular cell, or a non-GABAergic neuron (e.g., a cell that does not express one or more of GAD2, GAD1, NKX2.1, DLX1, DLX5, SST and VIP), a non-PV neuron (e.g., a GABAergic neuron that does not express parvalbumin), or other CNS cells (e.g., CNS cell types that have never expressed any of PV, GAD2, GAD1, NKX2.1, DLX1, DLX5, SST and VIP). In an exemplary embodiment, a PV selective microRNA binding site provided herein result in increased selectivity in gene expression in PV neurons as compared to excitatory neurons (e.g., neurons that express one or more of STAC, Slc17a7, Car12, Syt17, ITPKA, Col6a1, CamKII, Sv2b, INHBA, and/or DKK3) by decreasing expression in the excitatory neurons. In some cases, cell types are distinguished by having a different cell marker, morphology, phenotype, genotype, function, and/or any other means for classifying cell types.

Selectivity of expression driven by a PV selective microRNA binding site can be measured in a number of ways. In one embodiment, selectivity of gene expression in a PV cell over non-PV cells can be measured by comparing the number of PV cells that express a detectable level of a transcript from a gene that contains a PV selective microRNA binding site to the total number of cells that express the gene (e.g., the ratio of PV vs. total cells (PV + non-PV cells) expressing the gene). For example, selectivity for PV neurons can be determined using an immunohistochemistry based colocalization assay using an expression cassette comprising a gene encoding a fluorescent protein (e.g., eGFP) and a PV selective microRNA binding site to measure gene expression and an antibody that identifies PV cells (e.g., an anti-PV antibody that interacts specifically with PV neurons) linked to a second fluorescence label (e.g., red fluorescent protein). Selectivity of expression in PV cells can be calculated by dividing the number of cells that express both PV and eGFP (e.g., PV cells) by the total number of cells that express eGFP (e.g., PV cells and non-PV cells), and multiplying by 100 to convert into a percentage. In another example, selectivity for PV neurons can be determined using an immunohistochemistry based colocalization assay using an expression cassette comprising a gene encoding a fluorescent protein (e.g., eGFP) and a PV selective microRNA binding site to measure gene expression and a first antibody that identifies PV cells (e.g., an anti-PV antibody that interacts specifically with PV neurons) linked to a second fluorescence label (e.g., red fluorescent protein) and a second antibody that identifies excitatory cells (e.g., an anti-CamKII antibody that interacts specifically with excitatory neurons). Selectivity of expression in PV cells can be calculated by dividing the number of cells that express both PV and eGFP (e.g., PV cells) by the number of cells that express eGFP + PV and eGFP + CamKII (e.g., PV cells and excitatory cells), and multiplying by 100 to convert into a percentage. The higher the percentage of PV cells that express the transgene, the more selective the microRNA binding site is for the PV cells. In certain embodiments, a PV selective microRNA binding site provided herein can be highly selective for expression in PV cells. For example, a PV selective microRNA binding site provided herein can exhibit about 50%, 55%, 60%, 65%, 70%, 75%, 80%, 85%, 90%, 91%, 92%, 93%, 94%, 95%, 96%, 97%, 98%, 99%, or greater than about 99% selectivity for PV neurons (e.g., PV neurons/total cells x 100 or PV neurons/PV + excitatory neurons x 100).

In some cases, a PV selective microRNA binding site provided herein is short. In some cases, the size of the PV selective microRNA binding site is compatible with the cloning capacity of a vector, e.g., a viral vector or rAAV, such that the combined size of a transgene, a promoter (and optional enhancer) and microRNA binding site does not exceed the cloning capacity of a vector. In some cases, a PV selective microRNA binding site has a length of up to about 500bp, 400bp, 300bp, 250 bp, 225 bp, 215 bp, 210 bp, 200bp, 150 bp, 140 bp, 135 bp, 130 bp, 125 bp, 120 bp, 115 bp, 110 bp, 100bp, 90 bp, 80 bp, 75 bp, 70 bp, 65 bp, 60 bp or 50 bp. In some cases, a PV selective microRNA binding site is between about 50-500 bp, 50-400 bp, 50-300 bp, 50-250 bp, 50-200 bp, 50-100 bp, 50-75 bp, 50-70 bp, 100-500 bp, 100-400 bp, 100-300 bp, 100-250 bp, 100-200 bp, 100-150 bp, 100-140 bp, 100-135 bp, 200-500 bp, 200-400 bp, 200-300 bp, or 200-250 bp.

In exemplary embodiments, a polynucleotide provided herein that comprises one or more PV selective microRNA binding sites does not comprise SEQ ID NO: 67.

Expression Cassettes

In another aspect, the application provides expression cassettes comprising a polynucleotide provided herein (e.g., a polynucleotide comprising a sequence encoding an eTF that upregulates SCN1A and/or contains a PV selective microRNA binding site) and one or more regulatory elements. In certain embodiments, the application provides expression cassettes comprising a polynucleotide provided herein (e.g., a polynucleotide comprising a sequence encoding an eTF that upregulates SCN1A and/or contains a PV selective microRNA binding site) and a PV selective promoter.

In certain embodiments, a polynucleotide provided herein (e.g., a polynucleotide comprising a sequence encoding an eTF that upregulates SCN1A and/or contains a PV selective microRNA binding site) is part of an expression cassette comprising one or more regulatory elements in addition to the sequence encoding the eTF. In exemplary embodiments, a polynucleotide provided herein (e.g., a polynucleotide comprising a sequence encoding an eTF that upregulates SCN1A and/or contains a PV selective microRNA binding site) is part of an expression cassette comprising a promoter situated upstream of the transgene sequence so as to be capable of driving expression of the transgene (e.g., an eTF that selectively upregulates SCN1A) in a cell.

In certain embodiments, an expression cassette disclosed herein comprises a polynucleotide provided herein (e.g., a polynucleotide comprising a sequence encoding an eTF that upregulates SCN1A and/or contains a PV selective microRNA binding site) and a constitutive promoter situated upstream of the sequence encoding the transgene so as to be capable of driving expression of the transgene (e.g., an eTF that selectively upregulates SCN1A) in a cell. Examples of constitutive promoters include, a GAD2 promoter, a human synapsin promoter, CBA promoter, a CMV promoter, a minCMV promoter, a TATA box, a super core promoter, or an EF1$\alpha$ promoter, or a combination thereof.

In certain embodiments, an expression cassette disclosed herein comprises a polynucleotide provided herein (e.g., a polynucleotide comprising a sequence encoding an eTF that upregulates SCN1A and/or contains a PV selective microRNA binding site) and a short promoter capable of driving expression of the transgene (e.g., an eTF that selectively upregulates SCN1A) in a cell. In certain embodiments, a short promoter suitable for use in accordance with the nucleic acid molecules described herein comprises less than 500 bp, 450 bp, 400 bp, 350 bp, 300 bp, 250 bp, 225 bp, 200 bp, 175 bp, 150 bp, 145 bp, 140 bp, 135 bp, 130 bp, 125 bp, 120 bp, 115 bp, 110 bp, 105 bp, 100 bp, 95 bp, 90 bp, 85 bp, 80 bp or 75 bp, or from about 80-300 bp, 80-275 bp, 80-250 bp, 80-200 bp, 80-150 bp, 80-125 bp, 80-120 bp, 80-115 bp, 80-110 bp, 80-105 bp, 80-100 bp, 85-300 bp, 85-275 bp, 85-250 bp, 85-200 bp, 85-150 bp, 85-125 bp, 85-120 bp, 85-115 bp, 85-110 bp, 85-105 bp, 85-100 bp, 90-300 bp, 90-275 bp, 90-250 bp, 90-200 bp, 90-150 bp, 90-125 bp, 90-120 bp, 90-115 bp, 90-110 bp, 90-105 bp, 90-100 bp, 95-300 bp, 95-275 bp, 95-250 bp, 95-200 bp, 95-150 bp, 95-125 bp, 95-120 bp, 95-115 bp, 95-110 bp, 95-105 bp, 95-100 bp, 100-300 bp, 100-275 bp, 100-250 bp, 100-200 bp, 100-150 bp, 100-125 bp, 100-120 bp, 100-115 bp, 100-110 bp, or 100-105 bp. In exemplary embodiments, a short promoter suitable for use in accordance with the expression cassettes described herein comprises from about 100-120 bp, about 117 bp, or about 100 bp.

In certain embodiments, an expression cassette disclosed herein comprises a short promoter comprising or consisting of any one of (i) SEQ ID NO: 1; (ii) a variant or functional fragment thereof; or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii) operably linked to a polynucleotide encoding any one of the eTFs that selectively upregulate SCN1A as disclosed herein, and optionally containing a microRNA binding site as disclosed herein. Other examples of short promoter sequence may be found in PCT Publication No. WO 2018/213786.

In certain embodiments, an expression cassette disclosed herein comprises a polynucleotide provided herein (e.g., a polynucleotide comprising a sequence encoding an eTF that upregulates SCN1A and/or contains a PV selective microRNA binding site) and a cell type selective promoter situated upstream of the sequence encoding the transgene (e.g., an eTF that selectively upregulates SCN1A) so as to be capable of driving expression of the transgene selectively in a cell of interest. In certain embodiments, a cell type selective promoter may be selective for (e.g., selectively drive expression in) any cell type of interest, such as, for example, a heart cell, liver cell, muscle cell, bone cell, neuron, or sub populations thereof. In an exemplary embodiment, an expression cassette disclosed herein comprises a polynucleotide encoding an eTF that selectively upregulates SCN1A and a PV selective regulatory element (e.g., a promoter, enhancer, and/or promoter and enhancer) situated upstream of the sequence encoding the eTF so as to be capable of driving expression of the eTF selectively in a PV cell, and optionally a PV selective microRNA binding site. A PV selective regulatory element refers to a regulatory element that specifically modulates gene expression in a PV neuron. In certain embodiments, PV selective regulatory elements enhance expression in a PV neuron relative to one or more other CNS cell types. In certain embodiments, a PV selective regulatory element suppresses transcription and/or translation processes in off target cell-types.

In certain embodiments, a PV selective regulatory element provided herein results in selective gene expression in a PV cell as compared to off target cell types. In some cases, off target cell types include, but are not limited to, excitatory neurons, non-PV CNS cell-types, and non-neuronal CNS cell types. In certain embodiments, PV selective regulatory elements result in selective gene expression in PV neurons over at least one, two, three, four, five, or more non-PV CNS cell types. In some instances, a non-PV CNS cell is an excitatory neuron, a dopaminergic neuron, an astrocyte, a microglia, a motor neuron, a vascular cell, or a non-GABAergic neuron (e.g., a cell that does not express one or more of GAD2, GAD1, NKX2.1, DLX1, DLX5, SST and VIP), a non-PV neuron (e.g., a GABAergic neuron that does not express parvalbumin), or other CNS cells (e.g., CNS cell types that have never expressed any of PV, GAD2, GAD1, NKX2.1, DLX1, DLX5, SST and VIP). In some cases, a PV selective regulatory element provided herein result in increased selectivity in gene expression in PV neurons as compared to non-PV GABAergic cells. In some cases, cell types are distinguished by having a different cell marker, morphology, phenotype, genotype, function, and/or any other means for classifying cell types.

Selectivity of expression driven by a PV selective regulatory element can be measured in a number of ways. In one embodiment, selectivity of gene expression in a PV cell over non-PV cells can be measured by comparing the number of PV cells that express a detectable level of a transcript from a gene that is operably linked to a PV selective regulatory element to the total number of cells that express the gene (e.g., the ratio of PV vs. total cells (PV + non-PV cells) expressing the gene). For example, selectivity for PV neurons can be determined using an immunohistochemistry based colocalization assay using a gene encoding a fluorescent protein (e.g., eGFP) operably linked to a PV selective regulatory element to measure gene expression and an antibody that identifies PV cells (e.g., an anti-PV antibody that interacts specifically with PV neurons) linked to a second fluorescence label (e.g., red fluorescent protein). Selectivity of expression in PV cells can be calculated by dividing the number of cells that express both PV and eGFP (e.g., PV cells) by the total number of cells that express eGFP (e.g., PV cells and non-PV cells), and multiplying by 100 to convert into a percentage. The higher the percentage of PV cells that express the transgene, the more selective the regulatory element is for the PV cells. In certain embodiments, a PV selective regulatory element provided herein can be highly selective for expression in PV cells. For example, a PV selective regulatory element provided herein can exhibit about 50%, 55%, 60%, 65%, 70%, 75%, 80%, 85%, 90%, 91%, 92%, 93%, 94%, 95%, 96%, 97%, 98%, 99%, or greater than about 99% selectivity for PV neurons (e.g., PV neurons/total cells x 100).

In some cases, a PV selective regulatory element provided herein is short. In some cases, the size of the PV selective regulatory element is compatible with the cloning capacity of a vector, e.g., a viral vector or rAAV, such that the combined size of a transgene and one or more PV selective regulatory elements does not exceed the cloning capacity of a vector. In some cases, a PV selective regulatory element has a length of up to about 2050bp, 2000bp, 1900bp, 1800bp, 1700bp, 1600bp, 1500bp, 1400bp, 1300bp, 1200bp, 1100bp, 1000bp, 900bp, 800bp, 700bp, 600bp, 500bp, 400bp, 300bp, 200bp, or 100bp. In some cases, a PV selective regulatory element is between about 500-600bp, 500-700bp, 500-800bp, 500-900bp, 500-1000bp, 500-1500bp, 500-2000bp, or 500-2050bp.

In certain embodiments, a PV selective regulatory element provided herein comprises or consists of any one of (i) SEQ ID NOs: 2-4; (ii) a variant, functional fragment, or a combination thereof; or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In some cases, a regulatory element comprises any one of SEQ ID NOs: 2-4. Other examples of PV selective regulatory elements may be found in PCT Publication No. WO 2018/187363.

In exemplary embodiments, the application provides expression cassettes comprising a nucleic acid sequence encoding an eTF that selectively upregulates SCN1A under the control of a PV selective regulatory element. In certain embodiments, the application provides expression cassettes comprising a nucleic acid sequence encoding an eTF that selectively upregulates SCN1A comprising a DBD having any one of the following sequences: SEQ ID NOs: 77-98 under the control of a PV selective regulatory element having any one of SEQ ID NOs: 2-4. In certain embodiments, the application provides expression cassettes comprising a nucleic acid sequence encoding an eTF that selectively upregulates SCN1A comprising any one of the following sequences: SEQ ID NOs: 99-131, 205, 207, 209, 213, 217, 219, 221, or 223 under the control of a PV selective regulatory element having any one of SEQ ID NOs: 2-4. In certain embodiments, the application provides expression cassettes comprising a nucleic acid sequence comprising any one of the following sequences: SEQ ID NOs: 67-73 under the control of a PV selective regulatory element having any one of SEQ ID NOs: 2-4. In certain embodiments, the application provides expression cassettes comprising a nucleic acid sequence encoding an eTF that selectively upregulates SCN1A comprising a DBD having any one of the following sequences: SEQ ID NOs: 148-151 under the control of a PV selective regulatory element having any one of SEQ ID NO: 2. In certain embodiments, the application provides expression cassettes comprising a nucleic acid sequence encoding an eTF that selectively upregulates SCN1A comprising any one of the following sequences: SEQ ID NOs: 99-131, 205, 207, 209, 213, 217, 219, 221, or 223 under the control of a PV selective regulatory element having any one of SEQ ID NO: 2. In certain embodiments, the application provides expression cassettes comprising a nucleic acid sequence comprising any one of the following sequences: SEQ ID NOs: 67-76 or 316 under the control of a PV selective regulatory element having any one of SEQ ID NO: 2.

In certain embodiments, the application provides expression cassettes comprising a PV selective miroRNA binding site and a promoter and/or enhancer sequence. Any of the promoters described herein may be included in the expression cassette. In an exemplary embodiment, an expression cassette provided herein comprises a PV selective microRNA binding site and a PV selective regulatory element. In certain embodiments, an expression cassette provided herein comprises a PV selective microRNA binding site and a PV selective regulatory element comprising (i) any one of SEQ ID NOs: 2-4; (ii) a variant, functional fragment, or a combination thereof; or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In certain embodiments, an expression cassette provided herein comprises (1) a PV selective microRNA binding site comprising (i) any one of SEQ ID NOs: 7, 14 or 15; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (2) a PV selective regulatory element comprising (i) any one of SEQ ID NOs: 2-4; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In certain embodiments, an expression cassette provided herein comprises (1) a PV selective microRNA binding site comprising (i) SEQ ID NO: 7; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (2) a PV selective regulatory element comprising (i) SEQ ID NO: 2; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In one embodiment, an expression cassette provided herein comprises a microRNA binding site comprising SEQ ID NO: 7 and a PV selective regulatory element comprising SEQ ID NO: 2. In certain embodiments, a polynucleotide provided herein comprises (1) a PV selective microRNA binding site comprising (i) SEQ ID NO: 14; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (2) a PV selective regulatory element comprising (i) SEQ ID NO: 2; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In one embodiment, an expression cassette provided herein comprises a microRNA binding site comprising SEQ ID NO: 15 and a PV selective regulatory element comprising SEQ ID NO: 2. In certain embodiments, an expression cassette provided herein comprises (1) a PV selective microRNA binding site comprising (i) SEQ ID NO: 15; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (2) a PV selective regulatory element comprising (i) SEQ ID NO: 2; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In one embodiment, an expression cassette provided herein comprises a microRNA binding site comprising SEQ ID NO: 15 and a PV selective regulatory element comprising SEQ ID NO: 2.

In certain embodiments, an expression cassette provided herein comprising a PV selective microRNA binding site and a sequence encoding an eTF that upregulates expression of SCN1A as provided herein. In an exemplary embodiment, the application provides an expression cassette comprising a PV selective regulatory element, an eTF that upregulates expression of SCN1A as provided herein, and a PV selective miroRNA binding site. In an exemplary embodiment, the application provides an expression cassette comprising (1) a PV selective regulatory element comprising (i) any one of SEQ ID NOs: 2-4; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), (2) a sequence encoding an eTF that upregulates SCN1A comprising (i) any one of SEQ ID NOs: 77-131, 205, 207, 209, 213, 217, 219, 221, or 223; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (3) a PV selective microRNA binding site comprising (i) any one of SEQ ID NOs: 7, 14 or 15; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In another exemplary embodiment, the application provides an expression cassette comprising (1) a PV selective regulatory element comprising (i) SEQ ID NO: 2; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), (2) a sequence encoding an eTF that upregulates SCN1A comprising (i) any one of SEQ ID NOs: 77 or 127; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (3) a PV selective microRNA binding site comprising (i) SEQ ID NO: 7; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In another exemplary embodiment, the application provides an expression cassette comprising (1) a PV selective regulatory element comprising (i) SEQ ID NO: 2; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), (2) a sequence encoding an eTF that upregulates SCN1A comprising (i) any one of SEQ ID NOs: 77 or 127; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (3) a PV selective microRNA binding site comprising (i) SEQ ID NO: 14; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In another exemplary embodiment, the application provides an expression cassette comprising (1) a PV selective regulatory element comprising (i) SEQ ID NO: 2; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), (2) a sequence encoding an eTF that upregulates SCN1A comprising (i) any one of SEQ ID NOs: 77 or 127; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (3) a PV selective microRNA binding site comprising (i) SEQ ID NO: 15; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In another exemplary embodiment, the application provides an expression cassette comprising (1) a PV selective regulatory element comprising (i) SEQ ID NO: 2; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), (2) a sequence encoding an eTF that upregulates SCN1A comprising (i) any one of SEQ ID NOs: 92 or 106; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (3) a PV selective microRNA binding site comprising (i) SEQ ID NO: 7; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In another exemplary embodiment, the application provides an expression cassette comprising (1) a PV selective regulatory element comprising (i) SEQ ID NO: 2; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), (2) a sequence encoding an eTF that upregulates SCN1A comprising (i) any one of SEQ ID NOs: 92 or 106; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (3) a PV selective microRNA binding site comprising (i) SEQ ID NO: 14; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii). In another exemplary embodiment, the application provides an expression cassette comprising (1) a PV selective regulatory element comprising (i) SEQ ID NO: 2; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), (2) a sequence encoding an eTF that upregulates SCN1A comprising (i) any one of SEQ ID NOs: 92 or 106; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii), and (3) a PV selective microRNA binding site comprising (i) SEQ ID NO: 15; (ii) a variant, functional fragment, or a combination thereof of (i); or (iii) a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity to any one of (i) or (ii).

In certain embodiments, an expression cassette provided herein comprising a PV selective regulatory element and a PV selective microRNA binding site is less than 5 kb, 4.9 kb, 4.8 kb, 4.7 kb, 4.6 kb, 4.5 kb, 4.4 kb, 4.3 kb, 4.2 kb, 4.1 kb, 4.0 kb, 3.9 kb, 3.8 kb, 3.7 kb, 3.6 kb, 3.5 kb, 3.4 kb, 3.3 kb, 3.2 kb, 3.1 kb, 3.0 kb, 2.9 kb, 2.8 kb, 2.7 kb, 2.6 kb, 2.5 kb, 2.4 kb, 2.3 kb, 2.2 kb, 2.1 kb, 2.0 kb, 1.9 kb, 1.8 kb, 1.7 kb, 1.6 kb, or 1.5 kb or less, or is from about 1.5-5 kb, 1.5-4.7 kb, 1.5-4.5 kb, 1.5-4.0 kb, 1.5-3.5 kb, 1.5-3.0 kb, 1.5-2.5 kb, 1.5-2.0 kb in size.

In certain embodiments, an expression cassette provided herein may comprise one more additional regulatory elements in an addition to a promoter, such as, for example, sequences associated with transcription initiation or termination, enhancer sequences, and efficient RNA processing signals. Exemplary regulatory elements include, for example, an intron, an enhancer, UTR, stability element, WPRE sequence, a Kozak consensus sequence, posttranslational response element, a microRNA binding site, or a polyadenylation (polyA) sequence, or a combination thereof. Regulatory elements can function to modulate gene expression at the transcriptional phase, post-transcriptional phase, or at the translational phase of gene expression. At the RNA level, regulation can occur at the level of translation (e.g., stability elements that stabilize mRNA for translation), RNA cleavage, RNA splicing, and/or transcriptional termination. In various embodiments, regulatory elements can recruit transcription factors to a coding region that increase gene expression selectivity in a cell type of interest, increases the rate at which RNA transcripts are produced, increase the stability of RNA produced, and/or increase the rate of protein synthesis from RNA transcripts. In an exemplary embodiment, an expression cassette provided herein comprises at least one PV selective microRNA binding site as provided herein.

In certain embodiments, the expression cassettes described herein further comprise a polyA sequence. Suitable polyA sequences include, for example, an artificial polyA that is about 75 bp in length (PA75) (see e.g., WO 2018/126116), the bovine growth hormone polyA, SV40 early polyA signal, SV40 late polyA signal, rabbit beta globin polyA, HSV thymidine kinase polyA, protamine gene polyA, adenovirus 5 EIb polyA, growth hormone polyA, or a PBGD polyA. In exemplary embodiments, a polyA sequence suitable for use in the expression cassettes provided herein is an hGH polyA (SEQ ID NO: 17) or a synthetic polyA (SEQ ID NO: 16). Typically, the polyA sequence is positioned downstream of the polynucleotide encoding the eTF in the expression cassettes described herein.

In certain embodiments, the expression cassettes provided herein further comprise one or more nucleic acid sequences encoding one or more nuclear localization signals (NLS). Any NLS peptide that facilitates import of the protein to which is attached into the cell nucleus may be used. Examples of NLS include, for example, the SV40 large T-antigen NLS, the nucleoplasmin NLS, EGL-13 NLS, c-Myc NLS and TUS-protein NLS. See e.g., C. Dingwall et al., J. Cell Biol. 107: 841-9 (1988); J.P. Makkerh et al., Curr Biol. 6: 1025-7 (1996); and M. Ray et al., Bioconjug. Chem. 26: 1004-7 (2015). The NLS may be located anywhere on the eTF protein sequence, but in preferred embodiments is conjugated to the N-terminus of the eTF or a domain of the eTF. In exemplary embodiments, the nucleic acid cassettes provided herein encode an eTF with an NLS fused to the N-terminus of the eTF. In other embodiments, the nucleic acid cassettes provided herein encode an eTF with a first NLS fused to the N-terminus of the eTF and a second NLS located between the DBD and the TAD domain of the eTF.

## Expression Vectors

In certain embodiments, the expression cassettes described herein may be incorporated into an expression vector. Expression vectors may be used to deliver an expression cassette to a target cell via transfection or transduction. A vector may be an integrating or non-integrating vector, referring to the ability of the vector to integrate the expression cassette or transgene into the genome of the host cell. Examples of expression vectors include, but are not limited to, (a) non-viral vectors such as nucleic acid vectors including linear oligonucleotides and circular plasmids; artificial chromosomes such as human artificial chromosomes (HACs), yeast artificial chromosomes (YACs), and bacterial artificial chromosomes (BACs or PACs)); episomal vectors; transposons (e.g., PiggyBac); and (b) viral vectors such as retroviral vectors, lentiviral vectors, adenoviral vectors, and adeno-associated viral vectors.

Expression vectors may be linear oligonucleotides or circular plasmids and can be delivered to a cell via various transfection methods, including physical and chemical methods. Physical methods generally refer to methods of delivery employing a physical force to counteract the cell membrane barrier in facilitating intracellular delivery of genetic material. Examples of physical methods include the use of a needle, ballistic DNA, electroporation, sonoporation, photoporation, magnetofection, and hydroporation. Chemical methods generally refer to methods in which chemical carriers deliver a nucleic acid molecule to a cell and may include inorganic particles, lipid-based vectors, polymer-based vectors and peptide-based vectors.

In some embodiments, an expression vector is administered to a target cell using a cationic lipid (e.g., cationic liposome). Various types of lipids have been investigated for gene delivery, such as, for example, a lipid nano emulsion (e.g., which is a dispersion of one immiscible liquid in another stabilized by emulsifying agent) or a solid lipid nanoparticle.

In some embodiments, an expression vector is administered to a target cell using a peptide based delivery vehicle. Peptide based delivery vehicles can have advantages of protecting the genetic material to be delivered, targeting specific cell receptors, disrupting endosomal membranes and delivering genetic material into a nucleus. In some embodiments, an expression vector is administered to a target cell using a polymer based delivery vehicle. Polymer based delivery vehicles may comprise natural proteins, peptides and/or polysaccharides or synthetic polymers. In one embodiment, a polymer based delivery vehicle comprises polyethylenimine (PEI). PEI can condense DNA into positively charged particles which bind to anionic cell surface residues and are brought into the cell via endocytosis. In other embodiments, a polymer based delivery vehicle may comprise poly-L-lysine (PLL), poly (DL-lactic acid) (PLA), poly ( DL-lactide-co-glycoside) (PLGA), polyornithine, polyarginine, histones, protamines, dendrimers, chitosans, synthetic amino derivatives of dextran, and/or cationic acrylic polymers. In certain embodiments, polymer based delivery vehicles may comprise a mixture of polymers, such as, for example PEG and PLL.

In certain embodiments, an expression vector may be a viral vector suitable for gene therapy. Preferred characteristics of viral gene therapy vectors or gene delivery vectors may include the ability to be reproducibly and stably propagated and purified to high titers; to mediate targeted delivery (e.g., to deliver the transgene specifically to the tissue or organ of interest without widespread vector dissemination elsewhere); and to mediate gene delivery and transgene expression without inducing harmful side effects.

Several types of viruses, for example the non-pathogenic parvovirus referred to as adeno-associated virus, have been engineered for the purposes of gene therapy by harnessing the viral infection pathway but avoiding the subsequent expression of viral genes that can lead to replication and toxicity. Such viral vectors can be obtained by deleting all, or some, of the coding regions from the viral genome, but leaving intact those sequences (e.g., terminal repeat sequences) that may be necessary for functions such as packaging the vector genome into the virus capsid or the integration of vector nucleic acid (e.g., DNA) into the host chromatin.

In various embodiments, suitable viral vectors include retroviruses (e.g., A-type, B-type, C-type, and D-type viruses), adenovirus, parvovirus (e.g. adeno-associated viruses or AAV), coronavirus, negative strand RNA viruses such as orthomyxovirus (e.g., influenza virus), rhabdovirus (e.g., rabies and vesicular stomatitis virus), paramyxovirus (e. g.

measles and Sendai), positive strand RNA viruses such as picornavirus and alphavirus, and double-stranded DNA viruses including adenovirus, herpesvirus (e.g., Herpes Simplex virus types 1 and 2, Epstein-Barr virus, cytomegalovirus), and poxvirus (e.g., vaccinia, fowlpox and canarypox). Examples of retroviruses include avian leukosis-sarcoma virus, human T-lymphotrophic virus type 1 (HTLV-1), bovine leukemia virus (BLV), lentivirus, and spumavirus. Other viruses include Norwalk virus, togavirus, flavivirus, reoviruses, papovavirus, hepadnavirus, and hepatitis virus, for example. Viral vectors may be classified into two groups according to their ability to integrate into the host genome - integrating and non-integrating. Oncoretroviruses and lentiviruses can integrate into host cellular chromatin while adenoviruses, adeno-associated viruses, and herpes viruses predominantly persist in the cell nucleus as extrachromosomal episomes.

In certain embodiments, a suitable viral vector is a retroviral vector. Retroviruses refer to viruses of the family Retroviridae. Examples of retroviruses include oncoretroviruses, such as murine leukemia virus (MLV), and lentiviruses, such as human immunodeficiency virus 1 (HIV-1). Retroviral genomes are single-stranded (ss) RNAs and comprise various genes that may be provided in cis or trans. For example, retroviral genome may contain cis-acting sequences such as two long terminal repeats (LTR), with elements for gene expression, reverse transcription and integration into the host chromosomes. Other components include the packaging signal (psi or $\psi$), for the specific RNA packaging into newly formed virions and the polypurine tract (PPT), the site of the initiation of the positive strand DNA synthesis during reverse transcription. In addition, the retroviral genome may comprise gag, pol and env genes. The gag gene encodes the structural proteins, the pol gene encodes the enzymes that accompany the ssRNA and carry out reverse transcription of the viral RNA to DNA, and the env gene encodes the viral envelope. Generally, the gag, pol and env are provided in trans for viral replication and packaging.

In certain embodiments, a retroviral vector provided herein may be a lentiviral vector. At least five serogroups or serotypes of lentiviruses are recognized. Viruses of the different serotypes may differentially infect certain cell types and/or hosts. Lentiviruses, for example, include primate retroviruses and non-primate retroviruses. Primate retroviruses include HIV and simian immunodeficiency virus (SIV). Non-primate retroviruses include feline immunodeficiency virus (FIV), bovine immunodeficiency virus (BIV), caprine arthritis-encephalitis virus (CAEV), equine infectious anemia virus (EIAV) and visnavirus. Lentiviruses or lentivectors may be capable of transducing quiescent cells. As with oncoretrovirus vectors, the design of lentivectors may be based on the separation of cis- and trans-acting sequences.

In certain embodiments, the application provides expression vectors that have been designed for delivery by an optimized therapeutic retroviral vector. The retroviral vector can be a lentivirus comprising a left (5') LTR; sequences which aid packaging and/or nuclear import of the virus; a promoter; optionally one or more additional regulatory elements (such as, for example, an enhancer or polyA sequence); optionally a lentiviral reverse response element (RRE); a construct comprising PV selective regulatory element operably linked to a sequence encoding an eTF; optionally an insulator; and a right (3') retroviral LTR.

In exemplary embodiments, a viral vector provided herein is an adeno-associated virus (AAV). AAV is a small, replication-defective, non-enveloped animal virus that infects humans and some other primate species. AAV is not known to cause human disease and induces a mild immune response. AAV vectors can also infect both dividing and quiescent cells without integrating into the host cell genome.

The AAV genome consists of a linear single stranded DNA which is ~4.7kb in length. The genome consists of two open reading frames (ORF) flanked by an inverted terminal repeat (ITR) sequence that is about 145bp in length. The ITR consists of a nucleotide sequence at the 5' end (5' ITR) and a nucleotide sequence located at the 3' end (3' ITR) that contain palindromic sequences. The ITRs function in cis by folding over to form T-shaped hairpin structures by complementary base pairing that function as primers during initiation of DNA replication for second strand synthesis. The two open reading frames encode for rep and cap genes that are involved in replication and packaging of the virion. In an exemplary embodiment, an AAV vector provided herein does not contain the rep or cap genes. Such genes may be provided in trans for producing virions as described further below.

In certain embodiments, an AAV vector may include a stuffer nucleic acid. In some embodiments, the stuffer nucleic acid may encode a green fluorescent protein or antibiotic resistance gene such as kanamycin or ampicillin. In certain embodiments, the stuffer nucleic acid may be located outside of the ITR sequences (e.g., as compared to the eTF transgene sequence and regulatory sequences, which are located between the 5' and 3' ITR sequences).

Various serotypes of AAV exist, including AAV1, AAV2, AAV3, AAV4, AAV5, AAV6, AAV7, AAV8, AAV9, AAV10, AAV11, AAV12, and AAV13. These serotypes differ in their tropism, or the types of cells they infect. AAVs may comprise the genome and capsids from multiple serotypes (e.g., pseudotypes). For example, an AAV may comprise the genome of serotype 2 (e.g., ITRs) packaged in the capsid from serotype 5 or serotype 9. Pseudotypes may improve transduction efficiency as well as alter tropism.

In some cases, an AAV serotype that can cross the blood brain barrier or infect cells of the CNS is preferred. In some cases, AAV9 or a variant thereof is used to deliver an expression cassette of this disclosure, comprising a PV selective regulatory element operably linked to a transgene encoding an eTF that selectively upregulates SCN1A. In some cases, AAV9 or a variant thereof is used to deliver an expression cassette of this disclosure, comprising a PV selective microRNA binding site. In some cases, AAV9 or a variant thereof is used to deliver an expression cassette of this disclosure, comprising a PV selective regulatory element operably linked to a transgene encoding an eTF that selectively upregulates SCN1A, and a PV selective microRNA binding site

In exemplary embodiments, the application provides expression vectors that have been designed for delivery by an AAV. The AAV can be any serotype, for examples, AAV1, AAV2, AAV3, AAV3b, AAV4, AAV5, AAV6, AAV7, AAV8, AAV9, AAV10, AAV11, AAV12, AAV-DJ, or a chimeric, hybrid, or variant AAV. The AAV can also be a self-complementary AAV (scAAV). In certain embodiments, an expression vector designed for delivery by an AAV comprises a 5' ITR and a 3' ITR. In certain embodiments, an expression vector designed for delivery by an AAV comprises a 5' ITR, a promoter, a transgene encoding an eTF, and a 3' ITR. In certain embodiments, an expression vector designed for delivery by an AAV comprises a 5' ITR, an enhancer, a promoter, a transgene encoding an eTF, a polyA sequence, and a 3' ITR.

**Host Cells**

In another aspect, the invention relates to a host cell comprising an expression cassette or expression vector as disclosed herein. Host cells may be a bacterial cell, a yeast cell, an insect cell or a mammalian cell. In an exemplary embodiment, a host cell refers to any cell line that is susceptible to infection by a virus of interest, and amenable to culture *in vitro.*

In certain embodiments, a host cell provided herein may be used for *ex vivo* gene therapy purposes. In such embodiments, the cells are transfected with a nucleic acid molecule or expression vector comprising a PV selective microRNA binding site and/or a sequence encoding an eTF that selectively upregulates SCN1A as disclosed herein and subsequently transplanted into the patient or subject. Transplanted cells can have an autologous, allogenic or heterologous origin. For clinical use, cell isolation will generally be carried out under Good Manufacturing Practices (GMP) conditions. Before transplantation, cell quality and absence of microbial or other contaminants is typically checked and preconditioning, such as with radiation and/or an immunosuppressive treatment, may be carried out. Furthermore, the host cells may be transplanted together with growth factors to stimulate cell proliferation and/or differentiation.

In certain embodiments, a host cell may be used for *ex vivo* gene therapy. Preferably, said cells are eukaryotic cells such as mammalian cells, these include, but are not limited to, humans, non-human primates such as apes; chimpanzees; monkeys, and orangutans, domesticated animals, including dogs and cats, as well as livestock such as horses, cattle, pigs, sheep, and goats, or other mammalian species including, without limitation, mice, rats, guinea pigs, rabbits, hamsters, and the like. A person skilled in the art will choose the more appropriate cells according to the patient or subject to be transplanted.

In certain embodiments, a host cell provided herein may be a cell with self-renewal and pluripotency properties, such as stem cells or induced pluripotent stem cells. Stem cells are preferably mesenchymal stem cells. Mesenchymal stem cells (MSCs) are capable of differentiating into at least one of an osteoblast, a chondrocyte, an adipocyte, or a myocyte and may be isolated from any type of tissue. Generally, MSCs will be isolated from bone marrow, adipose tissue, umbilical cord, or peripheral blood. Methods for obtaining thereof are well known to a person skilled in the art. Induced pluripotent stem cells (also known as iPS cells or iPSCs) are a type of pluripotent stem cell that can be generated directly from adult cells. Yamanaka et al. induced iPS cells by transferring the Oct3/4, Sox2, Klf4 and c-Myc genes into mouse and human fibroblasts, and forcing the cells to express the genes (WO 2007/069666). Thomson et al. subsequently produced human iPS cells using Nanog and Lin28 in place of Klf4 and c-Myc (WO 2008/118820).

In an exemplary embodiment, a host cell provided herein is a packaging cell. Said cells can be adherent or suspension cells. The packaging cell, and helper vector or virus or DNA construct(s) provide together in trans all the missing functions which are required for the complete replication and packaging of the viral vector.

Preferably, said packaging cells are eukaryotic cells such as mammalian cells, including simian, human, dog and rodent cells. Examples of human cells are PER.C6 cells (WO01/38362), MRC-5 (ATCC CCL-171), WI-38 (ATCC CCL-75), HEK-293 cells (ATCC CRL-1573), HeLa cells (ATCC CCL2), and fetal rhesus lung cells (ATCC CL-160). Examples of non-human primate cells are Vero cells (ATCC CCL81), COS-1 cells (ATCC CRL-1650) or COS-7 cells (ATCC CRL-1651). Examples of dog cells are MDCK cells (ATCC CCL-34). Examples of rodent cells are hamster cells, such as BHK21-F, HKCC cells, or CHO cells.

As an alternative to mammalian sources, cell lines for use in the invention may be derived from avian sources such as chicken, duck, goose, quail or pheasant. Examples of avian cell lines include avian embryonic stem cells (WO01/85938 and WO03/076601), immortalized duck retina cells (WO2005/042728), and avian embryonic stem cell derived cells, including chicken cells (WO2006/108846) or duck cells, such as EB66 cell line (WO2008/129058 & WO2008/142124).

In another embodiment, said host cell are insect cells, such as SF9 cells (ATCC CRL-1711), Sf21 cells (IPLB-Sf21), MG1 cells (BTI-TN-MG1) or High Five™ cells (BTI-TN-5B1-4).

In certain embodiments, the host cells provided herein comprising the recombinant AAV vector/genome of the invention (e.g., comprising a PV selective microRNA binding site and/or a sequence encoding an eTF that selectively upregulates SCN1A) may further comprise one or more additional nucleic acid constructs, such as, for example (i) a nucleic acid construct (e.g., an AAV helper plasmid) that encodes rep and cap genes, but does not carry ITR sequences; and/or (ii) a nucleic acid construct (e.g., a plasmid) providing the adenoviral functions necessary for AAV replication. In an exemplary embodiment, a host cell provided herein comprises: i) an expression vector comprising a PV selective microRNA binding site and/or a sequence encoding an eTF that selectively upregulates SCN1A as provided herein (i.e., the recombinant AAV genome); ii) a nucleic acid construct encoding AAV rep and cap genes which does not carry the ITR sequences; and iii) a nucleic acid construct comprising adenoviral helper genes (as described further below).

In certain embodiments, the rep, cap, and adenoviral helper genes can be combined on a single plasmid (Blouin V et al. J Gene Med. 2004; 6(suppl): S223-S228; Grimm D. et al. Hum. Gene Ther. 2003; 7: 839-850). Thus, in another exemplary embodiment, a host cell provided herein comprises: i) an expression vector comprising a PV selective microRNA binding site and/or a sequence encoding an eTF that selectively upregulates SCN1A as disclosed herein (i.e., the recombinant AAV genome); and ii) a plasmid encoding AAV rep and cap genes which does not carry the ITR sequences and further comprising adenoviral helper genes.

In another embodiment, a host cell provided herein comprises: a) an expression vector comprising a PV selective microRNA binding site and/or a sequence encoding an eTF that selectively upregulates SCN1A as disclosed herein (i.e., the recombinant AAV genome); b) a plasmid encoding AAV rep and cap genes which does not carry the ITR sequences; and c) a plasmid comprising adenoviral helper genes E2a, E4, and VA RNAs; wherein co-transfection is performed in cells, preferably mammalian cells, that constitutively express and transcomplement the adenoviral E1 gene, like HEK-293 cells (ATCC CRL-1573).

In certain embodiments, a host cell suitable for large-scale production of AAV vectors is an insect cells that can be infected with a combination of recombinant baculoviruses (Urabe et al. Hum. Gene Ther. 2002; 13: 1935-1943). For example, SF9 cells may be co-infected with three baculovirus vectors respectively expressing AAV rep, AAV cap and the AAV vector to be packaged. The recombinant baculovirus vectors will provide the viral helper gene functions required for virus replication and/or packaging.

Further guidance for the construction and production of virions for gene therapy according to the invention can be found in: Viral Vectors for Gene Therapy, Methods and Protocols. Series: Methods in Molecular Biology, Vol. 737. Merten and Al-Rubeai (Eds.); 2011 Humana Press (Springer); Gene Therapy. M. Giacca. 2010 Springer-Verlag; Heilbronn R. and Weger S. Viral Vectors for Gene Transfer: Current Status of Gene Therapeutics. In: Drug Delivery, Handbook of Experimental Pharmacology 197; M. Schafer-Korting (Ed.). 2010 Springer-Verlag; pp. 143-170; Adeno-Associated Virus: Methods and Protocols. R. O. Snyder and P. Moulllier (Eds). 2011 Humana Press (Springer); Bunning H. et al. Recent developments in adeno-associated virus technology. J. Gene Med. 2008; 10:717-733; and Adenovirus: Methods and Protocols. M. Chillon and A. Bosch (Eds.); Third. Edition. 2014 Humana Press (Springer).

**Virions & Methods of Producing Virions**

In certain embodiments, the application provides viral particles comprising a viral vector comprising a PV selective microRNA binding site and/or a sequence encoding an eTF that selectively upregulates SCN1A as disclosed herein. The terms "viral particle", and "virion" are used herein interchangeably and relate to an infectious and typically replication-defective virus particle comprising the viral genome (e.g., the viral expression vector) packaged within a capsid and, as the case may be e.g., for retroviruses, a lipidic envelope surrounding the capsid. A "capsid" refers to the structure in which the viral genome is packaged. A capsid consists of several oligomeric structural subunits made of proteins. For example, AAV have an icosahedral capsid formed by the interaction of three capsid proteins: VP1, VP2 and VP3. In one embodiment, a virion provided herein is a recombinant AAV virion or rAAV virion obtained by packaging an AAV vector comprising a PV selective regulatory element and a PV selective microRNA binding site. In another embodiment, a virion provided herein is a recombinant AAV virion or rAAV virion obtained by packaging an AAV vector comprising a PV selective regulatory element operably linked to a sequence encoding an eTF that selectively upregulates SCN1A as described herein in a protein shell. In another embodiment, a virion provided herein is a recombinant AAV virion or rAAV virion obtained by packaging an AAV vector comprising a PV selective regulatory element operably linked to a sequence encoding an eTF that selectively upregulates SCN1A as described herein and a PV selective microRNA binding site in a protein shell.

In certain embodiments, a recombinant AAV virion provided herein may be prepared by encapsidating an AAV genome derived from a particular AAV serotype in a viral particle formed by natural Cap proteins corresponding to an AAV of the same particular serotype. In other embodiments, an AAV viral particle provided herein comprises a viral vector comprising ITR(s) of a given AAV serotype packaged into proteins from a different serotype. See e.g., Bunning H et al. J Gene Med 2008; 10: 717-733. For example, a viral vector having ITRs from a given AAV serotype may be packaged into: a) a viral particle constituted of capsid proteins derived from a same or different AAV serotype (e.g. AAV2 ITRs and AAV9 capsid proteins; AAV2 ITRs and AAV8 capsid proteins; etc.); b) a mosaic viral particle constituted of a mixture of capsid proteins from different AAV serotypes or mutants (e.g. AAV2 ITRs with AAV1 and AAV9 capsid proteins); c) a chimeric viral particle constituted of capsid proteins that have been truncated by domain swapping between different AAV serotypes or variants (e.g. AAV2 ITRs with AAV8 capsid proteins with AAV9 domains); or d) a targeted viral particle engineered to display selective binding domains, enabling stringent interaction with target cell specific receptors (e.g. AAV5 ITRs with AAV9 capsid proteins genetically truncated by insertion of a peptide ligand; or AAV9 capsid proteins non-genetically modified by coupling of a peptide ligand to the capsid surface).

The skilled person will appreciate that an AAV virion provided herein may comprise capsid proteins of any AAV serotype. In one embodiment, the viral particle comprises capsid proteins from an AAV serotype selected from the group consisting of an AAV1, an AAV2, an AAV5, an AAV8, and an AAV9, which are more suitable for delivery to the CNS (M. Hocquemiller et al., Hum Gene Ther 27(7): 478-496 (2016)). In a particular embodiment, the viral particle comprises an expression cassette of the invention wherein the 5'ITR and 3'ITR sequences of the expression cassette are of an AAV2 serotype and the capsid proteins are of an AAV9 serotype.

Numerous methods are known in the art for production of rAAV virions, including transfection, stable cell line production, and infectious hybrid virus production systems which include adenovirus-AAV hybrids, herpesvirus-AAV hybrids (Conway, J E et al., (1997) J. Virology 71(11):8780-8789) and baculovirus-AAV hybrids. rAAV production cultures for the production of rAAV virus particles all require; 1) suitable host cells, including, for example, human-derived cell lines such as HeLa, A549, or 293 cells, or insect-derived cell lines such as SF-9, in the case of baculovirus production systems; 2) suitable helper virus function, provided by wild-type or mutant adenovirus (such as temperature sensitive adenovirus), herpes virus, baculovirus, or a plasmid construct providing helper functions; 3) AAV rep and cap genes and gene products; 4) a transgene (e.g., comprising one or more of: a PV selective microRNA binding site, a sequence encoding an eTF that selectively upregulates SCN1A as described herein, and/or a PV selective promoter) flanked by AAV ITR sequences; and 5) suitable media and media components to support rAAV production.

In various embodiments, the host cells described herein comprise the following three components: (1) a rep gene and a cap gene, (2) genes providing helper functions, and (3) a transgene (e.g., comprising one or more of: a PV selective microRNA binding site, a sequence encoding an eTF that selectively upregulates SCN1A as described herein, and/or a PV selective promoter) flanked by ITRs. The AAV rep gene, AAV cap gene, and genes providing helper functions can be introduced into the cell by incorporating said genes into a vector such as, for example, a plasmid, and introducing said vector into the host cell. The rep, cap and helper function genes can be incorporated into the same plasmid or into different plasmids. In a preferred embodiment, the AAV rep and cap genes are incorporated into one plasmid and the genes providing helper functions are incorporated into another plasmid. The various plasmids for creation of a host cell for virion production (e.g., comprising AAV rep and cap genes, helper functions, or a transgene) can be introduced into the cell by using any suitable method well known in the art. Examples of transfection methods include, but are not limited to, coprecipitation with calcium phosphate, DEAE-dextran, polybrene, electroporation, microinjection, liposome-mediated fusion, lipofection, retrovirus infection and biolistic transfection. In certain embodiments, the plasmids providing the rep and cap genes, the helper functions and the transgene flanked by ITRs can be introduced into the cell simultaneously. In another embodiment, the plasmids providing the rep and cap genes and the helper functions can be introduced in the cell before or after the introduction of plasmid comprising the transgene. In an exemplary embodiment, the cells are transfected simultaneously with three plasmids (e.g., a triple transfection method): (1) a plasmid comprising the transgene (e.g., comprising one or more of: a PV selective microRNA binding site, a sequence encoding an eTF that selectively upregulates SCN1A as described herein, and/or a PV selective promoter) flanked by ITRs, (2) a plasmid comprising the AAV rep and cap genes, and (3) a plasmid comprising the genes providing the helper functions. Exemplary host cells may be 293, A549 or HeLa cells.

In other embodiments, one or more of (1) the AAV rep and cap genes, (2) genes providing helper functions, and (3) the transgene (e.g., comprising one or more of: a PV selective microRNA binding site, a sequence encoding an eTF that selectively upregulates SCN1A as described herein, and/or a PV selective promoter) flanked by ITRs, may be carried by the packaging cell, either episomally and/or integrated into the genome of the packaging cell. In one embodiment, host cells may be packaging cells in which the AAV rep and cap genes and helper functions are stably maintained in the host cell and the host cell is transiently transfected with a plasmid containing a transgene (e.g., comprising one or more of: a PV selective microRNA binding site, a sequence encoding an eTF that selectively upregulates SCN1A as described herein, and/or a PV selective promoter) flanked by ITRs. In another embodiment, host cells are packaging cells in which the AAV rep and cap genes are stably maintained in the host cell and the host cell is transiently transfected with a plasmid containing a transgene (e.g., comprising one or more of: a PV selective microRNA binding site, a sequence encoding an eTF that selectively upregulates SCN1A as described herein, and/or a PV selective promoter) flanked by ITRs and a plasmid containing the helper functions. In another embodiment, host cells may be packaging cells in which the helper functions are stably maintained in the host cell and the host cell is transiently transfected with a plasmid containing a transgene (e.g., comprising one or more of: a PV selective microRNA binding site, a sequence encoding an eTF that selectively upregulates SCN1A as described herein, and/or a PV selective promoter) flanked by ITRs and a plasmid containing rep and cap genes. In another embodiment, host cells may be producer cell lines that are stably transfected with rep and cap genes, helper functions and the transgene sequence (e.g., comprising one or more of: a PV selective microRNA binding site, a sequence encoding an eTF that selectively upregulates SCN1A as described herein, and/or a PV selective promoter) flanked by ITRs. Exemplary packaging and producer cells may be derived from 293, A549 or HeLa cells.

In another embodiment, the producer cell line is an insect cell line (typically Sf9 cells) that is infected with baculovirus expression vectors that provide Rep and Cap proteins. This system does not require adenovirus helper genes (Ayuso E, et al., Curr. Gene Ther. 2010, 10:423-436).

The term "cap protein", as used herein, refers to a polypeptide having at least one functional activity of a native AAV Cap protein (e.g. VP1, VP2, VP3). Examples of functional activities of cap proteins include the ability to induce formation of a capsid, facilitate accumulation of single-stranded DNA, facilitate AAV DNA packaging into capsids (i.e. encapsidation), bind to cellular receptors, and facilitate entry of the virion into host cells. In principle, any Cap protein can be used in the context of the present invention.

Cap proteins have been reported to have effects on host tropism, cell, tissue, or organ specificity, receptor usage, infection efficiency, and immunogenicity of AAV viruses. Accordingly, an AAV cap for use in an rAAV may be selected taking into consideration, for example, the subject's species (e.g. human or non-human), the subject's immunological state, the subject's suitability for long or short-term treatment, or a particular therapeutic application (e.g. treatment of a particular disease or disorder, or delivery to particular cells, tissues, or organs). In certain embodiments, the cap protein is derived from the AAV of the group consisting of AAV1, AAV2, AAV5, AAV8, and AAV9 serotypes. In an exemplary embodiment, the cap protein is derived from AAV9.

In some embodiments, an AAV Cap for use in the method of the invention can be generated by mutagenesis (i.e. by insertions, deletions, or substitutions) of one of the aforementioned AAV caps or its encoding nucleic acid. In some embodiments, the AAV cap is at least 70%, 75%, 80%, 85%, 90%, 95%, 98%, or 99% or more similar to one or more of the aforementioned AAV caps.

In some embodiments, the AAV cap is chimeric, comprising domains from two, three, four, or more of the aforementioned AAV caps. In some embodiments, the AAV cap is a mosaic of VP1, VP2, and VP3 monomers originating from two or three different AAV or a recombinant AAV. In some embodiments, a rAAV composition comprises more than one of the aforementioned caps.

In some embodiments, an AAV cap for use in a rAAV virion is engineered to contain a heterologous sequence or other modification. For example, a peptide or protein sequence that confers selective targeting or immune evasion may be engineered into a cap protein. Alternatively or in addition, the cap may be chemically modified so that the surface of the rAAV is polyethylene glycolated (i.e., pegylated), which may facilitate immune evasion. The cap protein may also be mutagenized (e.g., to remove its natural receptor binding, or to mask an immunogenic epitope).

The term "rep protein", as used herein, refers to a polypeptide having at least one functional activity of a native AAV rep protein (e.g. rep 40, 52, 68, 78). Examples of functional activities of a rep protein include any activity associated with the physiological function of the protein, including facilitating replication of DNA through recognition, binding and nicking of the AAV origin of DNA replication as well as DNA helicase activity. Additional functions include modulation of transcription from AAV (or other heterologous) promoters and site-specific integration of AAV DNA into a host chromosome. In a particular embodiment, AAV rep genes may be from the serotypes AAV1, AAV2, AAV4, AAV5, AAV6, AAV7, AAV8, AAV9, AAV10 or AAVrh10; more preferably from an AAV serotype selected from the group consisting of AAV1, AAV2, AAV5, AAV8, and AAV9.

In some embodiments, an AAV rep protein for use in the method of the invention can be generated by mutagenesis (i.e. by insertions, deletions, or substitutions) of one of the aforementioned AAV reps or its encoding nucleic acid. In some embodiments, the AAV rep is at least 70%, 75%, 80%, 85%, 90%, 95%, 98%, or 99% or more similar to one or more of the aforementioned AAV reps.

The expressions "helper functions" or "helper genes", as used herein, refer to viral proteins upon which AAV is dependent for replication. The helper functions include those proteins required for AAV replication including, without limitation, those proteins involved in activation of AAV gene transcription, stage specific AAV mRNA splicing, AAV DNA replication, synthesis of cap expression products, and AAV capsid assembly. Viral-based accessory functions can be derived from any of the known helper viruses such as adenovirus, herpesvirus (other than herpes simplex virus type-1), and vaccinia virus. Helper functions include, without limitation, adenovirus E1, E2a, VA, and E4 or herpesvirus UL5, ULB, UL52, and UL29, and herpesvirus polymerase. In a preferred embodiment, the proteins upon which AAV is dependent for replication are derived from adenovirus.

In some embodiments, a viral protein upon which AAV is dependent for replication for use in the method of the invention can be generated by mutagenesis (i.e. by insertions, deletions, or substitutions) of one of the aforementioned viral proteins or its encoding nucleic acid. In some embodiments, the viral protein is at least 70%, 75%, 80%, 85%, 90%, 95%, 98%, or 99% or more similar to one or more of the aforementioned viral proteins.

Methods for assaying the functions of cap proteins, rep proteins and viral proteins upon which AAV is dependent for replication are well known in the art.

Host cells for expressing a transgene of interest (e.g., comprising one or more of: a PV selective microRNA binding site, a sequence encoding an eTF that selectively upregulates SCN1A as described herein, and/or a PV selective promoter) may be grown under conditions adequate for assembly of the AAV virions. In certain embodiments, host cells are grown for a suitable period of time in order to promote the assembly of the AAV virions and the release of virions into the media. Generally, cells may be grown for about 24 hours, about 36 hours, about 48 hours, about 72 hours, about 4 days, about 5 days, about 6 days, about 7 days, about 8 days, about 9 days, or up to about 10 days. After about 10 days (or sooner, depending on the culture conditions and the particular host cell used), the level of production generally decreases significantly. Generally, time of culture is measured from the point of viral production. For example, in the case of AAV, viral production generally begins upon supplying helper virus function in an appropriate host cell as described herein. Generally, cells are harvested about 48 to about 100, preferably about 48 to about 96, preferably about 72 to about 96, preferably about 68 to about 72 hours after helper virus infection (or after viral production begins).

rAAV production cultures can be grown under a variety of conditions (over a wide temperature range, for varying lengths of time, and the like) suitable to the particular host cell being utilized. rAAV production cultures include attachment-dependent cultures which can be cultured in suitable attachment-dependent vessels such as, for example, roller bottles, hollow fiber filters, microcarriers, and packed-bed or fluidized-bed bioreactors. rAAV vector production cultures may also include suspension-adapted host cells such as HeLa, 293, and SF-9 cells which can be cultured in a variety of ways including, for example, spinner flasks, stirred tank bioreactors, and disposable systems such as the Wave bag system.

Suitable media known in the art may be used for the production of rAAV virions. These media include, without limitation, media produced by Hyclone Laboratories and JRH including Modified Eagle Medium (MEM), Dulbecco's Modified Eagle Medium (DMEM), each of which is incorporated herein by reference in its entirety. In certain embodiments, rAAV production culture media may be supplemented with serum or serum-derived recombinant proteins at a level of 0.5%-20% (v/v or w/v). Alternatively, rAAV vectors may be produced in serum-free conditions which may also be referred to as media with no animal-derived products.

After culturing the host cells to allow AAV virion production, the resulting virions may be then be harvested and purified. In certain embodiments, the AAV virions can be obtained from (1) the host cells of the production culture by lysis of the host cells, and/or (2) the culture medium of said cells after a period of time post-transfection, preferably 72 hours. The rAAV virions may be harvested from the spent media from the production culture, provided the cells are cultured under conditions that cause release of rAAV virions into the media from intact cells (see e.g., U.S. Pat. No. 6,566,118). Suitable methods of lysing cells are also known in the art and include for example multiple freeze/thaw cycles, sonication, microfluidization, and treatment with chemicals, such as detergents and/or proteases.

After harvesting, the rAAV virions may be purified. The term "purified" as used herein includes a preparation of rAAV virions devoid of at least some of the other components that may also be present where the rAAV virions naturally occur or are initially prepared from. Thus, for example, purified rAAV virions may be prepared using an isolation technique to enrich it from a source mixture, such as a culture lysate or production culture supernatant. Enrichment can be measured in a variety of ways, such as, for example, by the proportion of DNase-resistant particles (DRPs) or genome copies (gc) present in a solution, or by infectivity, or it can be measured in relation to a second, potentially interfering substance present in the source mixture, such as contaminants, including production culture contaminants or in-process contaminants, including helper virus, media components, and the like.

In certain embodiments, the rAAV production culture harvest may be clarified to remove host cell debris. In some embodiments, the production culture harvest may be clarified using a variety of standard techniques, such as, centrifugation or filtration through a filter of 0.2 $\mu$m or greater pore size (e.g., a cellulose acetate filter or a series of depth filters).

In certain embodiments, the rAAV production culture harvest is further treated with Benzonase™ to digest any high molecular weight DNA present in the production culture. In some embodiments, the Benzonase™ digestion is performed under standard conditions, for example, a final concentration of 1-2.5 units/ml of Benzonase™ at a temperature ranging from ambient to 37°C for a period of 30 minutes to several hours.

In certain embodiments, the rAAV virions may be isolated or purified using one or more of the following purification steps: equilibrium centrifugation; flow-through anionic exchange filtration; tangential flow filtration (TFF) for concentrating the rAAV particles; rAAV capture by apatite chromatography; heat inactivation of helper virus; rAAV capture by hydrophobic interaction chromatography; buffer exchange by size exclusion chromatography (SEC); nanofiltration; and rAAV capture by anionic exchange chromatography, cationic exchange chromatography, or affinity chromatography. These steps may be used alone, in various combinations, or in different orders. Methods to purify rAAV particles are found, for example, in Xiao et al., (1998) Journal of Virology 72:2224-2232; U.S. Pat. Nos. 6,989,264 and 8,137,948; and WO 2010/148143.

In certain embodiments, purified AAV virions can be dialyzed against PBS, filtered and stored at -80°C. Titers of viral genomes can be determined by quantitative PCR using linearized plasmid DNA as standard curve (see e.g., Lock M, et al., Hum. Gene Ther. 2010; 21:1273-1285).

**Pharmaceutical Compositions**

In certain embodiments, the application provides compositions comprising a PV selective microRNA binding site and/or a sequence encoding an eTF that selectively upregulates SCN1A and a pharmaceutically acceptable carrier. In other embodiments, the application provides virions comprising a PV selective microRNA binding site and/or a sequence encoding an eTF that selectively upregulates SCN1A and a pharmaceutically acceptable carrier. In exemplary embodiments, such compositions are suitable for gene therapy applications. Pharmaceutical compositions are preferably sterile and stable under conditions of manufacture and storage. Sterile solutions may be accomplished, for example, by filtration through sterile filtration membranes.

Acceptable carriers and excipients in the pharmaceutical compositions are preferably nontoxic to recipients at the dosages and concentrations employed. Acceptable carriers and excipients may include buffers such as phosphate, citrate, HEPES, and TAE, antioxidants such as ascorbic acid and methionine, preservatives such as hexamethonium chloride, octadecyldimethylbenzyl ammonium chloride, resorcinol, and benzalkonium chloride, proteins such as human serum albumin, gelatin, dextran, and immunoglobulins, hydrophilic polymers such as polyvinylpyrrolidone, amino acids such as glycine, glutamine, histidine, and lysine, and carbohydrates such as glucose, mannose, sucrose, and sorbitol. Pharmaceutical compositions of the disclosure can be administered parenterally in the form of an injectable formulation. Pharmaceutical compositions for injection can be formulated using a sterile solution or any pharmaceutically acceptable liquid as a vehicle. Pharmaceutically acceptable vehicles include, but are not limited to, sterile water and physiological saline.

The pharmaceutical compositions of the disclosure may be prepared in microcapsules, such as hydroxylmethylcellulose or gelatin-microcapsules and polymethylmethacrylate microcapsules. The pharmaceutical compositions of the disclosure may also be prepared in other drug delivery systems such as liposomes, albumin microspheres, microemulsions, nano-particles, and nanocapsules. The pharmaceutical composition for gene therapy can be in an acceptable diluent, or can comprise a slow release matrix in which the gene delivery vehicle is imbedded.

Pharmaceutical compositions provided herein may be formulated for parenteral administration, subcutaneous administration, intravenous administration, intramuscular administration, intra-arterial administration, intraparenchymal administration, intrathecal administration, intra-cisterna magna administration, intracerebroventricular administration, or intraperitoneal administration. The pharmaceutical composition may also be formulated for, or administered via, nasal, spray, oral, aerosol, rectal, or vaginal administration. In one embodiment, a pharmaceutical composition provided herein is administered to the CNS or cerebral spinal fluid (CSF), i.e. by intraparenchymal injection, intrathecal injection, intra-cisterna magna injection, or intracerebroventricular injection. The tissue target may be specific, for example the CNS, or it may be a combination of several tissues, for example the muscle and CNS tissues. Exemplary tissue or other targets may include liver, skeletal muscle, heart muscle, adipose deposits, kidney, lung, vascular endothelium, epithelial, hematopoietic cells, CNS and/or CSF. In a preferred embodiment, a pharmaceutical composition provided herein comprising a PV selective microRNA binding site and/or an eTF that selectively upregulates SCN1A is administered to the CNS or CSF injection, i.e. by intraparenchymal injection, intrathecal injection, intra-cisterna magna injection, or intracerebroventricular injection. One or more of these methods may be used to administer a pharmaceutical composition of the disclosure.

In certain embodiments, a pharmaceutical composition provided herein comprises an "effective amount" or a "therapeutically effective amount." As used herein, such amounts refer to an amount effective, at dosages and for periods of time necessary to achieve the desired therapeutic result, such as increasing the level of SCN1A expression and/or decreasing the frequency and/or duration of seizures.

The dosage of the pharmaceutical compositions of the disclosure depends on factors including the route of administration, the disease to be treated, and physical characteristics (e.g., age, weight, general health) of the subject. Dosage may be adjusted to provide the optimum therapeutic response. Typically, a dosage may be an amount that effectively treats the disease without inducing significant toxicity. In one embodiment, an AAV vector provided herein can be administered to the patient for the treatment of an SCN1A deficiency (including for example, Dravet syndrome) in an amount or dose within a range of $5 \times 10^{11}$ to $1 \times 10^{14}$ gc/kg (genome copies per kilogram of patient body weight (gc/kg)). In a more particular embodiment, the AAV vector is administered in an amount comprised within a range of about $5 \times 10^{11}$ gc/kg to about $3 \times 10^{13}$ gc/kg, or about $1 \times 10^{12}$ to about $1 \times 10^{14}$ gc/kg, or about $1 \times 10^{12}$ to about $1 \times 10^{13}$ gc/kg, or about $5 \times 10^{11}$ gc/kg, $1 \times 10^{12}$ gc/kg, $1.5 \times 10^{12}$ gc/kg, $2.0 \times 10^{12}$ gc/kg, $2.5 \times 10^{12}$ gc/kg, $3 \times 10^{12}$ gc/kg, $3.5 \times 10^{12}$ gc/kg, $4 \times 10^{12}$ gc/kg, $4.5 \times 10^{12}$ gc/kg, $5 \times 10^{12}$ gc/kg, $5.5 \times 10^{12}$ gc/kg, $6 \times 10^{12}$ gc/kg, $6.5 \times 10^{12}$ gc/kg, $7 \times 10^{12}$ gc/kg, $7.5 \times 10^{12}$ gc/kg, $8 \times 10^{12}$ gc/kg, $8.5 \times 10^{12}$ gc/kg, $9 \times 10^{12}$ gc/kg or $9.5 \times 10^{12}$ gc/kg. The gc/kg may be determined, for example, by qPCR or digital droplet PCR (ddPCR) (see e.g., M. Lock et al, Hum Gene Ther Methods. 2014 Apr;25(2): 115-25). In another embodiment, an AAV vector provided herein can be administered to the patient for the treatment of an SCN1A deficiency (including for example, Dravet syndrome) in an amount or dose within a range of $1 \times 10^9$ to $1 \times 10^{11}$ iu/kg (infective units of the vector (iu)/subject's or patient's body weight (kg)). In certain embodiments, the pharmaceutical composition may be formed in a unit dose as needed. Such single dosage units may contain about $1 \times 10^9$ gc to about $1 \times 10^{15}$ gc.

Pharmaceutical compositions of the disclosure may be administered to a subject in need thereof, for example, one or more times (e.g., 1-10 times or more) daily, weekly, monthly, biannually, annually, or as medically necessary. In an exemplary embodiment, a single administration is sufficient. In one embodiment, a pharmaceutical composition comprising an expression cassette encoding a PV selective microRNA binding site and/or an eTF that selectively upregulates SCN1A is suitable for use in human subjects and is administered by intraparenchymal injection, intrathecal injection, intra-cisterna magna injection, or intracerebroventricular injection. In one embodiment, the pharmaceutical composition is delivered via a peripheral vein by bolus injection. In other embodiments, the pharmaceutical composition is delivered via a peripheral vein by infusion over about 10 minutes ($\pm5$ minutes), over about 20 minutes ($\pm5$ minutes), over about 30 minutes ($\pm5$ minutes), over about 60 minutes ($\pm5$ minutes), or over about 90 minutes ($\pm10$ minutes).

In another aspect, the application further provides a kit comprising a nucleic acid molecule, vector, host cell, virion or pharmaceutical composition as described herein in one or more containers. A kit may include instructions or packaging materials that describe how to administer a nucleic acid molecule, vector, host cell or virion contained within the kit to a patient. Containers of the kit can be of any suitable material, e.g., glass, plastic, metal, etc., and of any suitable size, shape, or configuration. In certain embodiments, the kits may include one or more ampoules or syringes that contain a nucleic acid molecule, vector, host cell, virion or pharmaceutical composition in a suitable liquid or solution form.

## Methods of Treatment

In one aspect, the application provides methods for using the eTFs that selectively upregulate SCN1A as disclosed herein. In certain embodiments, the application provides methods for administering an expression cassette, an expression vector, or a viral particle comprising a polynucleotide encoding an eTF that selectively upregulates SCN1A as disclosed herein to upregulate expression of SCN1A in a cell. In various embodiments, an eTF that selectively upregulates SCN1A as disclosed herein may be used to modulate expression of SCN1A in a cell *in vitro, in vivo,* or *ex vivo.*

In certain embodiments, the application provides methods for treating a disease or disorder associated with SCN1A by administering an expression cassette, an expression vector, or a viral particle comprising a polynucleotide encoding an eTF that selectively upregulate SCN1A as disclosed herein to a subject in need thereof. In certain embodiments, the disorder is a central nervous system disorder. In exemplary embodiments, the disease or disorder is associated with haploinsufficiency of SCN1A. In certain embodiments, the disorder is epilepsy associated with SCN1A haploinsufficiency. In certain embodiments, the haploinsufficiency is the result of the subject being heterozygous for a loss of function mutation of the SCN1A gene. In certain embodiments, the disorder is epilepsy associated with an insertion, deletion, or substitution in the SCN1A gene. In certain embodiments, the disorder is epilepsy associated with a point mutation in the SCN1A gene. In certain embodiments, a method of treating a disease or disorder comprises administering an expression cassette, an expression vector, or a viral particle comprising a polynucleotide encoding an eTF that selectively upregulates SCN1A as disclosed herein such that under-expression of SCN1A is corrected, brought within a level of a healthy individual, or brought within a normal range as defined by a standard of medical care. In certain embodiments, the methods disclosed herein are used to treat a disease or disorder associated with endogenous SCN1A comprising one or more mutations that results in abnormal expression of SCN1A.

In certain embodiments, the application provides methods for ameliorating a symptom associated with a disease or disorder by administering an expression cassette, an expression vector, or a viral particle comprising a polynucleotide encoding an eTF that selectively upregulates SCN1A as disclosed herein to a subject in need thereof.

In an exemplary embodiment, the application provides methods for treating a disease, disorder or symptom associated with a mutation in SCN1A (e.g., point mutation, substitution, deletion, inversion, etc.), a deficiency in Nav1.1 and/or reduced activity of Nav1.1 by administering to a subject in need thereof an expression cassette, an expression vector, or a viral particle comprising a polynucleotide encoding an eTF that selectively upregulates expression of the SCN1A gene or its protein product Nav1.1. Voltage-gated sodium ion channels are important for the generation and propagation of action potentials in striated muscle and neuronal tissues. Voltage-gated sodium ion channels are heteromeric complexes consisting of a large central pore-forming glycosylated alpha subunit and 2 smaller auxiliary beta subunits. The large alpha subunit Nav1.1 subunit, encoded by the SCN1A gene, is relevant for a variety of diseases or disorders such as Dravet syndrome. Nav1.1 is expressed in neurons, and can be assembled with various beta subunits, including Navβ1 expressed by SCN1B gene.

In certain embodiments, the application provides methods for treating diseases associated with a mutation in SCN1A (e.g., deletion, insertion, inversion, point mutation (e.g., nonsense mutation, missense mutation), etc.) or reduced activity of Nav1.1 using an eTF that selectively upregulates expression of the endogenous SCN1A gene. Diseases and disorders associated with SCN1A mutations include, but are not limited to: Dravet syndrome, Ohtahara syndrome, epilepsy, early infantile epileptic encephalopathy 6 (EIEE6), familial febrile seizures 3A (FEB3A), intractable childhood epilepsy with generalized tonic-clonic seizures (ICEGTC), migraine, familial hemiplegic 3 (FHM3), Panayiotopoulos syndrome, familial atrial fibrillation 13 (ATFB13), generalized epilepsy with febrile seizures plus type 1 (gefs+ type 1), Brugada syndrome, nonspecific cardiac conduction defect, generalized epilepsy with febrile seizures plus, benign familial infantile seizures, early infantile epileptic encephalopathy11 (EIEE11), benign familial infantile epilepsy, neurodegeneration, tauopathies and Alzheimer's disease. In some cases, the neurological condition is Dravet syndrome. Mutations or abnormalities in SCN1A have also been associated with seizure disorders, epilepsy, autism, familial hemiplegic migraine type 3 (FHM3), genetic epilepsy with febrile seizures plus (GEFS+), and effectiveness of certain anti-seizure medications. For instance, ICS5N+5G>A mutation in SCN1A is associated with the maximum safe amount (dose) of the anti-seizure drugs phenytoin and carbamazepine.

In certain embodiments, the application provides a method for treating a subject with, or at risk of developing, Dravet syndrome by administering an expression cassette, expression vector, or viral particle comprising a polynucleotide encoding an eTF that selectively upregulates SCN1A. Dravet syndrome has been characterized by prolonged febrile and non-febrile seizures within the first year of a child's life. This disease progresses to other seizure types like myoclonic and partial seizures, psychomotor delay, and ataxia. It is characterized by cognitive impairment, behavioral disorders, and motor deficits. Behavioral deficits often include hyperactivity and impulsiveness, and in more rare cases, autistic-like behaviors. Dravet syndrome is also associated with sleep disorders including somnolence and insomnia. In many patients, Dravet syndrome is caused by genetic mutations that lead to the production of non-functional proteins. Many challenges exist in treating disorders associated with genetic causes. Thus, most of the existing treatments have been drawn to the prophylactic medical management of seizures and other symptoms.

In 70-90% of patients, Dravet syndrome is caused by nonsense mutations in the SCN1A gene resulting in a premature stop codon and thus a non-functional protein. Typically, a missense mutation in either the S5 or S6 segment of the sodium channel pore results in a loss of channel function and the development of Dravet syndrome. A heterozygous inheritance of an SCN1A mutation, e.g., a nonsense mutation, a missense mutation, deletion, insertion, inversion, etc., is all that is necessary to develop a defective sodium channel; patients with Dravet syndrome will still have one normal copy of the gene. Thus, the disease is characterized as one of haploinsufficiency and thus increasing expression of the functioning copy of SCN1A could restore normal production levels of Nav1.1.

Symptoms associated with Dravet syndrome include seizures, memory defects, developmental delay, poor muscle tone and/or cognitive problems. Treatment with an expression cassette, expression vector, or virial particle described herein can result in an improvement of one or more symptoms, such as a reduction in number, duration, and/or intensity of seizures. Administration of a gene therapy as described herein to a subject at risk of developing Dravet syndrome can prevent the development of or slow the progression of one or more symptoms of Dravet.

In certain embodiments, treatment with an expression cassette, expression vector, or virial particle comprising a polynucleotide encoding an eTF that selectively upregulates SCN1A as described herein reduces seizure duration and/or frequency, e.g., seizures associated with Dravet syndrome, by at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 45%, 50%, 55%, 60%, 65%, 70%, 75%, 80%, 85%, 90%, 95% or more as compared to an untreated control or as compared to the level before treatment.

In some Alzheimer's patients, production of amyloid β (Aβ) involving many peptides and proteases that can affect excitability of neurons, causing seizures and downregulation of the Nav1.1 sodium channel in PV neurons. In another embodiment, the application provides methods for treating a subject suffering from Alzheimer's disease by administering an expression cassette, expression vector, or viral particle described herein that comprises a polynucleotide encoding an eTF that selectively upregulates SCN1A. Symptoms associated with Alzheimer's disease include short term memory loss, cognitive difficulties, seizures, and difficulties with language, executive functions, perception (agnosia), and execution of movements (apraxia). Treatment with an expression cassette, expression vector, or viral particle comprising a polynucleotide encoding an eTF that selectively upregulates SCN1A can result in an improvement of one or more Alzheimer's disease symptoms, such as a reduction in progression of memory loss, or the prevention of one or more symptoms. In some cases, the treatment can result in a correction of high gamma power brain activity. The treatment can result in a decrease in seizure frequency and/or seizure severity, or a decrease in high gamma power activity by at least 10%, 20%, 30%, 40%, 50%, 60%, 70% or more as compared to no treatment. In some cases, the treatment can result in an improvement in cognitive function. Learning and/or memory can be improved by at least 10%, 20%, 30%, 40%, 50%, 60%, 70%, 80%, 90%, 100% or more than 100% as compared to no treatment, or before the treatment with a polynucleotide encoding an eTF that selectively upregulates SCN1A as disclosed herein.

In some cases, treatment with an expression cassette, expression vector, or viral particle comprising a polynucleotide encoding an eTF that selectively upregulates SCN1A reduces high gamma power activity (e.g., high gamma power activity associated with Alzheimer's disease) by at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 45%, 50%, 55%, 60%, 65%, 70%, 75%, 80%, 85%, 90%, or 95% as compared to an untreated control or as compared to the level before treatment.

Parkinsonism refers to a collection of signs and symptoms found in Parkinson's disease (PD), including slowness (bradykinesia), stiffness (rigidity), tremor and imbalance (postural instability). In some cases, administration of an expression cassette, expression vector, or viral particle comprising a polynucleotide encoding an eTF that selectively upregulates SCN1A as described herein to a subject at risk of developing or suffering from Parkinson's disease can prevent the development of one or more symptoms thereof or slow down the progression of Parkinson's disease by at least 10%, at least 20%, at least 30%, at least 40%, at least 50%, at least 60%, at least 70%, at least 80%, or at least 90% as compared to no treatment.

In certain embodiments, the application provides methods that can be used to treat a subject who is at risk of developing a disease. The subject can be known to be predisposed to a disease, for example, a neurological disease or a disease associated with epilepsy, seizures and/or encephalopathy. The subject can be predisposed to a disease due to a genetic event, or due to known risk factors. For example, a subject can carry a mutation in SCN1A which is associated with epilepsy (such as, for example, Dravet syndrome). Any mutation in the SCN1A gene that reduces its activity (by reducing expression levels, impairing protein function, or a combination of both) can predispose a subject to a disease, including any one or more of insertions, deletions, inversions, translocations, or substitutions (e.g., point mutations including nonsense mutations and/or missense mutations) in the SCN1A gene. In some cases the subject can be predisposed to a disease such as Alzheimer's disease due to the age of the subject. In some cases, the subject may have an insufficient amount of SCN1A protein and treating a disease associated with SCN1A involves administering an expression cassette, expression vector, or viral particle comprising a polynucleotide encoding an eTF that selectively upregulates endogenous SCN1A as described herein.

In certain embodiments, treatments using an expression cassette, expression vector, or viral particle comprising a polynucleotide encoding an eTF that selectively upregulates endogenous SCN1A provided herein can result in a decrease or cessation of symptoms associated with Dravet or other SCN1A associated disease or disorders, e.g., epilepsy associated with SCN1A haploinsufficiency. For example, treatment can improve learning, memory, cognitive function, and/or motor function; reduce frequency and/or duration of seizures; and/or reduce temperature sensitivity (or increase the temperature threshold for triggering a seizure).

In another aspect, the application provides methods for selective expression of a transgene in PV neurons by administering an expression cassette, an expression vector, or a viral particle comprising at least one PV selective microRNA binding site. In certain embodiments, the application provides methods for selective expression of a transgene in PV neurons of a primate by administering an expression cassette, an expression vector, or a viral particle comprising a transgene and at least one PV selective microRNA binding site. In certain embodiments, the application provides methods for selective expression of a transgene in PV neurons by administering an expression cassette, an expression vector, or a viral particle comprising a PV selective regulatory element operably linked to a transgene and at least one PV selective microRNA binding site. In exemplary embodiments, the transgene comprises a sequence encoding any of the eTFs that selectively upregulate SCN1A as described herein.

In certain embodiments, the application provides a method for gene therapy comprising administering to a subject an expression cassette, an expression vector, or a viral particle comprising a transgene and at least one PV selective microRNA binding site. In certain embodiments, the application provides methods for gene therapy comprising administering to a subject an expression cassette, an expression vector, or a viral particle comprising a PV selective regulatory element operably linked to a transgene and at least one PV selective microRNA binding site. In exemplary embodiments, the transgene comprises a sequence encoding any of the eTFs that selectively upregulate SCN1A as described herein.

In certain embodiments, the application provides a method for treating a disease or disorder comprising administering to an expression cassette, an expression vector, or a viral particle comprising a transgene and at least one PV selective microRNA binding site. In certain embodiments, the application provides methods for treating a disease or disorder comprising administering to a subject an expression cassette, an expression vector, or a viral particle comprising a PV selective regulatory element operably linked to a transgene and at least one PV selective microRNA binding site. In exemplary embodiments, the transgene comprises a sequence encoding any of the eTFs that selectively upregulate SCN1A as described herein. In certain embodiments, the expression cassette, expression vector, or viral particle comprising a transgene and a PV selective microRNA binding site and optionally a PV selective regulatory element may be used to treat a disease or disorder in which PV neurons are implicated. In certain embodiments, the expression cassette, expression vector, or viral particle comprising a transgene and a PV selective microRNA binding site and optionally a PV selective regulatory element are used to treat a neuronal condition. Neuronal diseases or disorders appropriate for treatment include, but are not limited to, Dravet Syndrome, Alzheimer's disease, Parkinson's disease, Huntington's disease, amyotrophic lateral sclerosis (ALS), spinal muscular atrophy (SMA), epilepsy, neurodegenerative disorders, motor disorders, movement disorders, mood disorders, motor neuron diseases, progressive muscular atrophy (PMA), progressive bulbar palsy, pseudobulbar palsy, primary lateral sclerosis, neurological consequences of AIDS, developmental disorders, multiple sclerosis, neurogenetic disorders, stroke, spinal cord injury, traumatic brain injury, tauopathy, neuronal hypoexcitability and/or seizures. In some embodiments, a viral vector, viral particle or pharmaceutical composition comprising a transgene and a PV selective microRNA binding site and optionally a PV selective regulatory element are used to treat a psychiatric disorder (e.g., schizophrenia, obsessive compulsive disorder, addiction, depression, anxiety, psychosis); an autism spectrum disorder (e.g., Fragile X syndrome, Rett syndrome); epilepsy (e.g., Dravet syndrome, chronic traumatic encephalopathy, generalized epilepsy with febrile seizures plus (GEFS+), epileptic encephalopathy, temporal lobe epilepsy, focal epilepsy, tuberous sclerosis, epilepsy associated with SCN1A haploinsufficiency); and/or neurodegeneration (e.g., Alzheimer's disease, Parkinson's disease). Diseases associated with dysfunctional PV neurons such as those due to loss of function mutations in SCN1A or Nav1.1 include: Dravet syndrome, Ohtahara syndrome, epilepsy, early infantile epileptic encephalopathy 6 (EIEE6), familial febrile seizures 3A (FEB3A), intractable childhood epilepsy with generalized tonic-clonic seizures (ICEGTC), migraine, familial hemiplegic 3 (FHM3), Panayiotopoulos syndrome, familial atrial fibrillation 13 (ATFB13), generalized epilepsy with febrile seizures plus type 1 (gefs+ type 1), Brugada syndrome, nonspecific cardiac conduction defect, generalized epilepsy with febrile seizures plus, benign familial infantile seizures, early infantile epileptic encephalopathy11 (EIEE11), benign familial infantile epilepsy, neurodegeneration, tauopathies and Alzheimer's disease.

In certain embodiments, treatment using an expression cassette, an expression vector, or a viral particle comprising a transgene and a PV selective microRNA binding site and optionally a PV selective regulatory element described herein results in improved symptoms associated with a neuronal disease or disorder. For instance, a Parkinson's patient can be monitored symptomatically for improved motor functions indicating positive response to treatment. Administration of a therapy using a method as described herein to a subject at risk of developing a neuronal disorder can prevent the development of or slow the progression of one or more symptoms.

In certain embodiments, an expression cassette, an expression vector, or a viral particle comprising a transgene and a PV selective microRNA binding site and optionally a PV selective regulatory element provided herein can be used to treat a subject who has been diagnosed with a neuronal disease, for example, epilepsy associated with SCN1A haploinsufficiency such as, for example, Dravet syndrome. In various embodiments, any of the neuronal diseases or disorders disclosed herein are caused by a known genetic event (*e.g.,* any of the *SCN1A* mutations known in the art) or have an unknown cause.

In certain embodiments, an expression cassette, an expression vector, or a viral particle comprising a transgene and a PV selective microRNA binding site and optionally a PV selective regulatory element provided herein can be used to treat a subject who is at risk of developing a disease or disorder. In some embodiments, the subject can be known to be predisposed to a disease, for example, a neuronal disease (e.g. epilepsy associated with SCN1A haploinsufficiency such as, for example, Dravet syndrome). In some embodiments, the subject can be predisposed to a disease due to a genetic event, or due to known risk factors. For example, a subject can carry a mutation in *SCN1A* which is associated with epilepsy or Dravet syndrome, e.g., an insertion, deletion, inversion, translocation, or substitution (e.g., a point mutation including a nonsense mutation and/or a missense mutation).

In certain embodiments, an expression cassette, an expression vector, or a viral particle comprising a transgene and a PV selective microRNA binding site and optionally a PV selective regulatory element provided herein can be used to reduce one or more symptoms associated with a disease or disorder. For example, symptoms associated with Dravet syndrome include seizures, memory defects, developmental delay, poor muscle tone and/or cognitive problems. Treatment with a viral vector, viral particle or pharmaceutical composition comprising a transgene and a PV selective microRNA binding site and optionally a PV selective regulatory element provided herein can result in an improvement of one or more symptoms, such as a reduction in number, duration, and/or intensity of seizures.

In certain embodiments, the methods described herein are used for increasing expression of a transgene in a PV neuron, gene therapy, or treating a disease or disorder in a primate. In certain embodiments, the primate is a human. In certain embodiments, the primate is a non-human primate. In certain embodiments, the non-human primate is an old world monkey, an orangutan, a gorilla, a chimpanzee, a crab-eating macaque, a rhesus macaque or a pig-tailed macaque.

The terms "subject" and "individual" are used interchangeably herein to refer to a vertebrate, preferably a mammal, more preferably a human. The methods described herein can be useful in human therapeutics, veterinary applications, and/or preclinical studies in animal models of a disease or condition. In various embodiments, a subject that can be treated in accordance with the methods described herein is a mammal, such as, for example, a mouse, rat, hamster, guinea pig, gerbil, cow, sheep, pig, goat, donkey, horse, dog, cat, llama, monkey (e.g., an old world monkey, a marmoset, or a macaque such as a Rhesus macaque, a pig-tailed macaque or a crab-eating macaque (i.e., a cynomolgus monkey)), ape (e.g., an orangutan, gorilla or chimpanzee) or human. In an exemplary embodiment, a subject is a human.

The following tables provide sequences disclosed herein.

**TABLE 1.** Exemplary engineered transcription factors disclosed herein. Sequences of the regulatory elements (RE) are disclosed below in **TABLES 2** and **8.** For the RE, when m1 is indicated it means that the m1 microRNA binding site (SEQ ID NO: 7, **TABLE 8)** is included between the coding region and the polyA tail. Sequences for the DNA binding domains (DBD) are disclosed below in **TABLE 3.** For the DBD, engineered zinc finger (eZF) indicates that the construct has the formula set forth in SEQ ID NO: 147 **(TABLE 10);** EGR1 indicates that the DBD is derived from wild-type human EGR1 (SEQ ID NO: 176; **TABLE 12);** and EGR3 indicates that the DBD is derived from wild-type human EGR3 (SEQ ID NO: 175, **TABLE 12).** Sequences for the target sites (e.g., the sequences bound by the DBDs) are provided in **TABLE 4** below. Sequences for the transcriptional activation domains (TAD) are disclosed below in **TABLE 5.** For the TAD, (c) indicates that the TAD is located at the c-terminus of the DBD, (n) indicates that the TAD is located at the n-terminus of the DBD, (n/c) means that there is a TAD located at both the n-terminus and c-terminus of the DBD, and 2x CITED4 (n) indicates that there are 2 copies of the CITED4 TAD located at the n-terminus of the DBD. Sequences for the full length engineered transcription factors (DBD + TAD) are provided in **TABLE 6** below.

| Construct | RE | DBD | Target Site | TAD (location) | SEQ ID NO (DBD) | SEQ ID NO (DBD + TAD) |
|---|---|---|---|---|---|---|
| 1 | RE 1 | eZF | Z13 | VPR (c) | 89 | 99 |
| 2 | RE 1 | eZF | Z1 | VPR (c) | 77 | 100 |
| 3 | RE 1 | eZF | Z13 | VP64 (c) | 89 | 101 |
| 4 | RE 1 | eZF | Z1 | VP64 (c) | 77 | 102 |
| 5 | RE 1 | EGR1 | Z13 | CITED4 (n/c) | 93 | 103 |
| 6 | RE 1 | EGR1 | Z13 | CITED4 (n) | 93 | 104 |
| 7 | RE 1 | EGR1 | Z1 | CITED4 (n/c) | 92 | 105 |
| 8 | RE 1 | EGR1 | Z1 | CITED4 (n) | 92 | 106 |
| 9 | RE 1 | EGR1 | Z1 | CITED4 (c) | 92 | 107 |
| 10 | RE 1 | EGR1 | Z1 | CITED2 (c) | 92 | 108 |
| 11 | RE 1 | EGR1 | Z1 | CITED2 (n) | 92 | 109 |
| 12 | RE 1 | EGR3 | Z1 | CITED4 (n/c) | 96 | 110 |
| 13 | RE 1 | EGR3 | Z1 | CITED4 (c) | 96 | 111 |
| 14 | RE 1 | EGR3 | Z1 | CITED2 (c) | 96 | 112 |
| 15 | RE 1 | EGR3 | Z1 | CITED2 (n) | 96 | 113 |
| 16 | CBA | EGR3 | Z15 | N/A | 98 | 114 |
| 17 | RE 1 | EGR1 | Z13 | N/A | 93 | 115 |
| 18 | RE 1 | EGR1 | Z15 | N/A | 94 | 116 |
| 19 | RE 1 | EGR1 | Z13 | N/A | 93 | 117 |
| 20 | RE 1 | EGR1 | Z13 | N/A | 93 | 115 |
| 21 | RE 1 | EGR1 | Z1 | N/A | 92 | 118 |
| 22 | CBA | EGR3 | Z13 | N/A | 97 | 119 |
| 23 | RE 1 | EGR1 | Z17 | N/A | 95 | 120 |
| 24 | RE 1 | EGR1 | Z13 | N/A | 93 | 121 |
| 25 | CBA | EGR3 | Z1 | N/A | 96 | 122 |
| 26 | RE 1 | EGR1 | Z1 | N/A | 92 | 123 |
| 27 | RE 1 | EGR1 | Z1 | N/A | 92 | 124 |
| 28 | RE 1 | eZF | Z8 | VP64 (c) | 84 | 125 |
| 29 | RE 1 | eZF | Z14 | VP64 (c) | 90 | 126 |
| 30 | CBA | eZF | Z13 | VPR (c) | 89 | 99 |
| 31 | RE 2 (m1) | eZF | Z1 | VP64 (c) | 77 | 102 |

(continued)

| Construct | RE | DBD | Target Site | TAD (location) | SEQ ID NO (DBD) | SEQ ID NO (DBD + TAD) |
|---|---|---|---|---|---|---|
| 32 | RE 2 | eZF | Z1 | VP64 (c) | 77 | 102 |
| 33 | RE 2 | eZF | Z1 | VPR (c) | 77 | 100 |
| 34 | RE 2 | eZF | Z1 | VP64 (c) | 77 | 127 |
| 35 | RE 2 (m1) | eZF | Z1 | VP64 (c) | 77 | 127 |
| 36 | RE 2 | EGR1 | Z1 | CITED4 (n) | 92 | 128 |
| 37 | RE 2 (m1) | EGR1 | Z1 | CITED4 (n) | 92 | 106 |
| 38 | RE 2 (m1) | EGR1 | Z1 | CITED4 (n) | 92 | 129 |
| 39 | RE 2 (m1) | EGR1 | Z1 | CITED4 (n/c) | 92 | 105 |
| 40 | RE 2 (m1) | EGR1 | Z1 | 2x CITED4 (n) | 92 | 130 |
| 41 | RE 2 (m1) | EGR1 | Z1 | 2x CITED4 (n) | 92 | 131 |
| 42 | RE 2 (m2) | eZF | Z1 | VP64 (c) | 77 | 102 |
| 43 | RE 2 (m3) | eZF | Z1 | VP64 (c) | 77 | 102 |
| 44 | RE 2 (m2) | EGR1 | Z1 | CITED4 (n) | 92 | 106 |
| 45 | RE 2 (m3) | EGR1 | Z1 | CITED4 (n) | 92 | 106 |
| 46 | RE 1 | EGR1 | Z1 | CITED4 (n) | 92 | 205 |
| 47 | RE 1 | EGR1 | Z1 | 2x CITED4 (n) | 92 | 207 |
| 48 | RE 1 | EGR1 | Z1 | 2x CITED4 (n) | 92 | 209 |
| 49 | CBA | eZF | Z1 | CREB3 (n) | 77 | 213 |
| 50 | CBA | EGR1 | Z1 | CREB3 (n) | 92 (without C-term Lys) | 217 |
| 51 | CBA | EGR1 | Z13 | CREB3 (n) | 93 (without C-term Lys) | 219 |
| 52 | CBA | eZF | Z1 | CREB3 (n); no TM domain at (c) | 77 | 221 |
| 53 | CBA | EGR1 | Z1 | CREB3 (n); no TM domain at (c) | 92 (without C-term Lys) | 223 |

TABLE 2. Nucleic acid sequences for various regulatory elements (RE) disclosed herein.

| RE | SEQUENCE | SEQ ID NO |
|---|---|---|
| RE 1 | GTGATGCGGTTTTGGCAGTACATCAATGGGCGTGGATAG CGGTTTGACTCACGGGGATTTCCAAGTCTCCACCCCATTG ACGTCAATGGGAGTTTGTTTTGGCACCAAAATCAACGGG ACTTTCCAAAATGTCGTAACAACTCCGCCCCATTGACGCA AATGGGCGGTAGGCGTGTACGGTGGGAGGTCTATATAAG CAGAGCTGGTACCGTGTGTATGCTCAGGGGCTGGGAAAG GAGGGGAGGGAGCTCCGGCTCAG | 1 |

(continued)

| RE | SEQUENCE | SEQ ID NO |
|---|---|---|
| RE 2 | ggaggaagccatcaactaaactacaatgactgtaagatacaaaattgggaatggtaacatatttt gaagttctgttgacataaagaatcatgatattaatgcccatggaaatgaaagggcgatcaacact atggtttgaaaagggggaaattgtagagcacagatgtgttcgtgtggcagtgtgctgtctctagc aatactcagagaagagagagaacaatgaaattctgattggccccagtgtgagcccagatgagg ttcagctgccaactttctctttcacatcttatgaaagtcatttaagcacaactaactttttttttttttttttttt tttttgagacagagtcttgctctgttgcccaggacagagtgcagtagtgactcaatctcggctcac tgcagcctccacctcctaggctcaaacggtcctcctgcatcagcctcccaagtagctggaattac aggagtggcccaccatgcccagctaattttttgtatttttaatagatacggggggtttcaccatatcac ccaggctggtctcgaactcctggcctcaagtgatccacctgcctcggcctcccaaagtgctggg attataggcgtcagccactatgcccaacccgaccaaccttttttaaaataaatatttaaaaaattgg tatttcacatatatactagtatttacatttatccacacaaaacggacgggcctccgctgaaccagtg aggccccagacgtgcgcataaataacccctgcgtgctgcaccacctggggagagggggagg accacggtaaatggagcgagcgcatagcaaaagggacgcggggtcctttttctctgccggtgg cactgggtagctgtggccaggtgtggtactttgatggggcccagggctggagctcaaggaag cgtcgcagggtcacagatctggggggaaccccggggaaaagcactgaggcaaaaccgccgc tcgtctcctacaatatatgggagggggaggttgagtacgttctggattactcataagaccttttttttt ttccttccgggcgcaaaaccgtgagctggatttataatcgccctataaagctccagaggcggtc aggcacctgcagaggagccccgccgctccgccgactagctgcccccgcgagcaacggcct cgtgatttccccgccgatccggtccccgcctcccactctgcccccgcctacccccggagccgt gcagccgcctctccgaatctctctcttctcctggcgctcgcgtgcgagagggaactagcgaga acgaggaagcagctggaggtgacgccgggcagattacgcctgtcagggccgagccgagcg gatcgctgggcgctgtgcagaggaaaggcgggagtgcccggctcgctgtcgcagagccga ggtgggtaagctagcgaccacctggacttcccagcgcccaaccgtggcttttcagccaggtcc tctcctcccgcggcttctcaaccaaccccatcccagcgccggccacccaacctcccgaaatga gtgcttcctgccccagcagccgaaggcgctactaggaacggtaacctgttacttttccaggggc cgtagtcgacccgctgcccgagttgctgtgcgactgcgcgcgcggggctagagtgcaaggtg actgtggttcttctctggccaagtccgagggagaacgtaaagatatgggcctttttcccctctca ccttgtctcaccaaagtccctagtccccggagcagttagcctctttctttccagggaattagccag acacaacaacgggaaccagacaccgaaccagacatgcccgccccgtgcgccctccccgctc gctgcctttcctccctcttgtctctccagagccggatcttcaaggggagcctccgtgcccccggc tgctcagtccctccggtgtgcaggaccccggaagtcctccccgcacagctctcgcttctctttgc agcctgtttctgcgccggaccagtcgaggactctggacagtagaggccccgggacgaccga gctg | 2 |

(continued)

| RE | SEQUENCE | SEQ ID NO |
|---|---|---|
| RE 3 | TCAACAGGGGGACACTTGGGAAAGAAGGATGGGGACAG AGCCGAGAGGACTGTTACACATTAGAGAAACATCAGTGA CTGTGCCAGCTTTGGGGTAGACTGCACAAAAGCCCTGAG GCAGCACAGGCAGGATCCAGTCTGCTGGTCCCAGGAAGC TAACCGTCTCAGACAGAGCACAAAGCACCGAGACATGTG CCACAAGGCTTGTGTAGAGAGGTCAGAGGACAGCGTACA GGTCCCAGAGATCAAACTCAACCTCACCAGGCTTGGCAG CAAGCCTTTACCAACCCACCCCCACCCCACCCACCCTGCA CGCGCCCCTCTCCCCTCCCCATGGTCTCCCATGGCTATCT CACTTGGCCCTAAAATGTTTAAGGATGACACTGGCTGCTG AGTGGAAATGAGACAGCAGAAGTCAACAGTAGATTTTAG GAAAGCCAGAGAAAAGGCTTGTGCTGTTTTTAGAAAGC CAAGGGACAAGCTAAGATAGGGCCCAAGTAATGCTAGTA TTTACATTTATCCACACAAAACGGACGGGCCTCCGCTGAA CCAGTGAGGCCCCAGACGTGCGCATAAATAACCCCTGCG TGCTGCACCACCTGGGGAGAGGGGGAGGACCACGGTAAA TGGAGCGAGCGCATAGCAAAAGGGACGCGGGGTCCTTTT CTCTGCCGGTGGCACTGGGTAGCTGTGGCCAGGTGTGGT ACTTTGATGGGGCCCAGGGCTGGAGCTCAAGGAAGCGTC GCAGGGTCACAGATCTGGGGGAACCCCGGGGAAAAGCA CTGAGGCAAAACCGCCGCTCGTCTCCTACAATATATGGG AGGGGGAGGTTGAGTACGTTCTGGATTACTCATAAGACC TTTTTTTTTTCCTTCCGGGCGCAAAACCGTGAGCTGGATTT ATAATCGCCCTATAAAGCTCCAGAGGCGGTCAGGCACCT GCAGAGGAGCCCCGCCGCTCCGCCGACTAGCTGCCCCCG CGAGCAACGGCCTCGTGATTTCCCCGCCGATCCGGTCCCC GCCTCCCCACTCTGCCCCCGCCTACCCCGGAGCCGTGCAG CCGCCTCTCCGAATCTCTCTCTTCTCCTGGCGCTCGCGTGC GAGAGGGAACTAGCGAGAACGAGGAAGCAGCTGGAGGT GACGCCGGGCAGATTACGCCTGTCAGGGCCGAGCCGAGC GGATCGCTGGGCGCTGTGCAGAGGAAAGGCGGGAGTGCC CGGCTCGCTGTCGCAGAGCCGAGGTGGGTAAGCTAGCGA CCACCTGGACTTCCCAGCGCCCAACCGTGGCTTTTCAGCC AGGTCCTCTCCTCCCGCGGCTTCTCAACCAACCCCATCCC AGCGCCGGCCACCCAACCTCCCGAAATGAGTGCTTCCTG CCCCAGCAGCCGAAGGCGCTACTAGGAACGGTAACCTGT TACTTTTCCAGGGGCCGTAGTCGACCCGCTGCCCGAGTTG CTGTGCGACTGCGCGCGCGGGGCTAGAGTGCAAGGTGAC TGTGGTTCTTCTCTGGCCAAGTCCGAGGGAGAACGTAAA GATATGGGCCTTTTTCCCCCTCTCACCTTGTCTCACCAAA GTCCCTAGTCCCCGGAGCAGTTAGCCTCTTTCTTTCCAGG GAATTAGCCAGACACAACAACGGGAACCAGACACCGAA | 3 |

(continued)

| RE | SEQUENCE | SEQ ID NO |
|---|---|---|
| | CCAGACATGCCCGCCCCGTGCGCCCTCCCCGCTCGCTGCC TTTCCTCCCTCTTGTCTCTCCAGAGCCGGATCTTCAAGGG GAGCCTCCGTGCCCCGGCTGCTCAGTCCCTCCGGTGTGC AGGACCCCGGAAGTCCTCCCCGCACAGCTCTCGCTTCTCT TTGCAGCCTGTTTCTGCGCCGGACCAGTCGAGGACTCTGG ACAGTAGAGGCCCCGGGACGACCGAGCTG | |
| RE 4 | GCCCTCTAGGCCACCTGACCAGGTCCCCTCAGTCCCCCCC TTCCCACACTCCCACACTCAGCCCCCCTCCCCCCCCCCCG ACCCCTGCAGGATTATCCTGTCTGTGTTCCTGACTCAGCC TGGGAGCCACCTGGGCAGCAGGGGCCAAGGGTGTCCTAG AAGGGACCTGGAGTCCACGCTGGGCCAAGCCTGCCCTTT CTCCCTCTGTCTTCCGTCCCTGCTTGCGGTTCTGCTGAATG TGGTTATTTCTCTGGCTCCTTTTACAGAGAATGCTGCTGCT AATTTTATGTGGAGCTCTGAGGCAGTGTAATTGGAAGCC AGACACCCTGTCAGCAGTGGGCTCCCGTCCTGAGCTGCC ATGCTTCCTGCTCTCCTCCCGTCCCGGCTCCTCATTTCATG CAGCCACCTGTCCCAGGGAGAGAGGAGTCACCCAGGCCC CTCAGTCCGCCCCTTAAATAAGAAAGCCTCCGTTGCTCGG CACACATACCAAGCAGCCGCTGGTGCAATCT | 4 |

(continued)

| RE | SEQUENCE | SEQ ID NO |
|---|---|---|
| CBA (CMV enhancer + Chicken beta actin promoter) | CGTTACATAACTTACGGTAAATGGCCCGCCTGGCTGACCG CCCAACGACCCCCGCCCATTGACGTCAATAATGACGTAT GTTCCCATAGTAACGCCAATAGGGACTTTCCATTGACGTC AATGGGTGGAGTATTTACGGTAAACTGCCCACTTGGCAG TACATCAAGTGTATCATATGCCAAGTACGCCCCCTATTGA CGTCAATGACGGTAAATGGCCCGCCTGGCATTATGCCCA GTACATGACCTTATGGGACTTTCCTACTTGGCAGTACATC TACGTATTAGTCATCGCTATTACCATGggtcgaggtgagccccacgtt ctgcttcactctccccatctccccccccctccccaccccccaattttgtatttatttattttttaattattttg tgcagcgatggggggcggggggggggggggcgcgcgccaggcggggcggggcggggcg aggggcggggcggggcgaggcggagaggtgcggcggcagccaatcagagcggcgcgct ccgaaagtttccttttatggcgaggcggcggcggcggcggcccctataaaaagcgaagcgcgc ggcgggcgggagtcgctgcgttgccttcgccccgtgccccgctccgcgccgcctcgcgccg cccgcccccggctctgactgaccgcgttactcccacaggtgagcgggcgggacggcccttctc ctccgggctgtaattagcgcttggtttaatgacggctcgtttcttttctgtggctgcgtgaaagcctt aaagggctccgggagggccctttgtgcggggggggagcggctcggggggtgcgtgcgtgtgt gtgtgcgtggggagcgccgcgtgcggcccgcgctgcccggcggctgtgagcgctgcgggc gcggcgcggggctttgtgcgctccgcgtgtgcgcgaggggagcgcggccggggggcggtgc cccgcggtgcggggggggctgcgaggggaacaaaggctgcgtgcggggtgtgtgcgtgggg gggtgagcagggggtgtgggcgcggcggtcgggctgtaacccccccctgcacccccctccc cgagttgctgagcacggcccggcttcgggtgcggggctccgtgcggggcgtggcgcggggg ctcgccgtgccgggcggggggtggcggcaggtggggggtgccgggcggggcggggccgc ctcgggccggggagggctcggggggaggggcgcggcggccccggagcgccggcggctgt |  |
|  | cgaggcgcggcgagccgcagccattgccttttatggtaatcgtgcgagagggcgcagggact tcctttgtcccaaatctggcggagccgaaatctgggaggcgccgccgcaccccctctagcggg cgcgggcgaagcggtgcggcgccggcaggaaggaaatgggcggggagggccttcgtgcg tcgccgcgccgccgtccccttctccatctccagcctcggggctgccgcaggggggacggctgc cttcggggggggacggggcagggcgggggttcggcttctggcgtgtgaccggcggctctagag cctctgctaaccatgttcatgccttcttcttttttcctacagctcctgggcaacgtgctggttgttgtgc tgtctcatcattttggcaaagaatt |  |

(continued)

| RE | SEQUENCE | SEQ ID NO |
|---|---|---|
| EF1alpha | GAGTAATTCATACAAAAGGACTCGCCCCTGCCTTGGGGA ATCCCAGGGACCGTCGTTAAACTCCCACTAACGTAGAAC CCAGAGATCGCTGCGTTCCCGCCCCCTCACCCGCCCGCTC TCGTCATCACTGAGGTGGAGAAGAGCATGCGTGAGGCTC CGGTGCCCGTCAGTGGGCAGAGCGCACATCGCCCACAGT CCCCGAGAAGTTGGGGGGAGGGGTCGGCAATTGAACCGG TGCCTAGAGAAGGTGGCGCGGGGTAAACTGGGAAAGTGA TGTCGTGTACTGGCTCCGCCTTTTTCCCGAGGGTGGGGGA GAACCGTATATAAGTGCAGTAGTCGCCGTGAACGTTCTTT TTCGCAACGGGTTTGCCGCCAGAACACAGGTAAGTGCCG TGTGTGGTTCCCGCGGGCCTGGCCTCTTTACGGGTTATGG CCCTTGCGTGCCTTGAATTACTTCCACGCCCCTGGCTGCA GTACGTGATTCTTGATCCCGAGCTTCGGGTTGGAAGTGGG TGGGAGAGTTCGAGGCCTTGCGCTTAAGGAGCCCCTTCG CCTCGTGCTTGAGTTGAGGCCTGGCTTGGGCGCTGGGGCC GCCGCGTGCGAATCGGTGGCACCTTCGCGCCTGTCTCGC TGCTTTCGATAAGTCTCTAGCCATTTAAAATTTTTGATGA CCTGCTGCGACGCTTTTTTTCTGGCAAGATAGTCTTGTAA ATGCGGGCCAAGATCTGCACACTGGTATTTCGGTTTTTGG GGCCGCGGGCGGCGACGGGGCCCGTGCGTCCCAGCGCAC ATGTTCGGCGAGGCGGGGCCTGCGAGCGCGGCCACCGAG AATCGGACGGGGGTAGTCTCAAGCTGGCCGGCCTGCTCT GGTGCCTGGCCTCGCGCCGCCGTGTATCGCCCCGCCCTGG GCGGCAAGGCTGGCCCGGTCGGCACCAGTTGCGTGAGCG GAAAGATGGCCGCTTCCCGGCCCTGCTGCAGGGAGCTCA AAATGGAGGACGCGGCGCTCGGGAGAGCGGGCGGGTGA GTCACCCACACAAAGGAAAAGGGCCTTTCCGTCCTCAGC CGTCGCTTCATGTGACTCCACGGAGTACCGGGCGCCGTCC AGGCACCTCGATTAGTTCTCGAGCTTTTGGAGTACGTCGT CTTTAGGTTGGGGGGAGGGGTTTTATGCGATGGAGTTTCC CCACACTGAGTGGGTGGAGACTGAAGTTAGGCCAGCTTG GCACTTGATGTAATTCTCCTTGGAATTTGCCCTTTTTGAGT TTGGATCTTGGTTCATTCTCAAGCCTCAGACAGTGGTTCA AAGTTTTTTTCTTCCATTTCAGGTGTCGTGA | 6 |

**TABLE 3.** Amino acid sequences for exemplary DNA Binding Domains (DBD) provided herein. For the DBD, engineered zinc finger (eZF) indicates that the construct has the formula set forth in SEQ ID NO: 147 **(TABLE 10);** EGR1 indicates that the DBD is derived from wild-type human EGR1 (SEQ ID NO: 176; **TABLE 12);** and EGR3 indicates that the DBD is derived from wild-type human EGR3 (SEQ ID NO: 175, **TABLE 12).** The target sites are the sequences bound by the DBD and are provided in **TABLE 4** below.

| DBD/Target site | SEQUENCE | SEQ ID NO |
|---|---|---|
| eZF/z1 | LEPGEKPYKCPECGKSFSRSDNLVRHQRTHTGEKPYKCPECGKSFSREDNLHTHQRTHTGEKPYKCPECGKSFSRSDELVRHQRTHTGEKPYKCPECGKSFSQSGNLTEHQRTHTGEKPYKCPECGKSFSTSGHLVRHQRTHTGEKPYKCPECGKSFSQNSTLTEHQRTHTGKKTS | 77 |
| eZF/z2 | LEPGEKPYKCPECGKSFSTKNSLTEHQRTHTGEKPYKCPECGKSFSRADNLTEHQRTHTGEKPYKCPECGKSFSQLAHLRAHQRTHTGEKPYKCPECGKSFSTKNSLTEHQRTHTGEKPYKCPECGKSFSQAGHLASHQRTHTGEKPYKCPECGKSFSTHLDLIRHQRTHTGKKTS | 78 |
| eZF/z3 | LEPGEKPYKCPECGKSFSQAGHLASHQRTHTGEKPYKCPECGKSFSREDNLHTHQRTHTGEKPYKCPECGKSFSTSGNLTEHQRTHTGEKPYKCPECGKSFSTHLDLIRHQRTHTGEKPYKCPECGKSFSQKSSLIAHQRTHTGEKPYKCPECGKSFSQAGHLASHQRTHTGKKTS | 79 |
| eZF/z4 | LEPGEKPYKCPECGKSFSTTGNLTVHQRTHTGEKPYKCPECGKSFSTSGELVRHQRTHTGEKPYKCPECGKSFSREDNLHTHQRTHTGEKPYKCPECGKSFSTSGNLTEHQRTHTGEKPYKCPECGKSFSQSSSLVRHQRTHTGEKPYKCPECGKSFSQRANLRAHQRTHTGKKTS | 80 |
| eZF/z5 | LEPGEKPYKCPECGKSFSSRRTCRAHQRTHTGEKPYKCPECGKSFSTTGALTEHQRTHTGEKPYKCPECGKSFSRSDELVRHQRTHTGEKPYKCPECGKSFSRNDALTEHQRTHTGEKPYKCPECGKSFSQSGDLRRHQRTHTGEKPYKCPECGKSFSTSHSLTEHQRTHTGKKTS | 81 |
| eZF/z6 | LEPGEKPYKCPECGKSFSRKDNLKNHQRTHTGEKPYKCPECGKSFSDPGALVRHQRTHTGEKPYKCPECGKSFSREDNLHTHQRTHTGEKPYKCPECGKSFSDPGALVRHQRTHTGEKPYKCPECGKSFSTSGELVRHQRTHTGEKPYKCPECGKSFSRKDNLKNHQRTHTGKKTS | 82 |
| eZF/z7 | LEPGEKPYKCPECGKSFSSKKALTEHQRTHTGEKPYKCPECGKSFSSPADLTRHQRTHTGEKPYKCPECGKSFSRSDNLVRHQRTHTGEKPYKCPECGKSFSREDNLHTHQRTHTGEKPYKCPECGKSFSRSDELVRHQRTHTGEKPYKCPECGKSFSQSGNLTEHQRTHTGEKPYKCPECGKSFSTSGHLVRHQRTHTGEKPYKCPECGKSFSQNSTLTEHQRTHTGKKTS | 83 |

(continued)

| DBD/Target site | SEQUENCE | SEQ ID NO |
|---|---|---|
| eZF/z8 | LEPGEKPYKCPECGKSFSSPADLTRHQRTHTGEKPYKCPEC GKSFSRSDNLVRHQRTHTGEKPYKCPECGKSFSREDNLHT HQRTHTGEKPYKCPECGKSFSRSDELVRHQRTHTGEKPYK CPECGKSFSQSGNLTEHQRTHTGEKPYKCPECGKSFSTSGH LVRHQRTHTGKKTS | 84 |
| eZF/z9 | LEPGEKPYKCPECGKSFSSKKALTEHQRTHTGEKPYKCPEC GKSFSSPADLTRHQRTHTGEKPYKCPECGKSFSRSDNLVRH QRTHTGEKPYKCPECGKSFSREDNLHTHQRTHTGEKPYKC PECGKSFSRSDELVRHQRTHTGEKPYKCPECGKSFSQSGNL TEHQRTHTGKKTS | 85 |
| eZF/z10 | LEPGEKPYKCPECGKSFSDCRDLARHQRTHTGEKPYKCPEC GKSFSRNDALTEHQRTHTGEKPYKCPECGKSFSRNDALTE HQRTHTGEKPYKCPECGKSFSSPADLTRHQRTHTGEKPYK CPECGKSFSDPGNLVRHQRTHTGEKPYKCPECGKSFSQRA HLERHQRTHTGEKPYKCPECGKSFSQSSSLVRHQRTHTGEK PYKCPECGKSFSHRTTLTNHQRTHTGKKTS | 86 |
| eZF/z 11 | LEPGEKPYKCPECGKSFSRNDALTEHQRTHTGEKPYKCPEC GKSFSSPADLTRHQRTHTGEKPYKCPECGKSFSDPGNLVRH QRTHTGEKPYKCPECGKSFSQRAHLERHQRTHTGEKPYKC PECGKSFSQSSSLVRHQRTHTGEKPYKCPECGKSFSHRTTL TNHQRTHTGKKTS | 87 |
| eZF/z12 | LEPGEKPYKCPECGKSFSRNDALTEHQRTHTGEKPYKCPEC GKSFSDPGHLVRHQRTHTGEKPYKCPECGKSFSTSGELVRH QRTHTGEKPYKCPECGKSFSTHLDLIRHQRTHTGEKPYKCP ECGKSFSSKKALTEHQRTHTGEKPYKCPECGKSFSQLAHLR AHQRTHTGEKPYKCPECGKSFSRSDHLTNHQRTHTGKKTS | 88 |
| eZF/z13 | LEPGEKPYKCPECGKSFSRSDNLVRHQRTHTGEKPYKCPEC GKSFSHRTTLTNHQRTHTGEKPYKCPECGKSFSREDNLHTH QRTHTGEKPYKCPECGKSFSTSHSLTEHQRTHTGEKPYKCP ECGKSFSQSSSLVRHQRTHTGEKPYKCPECGKSFSREDNLH THQRTHTGKKTS | 89 |
| eZF/z14 | LEPGEKPYKCPECGKSFSDPGALVRHQRTHTGEKPYKCPEC GKSFSRSDNLVRHQRTHTGEKPYKCPECGKSFSQSGDLRR HQRTHTGEKPYKCPECGKSFSTHLDLIRHQRTHTGEKPYKC PECGKSFSTSGNLVRHQRTHTGEKPYKCPECGKSFSRSDNL VRHQRTHTGKKTS | 90 |

(continued)

| DBD/Target site | SEQUENCE | SEQ ID NO |
|---|---|---|
| eZF/z15 | LEPGEKPYKCPECGKSFSRRDELNVHQRTHTGEKPYKCPEC GKSFSRSDHLTNHQRTHTGEKPYKCPECGKSFSRSDDLVRH QRTHTGEKPYKCPECGKSFSRSDNLVRHQRTHTGEKPYKC PECGKSFSHRTTLTNHQRTHTGEKPYKCPECGKSFSREDNL HTHQRTHTGEKPYKCPECGKSFSTSHSLTEHQRTHTGEKPY KCPECGKSFSQSSSLVRHQRTHTGEKPYKCPECGKSFSRED NLHTHQRTHTGKKTS | 91 |
| EGR1/z1 | RPYACPVESCDRRFSRSDNLVRHIRIHTGQKPFQCRICMRNF SREDNLHTHIRTHTGEKPFACDICGRKFARSDELVRHTKIHL RQKDRPYACPVESCDRRFSQSGNLTEHIRIHTGQKPFQCRIC MRNFSTSGHLVRHIRTHTGEKPFACDICGRKFAQNSTLTEH TKIHLRQKDK | 92 |
| EGR1/z13 | RPYACPVESCDRRFSRSDNLVRHIRIHTGQKPFQCRICMRNF SHRTTLTNHIRTHTGEKPFACDICGRKFAREDNLHTHTKIHL RQKDRPYACPVESCDRRFSTSHSLTEHIRIHTGQKPFQCRIC MRNFSQSSSLVRHIRTHTGEKPFACDICGRKFAREDNLHTH TKIHLRQKDK | 93 |
| EGR1/z15 | RPYACPVESCDRRFSRRDELNVHIRIHTGQKPFQCRICMRN FSRSDHLTNHIRTHTGEKPFACDICGRKFARSDDLVRHTKIH LRQKDRPYACPVESCDRRFSRSDNLVRHIRIHTGQKPFQCRI CMRNFSHRTTLTNHIRTHTGEKPFACDICGRKFAREDNLHT HTKIHLRQKDRPYACPVESCDRRFSTSHSLTEHIRIHTGQKP FQCRICMRNFSQSSSLVRHIRTHTGEKPFACDICGRKFARED NLHTHTKIHLRQKD | 94 |
| EGr1/z17 | RPYACPVESCDRRFSDPGALVRHIRIHTGQKPFQCRICMRN FSRSDNLVRHIRTHTGEKPFACDICGRKFAQSGDLRRHTKI HLRQKDRPYACPVESCDRRFSTHLDLIRHIRIHTGQKPFQCR ICMRNFSTSGNLVRHIRTHTGEKPFACDICGRKFARSDNLV RHTKIHLRQKDRPYACPVESCDRRFSQSGHLTEHIRIHTGQ KPFQCRICMRNFSERSHLREHIRTHTGEKPFACDICGRKFAQ AGHLASHTKIHLRQKD | 95 |
| EGR3/z1 | RPHACPAEGCDRRFSRSDNLVRHLRIHTGHKPFQCRICMRS FSREDNLHTHIRTHTGEKPFACEFCGRKFARSDELVRHAKI HLKQKEHACPAEGCDRRFSQSGNLTEHLRIHTGHKPFQCRI CMRSFSTSGHLVRHIRTHTGEKPFACEFCGRKFAQNSTLTE HAKIHLKQKEK | 96 |
| EGR3/z13 | RPHACPAEGCDRRFSRSDNLVRHLRIHTGHKPFQCRICMRS FSHRTTLTNHIRTHTGEKPFACEFCGRKFAREDNLHTHAKI HLKQKEHACPAEGCDRRFSTSHSLTEHLRIHTGHKPFQCRI CMRSFSQSSSLVRHIRTHTGEKPFACEFCGRKFAREDNLHT HAKIHLKQKEK | 97 |

(continued)

| DBD/Target site | SEQUENCE | SEQ ID NO |
|---|---|---|
| EGR3/z15 | RPHACPAEGCDRRFSRRDELNVHLRIHTGHKPFQCRICMRS FSRSDHLTNHIRTHTGEKPFACEFCGRKFARSDDLVRHAKI HLKQKEHACPAEGCDRRFSRSDNLVRHLRIHTGHKPFQCRI CMRSFSHRTTLTNHIRTHTGEKPFACEFCGRKFAREDNLHT HAKIHLKQKEHACPAEGCDRRFSTSHSLTEHLRIHTGHKPF QCRICMRSFSQSSSLVRHIRTHTGEKPFACEFCGRKFAREDN LHTHAKIHLKQKEK | 98 |

**TABLE 4.** Target site sequences and chromosomal location for exemplary target sites bound by DNA binding domains disclosed herein.

| Chr 2 Start Position | Target Site Sequence | Target Site | SEQ ID NO for Target Site Sequence |
|---|---|---|---|
| 166149168 | CTAGGTCAAGTGTAGGAG | z1 | 18 |
| 166149158 | ACTTGACCTAGACAGCCT | z2 | 19 |
| 166073978 | TGAATAACTCATTAGTGA | z3 | 20 |
| 166073933 | AAAGTACATTAGGCTAAT | z4 | 21 |
| 166149199 | CCAGCACTGGTGCTTCGT | z5 | 22 |
| 166149176 | AAGGCTGTCTAGGTCAAG | z6 | 23 |
| 166149168 | CTAGGTCAAGTGTAGGAGACACAC | z7 | 24 |
| 166149165 | GGTCAAGTGTAGGAGACA | z8 | 25 |
| 166149162 | CAAGTGTAGGAGACACAC | z9 | 26 |
| 166149160 | AGTGTAGGAGACACACTGCTGGCC | z10 | 27 |
| 166149160 | AGTGTAGGAGACACACTG | z11 | 28 |
| 166149155 | AGGAGACACACTGCTGGCCTG | z12 | 29 |
| 166128025 | TAGGTACCATAGAGTGAG | z13 | 30 |
| 166127991 | GAGGATACTGCAGAGGTC | z14 | 31 |
| 166127999 | TAGGTACCATAGAGTGAGGCGAGGATG | z15 | 32 |
| 166127991 | ATAGAGTGAGGCGAGGATGAAGCCGAG | z16 | 33 |
| 166127974 | TGAAGCCGAGAGGATACTGCAGAGGTC | z17 | 34 |

**TABLE 5.** Amino acid sequences for exemplary transcriptional activation domains (TADs) disclosed herein.

| TAD | SEQUENCE | SEQ ID NO |
|---|---|---|
| VPR | DALDDFDLDMLGSDALDDFDLDMLGSDALDDFDLDMLGSDA LDDFDLDMLINSRSSGSPKKKRKVGSQYLPDTDDRHRIEEKRK RTYETFKSIMKKSPFSGPTDPRPPPRRIAVPSRSSASVPKPAPQPY PFTSSLSTINYDEFPTMVFPSGQISQASALAPAPPQVLPQAPAPA PAPAMVSALAQAPAPVPVLAPGPPQAVAPPAPKPTQAGEGTLS EALLQLQFDDEDLGALLGNSTDPAVFTDLASVDNSEFQQLLNQ GIPVAPHTTEPMLMEYPEAITRLVTGAQRPPDPAPAPLGAPGLP NGLLSGDEDFSSIADMDFSALLGSGSGSRDSREGMFLPKPEAGS AISDVFEGREVCQPKRIRPFHPPGSPWANRPLPASLAPTPTGPVH EPVGSLTPAPVPQPLDPAPAVTPEASHLLEDPDEETSQAVKALR EMADTVIPQKEEAAICGQMDLSHPPPRGHLDELTTTLESMTEDL NLDSPLTPELNEILDTFLNDECLLHAMHISTGLSIFDTSLF | 132 |
| VP64 | DALDDFDLDMLGSDALDDFDLDMLGSDALDDFDLDMLGSDA LDDFDLDML | 133 |
| CITED2 | MSGLEMADHMMAMNHGRFPDGTNGLHHHPAHRMGMGQFPS PHHHQQQQPQHAFNALMGEHIHYGAGNMNATSGIRHAMGPG TVNGGHPPSALAPAARFNNSQFMGPPVASQGGSLPASMQLQKL NNQYFNHHPYPHNHYMPDLHPAAGHQMNGTNQHFRDCNPKH SGGSSTPGGSGGSSTPGGSGSSSGGGAGSSNSGGGSGSGNMPAS VAHVPAAMLPPNVIDTDFIDEEVLMSLVIEMGLDRIKELPELWL GQNEFDFMTDFVCKQQPSRVSC | 134 |
| CITED4 | ADHLMLAEGYRLVQRPPSAAAAHGPHALRTLPPYAGPGLDSG LRPRGAPLGPPPPRQPGALAYGAFGPPSSFQPFPAVPPPAAGIAH LQPVATPYPGRAAAPPNAPGGPPGPQPAPSAAAPPPPAHALGG MDAELIDEEALTSLELELGLHRVRELPELFLGQSEFDCFSDLGS APPAGSVSC | 135 |
| CREB3 | MELELDAGDQDLLAFLLEESGDLGTAPDEAVRAPLDWALPLSE VPSDWEVDDLLCSLLSPPASLNILSSSNPCLVHHDHTYSLPRET VSMDLESESCRKEGTQMTPQHMEELAEQEIARLVLTDEEKSLL EKEGLILPETLPLTKTEEQILKRVR | 224 |

**TABLE 6.** Amino acid sequences for exemplary engineered transcription factors (DBD + TAD) disclosed herein.

| CONSTRUCT | SEQUENCE | SEQ ID NO |
|---|---|---|
| 1 | MAPKKKRKVGIHGVPAALEPGEKPYKCPECGKSFS RSDNLVRHQRTHTGEKPYKCPECGKSFSHRTTLTNH QRTHTGEKPYKCPECGKSFSREDNLHTHQRTHTGE KPYKCPECGKSFSTSHSLTEHQRTHTGEKPYKCPEC GKSFSQSSSLVRHQRTHTGEKPYKCPECGKSFSRED NLHTHQRTHTGKKTSKRPAATKKAGQAKKKKGSY PYDVPDYALEEASGSGRADALDDFDLDMLGSDALD DFDLDMLGSDALDDFDLDMLGSDALDDFDLDMLIN SRSSGSPKKKRKVGSQYLPDTDDRHRIEEKRKRTYE TFKSIMKKSPFSGPTDPRPPPRRIAVPSRSSASVPKPA PQPYPFTSSLSTINYDEFPTMVFPSGQISQASALAPAP PQVLPQAPAPAPAPAMVSALAQAPAPVPVLAPGPPQ AVAPPAPKPTQAGEGTLSEALLQLQFDDEDLGALLG NSTDPAVFTDLASVDNSEFQQLLNQGIPVAPHTTEP MLMEYPEAITRLVTGAQRPPDPAPAPLGAPGLPNGL LSGDEDFSSIADMDFSALLGSGSGSRDSREGMFLPK PEAGSAISDVFEGREVCQPKRIRPFHPPGSPWANRPL PASLAPTPTGPVHEPVGSLTPAPVPQPLDPAPAVTPE ASHLLEDPDEETSQAVKALREMADTVIPQKEEAAIC GQMDLSHPPPRGHLDELTTTLESMTEDLNLDSPLTP ELNEILDTFLNDECLLHAMHISTGLSIFDTSLF | 99 |
| 2 | MAPKKKRKVGIHGVPAALEPGEKPYKCPECGKSFS RSDNLVRHQRTHTGEKPYKCPECGKSFSREDNLHT HQRTHTGEKPYKCPECGKSFSRSDELVRHQRTHTGE KPYKCPECGKSFSQSGNLTEHQRTHTGEKPYKCPEC GKSFSTSGHLVRHQRTHTGEKPYKCPECGKSFSQNS TLTEHQRTHTGKKTSKRPAATKKAGQAKKKKGSYP YDVPDYALEEASGSGRADALDDFDLDMLGSDALD DFDLDMLGSDALDDFDLDMLGSDALDDFDLDMLIN SRSSGSPKKKRKVGSQYLPDTDDRHRIEEKRKRTYE TFKSIMKKSPFSGPTDPRPPPRRIAVPSRSSASVPKPA PQPYPFTSSLSTINYDEFPTMVFPSGQISQASALAPAP PQVLPQAPAPAPAPAMVSALAQAPAPVPVLAPGPPQ AVAPPAPKPTQAGEGTLSEALLQLQFDDEDLGALLG NSTDPAVFTDLASVDNSEFQQLLNQGIPVAPHTTEP MLMEYPEAITRLVTGAQRPPDPAPAPLGAPGLPNGL LSGDEDFSSIADMDFSALLGSGSGSRDSREGMFLPK PEAGSAISDVFEGREVCQPKRIRPFHPPGSPWANRPL PASLAPTPTGPVHEPVGSLTPAPVPQPLDPAPAVTPE ASHLLEDPDEETSQAVKALREMADTVIPQKEEAAIC GQMDLSHPPPRGHLDELTTTLESMTEDLNLDSPLTP ELNEILDTFLNDECLLHAMHISTGLSIFDTSLF | 100 |
| 3 | MAPKKKRKVGIHGVPAALEPGEKPYKCPECGKSFS RSDNLVRHQRTHTGEKPYKCPECGKSFSHRTTLTNH QRTHTGEKPYKCPECGKSFSREDNLHTHQRTHTGE KPYKCPECGKSFSTSHSLTEHQRTHTGEKPYKCPEC | 101 |

(continued)

| CONSTRUCT | SEQUENCE | SEQ ID NO |
|---|---|---|
| | GKSFSQSSSLVRHQRTHTGEKPYKCPECGKSFSRED NLHTHQRTHTGKKTSKRPAATKKAGQAKKKKGSY PYDVPDYALEDALDDFDLDMLGSDALDDFDLDML GSDALDDFDLDMLGSDALDDFDLDML | |
| 4 | MAPKKKRKVGIHGVPAALEPGEKPYKCPECGKSFS RSDNLVRHQRTHTGEKPYKCPECGKSFSREDNLHT HQRTHTGEKPYKCPECGKSFSRSDELVRHQRTHTGE KPYKCPECGKSFSQSGNLTEHQRTHTGEKPYKCPEC GKSFSTSGHLVRHQRTHTGEKPYKCPECGKSFSQNS TLTEHQRTHTGKKTSKRPAATKKAGQAKKKKGSYP YDVPDYALEDALDDFDLDMLGSDALDDFDLDMLG SDALDDFDLDMLGSDALDDFDLDML | 102 |
| 5 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGQSQLIKPSRMRKYPNRPSKTPPHE RPYACPVESCDRRFSRSDNLVRHIRIHTGQKPFQCRI CMRNFSHRTTLTNHIRTHTGEKPFACDICGRKFARE DNLHTHTKIHLRQKDRPYACPVESCDRRFSTSHSLT EHIRIHTGQKPFQCRICMRNFSQSSSLVRHIRTHTGE KPFACDICGRKFAREDNLHTHTKIHLRQKDKLEMA DHLMLAEGYRLVQRPPSAAAAHGPHALRTLPPYAG PGLDSGLRPRGAPLGPPPPRQPGALAYGAFGPPSSFQ PFPAVPPPAAGIAHLQPVATPYPGRAAAPPNAPGGP PGPQPAPSAAAPPPPAHALGGMDAELIDEEALTSLE LELGLHRVRELPELFLGQSEFDCFSDLGSAPPAGSVS C | 103 |
| 6 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGQSQLIKPSRMRKYPNRPSKTPPHE RPYACPVESCDRRFSRSDNLVRHIRIHTGQKPFQCRI CMRNFSHRTTLTNHIRTHTGEKPFACDICGRKFARE DNLHTHTKIHLRQKDRPYACPVESCDRRFSTSHSLT EHIRIHTGQKPFQCRICMRNFSQSSSLVRHIRTHTGE KPFACDICGRKFAREDNLHTHTKIHLRQKDK | 104 |

(continued)

| CONSTRUCT | SEQUENCE | SEQ ID NO |
|---|---|---|
| 7 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGQSQLIKPSRMRKYPNRPSKTPPHE RPYACPVESCDRRFSRSDNLVRHIRIHTGQKPFQCRI CMRNFSREDNLHTHIRTHTGEKPFACDICGRKFARS DELVRHTKIHLRQKDRPYACPVESCDRRFSQSGNLT EHIRIHTGQKPFQCRICMRNFSTSGHLVRHIRTHTGE<br><br>KPFACDICGRKFAQNSTLTEHTKIHLRQKDKLEMAD HLMLAEGYRLVQRPPSAAAAHGPHALRTLPPYAGP GLDSGLRPRGAPLGPPPPRQPGALAYGAFGPPSSFQP FPAVPPPAAGIAHLQPVATPYPGRAAAPPNAPGGPP GPQPAPSAAAPPPPAHALGGMDAELIDEEALTSLEL ELGLHRVRELPELFLGQSEFDCFSDLGSAPPAGSVSC | 105 |
| 8 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGQSQLIKPSRMRKYPNRPSKTPPHE RPYACPVESCDRRFSRSDNLVRHIRIHTGQKPFQCRI CMRNFSREDNLHTHIRTHTGEKPFACDICGRKFARS DELVRHTKIHLRQKDRPYACPVESCDRRFSQSGNLT EHIRIHTGQKPFQCRICMRNFSTSGHLVRHIRTHTGE KPFACDICGRKFAQNSTLTEHTKIHLRQKDK | 106 |
| 9 | MQSQLIKPSRMRKYPNRPSKTPPHERPYACPVESCD RRFSRSDNLVRHIRIHTGQKPFQCRICMRNFSREDNL HTHIRTHTGEKPFACDICGRKFARSDELVRHTKIHLR QKDRPYACPVESCDRRFSQSGNLTEHIRIHTGQKPF QCRICMRNFSTSGHLVRHIRTHTGEKPFACDICGRKF AQNSTLTEHTKIHLRQKDKLEMADHLMLAEGYRLV QRPPSAAAAHGPHALRTLPPYAGPGLDSGLRPRGAP LGPPPPRQPGALAYGAFGPPSSFQPFPAVPPPAAGIA HLQPVATPYPGRAAAPPNAPGGPPGPQPAPSAAAPP PPAHALGGMDAELIDEEALTSLELELGLHRVRELPE LFLGQSEFDCFSDLGSAPPAGSVSC | 107 |

(continued)

| CONSTRUCT | SEQUENCE | SEQ ID NO |
|---|---|---|
| 10 | MQSQLIKPSRMRKYPNRPSKTPPHERPYACPVESCD RRFSRSDNLVRHIRIHTGQKPFQCRICMRNFSREDNL HTHIRTHTGEKPFACDICGRKFARSDELVRHTKIHLR QKDRPYACPVESCDRRFSQSGNLTEHIRIHTGQKPF QCRICMRNFSTSGHLVRHIRTHTGEKPFACDICGRKF AQNSTLTEHTKIHLRQKDKLEMSGLEMADHMMAM NHGRFPDGTNGLHHHPAHRMGMGQFPSPHHHQQQ QPQHAFNALMGEHIHYGAGNMNATSGIRHAMGPG TVNGGHPPSALAPAARFNNSQFMGPPVASQGGSLP ASMQLQKLNNQYFNHHPYPHNHYMPDLHPAAGHQ MNGTNQHFRDCNPKHSGGSSTPGGSGGSSTPGGSG SSSGGGAGSSNSGGGSGSGNMPASVAHVPAAMLPP NVIDTDFIDEEVLMSLVIEMGLDRIKELPELWLGQN EFDFMTDFVCKQQPSRVSC | 108 |
| 11 | MSGLEMADHMMAMNHGRFPDGTNGLHHHPAHRM GMGQFPSPHHHQQQQPQHAFNALMGEHIHYGAGN MNATSGVRHAMGPGTVNGGHPPSALAPAARFNNS QFMGPPVASQGGSLPASMQLQKLNNQYFNHHPYPH NHYMPDLHPAAGHQMNGTNQHFRDCNPKHSGGSS TPGGSGGSSTPGGSGSSSGGGAGSSNSGGGSGSGNM PASVAHVPAAMLPPNVIDTDFIDEEVLMSLVIEMGL DRIKELPELWLGQNEFDFMTDFVCKQQPSRVSCQSQ<br><br>LIKPSRMRKYPNRPSKTPPHERPYACPVESCDRRFSR SDNLVRHIRIHTGQKPFQCRICMRNFSREDNLHTHIR THTGEKPFACDICGRKFARSDELVRHTKIHLRQKDR PYACPVESCDRRFSQSGNLTEHIRIHTGQKPFQCRIC MRNFSTSGHLVRHIRTHTGEKPFACDICGRKFAQNS TLTEHTKIHLRQKDK | 109 |
| 12 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGRPHACPAEGCDRRFSRSDNLVRH LRIHTGHKPFQCRICMRSFSREDNLHTHIRTHTGEKP FACEFCGRKFARSDELVRHAKIHLKQKEHACPAEG CDRRFSQSGNLTEHLRIHTGHKPFQCRICMRSFSTSG HLVRHIRTHTGEKPFACEFCGRKFAQNSTLTEHAKI HLKQKEKLEMADHLMLAEGYRLVQRPPSAAAAHG PHALRTLPPYAGPGLDSGLRPRGAPLGPPPPRQPGA LAYGAFGPPSSFQPFPAVPPPAAGIAHLQPVATPYPG RAAAPPNAPGGPPGPQPAPSAAAPPPPAHALGGMD AELIDEEALTSLELELGLHRVRELPELFLGQSEFDCF SDLGSAPPAGSVSC | 110 |

(continued)

| CONSTRUCT | SEQUENCE | SEQ ID NO |
|---|---|---|
| 13 | MRPHACPAEGCDRRFSRSDNLVRHLRIHTGHKPFQC RICMRSFSREDNLHTHIRTHTGEKPFACEFCGRKFAR SDELVRHAKIHLKQKEHACPAEGCDRRFSQSGNLTE HLRIHTGHKPFQCRICMRSFSTSGHLVRHIRTHTGEK PFACEFCGRKFAQNSTLTEHAKIHLKQKEKLEMAD HLMLAEGYRLVQRPPSAAAAHGPHALRTLPPYAGP GLDSGLRPRGAPLGPPPPRQPGALAYGAFGPPSSFQP FPAVPPPAAGIAHLQPVATPYPGRAAAPPNAPGGPP GPQPAPSAAAPPPPAHALGGMDAELIDEEALTSLEL ELGLHRVRELPELFLGQSEFDCFSDLGSAPPAGSVSC | 111 |
| 14 | MRPHACPAEGCDRRFSRSDNLVRHLRIHTGHKPFQC RICMRSFSREDNLHTHIRTHTGEKPFACEFCGRKFAR SDELVRHAKIHLKQKEHACPAEGCDRRFSQSGNLTE HLRIHTGHKPFQCRICMRSFSTSGHLVRHIRTHTGEK PFACEFCGRKFAQNSTLTEHAKIHLKQKEKKAEKG GAPSASSAPPVSLAPVVTTCALEMSGLEMADHMMA MNHGRFPDGTNGLHHHPAHRMGMGQFPSPHHHQQ QQPQHAFNALMGEHIHYGAGNMNATSGIRHAMGP GTVNGGHPPSALAPAARFNNSQFMGPPVASQGGSL PASMQLQKLNNQYFNHHPYPHNHYMPDLHPAAGH QMNGTNQHFRDCNPKHSGGSSTPGGSGGSSTPGGS GSSSGGGAGSSNSGGGSGSGNMPASVAHVPAAMLP PNVIDTDFIDEEVLMSLVIEMGLDRIKELPELWLGQN EFDFMTDFVCKQQPSRVSC | 112 |
| 15 | MSGLEMADHMMAMNHGRFPDGTNGLHHHPAHRM GMGQFPSPHHHQQQQPQHAFNALMGEHIHYGAGN MNATSGVRHAMGPGTVNGGHPPSALAPAARFNNS QFMGPPVASQGGSLPASMQLQKLNNQYFNHHPYPH NHYMPDLHPAAGHQMNGTNQHFRDCNPKHSGGSS TPGGSGGSSTPGGSGSSSGGGAGSSNSGGGSGSGNM PASVAHVPAAMLPPNVIDTDFIDEEVLMSLVIEMGL DRIKELPELWLGQNEFDFMTDFVCKQQPSRVSCRPH ACPAEGCDRRFSRSDNLVRHLRIHTGHKPFQCRICM RSFSREDNLHTHIRTHTGEKPFACEFCGRKFARSDEL VRHAKIHLKQKEHACPAEGCDRRFSQSGNLTEHLRI HTGHKPFQCRICMRSFSTSGHLVRHIRTHTGEKPFA CEFCGRKFAQNSTLTEHAKIHLKQKEKKAEKGGAP SASSAPPVSLAPVVTTCA | 113 |

(continued)

| CONSTRUCT | SEQUENCE | SEQ ID NO |
|---|---|---|
| 16 | MTGKLAEKLPVTMSSLLNQLPDNLYPEEIPSALNLF SGSSDSVVHYNQMATENVMDIGLTNEKPNPELSYS GSFQPAPGNKTVTYLGKFAFDSPSNWCQDNIISLMS AGILGVPPASGALSTQTSTASMVQPPQGDVEAMYP ALPPYSNCGDLYSEPVSFHDPQGNPGLAYSPQDYQS AKPALDSNLFPMIPDYNLYHHPNDMGSIPEHKPFQG MDPIRVNPPPITPLETIKAFKDKQIHPGFGSLPQPPLT LKPIRPRKYPNRPSKTPLHERPHACPAEGCDRRFSRR DELNVHLRIHTGHKPFQCRICMRSFSRSDHLTNHIRT HTGEKPFACEFCGRKFARSDDLVRHAKIHLKQKEH ACPAEGCDRRFSRSDNLVRHLRIHTGHKPFQCRICM RSFSHRTTLTNHIRTHTGEKPFACEFCGRKFAREDNL HTHAKIHLKQKEHACPAEGCDRRFSTSHSLTEHLRI HTGHKPFQCRICMRSFSQSSSLVRHIRTHTGEKPFAC EFCGRKFAREDNLHTHAKIHLKQKEKKAEKGGAPS ASSAPPVSLAPVVTTCA | 114 |
| 17 | MAAAKAEMQLMSPLQISDPFGSFPHSPTMDNYPKL EEMMLLSNGAPQFLGAAGAPEGSGSNSSSSSSGGGG GGGGGSNSSSSSSTFNPQADTGEQPYEHLTAESFPDI SLNNEKVLVETSYPSQTTRLPPITYTGRFSLEPAPNS GNTLWPEPLFSLVSGLVSMTNPPASSSSAPSPAASSA SASQSPPLSCAVPSNDSSPIYSAAPTFPTPNTDIFPEPQ SQAFPGSAGTALQYPPPAYPAAKGGFQVPMIPDYLF PQQQGDLGLGTPDQKPFQGLESRTQQPSLTPLSTIKA FATQSGSQDLKALNTSYQSQLIKPSRMRKYPNRPSK TPPHERPYACPVESCDRRFSRSDNLVRHIRIHTGQKP FQCRICMRNFSHRTTLTNHIRTHTGEKPFACDICGRK FAREDNLHTHTKIHLRQKDRPYACPVESCDRRFSTS HSLTEHIRIHTGQKPFQCRICMRNFSQSSSLVRHIRTH TGEKPFACDICGRKFAREDNLHTHTKIHLRQKDKKA DKSVVASSATSSLSSYPSPVATSYPSPVTTSYPSPATT SYPSPVPTSFSSPGSSTYPSPVHSGFPSPSVATTYSSV PPAFPAQVSSFPSSAVTNSFSASTGLSDMTATFSPRTI EIC | 115 |
| 18 | MAAAKAEMQLMSPLQISDPFGSFPHSPTMDNYPKL EEMMLLSNGAPQFLGAAGAPEGSGSNSSSSSSGGGG GGGGGSNSSSSSSTFNPQADTGEQPYEHLTAESFPDI SLNNEKVLVETSYPSQTTRLPPITYTGRFSLEPAPNS GNTLWPEPLFSLVSGLVSMTNPPASSSSAPSPAASSA SASQSPPLSCAVPSNDSSPIYSAAPTFPTPNTDIFPEPQ | 116 |

(continued)

| CONSTRUCT | SEQUENCE | SEQ ID NO |
|---|---|---|
| | SQAFPGSAGTALQYPPPAYPAAKGGFQVPMIPDYLF PQQQGDLGLGTPDQKPFQGLESRTQQPSLTPLSTIKA FATQSGSQDLKALNTSYQSQLIKPSRMRKYPNRPSK TPPHERPYACPVESCDRRFSRRDELNVHIRIHTGQKP FQCRICMRNFSRSDHLTNHIRTHTGEKPFACDICGRK FARSDDLVRHTKIHLRQKDRPYACPVESCDRRFSRS DNLVRHIRIHTGQKPFQCRICMRNFSHRTTLTNHIRT HTGEKPFACDICGRKFAREDNLHTHTKIHLRQKDRP YACPVESCDRRFSTSHSLTEHIRIHTGQKPFQCRICM RNFSQSSSLVRHIRTHTGEKPFACDICGRKFAREDNL HTHTKIHLRQKDKKADKSVVASSATSSLSSYPSPVA TSYPSPVTTSYPSPATTSYPSPVPTSFSSPGSSTYPSPV HSGFPSPSVATTYSSVPPAFPAQVSSFPSSAVTNSFSA STGLSDMTATFSPRTIEIC | |
| 19 | MAAAKAEMQLMSPLQISDPFGSFPHSPTMDNYPKL EEMMLLSNGAPQFLGAAGAPEGSGSNSSSSSSGGGG GGGGGGSNSSSSSSTFNPQADTGEQPYEHLTAESFPDI SLNNEKVLVETSYPSQTTRLPPITYTGRFSLEPAPNS GNTLWPEPLFSLVSGLVSMTNPPASSSSAPSPAASSA SASQSPPLSCAVPSNDSSPIYSAAPTFPTPNTDIFPEPQ SQAFPGSAGTALQYPPPAYPAAKGGFQVPMIPDYLF PQQQGDLGLGTPDQKPFQGLESRTQQPSLTPLSTIKA FATQSGSQDLKALNTSYQSQLIKPSRMRKYPNRPSK TPPHERPYACPVESCDRRFSRSDNLVRHIRIHTGQKP FQCRICMRNFSHRTTLTNHIRTHTGEKPFACDICGRK FAREDNLHTHIRTHTGEKPFACDICGRKFSTSHSLTE HIRIHTGQKPFQCRICMRNFSQSSSLVRHIRTHTGEK PFACDICGRKFAREDNLHTHTKIHLRQKDKKADKS VVASSATSSLSSYPSPVATSYPSPVTTSYPSPATTSYP SPVPTSFSSPGSSTYPSPVHSGFPSPSVATTYSSVPPA FPAQVSSFPSSAVTNSFSASTGLSDMTATFSPRTIEIC | 117 |

(continued)

| CONSTRUCT | SEQUENCE | SEQ ID NO |
|---|---|---|
| 20 | MAAAKAEMQLMSPLQISDPFGSFPHSPTMDNYPKL EEMMLLSNGAPQFLGAAGAPEGSGSNSSSSSSGGGG GGGGGSNSSSSSSTFNPQADTGEQPYEHLTAESFPDI SLNNEKVLVETSYPSQTTRLPPITYTGRFSLEPAPNS GNTLWPEPLFSLVSGLVSMTNPPASSSSAPSPAASSA SASQSPPLSCAVPSNDSSPIYSAAPTFPTPNTDIFPEPQ SQAFPGSAGTALQYPPPAYPAAKGGFQVPMIPDYLF PQQQGDLGLGTPDQKPFQGLESRTQQPSLTPLSTIKA FATQSGSQDLKALNTSYQSQLIKPSRMRKYPNRPSK TPPHERPYACPVESCDRRFSRSDNLVRHIRIHTGQKP FQCRICMRNFSHRTTLTNHIRTHTGEKPFACDICGRK FAREDNLHTHTKIHLRQKDRPYACPVESCDRRFSTS HSLTEHIRIHTGQKPFQCRICMRNFSQSSSLVRHIRTH TGEKPFACDICGRKFAREDNLHTHTKIHLRQKDKKA DKSVVASSATSSLSSYPSPVATSYPSPVTTSYPSPATT SYPSPVPTSFSSPGSSTYPSPVHSGFPSPSVATTYSSV PPAFPAQVSSFPSSAVTNSFSASTGLSDMTATFSPRTI EIC | 115 |
| 21 | MAAAKAEMQLMSPLQISDPFGSFPHSPTMDNYPKL EEMMLLSNGAPQFLGAAGAPEGSGSNSSSSSSGGGG GGGGGSNSSSSSSTFNPQADTGEQPYEHLTAESFPDI SLNNEKVLVETSYPSQTTRLPPITYTGRFSLEPAPNS GNTLWPEPLFSLVSGLVSMTNPPASSSSAPSPAASSA SASQSPPLSCAVPSNDSSPIYSAAPTFPTPNTDIFPEPQ SQAFPGSAGTALQYPPPAYPAAKGGFQVPMIPDYLF PQQQGDLGLGTPDQKPFQGLESRTQQPSLTPLSTIKA FATQSGSQDLKALNTSYQSQLIKPSRMRKYPNRPSK TPPHERPYACPVESCDRRFSRSDNLVRHIRIHTGQKP FQCRICMRNFSREDNLHTHIRTHTGEKPFACDICGR KFARSDELVRHTKIHLRQKDRPYACPVESCDRRFSQ SGNLTEHIRIHTGQKPFQCRICMRNFSTSGHLVRHIR THTGEKPFACDICGRKFAQNSTLTEHTKIHLRQKDK KADKSVVASSATSSLSSYPSPVATSYPSPVTTSYPSP ATTSYPSPVPTSFSSPGSSTYPSPVHSGFPSPSVATTY SSVPPAFPAQVSSFPSSAVTNSFSASTGLSDMTATFS PRTIEIC | 118 |

**64**

(continued)

| CONSTRUCT | SEQUENCE | SEQ ID NO |
|---|---|---|
| 22 | MTGKLAEKLPVTMSSLLNQLPDNLYPEEIPSALNLF SGSSDSVVHYNQMATENVMDIGLTNEKPNPELSYS GSFQPAPGNKTVTYLGKFAFDSPSNWCQDNIISLMS AGILGVPPASGALSTQTSTASMVQPPQGDVEAMYP ALPPYSNCGDLYSEPVSFHDPQGNPGLAYSPQDYQS AKPALDSNLFPMIPDYNLYHHPNDMGSIPEHKPFQG MDPIRVNPPPITPLETIKAFKDKQIHPGFGSLPQPPLT LKPIRPRKYPNRPSKTPLHERPHACPAEGCDRRFSRS DNLVRHLRIHTGHKPFQCRICMRSFSHRTTLTNHIRT HTGEKPFACEFCGRKFAREDNLHTHAKIHLKQKEH ACPAEGCDRRFSTSHSLTEHLRIHTGHKPFQCRICMR SFSQSSSLVRHIRTHTGEKPFACEFCGRKFAREDNLH THAKIHLKQKEKKAEKGGAPSASSAPPVSLAPVVTT CA | 119 |
| 23 | MAAAKAEMQLMSPLQISDPFGSFPHSPTMDNYPKL EEMMLLSNGAPQFLGAAGAPEGSGSNSSSSSSGGGG GGGGGSNSSSSSSSTFNPQADTGEQPYEHLTAESFPDI SLNNEKVLVETSYPSQTTRLPPITYTGRFSLEPAPNS GNTLWPEPLFSLVSGLVSMTNPPASSSSAPSPAASSA SASQSPPLSCAVPSNDSSPIYSAAPTFPTPNTDIFPEPQ SQAFPGSAGTALQYPPPAYPAAKGGFQVPMIPDYLF PQQQGDLGLGTPDQKPFQGLESRTQQPSLTPLSTIKA FATQSGSQDLKALNTSYQSQLIKPSRMRKYPNRPSK TPPHERPYACPVESCDRRFSDPGALVRHIRIHTGQKP FQCRICMRNFSRSDNLVRHIRTHTGEKPFACDICGR KFAQSGDLRRHTKIHLRQKDRPYACPVESCDRRFST HLDLIRHIRIHTGQKPFQCRICMRNFSTGNLVRHIR THTGEKPFACDICGRKFARSDNLVRHTKIHLRQKDR PYACPVESCDRRFSQSGHLTEHIRIHTGQKPFQCRIC MRNFSERSHLREHIRTHTGEKPFACDICGRKFAQAG HLASHTKIHLRQKDKKADKSVVASSATSSLSSYPSP VATSYPSPVTTSYPSPATTSYPSPVPTSFSSPGSSTYP SPVHSGFPSPSVATTYSSVPPAFPAQVSSFPSSAVTNS FSASTGLSDMTATFSPRTIEIC | 120 |

(continued)

| CONSTRUCT | SEQUENCE | SEQ ID NO |
|---|---|---|
| 24 | MAAAKAEMQLMSPLQISDPFGSFPHSPTMDNYPKL EEMMLLSNGAPQFLGAAGAPEGSGSNSSSSSSGGGG GGGGGSNSSSSSSTFNPQADTGEQPYEHLTAESFPDI SLNNEKVLVETSYPSQTTRLPPITYTGRFSLEPAPNS GNTLWPEPLFSLVSGLVSMTNPPASSSSAPSPAASSA SASQSPPLSCAVPSNDSSPIYSAAPTFPTPNTDIFPEPQ SQAFPGSAGTALQYPPPAYPAAKGGFQVPMIPDYLF PQQQGDLGLGTPDQKPFGLESRTQQPSLTPLSTIKA FATQSGSQDLKALNTSYQSQLIKPSRMRKYPNRPSK TPPHERPYACPVESCDRRFSRSDNLTRHIRIHTGQKP FQCRICMRNFSHSTTLTNHIRTHTGEKPFACDICGRK FARSDNRKTHIRTHTGEKPFACDICGRKFSTSHSLTE HIRIHTGQKPFQCRICMRNFSQSSSLTRHIRTHTGEKP FACDICGRKFARSDNRKTHTKIHLRQKDKKADKSV VASSATSSLSSYPSPVATSYPSPVTTSYPSPATTSYPS PVPTSFSSPGSSTYPSPVHSGFPSPSVATTYSSVPPAF PAQVSSFPSSAVTNSFSASTGLSDMTATFSPRTIEIC | 121 |
| 25 | MTGKLAEKLPVTMSSLLNQLPDNLYPEEIPSALNLF SGSSDSVVHYNQMATENVMDIGLTNEKPNPELSYS GSFQPAPGNKTVTYLGKFAFDSPSNWCQDNIISLMS AGILGVPPASGALSTQTSTASMVQPPQGDVEAMYP ALPPYSNCGDLYSEPVSFHDPQGNPGLAYSPQDYQS AKPALDSNLFPMIPDYNLYHHPNDMGSIPEHKPFQG MDPIRVNPPITPLETIKAFKDKQIHPGFGSLPQPPLT LKPIRPRKYPNRPSKTPLHERPHACPAEGCDRRFSRS DNLVRHLRIHTGHKPFQCRICMRSFSREDNLHTHIRT HTGEKPFACEFCGRKFARSDELVRHAKIHLKQKEH ACPAEGCDRRFSQSGNLTEHLRIHTGHKPFQCRICM RSFSTSGHLVRHIRTHTGEKPFACEFCGRKFAQNSTL TEHAKIHLKQKEKKAEKGGAPSASSAPPVSLAPVVT TCA | 122 |

(continued)

| CONSTRUCT | SEQUENCE | SEQ ID NO |
|---|---|---|
| 26 | MAAAKAEMQLMSPLQISDPFGSFPHSPTMDNYPKL EEMMLLSNGAPQFLGAAGAPEGSGSNSSSSSSGGGG GGGGGSNSSSSSSTFNPQADTGEQPYEHLTAESFPDI SLNNEKVLVETSYPSQTTRLPPITYTGRFSLEPAPNS GNTLWPEPLFSLVSGLVSMTNPPASSSSAPSPAASSA SASQSPPLSCAVPSNDSSPIYSAAPTFPTPNTDIFPEPQ SQAFPGSAGTALQYPPPAYPAAKGGFQVPMIPDYLF PQQQGDLGLGTPDQKPFQGLESRTQQPSLTPLSTIKA FATQSGSQDLKALNTSYQSQLIKPSRMRKYPNRPSK TPPHERPYACPVESCDRRFSRSDNLTRHIRIHTGQKP FQCRICMRNFSRSDNLTTHIRTHTGEKPFACDICGRK FARSDERKRHIRTHTGEKPFACDICGRKFSQSGNLTE HIRIHTGQKPFQCRICMRNFSTSGHLTRHIRTHTGEK PFACDICGRKFAQSSTRKEHTKIHLRQKDKKADKSV VASSATSSLSSYPSPVATSYPSPVTTSYPSPATTSYPS PVPTSFSSPGSSTYPSPVHSGFPSPSVATTYSSVPPAF PAQVSSFPSSAVTNSFSASTGLSDMTATFSPRTIEIC | 123 |
| 27 | MAAAKAEMQLMSPLQISDPFGSFPHSPTMDNYPKL EEMMLLSNGAPQFLGAAGAPEGSGSNSSSSSSGGGG GGGGGSNSSSSSSTFNPQADTGEQPYEHLTAESFPDI SLNNEKVLVETSYPSQTTRLPPITYTGRFSLEPAPNS GNTLWPEPLFSLVSGLVSMTNPPASSSSAPSPAASSA SASQSPPLSCAVPSNDSSPIYSAAPTFPTPNTDIFPEPQ SQAFPGSAGTALQYPPPAYPAAKGGFQVPMIPDYLF PQQQGDLGLGTPDQKPFQGLESRTQQPSLTPLSTIKA FATQSGSQDLKALNTSYQSQLIKPSRMRKYPNRPSK TPPHERPYACPVESCDRRFSRSDNLVRHIRIHTGQKP FQCRICMRNFSREDNLHTHIRTHTGEKPFACDICGR KFARSDELVRHIRTHTGEKPFACDICGRKFSQSGNLT EHIRIHTGQKPFQCRICMRNFSTSGHLVRHIRTHTGE KPFACDICGRKFAQNSTLTEHTKIHLRQKDKKADKS VVASSATSSLSSYPSPVATSYPSPVTTSYPSPATTSYP SPVPTSFSSPGSSTYPSPVHSGFPSPSVATTYSSVPPA FPAQVSSFPSSAVTNSFSASTGLSDMTATFSPRTIEIC | 124 |
| 28 | MAPKKKRKVGIHGVPAALEPGEKPYKCPECGKSFSS PADLTRHQRTHTGEKPYKCPECGKSFSRSDNLVRH QRTHTGEKPYKCPECGKSFSREDNLHTHQRTHTGE KPYKCPECGKSFSRSDELVRHQRTHTGEKPYKCPEC GKSFSQSGNLTEHQRTHTGEKPYKCPECGKSFSTSG HLVRHQRTHTGKKTSKRPAATKKAGQAKKKKGSY PYDVPDYALEDALDDFDLDMLGSDALDDFDLDML GSDALDDFDLDMLGSDALDDFDLDML | 125 |

(continued)

| CONSTRUCT | SEQUENCE | SEQ ID NO |
|---|---|---|
| 29 | MAPKKKRKVGIHGVPAALEPGEKPYKCPECGKSFS DPGALVRHQRTHTGEKPYKCPECGKSFSRSDNLVR HQRTHTGEKPYKCPECGKSFSQSGDLRRHQRTHTG EKPYKCPECGKSFSTHLDLIRHQRTHTGEKPYKCPE CGKSFSTSGNLVRHQRTHTGEKPYKCPECGKSFSRS DNLVRHQRTHTGKKTSKRPAATKKAGQAKKKKGS YPYDVPDYALEDALDDFDLDMLGSDALDDFDLDM LGSDALDDFDLDMLGSDALDDFDLDML | 126 |
| 30 | MAPKKKRKVGIHGVPAALEPGEKPYKCPECGKSFS RSDNLVRHQRTHTGEKPYKCPECGKSFSHRTTLTNH QRTHTGEKPYKCPECGKSFSREDNLHTHQRTHTGE KPYKCPECGKSFSTSHSLTEHQRTHTGEKPYKCPEC GKSFSQSSSLVRHQRTHTGEKPYKCPECGKSFSRED NLHTHQRTHTGKKTSKRPAATKKAGQAKKKKGSY PYDVPDYALEEASGSGRADALDDFDLDMLGSDALD DFDLDMLGSDALDDFDLDMLGSDALDDFDLDMLIN SRSSGSPKKKRKVGSQYLPDTDDRHRIEEKRKRTYE TFKSIMKKSPFSGPTDPRPPPRRIAVPSRSSASVPKPA PQPYPFTSSLSTINYDEFPTMVFPSGQISQASALAPAP PQVLPQAPAPAPAMVSALAQAPAPVPVLAPGPPQ AVAPPAPKPTQAGEGTLSEALLQLQFDDEDLGALLG NSTDPAVFTDLASVDNSEFQQLLNQGIPVAPHTTEP MLMEYPEAITRLVTGAQRPPDPAPAPLGAPGLPNGL LSGDEDFSSIADMDFSALLGSGSGSRDSREGMFLPK PEAGSAISDVFEGREVCQPKRIRPFHPPGSPWANRPL<br><br>PASLAPTPTGPVHEPVGSLTPAPVPQPLDPAPAVTPE ASHLLEDPDEETSQAVKALREMADTVIPQKEEAAIC GQMDLSHPPPRGHLDELTTTLESMTEDLNLDSPLTP ELNEILDTFLNDECLLHAMHISTGLSIFDTSLF | 99 |
| 31 | MAPKKKRKVGIHGVPAALEPGEKPYKCPECGKSFS RSDNLVRHQRTHTGEKPYKCPECGKSFSREDNLHT HQRTHTGEKPYKCPECGKSFSRSDELVRHQRTHTGE KPYKCPECGKSFSQSGNLTEHQRTHTGEKPYKCPEC GKSFSTSGHLVRHQRTHTGEKPYKCPECGKSFSQNS TLTEHQRTHTGKKTSKRPAATKKAGQAKKKKGSYP YDVPDYALEDALDDFDLDMLGSDALDDFDLDMLG SDALDDFDLDMLGSDALDDFDLDML | 102 |
| 32 | MAPKKKRKVGIHGVPAALEPGEKPYKCPECGKSFS RSDNLVRHQRTHTGEKPYKCPECGKSFSREDNLHT HQRTHTGEKPYKCPECGKSFSRSDELVRHQRTHTGE KPYKCPECGKSFSQSGNLTEHQRTHTGEKPYKCPEC GKSFSTSGHLVRHQRTHTGEKPYKCPECGKSFSQNS TLTEHQRTHTGKKTSKRPAATKKAGQAKKKKGSYP YDVPDYALEDALDDFDLDMLGSDALDDFDLDMLG SDALDDFDLDMLGSDALDDFDLDML | 102 |

(continued)

| CONSTRUCT | SEQUENCE | SEQ ID NO |
|---|---|---|
| 33 | MAPKKKRKVGIHGVPAALEPGEKPYKCPECGKSFS RSDNLVRHQRTHTGEKPYKCPECGKSFSREDNLHT HQRTHTGEKPYKCPECGKSFSRSDELVRHQRTHTGE KPYKCPECGKSFSQSGNLTEHQRTHTGEKPYKCPEC GKSFSTSGHLVRHQRTHTGEKPYKCPECGKSFSQNS TLTEHQRTHTGKKTSKRPAATKKAGQAKKKKGSYP YDVPDYALEEASGSGRADALDDFDLDMLGSDALD DFDLDMLGSDALDDFDLDMLGSDALDDFDLDMLIN SRSSGSPKKKRKVGSQYLPDTDDRHRIEEKRKRTYE TFKSIMKKSPFSGPTDPRPPPRRIAVPSRSSASVPKPA PQPYPFTSSLSTINYDEFPTMVFPSGQISQASALAPAP PQVLPQAPAPAPAMVSALAQAPAPVPVLAPGPPQ AVAPPAPKPTQAGEGTLSEALLQLQFDDEDLGALLG NSTDPAVFTDLASVDNSEFQQLLNQGIPVAPHTTEP MLMEYPEAITRLVTGAQRPPDPAPAPLGAPGLPNGL LSGDEDFSSIADMDFSALLGSGSGSRDSREGMFLPK PEAGSAISDVFEGREVCQPKRIRPFHPPGSPWANRPL PASLAPTPTGPVHEPVGSLTPAPVPQPLDPAPAVTPE ASHLLEDPDEETSQAVKALREMADTVIPQKEEAAIC GQMDLSHPPPRGHLDELTTTLESMTEDLNLDSPLTP ELNEILDTFLNDECLLHAMHISTGLSIFDTSLF | 100 |
| 34 | MAPKKKRKVGIHGVPAALEPGEKPYKCPECGKSFS RSDNLVRHQRTHTGEKPYKCPECGKSFSREDNLHT HQRTHTGEKPYKCPECGKSFSRSDELVRHQRTHTGE KPYKCPECGKSFSQSGNLTEHQRTHTGEKPYKCPEC GKSFSTSGHLVRHQRTHTGEKPYKCPECGKSFSQNS TLTEHQRTHTGKKTSKRPAATKKAGQAKKKKGSD ALDDFDLDMLGSDALDDFDLDMLGSDALDDFDLD MLGSDALDDFDLDML | 127 |
| 35 | MAPKKKRKVGIHGVPAALEPGEKPYKCPECGKSFS RSDNLVRHQRTHTGEKPYKCPECGKSFSREDNLHT HQRTHTGEKPYKCPECGKSFSRSDELVRHQRTHTGE KPYKCPECGKSFSQSGNLTEHQRTHTGEKPYKCPEC GKSFSTSGHLVRHQRTHTGEKPYKCPECGKSFSQNS TLTEHQRTHTGKKTSKRPAATKKAGQAKKKKGSD ALDDFDLDMLGSDALDDFDLDMLGSDALDDFDLD MLGSDALDDFDLDML | 127 |

(continued)

| CONSTRUCT | SEQUENCE | SEQ ID NO |
|---|---|---|
| 36 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCQSQLIKPSRMRKYPNRPSKTPPHERPYACPVES CDRRFSRSDNLVRHIRIHTGQKPFQCRICMRNFSRED NLHTHIRTHTGEKPFACDICGRKFARSDELVRHTKIH LRQKDRPYACPVESCDRRFSQSGNLTEHIRIHTGQK PFQCRICMRNFSTSGHLVRHIRTHTGEKPFACDICGR KFAQNSTLTEHTKIHLRQKDK | 128 |
| 37 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGQSQLIKPSRMRKYPNRPSKTPPHE RPYACPVESCDRRFSRSDNLVRHIRIHTGQKPFQCRI CMRNFSREDNLHTHIRTHTGEKPFACDICGRKFARS DELVRHTKIHLRQKDRPYACPVESCDRRFSQSGNLT EHIRIHTGQKPFQCRICMRNFSTSGHLVRHIRTHTGE KPFACDICGRKFAQNSTLTEHTKIHLRQKDK | 106 |
| 38 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGGGSGGGSGQSQLIKPSRMRKYPN RPSKTPPHERPYACPVESCDRRFSRSDNLVRHIRIHT GQKPFQCRICMRNFSREDNLHTHIRTHTGEKPFACDI CGRKFARSDELVRHTKIHLRQKDRPYACPVESCDRR FSQSGNLTEHIRIHTGQKPFQCRICMRNFSTSGHLVR HIRTHTGEKPFACDICGRKFAQNSTLTEHTKIHLRQK DK | 129 |
| 39 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGQSQLIKPSRMRKYPNRPSKTPPHE RPYACPVESCDRRFSRSDNLVRHIRIHTGQKPFQCRI CMRNFSREDNLHTHIRTHTGEKPFACDICGRKFARS DELVRHTKIHLRQKDRPYACPVESCDRRFSQSGNLT | 105 |

(continued)

| CONSTRUCT | SEQUENCE | SEQ ID NO |
|---|---|---|
| | EHIRIHTGQKPFQCRICMRNFSTSGHLVRHIRTHTGE KPFACDICGRKFAQNSTLTEHTKIHLRQKDKLEMAD HLMLAEGYRLVQRPPSAAAAHGPHALRTLPPYAGP GLDSGLRPRGAPLGPPPPRQPGALAYGAFGPPSSFQP FPAVPPPAAGIAHLQPVATPYPGRAAAPPNAPGGPP GPQPAPSAAAPPPPAHALGGMDAELIDEEALTSLEL ELGLHRVRELPELFLGQSEFDCFSDLGSAPPAGSVSC | |
| 40 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCADHLMLAEGYRLVQRPPSAAAAHGPHALRTL PPYAGPGLDSGLRPRGAPLGPPPPRQPGALAYGAFG PPSSFQPFPAVPPPAAGIAHLQPVATPYPGRAAAPPN APGGPPGPQPAPSAAAPPPPAHALGGMDAELIDEEA LTSLELELGLHRVRELPELFLGQSEFDCFSDLGSAPP AGSVSCQSQLIKPSRMRKYPNRPSKTPPHERPYACP VESCDRRFSRSDNLVRHIRIHTGQKPFQCRICMRNFS REDNLHTHIRTHTGEKPFACDICGRKFARSDELVRH TKIHLRQKDRPYACPVESCDRRFSQSGNLTEHIRIHT GQKPFQCRICMRNFSTSGHLVRHIRTHTGEKPFACDI CGRKFAQNSTLTEHTKIHLRQKDK | 130 |
| 41 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGADHLMLAEGYRLVQRPPSAAAAH GPHALRTLPPYAGPGLDSGLRPRGAPLGPPPPRQPG ALAYGAFGPPSSFQPFPAVPPPAAGIAHLQPVATPYP GRAAAPPNAPGGPPGPQPAPSAAAPPPPAHALGGM DAELIDEEALTSLELELGLHRVRELPELFLGQSEFDC FSDLGSAPPAGSVSCGGSGGGSGQSQLIKPSRMRKY PNRPSKTPPHERPYACPVESCDRRFSRSDNLVRHIRI HTGQKPFQCRICMRNFSREDNLHTHIRTHTGEKPFA CDICGRKFARSDELVRHTKIHLRQKDRPYACPVESC DRRFSQSGNLTEHIRIHTGQKPFQCRICMRNFSTSGH LVRHIRTHTGEKPFACDICGRKFAQNSTLTEHTKIHL RQKDK | 131 |

(continued)

| CONSTRUCT | SEQUENCE | SEQ ID NO |
|---|---|---|
| 42 | MAPKKKRKVGIHGVPAALEPGEKPYKCPECGKSFS RSDNLVRHQRTHTGEKPYKCPECGKSFSREDNLHT HQRTHTGEKPYKCPECGKSFSRSDELVRHQRTHTGE KPYKCPECGKSFSQSGNLTEHQRTHTGEKPYKCPEC GKSFSTSGHLVRHQRTHTGEKPYKCPECGKSFSQNS TLTEHQRTHTGKKTSKRPAATKKAGQAKKKKGSYP YDVPDYALEDALDDFDLDMLGSDALDDFDLDMLG SDALDDFDLDMLGSDALDDFDLDML | 102 |
| 43 | MAPKKKRKVGIHGVPAALEPGEKPYKCPECGKSFS RSDNLVRHQRTHTGEKPYKCPECGKSFSREDNLHT<br><br>HQRTHTGEKPYKCPECGKSFSRSDELVRHQRTHTGE KPYKCPECGKSFSQSGNLTEHQRTHTGEKPYKCPEC GKSFSTSGHLVRHQRTHTGEKPYKCPECGKSFSQNS TLTEHQRTHTGKKTSKRPAATKKAGQAKKKKGSYP YDVPDYALEDALDDFDLDMLGSDALDDFDLDMLG SDALDDFDLDMLGSDALDDFDLDML | 102 |
| 44 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGQSQLIKPSRMRKYPNRPSKTPPHE RPYACPVESCDRRFSRSDNLVRHIRIHTGQKPFQCRI CMRNFSREDNLHTHIRTHTGEKPFACDICGRKFARS DELVRHTKIHLRQKDRPYACPVESCDRRFSQSGNLT EHIRIHTGQKPFQCRICMRNFSTSGHLVRHIRTHTGE KPFACDICGRKFAQNSTLTEHTKIHLRQKDK | 106 |
| 45 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGQSQLIKPSRMRKYPNRPSKTPPHE RPYACPVESCDRRFSRSDNLVRHIRIHTGQKPFQCRI CMRNFSREDNLHTHIRTHTGEKPFACDICGRKFARS DELVRHTKIHLRQKDRPYACPVESCDRRFSQSGNLT EHIRIHTGQKPFQCRICMRNFSTSGHLVRHIRTHTGE KPFACDICGRKFAQNSTLTEHTKIHLRQKDK | 106 |

(continued)

| CONSTRUCT | SEQUENCE | SEQ ID NO |
|---|---|---|
| 46 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGGGSGGGSGQSQLIKPSRMRKYPN RPSKTPPHERPYACPVESCDRRFSRSDNLVRHIRIHT GQKPFQCRICMRNFSREDNLHTHIRTHTGEKPFACDI CGRKFARSDELVRHTKIHLRQKDRPYACPVESCDRR FSQSGNLTEHIRIHTGQKPFQCRICMRNFSTSGHLVR HIRTHTGEKPFACDICGRKFAQNSTLTEHTKIHLRQK DK | 205 |
| 47 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCADHLMLAEGYRLVQRPPSAAAAHGPHALRTL PPYAGPGLDSGLRPRGAPLGPPPPRQPGALAYGAFG PPSSFQPFPAVPPPAAGIAHLQPVATPYPGRAAAPPN APGGPPGPQPAPSAAAPPPPAHALGGMDAELIDEEA LTSLELELGLHRVRELPELFLGQSEFDCFSDLGSAPP

AGSVSCQSQLIKPSRMRKYPNRPSKTPPHERPYACP VESCDRRFSRSDNLVRHIRIHTGQKPFQCRICMRNFS REDNLHTHIRTHTGEKPFACDICGRKFARSDELVRH TKIHLRQKDRPYACPVESCDRRFSQSGNLTEHIRIHT GQKPFQCRICMRNFSTSGHLVRHIRTHTGEKPFACDI CGRKFAQNSTLTEHTKIHLRQKDK | 207 |
| 48 | MAADHLMLAEGYRLVQRPPSAAAAHGPHALRTLPP YAGPGLDSGLRPRGAPLGPPPPRQPGALAYGAFGPP SSFQPFPAVPPPAAGIAHLQPVATPYPGRAAAPPNAP GGPPGPQPAPSAAAPPPPAHALGGMDAELIDEEALT SLELELGLHRVRELPELFLGQSEFDCFSDLGSAPPAG SVSCGGSGGGSGADHLMLAEGYRLVQRPPSAAAAH GPHALRTLPPYAGPGLDSGLRPRGAPLGPPPPRQPG ALAYGAFGPPSSFQPFPAVPPPAAGIAHLQPVATPYP GRAAAPPNAPGGPPGPQPAPSAAAPPPPAHALGGM DAELIDEEALTSLELELGLHRVRELPELFLGQSEFDC FSDLGSAPPAGSVSCGGSGGGSGQSQLIKPSRMRKY PNRPSKTPPHERPYACPVESCDRRFSRSDNLVRHIRI HTGQKPFQCRICMRNFSREDNLHTHIRTHTGEKPFA CDICGRKFARSDELVRHTKIHLRQKDRPYACPVESC DRRFSQSGNLTEHIRIHTGQKPFQCRICMRNFSTSGH LVRHIRTHTGEKPFACDICGRKFAQNSTLTEHTKIHL RQKDK | 209 |

(continued)

| CONSTRUCT | SEQUENCE | SEQ ID NO |
|---|---|---|
| 49 | MELELDAGDQDLLAFLLEESGDLGTAPDEAVRAPL DWALPLSEVPSDWEVDDLLCSLLSPPASLNILSSSNP CLVHHDHTYSLPRETVSMDLESESCRKEGTQMTPQ HMEELAEQEIARLVLTDEEKSLLEKEGLILPETLPLT KTEEQILKRVRLEPGEKPYKCPECGKSFSRSDNLVR HQRTHTGEKPYKCPECGKSFSREDNLHTHQRTHTG EKPYKCPECGKSFSRSDELVRHQRTHTGEKPYKCPE CGKSFSQSGNLTEHQRTHTGEKPYKCPECGKSFSTS GHLVRHQRTHTGEKPYKCPECGKSFSQNSTLTEHQ RTHTGKKTSVYVGGLESRVLKYTAQNMELQNKVQ LLEEQNLSLLDQLRKLQAMVIEISNKTSSSSTCILVLL VSFCLLLVPAMYSSDTRGSLPAEHGVLSRQLRALPS EDPYQLELPALQSEVPKDSTHQWLDGSDCVLQAPG NTSCLLHYMPQAPSAEPPLEWPFPDLFSEPLCRGPIL PLQANLTRKGGWLPTGSPSVILQDRYSG | 213 |
| 50 | MELELDAGDQDLLAFLLEESGDLGTAPDEAVRAPL DWALPLSEVPSDWEVDDLLCSLLSPPASLNILSSSNP CLVHHDHTYSLPRETVSMDLESESCRKEGTQMTPQ HMEELAEQEIARLVLTDEEKSLLEKEGLILPETLPLT KTEEQILKRVRRPYACPVESCDRRFSRSDNLVRHIRI HTGQKPFQCRICMRNFSREDNLHTHIRTHTGEKPFA CDICGRKFARSDELVRHTKIHLRQKDRPYACPVESC DRRFSQSGNLTEHIRIHTGQKPFQCRICMRNFSTSGH LVRHIRTHTGEKPFACDICGRKFAQNSTLTEHTKIHL RQKDVYVGGLESRVLKYTAQNMELQNKVQLLEEQ NLSLLDQLRKLQAMVIEISNKTSSSSTCILVLLVSFCL LLVPAMYSSDTRGSLPAEHGVLSRQLRALPSEDPYQ LELPALQSEVPKDSTHQWLDGSDCVLQAPGNTSCL LHYMPQAPSAEPPLEWPFPDLFSEPLCRGPILPLQAN LTRKGGWLPTGSPSVILQDRYSG | 217 |
| 51 | MELELDAGDQDLLAFLLEESGDLGTAPDEAVRAPL DWALPLSEVPSDWEVDDLLCSLLSPPASLNILSSSNP CLVHHDHTYSLPRETVSMDLESESCRKEGTQMTPQ HMEELAEQEIARLVLTDEEKSLLEKEGLILPETLPLT KTEEQILKRVRRPYACPVESCDRRFSRSDNLVRHIRI HTGQKPFQCRICMRNFSHRTTLTNHIRTHTGEKPFA CDICGRKFAREDNLHTHTKIHLRQKDRPYACPVESC DRRFSTSHSLTEHIRIHTGQKPFQCRICMRNFSQSSSL VRHIRTHTGEKPFACDICGRKFAREDNLHTHTKIHL RQKDVYVGGLESRVLKYTAQNMELQNKVQLLEEQ NLSLLDQLRKLQAMVIEISNKTSSSSTCILVLLVSFCL LLVPAMYSSDTRGSLPAEHGVLSRQLRALPSEDPYQ LELPALQSEVPKDSTHQWLDGSDCVLQAPGNTSCL LHYMPQAPSAEPPLEWPFPDLFSEPLCRGPILPLQAN LTRKGGWLPTGSPSVILQDRYSG | 219 |

(continued)

| CONSTRUCT | SEQUENCE | SEQ ID NO |
|---|---|---|
| 52 | MELELDAGDQDLLAFLLEESGDLGTAPDEAVRAPL DWALPLSEVPSDWEVDDLLCSLLSPPASLNILSSSNP CLVHHDHTYSLPRETVSMDLESESCRKEGTQMTPQ HMEELAEQEIARLVLTDEEKSLLEKEGLILPETLPLT KTEEQILKRVRLEPGEKPYKCPECGKSFSRSDNLVR HQRTHTGEKPYKCPECGKSFSREDNLHTHQRTHTG EKPYKCPECGKSFSRSDELVRHQRTHTGEKPYKCPE CGKSFSQSGNLTEHQRTHTGEKPYKCPECGKSFSTS GHLVRHQRTHTGEKPYKCPECGKSFSQNSTLTEHQ RTHTGKKTSVYVGGLESRVLKYTAQNMELQNKVQ LLEEQNLSLLDQLRKLQAMVIEIS | 221 |
| 53 | MELELDAGDQDLLAFLLEESGDLGTAPDEAVRAPL DWALPLSEVPSDWEVDDLLCSLLSPPASLNILSSSNP CLVHHDHTYSLPRETVSMDLESESCRKEGTQMTPQ HMEELAEQEIARLVLTDEEKSLLEKEGLILPETLPLT KTEEQILKRVRRPYACPVESCDRRFSRSDNLVRHIRI HTGQKPFQCRICMRNFSREDNLHTHIRTHTGEKPFA CDICGRKFARSDELVRHTKIHLRQKDRPYACPVESC DRRFSQSGNLTEHIRIHTGQKPFQCRICMRNFSTSGH LVRHIRTHTGEKPFACDICGRKFAQNSTLTEHTKIHL RQKDVYVGGLESRVLKYTAQNMELQNKVQLLEEQ NLSLLDQLRKLQAMVIEIS | 223 |

**TABLE 7.** Nucleic acid sequences encoding exemplary engineered transcription factors disclosed herein.

| CONSTRUCT | SEQUENCE | SEQ ID NO |
|---|---|---|
| 31 (RE + coding) | ggaggaagccatcaactaaactacaatgactgtaagatacaaaattgggaatggt aacatattttgaagttctgttgacataaagaatcatgatattaatgcccatggaaatg aaagggcgatcaacactatggtttgaaaaggggggaaattgtagagcacagatgt gttcgtgtggcagtgtgctgtctctagcaatactcagagaagagagagaacaatg aaattctgattggccccagtgtgagcccagatgaggttcagctgccaactttctctt | 67 |

| CONSTRUCT | SEQUENCE | SEQ ID NO |
|---|---|---|
| | tcacatcttatgaaagtcatttaagcacaactaacttttttttttttttttttttttttgagaca gagtcttgctctgttgcccaggacagagtgcagtagtgactcaatctcggctcact gcagcctccacctcctaggctcaaacggtcctcctgcatcagcctcccaagtagc tggaattacaggagtggcccaccatgcccagctaattttgtattttaatagatacg ggggtttcaccatatcacccaggctggtctcgaactcctggcctcaagtgatcca cctgcctcggcctcccaaagtgctgggattataggcgtcagccactatgcccaac ccgaccaaccttttttaaaataaatatttaaaaaattggtatttcacatatatactagta tttacatttatccacacaaaacggacgggcctccgctgaaccagtgaggcccca gacgtgcgcataaataaccccctgcgtgctgcaccacctggggagagggggag gaccacggtaaatggagcgagcgcatagcaaaagggacgcggggtcctttct ctgccggtggcactgggtagctgtggccaggtgtggtactttgatggggcccag ggctggagctcaaggaagcgtcgcagggtcacagatctgggggaaccccgg ggaaaagcactgaggcaaaaccgccgctcgtctcctacaatatatgggaggg gaggttgagtacgttctggattactcataagacctttttttttttccttccgggcgcaaa accgtgagctggatttataatcgccctataaagctccagaggcggtcaggcacct gcagaggagccccgccgctccgccgactagctgccccgcgagcaacggcct cgtgatttccccgccgatccggtccccgcctcccactctgcccccgcctacccc ggagccgtgcagccgcctctccgaatctctctcttctcctggcgctcgcgtgcga gagggaactagcgagaacgaggaagcagctggaggtgacgccgggcagatt acgcctgtcagggccgagccgagcggatcgctgggcgctgtgcagaggaaa ggcgggagtgccccggctcgctgtcgcagagccgaggtgggtaagctagcgac cacctggacttcccagcgcccaaccgtggcttttcagccaggtcctctcctcccg cggcttctcaaccaaccccatcccagcgccggccacccaacctcccgaaatga gtgcttcctgccccagcagccgaaggcgctactaggaacggtaacctgttacttt tccaggggccgtagtcgacccgctgcccgagttgctgtgcgactgcgcgcgcg gggctagagtgcaaggtgactgtggttcttctctggccaagtccgagggagaac gtaaagatatgggcctttttccccctctcaccttgtctcaccaaagtccctagtccc cggagcagttagcctctttctttccagggaattagccagacacaacaacgggaac cagacaccgaaccagacatgcccgccccgtgcgccctccccgctcgctgccttt cctccctcttgtctctccagagccggatcttcaaggggagcctccgtgccccccgg ctgctcagtccctccggtgtgcaggaccccggaagtcctccccgcacagctctc gcttctctttgcagcctgtttctgcgccggaccagtcgaggactctggacagtaga ggccccgggacgaccgagctgGAATTCGCCACCATGGCCCC AAAGAAGAAGCGGAAGGTCGGTATCCACGGAGT CCCAGCAGCCCTCGAACCAGGTGAAAAACCTTA CAAATGTCCTGAATGTGGGAAATCATTCAGTCGC AGCGACAACCTGGTGAGACATCAACGCACCCAT ACAGGAGAAAAACCTTATAAATGTCCAGAATGT GGAAAGTCCTTCTCACGAGAGGATAACTTGCAC ACTCATCAACGAACACATACTGGTGAAAAACCA TACAAGTGTCCCGAATGTGGTAAAAGTTTTAGCC GGAGCGATGAACTTGTCCGACACCAACGAACCC ATACAGGCGAGAAGCCTTACAAATGTCCCGAGT GTGGCAAGAGCTTCTCACAATCAGGGAATCTGA CTGAGCATCAACGAACTCATACCGGGGAAAAAC CTTACAAGTGTCCAGAGTGTGGGAAGAGCTTTTC CACAAGTGGACATCTGGTACGCCACCAGAGGAC ACATACAGGGGAGAAGCCCTACAAATGCCCCGA ATGCGGTAAAAGTTTCTCTCAGAATAGTACCCTG ACCGAACACCAGCGAACACACACTGGGAAAAAA | |

(continued)

| CONSTRUCT | SEQUENCE | SEQ ID NO |
|---|---|---|
| | ACGAGTAAAAGGCCGGCGGCCACGAAAAAGGCC GGCCAGGCAAAAAAGAAAAAGGGATCCTACCCA TACGACGTACCAGATTACGCTCTCGAGGACGCGC TGGACGATTTCGATCTCGACATGCTGGGTTCTGA TGCCCTCGATGACTTTGACCTGGATATGTTGGGA AGCGACGCATTGGATGACTTTGATCTGGACATGC TCGGCTCCGATGCTCTGGACGATTTCGATCTCGA TATGTTATAAACTAGTaaagagaccggttcactgtgacagtaaaa gagaccggttcactgtgagaatgaaagagaccggttcactgtgatcggaaaaga gaccggttcactgtgagcggccttgaaacccagcagacaatgtagctcagtagaa acccagcagacaatgtagctgaatggaaacccagcagacaatgtagcttcgg agaaacccagcagacaatgtagctAAGCTTGGGTGGCATCCC TGTGACCCCTCCCCAGTGCCTCTCCTGGCCCTGG AAGTTGCCACTCCAGTGCCCACCAGCCTTGTCCT AATAAAATTAAGTTGCATCATTTTGTCTGACTAG GTGTCCTTCTATAATATTATGGGGTGGAGGGGGG TGGTATGGAGCAAGGGGCAAGTTGGGAAGACAA CCTGTAGGGCCTGCGGGGTCTATTGGGAACCAA GCTGGAGTGCAGTGGCACAATCTTGGCTCACTGC AATCTCCGCCTCCTGGGTTCAAGCGATTCTCCTG CCTCAGCCTCCCGAGTTGTTGGGATTCCAGGCAT GCATGACCAGGCTCAGCTAATTTTTGTTTTTTTGG TAGAGACGGGGTTTCACCATATTGGCCAGGCTGG TCTCCAACTCCTAATCTCAGGTGATCTACCCACC TTGGCCTCCCAAATTGCTGGGATTACAGGCGTGA ACCACTGCTCCCTTCCCTGTCCTT | |

(continued)

| CONSTRUCT | SEQUENCE | SEQ ID NO |
|---|---|---|
| 32 (RE + coding) | ggaggaagccatcaactaaactacaatgactgtaagatacaaaattgggaatggt aacatattttgaagttctgttgacataaagaatcatgatattaatgcccatggaaatg aaagggcgatcaacactatggtttgaaaaggggggaaattgtagagcacagatgt gttcgtgtggcagtgtgctgtctctagcaatactcagagaagagagagagaacaatg aaattctgattggccccagtgtgagcccagatgaggttcagctgccaactttctctt tcacatcttatgaaagtcatttaagcacaactaactttttttttttttttttttttttgagaca gagtcttgctctgttgcccaggacagagtgcagtagtgactcaatctcggctcact gcagcctccacctcctaggctcaaacggtcctcctgcatcagcctcccaagtagc tggaattacaggagtggcccaccatgcccagctaattttgtattttaatagatacg ggggtttcaccatatcacccaggctggtctcgaactcctggcctcaagtgatcca cctgcctcggcctcccaaagtgctgggattataggcgtcagccactatgcccaac ccgaccaacctttttaaaataaatatttaaaaaattggtatttcacatatatactagta tttacatttatccacacaaaacggacgggcctccgctgaaccagtgaggcccca gacgtgcgcataaataacccctgcgtgctgcaccacctggggagagggggag gaccacggtaaatggagcgagcgcatagcaaaagggacgcggggtcctttct ctgccggtggcactgggtagctgtggccaggtgtggtactttgatggggcccag ggctggagctcaaggaagcgtcgcaggtcacagatctgggggaaccccgg ggaaaagcactgaggcaaaaccgccgctcgtctcctacaatatatgggagggg gaggttgagtacgttctggattactcataagacctttttttttttccttccgggcgcaaa accgtgagctggatttataatcgccctataaagctccagaggcggtcaggcacct gcagaggagccccgccgctccgccgactagctgccccgcgagcaacggcct cgtgatttccccgccgatccggtccccgcctcccactctgcccccgcctacccc ggagccgtgcagccgcctctccgaatctctctcttctcctggcgctcgcgtgcga gagggaactagcgagaacgaggaagcagctggaggtgacgccgggcagatt | 68 |

(continued)

| CONSTRUCT | SEQUENCE | SEQ ID NO |
|---|---|---|
| | acgcctgtcagggccgagccgagcggatcgctgggcgctgtgcagaggaaa ggcgggagtgcccggctcgctgtcgcagagccgaggtgggtaagctagcgac cacctggacttcccagcgcccaaccgtggcttttcagccaggtcctctcctcccg cggcttctcaaccaaccccatcccagcgccggccacccaacctcccgaaatga gtgcttcctgccccagcagccgaaggcgctactaggaacggtaacctgttacttt tccaggggccgtagtcgacccgctgcccgagttgctgtgcgactgcgcgcgcg gggctagagtgcaaggtgactgtggttcttctctggccaagtccgagggagaac gtaaagatatgggcctttttcccctctcaccttgtctcaccaaagtccctagtccc cggagcagttagcctctttctttccagggaattagccagacacaacaacgggaac cagacaccgaaccagacatgcccgccccgtgcgccctccccgctcgctgccttt cctccctcttgtctctccagagccggatcttcaaggggagcctccgtgcccccgg ctgctcagtccctccggtgtgcaggaccccggaagtcctccccgcacagctctc gcttctctttgcagcctgtttctgcgccggaccagtcgaggactctggacagtaga ggcccccgggacgaccgagctgGAATTCGCCACCATGGCCCC AAAGAAGAAGCGGAAGGTCGGTATCCACGGAGT CCCAGCAGCCCTCGAACCAGGTGAAAAACCTTA CAAATGTCCTGAATGTGGGAAATCATTCAGTCGC AGCGACAACCTGGTGAGACATCAACGCACCCAT ACAGGAGAAAAACCTTATAAATGTCCAGAATGT GGAAAGTCCTTCTCACGAGAGGATAACTTGCAC ACTCATCAACGAACACATACTGGTGAAAAACCA TACAAGTGTCCCGAATGTGGTAAAAGTTTTAGCC GGAGCGATGAACTTGTCCGACACCAACGAACCC ATACAGGCGAGAAGCCTTACAAATGTCCCGAGT GTGGCAAGAGCTTCTCACAATCAGGGAATCTGA CTGAGCATCAACGAACTCATACCGGGGAAAAAC CTTACAAGTGTCCAGAGTGTGGGAAGAGCTTTTC CACAAGTGGACATCTGGTACGCCACCAGAGGAC ACATACAGGGGAGAAGCCCTACAAATGCCCCGA ATGCGGTAAAAGTTTCTCTCAGAATAGTACCCTG ACCGAACACCAGCGAACACACACTGGGAAAAAA ACGAGTAAAAGGCCGGCGGCCACGAAAAAGGCC GGCCAGGCAAAAAAGAAAAAGGGATCCTACCCA TACGACGTACCAGATTACGCTCTCGAGGACGCGC TGGACGATTTCGATCTCGACATGCTGGGTTCTGA TGCCCTCGATGACTTTGACCTGGATATGTTGGGA AGCGACGCATTGGATGACTTTGATCTGGACATGC TCGGCTCCGATGCTCTGGACGATTTCGATCTCGA TATGTTATAAACTAGTAAGCTTGGGTGGCATCCC TGTGACCCCTCCCCAGTGCCTCTCCTGGCCCTGG AAGTTGCCACTCCAGTGCCCACCAGCCTTGTCCT AATAAAATTAAGTTGCATCATTTTGTCTGACTAG GTGTCCTTCTATAATATTATGGGGTGGAGGGGGG TGGTATGGAGCAAGGGGCAAGTTGGGAAGACAA CCTGTAGGGCCTGCGGGGTCTATTGGGAACCAA GCTGGAGTGCAGTGGCACAATCTTGGCTCACTGC AATCTCCGCCTCCTGGGTTCAAGCGATTCTCCTG CCTCAGCCTCCCGAGTTGTTGGGATTCCAGGCAT GCATGACCAGGCTCAGCTAATTTTTGTTTTTTTGG TAGAGACGGGGTTTCACCATATTGGCCAGGCTGG | |

(continued)

| CONSTRUCT | SEQUENCE | SEQ ID NO |
|---|---|---|
| | TCTCCAACTCCTAATCTCAGGTGATCTACCCACC TTGGCCTCCCAAATTGCTGGGATTACAGGCGTGA ACCACTGCTCCCTTCCCTGTCCTT | |

(continued)

| CONSTRUCT | SEQUENCE | SEQ ID NO |
|---|---|---|
| 33 (RE + coding) | ggaggaagccatcaactaaactacaatgactgtaagatacaaaattgggaatggt aacatattttgaagttctgttgacataaagaatcatgatattaatgcccatggaaatg aaagggcgatcaacactatggtttgaaaaggggggaaattgtagagcacagatgt gttcgtgtggcagtgtgctgtctctagcaatactcagagaagagagagaacaatg aaattctgattggcccccagtgtgtgagcccagatgaggttcagctgccaactttctctt tcacatcttatgaaagtcatttaagcacaactaacttttttttttttttttttttttttttgagaca gagtcttgctctgttgcccaggacagagtgcagtagtgactcaatctcggctcact gcagcctccacctcctaggctcaaacggtcctcctgcatcagcctcccaagtagc tggaattacaggagtggcccaccatgcccagctaattttgtatttttaatagatacg ggggtttcaccatatcacccaggctggtctcgaactcctggcctcaagtgatcca cctgcctcggcctcccaaagtgctgggattataggcgtcagccactatgcccaac ccgaccaaccttttttaaaataaatatttaaaaaattggtatttcacatatatactagta tttacatttatccacacaaaacggacgggcctccgctgaaccagtgaggcccca gacgtgcgcataaataaccccctgcgtgctgcaccacctggggagaggggggag gaccacggtaaatggagcgagcgcatagcaaaagggacgcggggtccttttct ctgccggtggcactgggtagctgtggccaggtgtggtactttgatgggggcccag ggctggagctcaaggaagcgtcgcagggtcacagatctggggggaacccccgg ggaaaagcactgaggcaaaaccgccgctcgtctcctacaatatatgggaggggg gaggttgagtacgttctggattactcataagaccttttttttttccttccgggcgcaaa accgtgagctggatttataatcgccctataaagctccagaggcggtcaggcacct gcagaggagccccgccgctccgccgactagctgcccccgcgagcaacggcct cgtgatttccccgccgatccggtccccgcctcccactctgcccccgcctaccc ggagccgtgcagccgcctctccgaatctctctcttctcctggcgctcgcgtgcga gagggaactagcgagaacgaggaagcagctggaggtgacgccgggcagatt acgcctgtcagggccgagccgagcggatcgctgggcgctgtgcagaggaaa ggcgggagtgcccggctcgctgtcgcagagccgaggtgggtaagctagcgac cacctggacttcccagcgcccaaccgtggctttcagccaggtcctctcctcccg cggcttctcaaccaaccccatcccagcgccggccacccaacctcccgaaatga gtgcttcctgccccagcagccgaaggcgctactaggaacggtaacctgttacttt tccaggggccgtagtcgacccgctgcccgagttgctgtgcgactgcgcgcgcg gggctagagtgcaaggtgactgtggttcttctctggccaagtccgagggagaac gtaaagatatgggcctttttcccccctctcaccttgtctcaccaaagtccctagtccc cggagcagttagcctctttctttccagggaattagccagacacaacaacgggaac cagacaccgaaccagacatgcccgcccccgtgcgccctccccgctcgctgcctt cctccctcttgtctctccagagccggatcttcaaggggagcctccgtgcccccgg ctgctcagtccctccggtgtgcaggaccccggaagtcctccccgcacagctctc gcttctctttgcagcctgtttctgcgccggaccagtcgaggactctggacagtaga ggccccgggacgaccgagctgGAATTCGCCACCATGGCCCC AAAGAAGAAGCGGAAGGTCGGTATCCACGGAGT CCCAGCAGCCCTCGAACCAGGTGAAAAACCTTA CAAATGTCCTGAATGTGGGAAATCATTCAGTCGC AGCGACAACCTGGTGAGACATCAACGCACCCAT ACAGGAGAAAACCTTATAAATGTCCAGAATGT GGAAAGTCCTTCTCACGAGAGGATAACTTGCAC ACTCATCAACGAACACATACTGGTGAAAAACCA TACAAGTGTCCCGAATGTGGTAAAAGTTTTAGCC GGAGCGATGAACTTGTCCGACACCAACGAACCC | 69 |

(continued)

| CONSTRUCT | SEQUENCE | SEQ ID NO |
|---|---|---|
| | ATACAGGCGAGAAGCCTTACAAATGTCCCGAGT<br>GTGGCAAGAGCTTCTCACAATCAGGGAATCTGA<br>CTGAGCATCAACGAACTCATACCGGGGAAAAAC<br>CTTACAAGTGTCCAGAGTGTGGGAAGAGCTTTTC<br>CACAAGTGGACATCTGGTACGCCACCAGAGGAC<br>ACATACAGGGGAGAAGCCCTACAAATGCCCCGA<br>ATGCGGTAAAAGTTTCTCTCAGAATAGTACCCTG<br>ACCGAACACCAGCGAACACACTGGGAAAAAA<br>ACGAGTAAAAGGCCGGCGGCCACGAAAAAGGCC<br>GGCCAGGCAAAAAAGAAAAAGGGATCCTACCCA<br>TACGACGTACCAGATTACGCTCTCGAGGAGGCC<br>AGCGGTTCCGGACGGGCTGACGCATTGGACGAT<br>TTTGATCTGGATATGCTGGGAAGTGACGCCCTCG<br>ATGATTTTGACCTTGACATGCTTGGTTCGGATGC<br>CCTTGATGACTTTGACCTCGACATGCTCGGCAGT<br>GACGCCCTTGATGATTCGACCTGGACATGCTGA<br>TTAACTCTAGAAGTTCCGGATCTCCGAAAAAGAA<br>ACGCAAAGTTGGTAGCCAGTACCTGCCCGACAC<br>CGACGACCGGCACCGGATCGAGGAAAAGCGGAA<br>GCGGACCTACGAGACATTCAAGAGCATCATGAA<br>GAAGTCCCCCTTCAGCGGCCCCACCGACCCTAGA<br>CCTCCACCTAGAAGAATCGCCGTGCCCAGCAGAT<br>CCAGCGCCAGCGTGCCAAAACCTGCCCCCCAGC<br>CTTACCCCTTCACCAGCAGCCTGAGCACCATCAA<br>CTACGACGAGTTCCCTACCATGGTGTTCCCCAGC<br>GGCCAGATCTCTCAGGCCTCTGCTCTGGCTCCAG<br>CCCCTCCTCAGGTGCTGCCTCAGGCTCCTGCTCC<br>TGCACCAGCTCCAGCCATGGTGTCTGCACTGGCT<br>CAGGCACCAGCACCCGTGCCTGTGCTGGCTCCTG<br>GACCTCCACAGGCTGTGGCTCCACCAGCCCCTAA<br>ACCTACACAGGCCGGCGAGGGCACACTGTCTGA<br>AGCTCTGCTGCAGCTGCAGTTCGACGACGAGGAT<br>CTGGGAGCCCTGCTGGGAAACAGCACCGATCCT<br>GCCGTGTTCACCGACCTGGCCAGCGTGGACAAC<br>AGCGAGTTCCAGCAGCTGCTGAACCAGGGCATC<br>CCTGTGGCCCCTCACACCACCGAGCCCATGCTGA<br>TGGAATACCCCGAGGCCATCACCCGGCTCGTGAC<br>AGGCGCTCAGAGGCCTCCTGATCCAGCTCCTGCC<br>CCTCTGGGAGCACCAGGCCTGCCTAATGGACTGC<br>TGTCTGGCGACGAGGACTTCAGCTCTATCGCCGA<br>TATGGATTTCTCAGCCTTGCTGGGCTCTGGCAGC<br>GGCAGCCGGGATTCCAGGGAAGGGATGTTTTTG<br>CCGAAGCCTGAGGCCGGCTCCGCTATTAGTGACG<br>TGTTTGAGGGCCGCGAGGTGTGCCAGCCAAAAC<br>GAATCCGGCCATTTCATCCTCCAGGAAGTCCATG<br>GGCCAACCGCCCACTCCCCGCCAGCCTCGCACCA<br>ACACCAACCGGTCCAGTACATGAGCCAGTCGGG<br>TCACTGACCCCGGCACCAGTCCCTCAGCCACTGG<br>ATCCAGCGCCCGCAGTGACTCCCGAGGCCAGTC<br>ACCTGTTGGAGGATCCCGATGAAGAGACGAGCC | |

(continued)

| CONSTRUCT | SEQUENCE | SEQ ID NO |
|---|---|---|
| | AGGCTGTCAAAGCCCTTCGGGAGATGGCCGATA CTGTGATTCCCCAGAAGGAAGAGGCTGCAATCT GTGGCCAAATGGACCTTTCCCATCCGCCCCCAAG GGGCCATCTGGATGAGCTGACAACCACACTTGA GTCCATGACCGAGGATCTGAACCTGGACTCACCC CTGACCCCGGAATTGAACGAGATTCTGGATACCT TCCTGAACGACGAGTGCCTCTTGCATGCCATGCA TATCAGCACAGGACTGTCCATCTTCGACACATCT CTGTTTTAAACTAGTaataaaagatctttattttcattagatctgtgtgt tggtttttttgtgtg | |

(continued)

| CONSTRUCT | SEQUENCE | SEQ ID NO |
|---|---|---|
| 34 (RE + coding) | ggaggaagccatcaactaaactacaatgactgtaagatacaaaattgggaatggt aacatattttgaagttctgttgacataaagaatcatgatattaatgcccatggaaatg aaagggcgatcaacactatggtttgaaaaggggggaaattgtagagcacagatgt gttcgtgtggcagtgtgctgtctctagcaatactcagagaagagagagagaacaatg aaattctgattggccccagtgtgagcccagatgaggttcagctgccaactttctctt tcacatcttatgaaagtcatttaagcacaactaactttttttttttttttttttttttgagaca gagtcttgctctgttgcccaggacagagtgcagtagtgactcaatctcggctcact gcagcctccacctcctaggctcaaacggtcctcctgcatcagcctcccaagtagc tggaattacaggagtggcccaccatgcccagctaattttgtatttttaatagatacg ggggtttcaccatatcacccaggctggtctcgaactcctggcctcaagtgatcca cctgcctcggcctcccaaagtgctgggattataggcgtcagccactatgcccaac ccgaccaacctttttaaaataaatatttaaaaaattggtatttcacatatatactagta tttacatttatccacacaaaacggacgggcctccgctgaaccagtgaggcccca gacgtgcgcataaataaccccctgcgtgctgcaccacctggggagagggggag gaccacggtaaatggagcgagcgcatagcaaaagggacgcggggtccttttct ctgccggtggcactgggtagctgtggccaggtgtggtactttgatggggcccag ggctggagctcaaggaagcgtcgcagggtcacagatctgggggaacccccgg ggaaaagcactgaggcaaaaccgccgctcgtctcctacaatatatgggagggg gaggttgagtacgttctggattactcataagaccttttttttttccttccgggcgcaaa accgtgagctggatttataatcgccctataaagctccagaggcggtcaggcacct gcagaggagccccgccgctccgccgactagctgccccgcgagcaacggcct cgtgatttccccgccgatccggtccccgcctcccactctgcccccgcctacccc ggagccgtgcagccgcctctccgaatctctctcttctcctggcgctcgcgtgcga gagggaactagcgagaacgaggaagcagctggaggtgacgccgggcagatt acgcctgtcagggccgagccgagcggatcgctgggcgctgtgcagaggaaa ggcgggagtgcccggctcgctgtcgcagagccgaggtgggtaagctagcgac cacctggacttcccagcgcccaaccgtggcttttcagccaggtcctctcctcccg cggcttctcaaccaaccccatcccagcgccggccacccaacctcccgaaatga gtgcttcctgccccagcagccgaaggcgctactaggaacggtaacctgttacttt tccaggggccgtagtcgacccgctgcccgagttgctgtgcgactgcgcgcgcg gggctagagtgcaaggtgactgtggttcttctctggccaagtccgagggagaac gtaaagatatgggcctttttccccctctcaccttgtctcaccaaagtccctagtccc cggagcagttagcctctttctttccagggaattagccagacacaacaacgggaac cagacaccgaaccagacatgcccgccccgtgcgccctccccgctcgctgccttt cctccctcttgtctctccagagccggatcttcaaggggagcctccgtgcccccgg ctgctcagtccctccggtgtgcaggaccccggaagtcctccccgcacagctctc gcttctctttgcagcctgtttctgcgccggaccagtcgaggactctggacagtaga ggccccgggacgaccgagctgGAATTCGCCACCATGGCCCC AAAGAAGAAGCGGAAGGTCGGTATCCACGGAGT CCCAGCAGCCCTCGAACCAGGTGAAAAACCTTA | 70 |

(continued)

| CONSTRUCT | SEQUENCE | SEQ ID NO |
|---|---|---|
|  | CAAATGTCCTGAATGTGGGAAATCATTCAGTCGC AGCGACAACCTGGTGAGACATCAACGCACCCAT ACAGGAGAAAAACCTTATAAATGTCCAGAATGT GGAAAGTCCTTCTCACGAGAGGATAACTTGCAC ACTCATCAACGAACACATACTGGTGAAAAACCA TACAAGTGTCCCGAATGTGGTAAAAGTTTTAGCC GGAGCGATGAACTTGTCCGACACCAACGAACCC ATACAGGCGAGAAGCCTTACAAATGTCCCGAGT GTGGCAAGAGCTTCTCACAATCAGGGAATCTGA CTGAGCATCAACGAACTCATACCGGGGAAAAAC CTTACAAGTGTCCAGAGTGTGGGAAGAGCTTTTC CACAAGTGGACATCTGGTACGCCACCAGAGGAC ACATACAGGGGAGAAGCCCTACAAATGCCCCGA ATGCGGTAAAAGTTTCTCTCAGAATAGTACCCTG ACCGAACACCAGCGAACACACACTGGGAAAAAA ACGAGTAAAAGGCCGGCGGCCACGAAAAAGGCC GGCCAGGCAAAAAAGAAAAAGGGATCCGACGC GCTGGACGATTTCGATCTCGACATGCTGGGTTCT GATGCCCTCGATGACTTTGACCTGGATATGTTGG GAAGCGACGCATTGGATGACTTTGATCTGGACAT GCTCGGCTCCGATGCTCTGGACGATTTCGATCTC GATATGTTATAAAGCTTGGGTGGCATCCCTGTG ACCCCTCCCCAGTGCCTCTCCTGGCCCTGGAAGT TGCCACTCCAGTGCCCACCAGCCTTGTCCTAATA AAATTAAGTTGCATCATTTTGTCTGACTAGGTGT CCTTCTATAATATTATGGGGTGGAGGGGGGTGGT ATGGAGCAAGGGGCAAGTTGGGAAGACAACCTG TAGGGCCTGCGGGGTCTATTGGGAACCAAGCTG GAGTGCAGTGGCACAATCTTGGCTCACTGCAATC TCCGCCTCCTGGGTTCAAGCGATTCTCCTGCCTC AGCCTCCCGAGTTGTTGGGATTCCAGGCATGCAT GACCAGGCTCAGCTAATTTTTGTTTTTTTGGTAG AGACGGGGTTTCACCATATTGGCCAGGCTGGTCT CCAACTCCTAATCTCAGGTGATCTACCCACCTTG GCCTCCCAAATTGCTGGGATTACAGGCGTGAACC ACTGCTCCCTTCCCTGTCCTT |  |

(continued)

| CONSTRUCT | SEQUENCE | SEQ ID NO |
|---|---|---|
| 26 (RE + coding) | ggaggaagccatcaactaaactacaatgactgtaagatacaaaattgggaatggt aacatattttgaagttctgttgacataaagaatcatgatattaatgcccatggaaatg aaagggcgatcaacactatggtttgaaaaggggggaaattgtagagcacagatgt gttcgtgtggcagtgtgctgtctctagcaatactcagagaagagagagaacaatg aaattctgattggccccagtgtgagcccagatgaggttcagctgccaactttctctt tcacatcttatgaaagtcatttaagcacaactaactttttttttttttttttttttttttgagaca gagtcttgctctgttgcccaggacagagtgcagtagtgactcaatctcggctcact gcagcctccacctcctaggctcaaacggtcctcctgcatcagcctcccaagtagc tggaattacaggagtggcccaccatgcccagctaattttttgtatttttaatagatacg ggggtttcaccatatcacccaggctggtctcgaactcctggcctcaagtgatcca cctgcctcggcctcccaaagtgctgggattataggcgtcagccactatgcccaac ccgaccaacctttttttaaaataaatatttaaaaaaattggtatttcacatatatactagta tttacatttatccacacaaaacggacgggcctccgctgaaccagtgaggcccca gacgtgcgcataaataacccctgcgtgctgcaccacctggggagagggggag | 71 |

(continued)

| CONSTRUCT | SEQUENCE | SEQ ID NO |
|---|---|---|
| | gaccacggtaaatggagcgagcgcatagcaaaagggacgcggggtcctttttct ctgccggtggcactgggtagctgtggccaggtgtggtactttgatggggcccag ggctggagctcaaggaagcgtcgcagggtcacagatctgggggaacccccgg ggaaaagcactgaggcaaaaccgccgctcgtctcctacaatatatgggaggggg gaggttgagtacgttctggattactcataagacctttttttttttccttccgggcgcaaa accgtgagctggatttataatcgccctataaagctccagaggcggtcaggcacct gcagaggagccccgccgctccgccgactagctgcccccgcgagcaacggcct cgtgatttcccccgccgatccggtccccgcctccccactctgcccccgcctacccc ggagccgtgcagccgcctctccgaatctctctcttctcctggcgctcgcgtgcga gagggaactagcgagaacgaggaagcagctggaggtgacgccgggcagatt acgcctgtcagggccgagccgagcggatcgctgggcgctgtgcagaggaaa ggcgggagtgcccggctcgctgtcgcagagccgaggtgggtaagctagcgac cacctggacttcccagcgcccaaccgtggctttcagccaggtcctctcctcccg cggcttctcaaccaaccccatcccagcgccggccacccaacctcccgaaatga gtgcttcctgccccagcagccgaaggcgctactaggaacggtaacctgttacttt tccaggggccgtagtcgacccgctgcccgagttgctgtgcgactgcgcgcgcg gggctagagtgcaaggtgactgtggttcttctctggccaagtccgagggagaac gtaaagatatgggcctttttccccctctcaccttgtctcaccaaagtccctagtccc cggagcagttagcctcttctctttccagggaattagccagacacaacaacgggaac cagacaccgaaccagacatgcccgccccgtgcgccctccccgctcgctgcctt cctccctcttgtctctccagagccggatcttcaaggggagcctccgtgcccccgg ctgctcagtccctccggtgtgcaggaccccggaagtcctccccgcacagctctc gcttctctttgcagcctgtttctgcgccggaccagtcgaggactctggacagtaga ggccccgggacgaccgagctgGAATTCGCCACCATGGCCCC AAAGAAGAAGCGGAAGGTCGGTATCCACGGAGT CCCAGCAGCCCTCGAACCAGGTGAAAAACCTTA CAAATGTCCTGAATGTGGGAAATCATTCAGTCGC AGCGACAACCTGGTGAGACATCAACGCACCCAT ACAGGAGAAAAACCTTATAAATGTCCAGAATGT GGAAAGTCCTTCTCACGAGAGGATAACTTGCAC ACTCATCAACGAACACATACTGGTGAAAAACCA TACAAGTGTCCCGAATGTGGTAAAAGTTTTAGCC GGAGCGATGAACTTGTCCGACACCAACGAACCC ATACAGGCGAGAAGCCTTACAAATGTCCCGAGT GTGGCAAGAGCTTCTCACAATCAGGGAATCTGA CTGAGCATCAACGAACTCATACCGGGGAAAAAC CTTACAAGTGTCCAGAGTGTGGGAAGAGCTTTTC CACAAGTGGACATCTGGTACGCCACCAGAGGAC ACATACAGGGGAGAAGCCCTACAAATGCCCCGA ATGCGGTAAAAGTTTCTCTCAGAATAGTACCCTG ACCGAACACCAGCGAACACACTGGGAAAAAA ACGAGTAAAAGGCCGGCGGCCACGAAAAAGGCC GGCCAGGCAAAAAAGAAAAAGGGATCCGACGC GCTGGACGATTTCGATCTCGACATGCTGGGTTCT GATGCCCTCGATGACTTTGACCTGGATATGTTGG GAAGCGACGCATTGGATGACTTTGATCTGGACAT GCTCGGCTCCGATGCTCTGGACGATTTCGATCTC GATATGTTATAAaaagagaccggttcactgtgacagtaaaagagac cggttcactgtgagaatgaaagagaccggttcactgtgatcggaaaagagaccg gttcactgtgagcggccttgaaacccagcagacaatgtagctcagtagaaaccc | |

(continued)

| CONSTRUCT | SEQUENCE | SEQ ID NO |
|---|---|---|
|  | agcagacaatgtagctgaatggaaacccagcagacaatgtagcttcggagaaacccagcagacaatgtagctAAGCTTGGGTGGCATCCCTGTGACCCCTCCCCAGTGCCTCTCCTGGCCCTGGAAGTTGCCACTCCAGTGCCCACCAGCCTTGTCCTAATAAAATTAAGTTGCATCATTTTGTCTGACTAGGTGTCCTTCTATAATATTATGGGGTGGAGGGGGGTGGTATGGAGCAAGGGGCAAGTTGGGAAGACAACCTGTAGGGCCTGCGGGGTCTATTGGGAACCAAGCTGGAGTGCAGTGGCACAATCTTGGCTCACTGCAATCTCCGCCTCCTGGGTTCAAGCGATTCTCCTGCCTCAGCCTCCCGAGTTGTTGGGATTCCAGGCATGCATGACCAGGCTCAGCTAATTTTTGTTTTTTTGGTAGAGACGGGGTTTCACCATATTGGCCAGGCTGGTCTCCAACTCCTAATCTCAGGTGATCTACCCACCTTGGCCTCCCAAATTGCTGGGATTACAGGCGTGAACCACTGCTCCCTTCCCTGTCCTT |  |

(continued)

| CONSTRUCT | SEQUENCE | SEQ ID NO |
|---|---|---|
| 40 (coding) | ATGGCCGCAGATCACCTGATGCTGGCTGAAGGCT ACAGACTGGTGCAGCGGCCTCCATCTGCCGCTGC CGCCCACGGCCCCCACGCCCTGAGAACACTGCCC CCCTACGCCGGCCCTGGTCTTGATAGCGGACTCA GACCTAGAGGCGCCCCTCTGGGCCCTCCACCTCC AAGACAGCCTGGAGCCCTGGCCTACGGCGCCTTC GGCCCTCCTTCTAGCTTCCAGCCCTTCCCCGCCGT GCCTCCTCCAGCcGCTGGCATCGCCCACCTGCAG CCTGTGGCCACCCCTTACCCCGGAAGAGCCGCCG CCCCTCCAAACGCCCCTGGCGGACCTCCTGGCCC CCAGCCTGCTCCAAGCGCCGCTGCCCCTCCACCT CCTGCTCATGCCCTGGGCGGCATGGACGCCGAGC TGATCGACGAGGAAGCCCTGACCAGCCTGGAAC TGGAACTGGGCCTGCACAGAGTGCGGGAACTGC CTGAGCTGTTCCTGGGACAGAGCGAGTTCGACTG CTTCAGCGACCTGGGCAGCGCCCTCCTGCCGGC TCTGTGTCCTGCgccgaccacctgatgctcgccgagggctaccgcct ggtgcagaggccgccgtccgccgccgccgcccatggccctcatgcgctccgg actctgccgccgtacgcgggccccgggcctggacagtgggctgaggccgcgg ggggctccgctggggccgccgccgccccgccaacccggggccctggcgtac ggggccttcgggccgccgtcctccttccagcccctttccggccgtgcctccgccg gccgcgggcatcgcgcacctgcagcctgtggcgacgccgtaccccggccgc gcCgccgcgccccccaacgctccgggagggcccccgggcccgcagccggc cccaagcgccgcagccccgccgccgcccgcgcacgccctgggcggcatgga cgccgaactcatcgacgaggaggcgctgacgtcgctggagctggagctgggg ctgcaccgcgtgcgcgagctgcccgagctgttcctgggccagagcgagttcga ctgcttctcggacttggggtccgcgccgcccgccggctccgtgagctgccagtc ccagctcatcaaacccagccgcatgcgcaagtaccccaaccggcccagcaag acgccccccacgaacgcccttacgcttgcccagtggagtcctgtgatcgccgc ttctccCGCAGCGACAACCTGGTGAGAcacatccgcatccac acaggccagaagcccttccagtgccgcatctgcatgAGAaacttcagcCG AGAGGATAACTTGCACACTcacatccgcacccacacaggcg aaaagcccttcgcctgcgacatctgtggaagaaagtttgccCGGAGCGA TGAACTTGTCCGAcataccaagatccacttgcggcagaaggaccgc<br><br>ccttacgcttgcccagtggagtcctgtgatcgccgcttctccCAATCAGG GAATCTGACTGAGcacatccgcatccacacaggccagaagcccttc cagtgccgcatctgcatgAGAaacttcagcACAAGTGGACATCT GGTACGCcacatccgcacccacacaggcgaaaagcccttcgcctgcgac atctgtggaagaaagtttgccCAGAATAGTACCCTGACCGAA cataccaagatccacttgcggcagaaggacaag | 72 |

(continued)

| CONSTRUCT | SEQUENCE | SEQ ID NO |
|---|---|---|
| 41 (coding) | ATGGCCGCAGATCACCTGATGCTGGCTGAAGGCT ACAGACTGGTGCAGCGGCCTCCATCTGCCGCTGC CGCCCACGGCCCCCACGCCCTGAGAACACTGCCC CCCTACGCCGGCCTGGTCTTGATAGCGGACTCA GACCTAGAGGCGCCCCTCTGGGCCCTCCACCTCC AAGACAGCCTGGAGCCCTGGCCTACGGCGCCTTC GGCCCTCCTTCTAGCTTCCAGCCCTTCCCCGCCGT GCCTCCTCCAGCTGCTGGCATCGCCCACCTGCAG CCTGTGGCCACCCCTTACCCCGGAAGAGCCGCCG CCCCTCCAAACGCCCCTGGCGGACCTCCTGGCCC CCAGCCTGCTCCAAGCGCCGCTGCCCCTCCACCT CCTGCTCATGCCCTGGGCGGCATGGACGCCGAGC TGATCGACGAGGAAGCCCTGACCAGCCTGGAAC TGGAACTGGGCCTGCACAGAGTGCGGGAACTGC CTGAGCTGTTCCTGGGACAGAGCGAGTTCGACTG CTTCAGCGACCTGGGCAGCGCCCTCCTGCCGGC TCTGTGTCCTGCGGCGGCAGCGGCGGCGGAAGC GGCgccgaccacctgatgctcgccgagggctaccgcctggtgcagaggcc gccgtccgccgccgccgcccatggccctcatgcgctccggactctgccgccgt acgcgggcccgggcctggacagtgggctgaggccgcggggggctccgctgg ggccgccgccgccccgccaacccggggccctggcgtacggggccttcgggc cgccgtcctccttccagccctttccggccgtgcctccgccggccgcgggcatcg cgcacctgcagcctgtggcgacgccgtaccccggccgcgcggccgcgcccc ccaacgctccgggaggccccccgggcccgcagccggcccccaagcgccgca gccccgccgccgcccgcgcacgccctgggcggcatggacgccgaactcatc gacgaggaggcgctgacgtcgctggagctggagctggggctgcaccgcgtgc gcgagctgcccgagctgttcctgggccagagcgagttcgactgcttctcggactt ggggtccgcgccgcccgccggctccgtgagctgcggtggttctggtggtggtt ctggtcagtcccagctcatcaaacccagccgcatgcgcaagtaccccaaccgg cccagcaagacgcccccccacgaacgcccttacgcttgcccagtggagtcctgt gatcgccgcttctccCGCAGCGACAACCTGGTGAGAcacatc cgcatccacacaggccagaagcccttccagtgccgcatctgcatgAGAaact tcagcCGAGAGGATAACTTGCACACTcacatccgcacccac acaggcgaaaagcccttcgcctgcgacatctgtggaagaaagtttgccCGG AGCGATGAACTTGTCCGAcataccaagatccacttgcggcaga aggaccgcccttacgcttgcccagtggagtcctgtgatcgccgcttctccCAA TCAGGGAATCTGACTGAGcacatccgcatccacacaggccag aagcccttccagtgccgcatctgcatgAGAaacttcagcACAAGTGG ACATCTGGTACGCcacatccgcacccacacaggcgaaaagcccttc gcctgcgacatctgtggaagaaagtttgccCAGAATAGTACCCTG ACCGAAcataccaagatccacttgcggcagaaggacaag | 73 |
| 42 (RE + Coding) | ggaggaagccatcaactaaactacaatgactgtaagatacaaaattgggaatggt aacatattttgaagttctgttgacataaagaatcatgatattaatgcccatggaaatg aaagggcgatcaacactatggtttgaaaagggggaaattgtagagcacagatgt | 74 |

(continued)

| CONSTRUCT | SEQUENCE | SEQ ID NO |
|-----------|----------|-----------|
| | gttcgtgtggcagtgtgctgtctctagcaatactcagagaagagagagaacaatg aaattctgattggccccagtgtgagcccagatgaggttcagctgccaactttctctt tcacatcttatgaaagtcatttaagcacaactaactttttttttttttttttttttttgagaca gagtcttgctctgttgcccaggacagagtgcagtagtgactcaatctcggctcact gcagcctccacctcctaggctcaaacggtcctcctgcatcagcctcccaagtagc tggaattacaggagtggcccaccatgcccagctaattttttgtattttttaatagatacg ggggtttcaccatatcacccaggctggtctcgaactcctggcctcaagtgatcca cctgcctcggcctcccaaagtgctgggattataggcgtcagccactatgcccaac ccgaccaaccttttttaaaataaatatttaaaaaattggtatttcacatatatactagta tttacatttatccacacaaaacggacgggcctccgctgaaccagtgaggcccca gacgtgcgcataaataacccctgcgtgctgcaccacctgggggagagggggag gaccacggtaaatggagcgagcgcatagcaaaagggacgcgggggtcctttct ctgccggtggcactgggtagctgtggccaggtgtggtactttgatgggggcccag ggctggagctcaaggaagcgtcgcagggtcacagatctgggggggaaccccgg ggaaaagcactgaggcaaaaccgccgctcgtctcctacaatatatgggagggg gaggttgagtacgttctggattactcataagacctttttttttttccttccgggcgcaaa accgtgagctggatttataatcgccctataaagctccagaggcggtcaggcacct gcagaggagccccgccgctccgccgactagctgcccccgcgagcaacggcct cgtgatttccccgccgatccggtccccgcctcccactctgcccccgcctacccc ggagccgtgcagccgcctctccgaatctctctcttctcctggcgctcgcgtgcga gagggaactagcgagaacgaggaagcagctggaggtgacgccgggcagatt acgcctgtcagggccgagccgagcggatcgctgggcgctgtgcagaggaaa ggcgggagtgcccggctcgctgtcgcagagccgaggtgggtaagctagcgac cacctggacttcccagcgcccaaccgtggcttttcagccaggtcctctcctcccg cggcttctcaaccaaccccatcccagcgccggccacccaacctcccgaaatga gtgcttcctgccccagcagccgaaggcgctactaggaacggtaacctgttacttt tccaggggccgtagtcgacccgctgcccgagttgctgtgcgactgcgcgcgcg gggctagagtgcaaggtgactgtggttcttctctggccaagtccgagggagaac gtaaagatatgggcctttttcccctctcaccttgtctcaccaaagtccctagtccc cggagcagttagcctctttctttccagggaattagccagacacaacaacgggaac cagacaccgaaccagacatgcccgccccgtgcgccctccccgctcgctgcctt cctccctcttgtctctccagagccggatcttcaagggggagcctccgtgccccgg ctgctcagtccctccggtgtgcaggaccccggaagtcctcccgcacagctctc gcttctctttgcagcctgtttctgcgccggaccagtcgaggactctggacagtaga ggccccgggacgaccgagctgGAATTCGCCACCATGGCCCC AAAGAAGAAGCGGAAGGTCGGTATCCACGGAGT CCCAGCAGCCCTCGAACCAGGTGAAAAACCTTA CAAATGTCCTGAATGTGGGAAATCATTCAGTCGC AGCGACAACCTGGTGAGACATCAACGCACCCAT ACAGGAGAAAAACCTTATAAATGTCCAGAATGT GGAAAGTCCTTCTCACGAGAGGATAACTTGCAC ACTCATCAACGAACACATACTGGTGAAAAACCA TACAAGTGTCCCGAATGTGGTAAAAGTTTTAGCC GGAGCGATGAACTTGTCCGACACCAACGAACCC ATACAGGCGAGAAGCCTTACAAATGTCCCGAGT GTGGCAAGAGCTTCTCACAATCAGGGAATCTGA CTGAGCATCAACGAACTCATACCGGGGAAAAAC CTTACAAGTGTCCAGAGTGTGGGAAGAGCTTTTC CACAAGTGGACATCTGGTACGCCACCAGAGGAC ACATACAGGGGAGAAGCCCTACAAATGCCCCGA | |

(continued)

| CONSTRUCT | SEQUENCE | SEQ ID NO |
|---|---|---|
|  | ATGCGGTAAAAGTTTCTCTCAGAATAGTACCCTG ACCGAACACCAGCGAACACACTGGGAAAAAA ACGAGTAAAAGGCCGGCGGCCACGAAAAAGGCC GGCCAGGCAAAAAGAAAAAGGGATCCTACCCA TACGACGTACCAGATTACGCTCTCGAGGACGCGC TGGACGATTTCGATCTCGACATGCTGGGTTCTGA TGCCCTCGATGACTTTGACCTGGATATGTTGGGA AGCGACGCATTGGATGACTTTGATCTGGACATGC TCGGCTCCGATGCTCTGGACGATTTCGATCTCGA TATGTTATAAACTAGTGAAACCCAGCAGACAAT GTAGCTAGACCCAGTAGCCAGATGTAGCTAAAG AGACCGGTTCACTGTGAAAGCTTGGGTGGCATCC CTGTGACCCCTCCCCAGTGCCTCTCCTGGCCCTG GAAGTTGCCACTCCAGTGCCCACCAGCCTTGTCC TAATAAAATTAAGTTGCATCATTTTGTCTGACTA GGTGTCCTTCTATAATATTATGGGGTGGAGGGGG GTGGTATGGAGCAAGGGGCAAGTTGGGAAGACA ACCTGTAGGGCCTGCGGGGTCTATTGGGAACCA AGCTGGAGTGCAGTGGCACAATCTTGGCTCACTG CAATCTCCGCCTCCTGGGTTCAAGCGATTCTCCT GCCTCAGCCTCCCGAGTTGTTGGGATTCCAGGCA TGCATGACCAGGCTCAGCTAATTTTTGTTTTTTTG GTAGAGACGGGGTTTCACCATATTGGCCAGGCTG GTCTCCAACTCCTAATCTCAGGTGATCTACCCAC CTTGGCCTCCCAAATTGCTGGGATTACAGGCGTG AACCACTGCTCCCTTCCCTGTCCTT |  |

(continued)

| CONSTRUCT | SEQUENCE | SEQ ID NO |
|---|---|---|
| 43 (RE + Coding) | ggaggaagccatcaactaaactacaatgactgtaagatacaaaattgggaatggt aacatattttgaagttctgttgacataaagaatcatgatattaatgcccatggaaatg aaagggcgatcaacactatggtttgaaaaggggggaaattgtagagcacagatgt gttcgtgtggcagtgtgctgtctctagcaatactcagagaagagagagagaacaatg aaattctgattggccccagtgtgagcccagatgaggttcagctgccaactttctctt tcacatcttatgaaagtcatttaagcacaactaactttttttttttttttttttttttgagaca gagtcttgctctgttgcccaggacagagtgcagtagtgactcaatctcggctcact gcagcctccacctcctaggctcaaacggtcctcctgcatcagcctcccaagtagc tggaattacaggagtggcccaccatgcccagctaattttgtattttaatagatacg ggggtttcaccatatcacccaggctggtctcgaactcctggcctcaagtgatcca cctgcctcggcctcccaaagtgctgggattataggcgtcagccactatgcccaac ccgaccaaccttttttaaaataaatatttaaaaaaattggtatttcacatatatactagta tttacatttatccacacaaaacggacgggcctccgctgaaccagtgaggcccca gacgtgcgcataaataaccccctgcgtgctgcaccacctggggagaggggggag gaccacggtaaatggagcgagcgcatagcaaaagggacgcggggtccttttct ctgccggtggcactgggtagctgtggccaggtgtggtactttgatggggcccag ggctggagctcaaggaagcgtcgcaggtcacagatctgggggaacccccgg ggaaaagcactgaggcaaaaccgccgctcgtctcctacaatatatgggagggg gaggttgagtacgttctggattactcataagaccttttttttttccttccgggcgcaaa accgtgagctggatttataatcgccctataaagctccagaggcggtcaggcacct gcagaggagccccgccgctccgccgactagctgccccgcgagcaacggcct cgtgatttccccgccgatccggtccccgcctccccactctgcccccgcctacccc ggagccgtgcagccgcctctccgaatctctctcttctcctggcgctcgcgtgcga gagggaactagcgagaacgaggaagcagctggaggtgacgccgggcagatt | 75 |

(continued)

| CONSTRUCT | SEQUENCE | SEQ ID NO |
|---|---|---|
| | acgcctgtcagggccgagccgagcggatcgctgggcgctgtgcagaggaaa ggcgggagtgcccggctcgctgtcgcagagccgaggtgggtaagctagcgac cacctggacttcccagcgcccaaccgtggcttttcagccaggtcctctcctcccg cggcttctcaaccaaccccatcccagcgccggccacccaacctcccgaaatga gtgcttcctgccccagcagccgaaggcgctactaggaacggtaacctgttacttt tccaggggccgtagtcgacccgctgcccgagttgctgtgcgactgcgcgcgcg gggctagagtgcaaggtgactgtggttcttctctggccaagtccgagggagaac gtaaagatatgggcctttttcccctctcaccttgtctcaccaaagtccctagtccc cggagcagttagcctctttctttccagggaattagccagacacaacaacgggaac cagacaccgaaccagacatgcccgccccgtgcgccctccccgctcgctgccttt cctccctcttgtctctccagagccggatcttcaaggggagcctccgtgcccccgg ctgctcagtccctccggtgtgcaggaccccggaagtcctcccccgcacagctctc gcttctctttgcagcctgtttctgcgccggaccagtcgaggactctggacagtaga ggccccgggacgaccgagctgGAATTCGCCACCATGGCCCC AAAGAAGAAGCGGAAGGTCGGTATCCACGGAGT CCCAGCAGCCCTCGAACCAGGTGAAAAACCTTA CAAATGTCCTGAATGTGGGAAATCATTCAGTCGC AGCGACAACCTGGTGAGACATCAACGCACCCAT ACAGGAGAAAAACCTTATAAATGTCCAGAATGT GGAAAGTCCTTCTCACGAGAGGATAACTTGCAC ACTCATCAACGAACACATACTGGTGAAAAACCA TACAAGTGTCCCGAATGTGGTAAAAGTTTTAGCC GGAGCGATGAACTTGTCCGACACCAACGAACCC ATACAGGCGAGAAGCCTTACAAATGTCCCGAGT GTGGCAAGAGCTTCTCACAATCAGGGAATCTGA CTGAGCATCAACGAACTCATACCGGGGAAAAAC CTTACAAGTGTCCAGAGTGTGGGAAGAGCTTTTC CACAAGTGGACATCTGGTACGCCACCAGAGGAC ACATACAGGGGAGAAGCCCTACAAATGCCCCGA ATGCGGTAAAAGTTTCTCTCAGAATAGTACCCTG ACCGAACACCAGCGAACACACACTGGGAAAAAA ACGAGTAAAAGGCCGGCGGCCACGAAAAAGGCC GGCCAGGCAAAAAAGAAAAAGGGATCCTACCCA TACGACGTACCAGATTACGCTCTCGAGGACGCGC TGGACGATTTCGATCTCGACATGCTGGGTTCTGA TGCCCTCGATGACTTTGACCTGGATATGTTGGGA AGCGACGCATTGGATGACTTTGATCTGGACATGC TCGGCTCCGATGCTCTGGACGATTTCGATCTCGA TATGTTATAAACTAGTGAAACCCAGCAGACAAT GTAGCTAGACCCAGTAGCCAGATGTAGCTAAAG AGACCGGTTCACTGTGAGAAACCCAGCAGACAA TGTAGCTAGACCCAGTAGCCAGATGTAGCTAAA GAGACCGGTTCACTGTGAAAGCTTGGGTGGCATC CCTGTGACCCCTCCCCAGTGCCTCTCCTGGCCCT GGAAGTTGCCACTCCAGTGCCCACCAGCCTTGTC CTAATAAAATTAAGTTGCATCATTTTGTCTGACT AGGTGTCCTTCTATAATATTATGGGGTGGAGGGG GGTGGTATGGAGCAAGGGGCAAGTTGGGAAGAC AACCTGTAGGGCCTGCGGGGTCTATTGGGAACC AAGCTGGAGTGCAGTGGCACAATCTTGGCTCACT | |

(continued)

| CONSTRUCT | SEQUENCE | SEQ ID NO |
|---|---|---|
| | GCAATCTCCGCCTCCTGGGTTCAAGCGATTCTCC TGCCTCAGCCTCCCGAGTTGTTGGGATTCCAGGC ATGCATGACCAGGCTCAGCTAATTTTTGTTTTTTT GGTAGAGACGGGGTTTCACCATATTGGCCAGGCT GGTCTCCAACTCCTAATCTCAGGTGATCTACCCA CCTTGGCCTCCCAAATTGCTGGGATTACAGGCGT GAACCACTGCTCCCTTCCCTGTCCTT | |

(continued)

| CONSTRUCT | SEQUENCE | SEQ ID NO |
|---|---|---|
| 44 (RE + Coding) | ggaggaagccatcaactaaactacaatgactgtaagatacaaaattgggaatggt aacatattttgaagttctgttgacataaagaatcatgatattaatgcccatggaaatg aaagggcgatcaacactatggtttgaaaaggggggaaattgtagagcacagatgt gttcgtgtggcagtgtgctgtctctagcaatactcagagaagagagagagaacaatg aaattctgattggccccagtgtgagcccagatgaggttcagctgccaactttctctt tcacatcttatgaaagtcatttaagcacaactaactttttttttttttttttttttttgagaca gagtcttgctctgttgcccaggacagagtgcagtagtgactcaatctcggctcact gcagcctccacctcctaggctcaaacggtcctcctgcatcagcctcccaagtagc tggaattacaggagtggcccaccatgcccagctaatttttgtattttaatagatacg ggggtttcaccatatcacccaggctggtctcgaactcctggcctcaagtgatcca cctgcctcggcctcccaaagtgctgggattataggcgtcagccactatgcccaac ccgaccaacctttttaaaataaatatttaaaaaattggtatttcacatatatactagta tttacatttatccacacaaaacggacgggcctccgctgaaccagtgaggcccca gacgtgcgcataaataacccctgcgtgctgcaccacctggggagagggggag gaccacggtaaatggagcgagcgcatagcaaaagggacgcggggtccttttct ctgccggtggcactgggtagctgtggccaggtgtggtactttgatggggcccag ggctggagctcaaggaagcgtcgcagggtcacagatctgggggaaccccgg ggaaaagcactgaggcaaaaccgccgctcgtctcctacaatatatgggaggg gaggttgagtacgttctggattactcataagacctttttttttttccttccgggcgcaaa accgtgagctggatttataatcgccctataaagctccagaggcggtcaggcacct gcagaggagccccgccgctccgccgactagctgcccccgcgagcaacggcct cgtgatttccccgccgatccggtccccgcctcccactctgcccccgcctaccc ggagccgtgcagccgcctctccgaatctctctcttctcctggcgctcgcgtgcga gagggaactagcgagaacgaggaagcagctggaggtgacgccgggcagatt acgcctgtcagggccgagccgagcggatcgctgggcgctgtgcagaggaaa ggcgggagtgcccggctcgctgtcgcagagccgaggtgggtaagctagcgac cacctggacttcccagcgcccaaccgtggcttttcagccaggtcctctcctcccg cggcttctcaaccaacccatcccagcgccggccacccaacctcccgaaatga gtgcttcctgcccccagcagccgaaggcgctactaggaacggtaacctgttacttt tccaggggccgtagtcgacccgctgcccgagttgctgtgcgactgcgcgcgcg gggctagagtgcaaggtgactgtggttcttctctggccaagtccgagggagaac gtaaagatatgggcctttttccccctctcaccttgtctcaccaaagtccctagtccc cggagcagttagcctctttctttccagggaattagccagacacaacaacgggaac cagacaccgaaccagacatgcccgccccgtgcgccctccccgctcgctgcctt cctccctcttgtctctccagagccggatcttcaggggagcctccgtgcccccgg ctgctcagtccctccggtgtgcaggaccccggaagtcctcccccgcacagctctc gcttctctttgcagcctgtttctgcgccggaccagtcgaggactctggacagtaga ggccccgggacgaccgagctg**GAATTCGCCACCATGGCC**gc cgaccacctgatgctcgccgagggctaccgcctggtgcagaggccgccgtcc gccgccgccgcccatggccctcatgcgctccggactctgccgccgtacgcggg cccggggcctggacagtgggctgaggccgcggggggctccgctggggccgcc gccgccccgccaacccggggccctggcgtacggggccttcgggccgccgtc ctccttccagccctttccggccgtgcctccgccggccgcgggcatcgcgcacct | 76 |

(continued)

| CONSTRUCT | SEQUENCE | SEQ ID NO |
|---|---|---|
| | gcagcctgtggcgacgccgtaccccggccgcgcggccgcgccccccaacgc tccgggaggccccccgggcccgcagccggccccaagcgccgcagccccgc cgccgcccgcgcacgccctgggcggcatggacgccgaactcatcgacgagg aggcgctgacgtcgctggagctggagctggggctgcaccgcgtgcgcgagct gcccgagctgttcctgggccagagcgagttcgactgcttctcggacttggggtc cgcgccgcccgccggctccgtgagctgcggtggttctggtggtggtggttctggtca gtcccagctcatcaaacccagccgcatgcgcaagtaccccaaccggcccagca agacgccccccacgaacgcccttacgcttgcccagtggagtcctgtgatcgcc gcttctccCGCAGCGACAACCTGGTGAGAcacatccgcatcc acacaggccagaagcccttccagtgccgcatctgcatgAGAaacttcagcC GAGAGGATAACTTGCACACTcacatccgcacccacacaggc gaaaagcccttcgcctgcgacatctgtggaagaaagtttgccCGGAGCG ATGAACTTGTCCGAcataccaagatccacttgcggcagaaggacc gcccttacgcttgcccagtggagtcctgtgatcgccgcttctccCAATCAG GGAATCTGACTGAGcacatccgcatccacacaggccagaagccct tccagtgccgcatctgcatgAGAaacttcagcACAAGTGGACATC TGGTACGCcacatccgcacccacacaggcgaaaagcccttcgcctgcg acatctgtggaagaaagtttgccCAGAATAGTACCCTGACCG AAcataccaagatccacttgcggcagaaggacaagtaaCTCGAGGA AACCCAGCAGACAATGTAGCTAGACCCAGTAGC CAGATGTAGCTAAAGAGACCGGTTCACTGTGAA AGCTTGGGTGGCATCCCTGTGACCCCTCCCCAGT GCCTCCTGGCCCTGGAAGTTGCCACTCCAGTG CCCACCAGCCTTGTCCTAATAAAATTAAGTTGCA TCATTTTGTCTGACTAGGTGTCCTTCTATAATATT ATGGGGTGGAGGGGGGTGGTATGGAGCAAGGGG CAAGTTGGGAAGACAACCTGTAGGGCCTGCGGG GTCTATTGGGAACCAAGCTGGAGTGCAGTGGCA CAATCTTGGCTCACTGCAATCTCCGCCTCCTGGG TTCAAGCGATTCTCCTGCCTCAGCCTCCCGAGTT GTTGGGATTCCAGGCATGCATGACCAGGCTCAGC TAATTTTTGTTTTTTTGGTAGAGACGGGGTTTCAC CATATTGGCCAGGCTGGTCTCCAACTCCTAATCT CAGGTGATCTACCCACCTTGGCCTCCCAAATTGC TGGGATTACAGGCGTGAACCACTGCTCCCTTCCC TGTCCTT | |

(continued)

| CONSTRUCT | SEQUENCE | SEQ ID NO |
|---|---|---|
| 45 (RE + Coding) | ggaggaagccatcaactaaactacaatgactgtaagatacaaaattgggaatggt aacatattttgaagttctgttgacataaagaatcatgatattaatgcccatggaaatg aaagggcgatcaacactatggtttgaaaaggggggaaattgtagagcacagatgt gttcgtgtggcagtgtgctgtctctagcaatactcagagaagagagagaacaatg aaattctgattggccccagtgtgagcccagatgaggttcagctgccaactttctctt tcacatcttatgaaagtcatttaagcacaactaactttttttttttttttttttttttgagaca gagtcttgctctgttgcccaggacagagtgcagtagtgactcaatctcggctcact gcagcctccacctcctaggctcaaacggtcctcctgcatcagcctcccaagtagc tggaattacaggagtggcccaccatgcccagctaattttgtattttaatagatacg ggggtttcaccatatcacccaggctggtctcgaactcctggcctcaagtgatcca cctgcctcggcctcccaaagtgctgggattataggcgtcagccactatgcccaac ccgaccaaccttttttaaaataaatatttaaaaaattggtatttcacatatatactagta tttacatttatccacacaaaacggacgggcctccgctgaaccagtgaggcccca gacgtgcgcataaataacccctgcgtgctgcaccacctggggagagggggag | 184 |

(continued)

| CONSTRUCT | SEQUENCE | SEQ ID NO |
|---|---|---|
| | gaccacggtaaatggagcgagcgcatagcaaaagggacgcggggtccttttct ctgccggtggcactgggtagctgtggccaggtgtggtactttgatggggcccag ggctggagctcaaggaagcgtcgcagggtcacagatctgggggaaccccgg ggaaaagcactgaggcaaaaccgccgctcgtctcctacaatatatgggaggg gaggttgagtacgttctggattactcataagacctttttttttttccttccgggcgcaaa accgtgagctggatttataatcgccctataaagctccagaggcggtcaggcacct gcagaggagccccgccgctccgccgactagctgcccccgcgagcaacggcct cgtgatttccccgccgatccggtccccgcctccccactctgcccccgcctacccc ggagccgtgcagccgcctctccgaatctctctcttctcctggcgctcgcgtgcga gagggaactagcgagaacgaggaagcagctggaggtgacgccgggcagatt acgcctgtcagggccgagccgagcggatcgctgggcgctgtgcagaggaaa ggcgggagtgccccggctcgctgtcgcagagccgaggtgggtaagctagcgac cacctggacttcccagcgcccaaccgtggcttttcagccaggtcctctcctcccg cggcttctcaaccaaccccatcccagcgccggccacccaacctcccgaaatga gtgcttcctgccccagcagccgaaggcgctactaggaacggtaacctgttacttt tccaggggccgtagtcgacccgctgcccgagttgctgtgcgactgcgcgcgcg gggctagagtgcaaggtgactgtggttcttctctggccaagtccgagggagaac gtaaagatatgggccttttttccccctctcaccttgtctcaccaaagtccctagtccc cggagcagttagcctctttctttccagggaattagccagacacaacaacgggaac cagacaccgaaccagacatgcccgccccgtgcgccctccccgctcgctgccttt cctccctcttgtctctccagagccggatcttcaaggggagcctccgtgcccccgg ctgctcagtccctccggtgtgcaggacccccggaagtcctccccgcacagctctc gcttctctttgcagcctgtttctgcgccggaccagtcgaggactctggacagtaga ggccccgggacgaccgagctgGAATTCGCCACCATGGCCgc cgaccacctgatgctcgccgagggctaccgcctggtgcagaggccgccgtcc gccgccgccgcccatggccctcatgcgctccggactctgccgccgtacgcggg cccgggcctggacagtgggctgaggccgcggggggctccgctggggccgcc gccgccccgccaacccggggccctggcgtacggggccttcgggccgccgtc ctccttccagcccctttccggccgtgcctccgccggccgcgggcatcgcgcacct gcagcctgtggcgacgccgtaccccggccgcgcggccgcgccccccaacgc tccgggaggcccccgggcccgcagccggccccaagcgccgcagccccgc cgccgcccgcgcacgccctgggcggcatggacgccgaactcatcgacgagg aggcgctgacgtcgctggagctggagctggggctgcaccgcgtgcgcgagct gccccgagctgttcctgggccagagcgagttcgactgcttctcggacttggggtc cgcgccgcccgccggctccgtgagctgcggtggttctggtggtggttctggtca gtcccagctcatcaaacccagccgcatgcgcaagtaccccaaccggcccagca agacgcccccccacgaacgcccttacgcttgcccagtggagtcctgtgatcgcc gcttctccCGCAGCGACAACCTGGTGAGAcacatccgcatcc acacaggccagaagcccttccagtgccgcatctgcatgAGAaacttcagcC GAGAGGATAACTTGCACACTcacatccgcacccacacaggc gaaaagcccttcgcctgcgacatctgtggaagaaagtttgccCGGAGCG ATGAACTTGTCCGAcataccaagatccacttgcggcagaaggacc gcccttacgcttgcccagtggagtcctgtgatcgccgcttctccCAATCAG GGAATCTGACTGAGcacatccgcatccacacaggccagaagccct tccagtgccgcatctgcatgAGAaacttcagcACAAGTGGACATC TGGTACGCcacatccgcacccacacaggcgaaaagcccttcgcctgcg acatctgtggaagaaagtttgccCAGAATAGTACCCTGACCG AAcataccaagatccacttgcggcagaaggacaagtaaCTCGAGGA AACCCAGCAGACAATGTAGCTAGACCCAGTAGC CAGATGTAGCTAAAGAGACCGGTTCACTGTGAG | |

(continued)

| CONSTRUCT | SEQUENCE | SEQ ID NO |
|---|---|---|
|  | AAACCCAGCAGACAATGTAGCTAGACCCAGTAG CCAGATGTAGCTAAAGAGACCGGTTCACTGTGA AAGCTTGGGTGGCATCCCTGTGACCCCTCCCCAG TGCCTCTCCTGGCCCTGGAAGTTGCCACTCCAGT GCCCACCAGCCTTGTCCTAATAAAATTAAGTTGC ATCATTTTGTCTGACTAGGTGTCCTTCTATAATAT TATGGGGTGGAGGGGGGTGGTATGGAGCAAGGG GCAAGTTGGGAAGACAACCTGTAGGGCCTGCGG GGTCTATTGGGAACCAAGCTGGAGTGCAGTGGC ACAATCTTGGCTCACTGCAATCTCCGCCTCCTGG GTTCAAGCGATTCTCCTGCCTCAGCCTCCCGAGT TGTTGGGATTCCAGGCATGCATGACCAGGCTCAG CTAATTTTTGTTTTTTTGGTAGAGACGGGGTTTCA CCATATTGGCCAGGCTGGTCTCCAACTCCTAATC TCAGGTGATCTACCCACCTTGGCCTCCCAAATTG CTGGGATTACAGGCGTGAACCACTGCTCCCTTCC CTGTCCTT |  |
| 8 (coding) | ATGGCCgccgaccacctgatgctcgccgagggctaccgcctggtgcaga ggccgccgtccgccgccgccgcccatggccctcatgcgctccggactctgccg ccgtacgcgggcccgggcctggacagtgggctgaggccgcgggggggctccg ctggggccgccgccgcccccgccaacccggggccctggcgtacggggccttc gggccgccgtcctccttccagccctttccggccgtgcctccgccggccgcggg catcgcgcacctgcagcctgtggcgacgccgtaccccggccgcgcggccgcg ccccccaacgctccggggaggcccccgggcccgcagccggccccaagcgcc gcagccccgccgccgcccgcgcacgccctgggcggcatggacgccgaactc atcgacgaggaggcgctgacgtcgctggagctggagctggggctgcaccgcg tgcgcgagctgcccgagctgttcctgggccagagcgagttcgactgcttctcgg acttggggtccgcgccgcccgccggctccgtgagctgcggtggttctggtggtg gttctggtcagtcccagctcatcaaacccagccgcatgcgcaagtaccccaacc ggcccagcaagacgcccccccacgaacgcccttacgcttgcccagtggagtcc tgtgatcgccgcttctccCGCAGCGACAACCTGGTGAGAcac atccgcatccacacaggccagaagccettccagtgccgcatctgcatgAGAa acttcagcCGAGAGGATAACTTGCACACTcacatccgcacc cacacaggcgaaaagcccttcgcctgcgacatctgtggaagaaagtttgccCG GAGCGATGAACTTGTCCGAcataccaagatccacttgcggca gaaggaccgcccttacgcttgcccagtggagtcctgtgatcgccgcttctccCA ATCAGGGAATCTGACTGAGcacatccgcatccacacaggcca gaagcccttccagtgccgcatctgcatgAGAaacttcagcACAAGTG GACATCTGGTACGCcacatccgcacccacacaggcgaaaagccct tcgcctgcgacatctgtggaagaaagtttgccCAGAATAGTACCCT GACCGAAcataccaagatccacttgcggcagaaggacaag | 203 |

(continued)

| CONSTRUCT | SEQUENCE | SEQ ID NO |
|---|---|---|
| 46 (coding) | ATGGCCgccgaccacctgatgctcgccgagggctaccgcctggtgcaga ggccgccgtccgccgccgccgcccatggccctcatgcgctccggactctgccg ccgtacgcggggcccgggcctggacagtgggctgaggccgcggggggctccg ctggggccgccgccgcccccgccaacccggggccctggcgtacggggccttc gggccgccgtcctccttccagccctttccggccgtgcctccgccggccgcggg catcgcgcacctgcagcctgtggcgacgccgtaccccggccgcgcggccgcg cccccaacgctccgggaggcccccgggcccgcagccggcccaagcgcc gcagccccgccgccgcccgcgcacgcctgggcggcatggacgccgaactc atcgacgaggaggcgctgacgtcgctggagctggagctggggctgcaccgcg tgcgcgagctgcccgagctgttcctgggccagagcgagttcgactgcttctcgg acttggggtccgcgccgcccgccggctccgtgagctgcggtggttctggtggtg gttctggtGGTGGCAGCGGGGGAGGTTCTGGTcagtccca gctcatcaaacccagccgcatgcgcaagtaccccaaccggcccagcaagacg cccccccacgaacgcccttacgcttgcccagtggagtcctgtgatcgccgcttct ccCGCAGCGACAACCTGGTGAGAcacatccgcatccacaca ggccagaagcccttccagtgccgcatctgcatgAGAaacttcagcCGAG AGGATAACTTGCACACTcacatccgcacccacacaggcgaaaa gcccttcgcctgcgacatctgtggaagaaagtttgccCGGAGCGATGA ACTTGTCCGAcataccaagatccacttgcggcagaaggaccgcccctta cgcttgcccagtggagtcctgtgatcgccgcttctccCAATCAGGGAA TCTGACTGAGcacatccgcatccacacaggccagaagcccttccagtg ccgcatctgcatgAGAaacttcagcACAAGTGGACATCTGGT ACGCcacatccgcacccacacaggcgaaaagcccttcgcctgcgacatctg tggaagaaagtttgccCAGAATAGTACCCTGACCGAAcatac caagatccacttgcggcagaaggacaag | 204 |

(continued)

| CONSTRUCT | SEQUENCE | SEQ ID NO |
|---|---|---|
| 47 (coding) | ATGGCCGCAGATCACCTGATGCTGGCTGAAGGCT ACAGACTGGTGCAGCGGCCTCCATCTGCCGCTGC CGCCCACGGCCCCCACGCCCTGAGAACACTGCCC CCCTACGCCGGCCCTGGTCTTGATAGCGGACTCA GACCTAGAGGCGCCCCTCTGGGCCCTCCACCTCC AAGACAGCCTGGAGCCCTGGCCTACGGCGCCTTC GGCCCTCCTTCTAGCTTCCAGCCCTTCCCCGCCGT GCCTCCTCCAGCcGCTGGCATCGCCCACCTGCAG CCTGTGGCCACCCCTTACCCCGGAAGAGCCGCCG CCCCTCCAAACGCCCCTGGCGGACCTCCTGGCCC CCAGCCTGCTCCAAGCGCCGCTGCCCCTCCACCT CCTGCTCATGCCCTGGGCGGCATGGACGCCGAGC TGATCGACGAGGAAGCCCTGACCAGCCTGGAAC TGGAACTGGGCCTGCACAGAGTGCGGGAACTGC CTGAGCTGTTCCTGGGACAGAGCGAGTTCGACTG CTTCAGCGACCTGGGCAGCGCCCTCCTGCCGGC TCTGTGTCCTGCgccgaccacctgatgctcgccgagggctaccgcct ggtgcagaggccgccgtccgccgccgccgcccatggccctcatgcgctccgg actctgccgccgtacgcgggccccgggcctggacagtgggctgaggccgcgg ggggctccgctggggccgccgccgccccgccaacccggggccctggcgtac ggggccttcgggccgccgtcctccttccagcccttccggccgtgcctccgccg gccgcgggcatcgcgcacctgcagcctgtggcgacgccgtaccccggccgc gcCgccgcgcccccaacgctccgggaggcccccgggcccgcagccggc cccaagcgccgcagccccgccgccgcccgcgcacgccctgggcggcatgga cgccgaactcatcgacgaggaggcgctgacgtcgctggagctggagctgggg ctgcaccgcgtgcgcgagctgcccgagctgttcctgggccagagcgagttcga ctgcttctcggacttggggtccgcgccgcccgccggctccgtgagctgccagtc ccagctcatcaaacccagccgcatgcgcaagtaccccaaccggcccagcaag acgccccccacgaacgcccttacgcttgcccagtggagtcctgtgatcgccgc ttctccCGCAGCGACAACCTGGTGAGAcacatccgcatccac acaggccagaagcccttccagtgccgcatctgcatgAGAaacttcagcCG AGAGGATAACTTGCACACTcacatccgcacccacacaggcg aaaagcccttcgcctgcgacatctgtggaagaaagtttgccCGGAGCGA TGAACTTGTCCGAcataccaagatccacttgcggcagaaggaccgc ccttacgcttgcccagtggagtcctgtgatcgccgcttctccCAATCAGG GAATCTGACTGAGcacatccgcatccacacaggccagaagcccttc cagtgccgcatctgcatgAGAaacttcagcACAAGTGGACATCT GGTACGCcacatccgcacccacacaggcgaaaagcccttcgcctgcgac atctgtggaagaaagtttgccCAGAATAGTACCCTGACCGAA cataccaagatccacttgcggcagaaggacaag | 206 |

(continued)

| CONSTRUCT | SEQUENCE | SEQ ID NO |
|---|---|---|
| 48 (coding) | ATGGCCGCAGATCACCTGATGCTGGCTGAAGGCT ACAGACTGGTGCAGCGGCCTCCATCTGCCGCTGC CGCCCACGGCCCCCACGCCCTGAGAACACTGCCC CCCTACGCCGGCCCTGGTCTTGATAGCGGACTCA GACCTAGAGGCGCCCCTCTGGGCCCTCCACCTCC AAGACAGCCTGGAGCCCTGGCCTACGGCGCCTTC GGCCCTCCTTCTAGCTTCCAGCCCTTCCCGCCGT GCCTCCTCCAGCTGCTGGCATCGCCCACCTGCAG CCTGTGGCCACCCCTTACCCCGGAAGAGCCGCCG CCCCTCCAAACGCCCCTGGCGGACCTCCTGGCCC CCAGCCTGCTCCAAGCGCCGCTGCCCCTCCACCT CCTGCTCATGCCCTGGGCGGCATGGACGCCGAGC TGATCGACGAGGAAGCCCTGACCAGCCTGGAAC TGGAACTGGGCCTGCACAGAGTGCGGGAACTGC CTGAGCTGTTCCTGGGACAGAGCGAGTTCGACTG CTTCAGCGACCTGGGCAGCGCCCTCCTGCCGGC TCTGTGTCCTGCGGCGGCAGCGGCGGCGGAAGC GGCgccgaccacctgatgctcgccgagggctaccgcctggtgcagaggcc gccgtccgccgccgccgcccatggccctcatgcgctccggactctgccgccgt acgcgggcccgggcctggacagtgggctgaggccgcggggggctccgctgg ggccgccgccgccccgccaacccggggccctggcgtacggggccttcgggc cgccgtcctccttccagcccttccggccgtgcctccgccggccgcgggcatcg cgcacctgcagcctgtggcgacgccgtaccccggccgcgcggccgcgccccc ccaacgctccgggaggcccccgggcccgcagccggcccccaagcgccgca gccccgccgccgcccgcgcacgccctgggcggcatggacgccgaactcatc gacgaggaggcgctgacgtcgctggagctggagctggggctgcaccgcgtgc gcgagctgcccgagctgttcctgggccagagcgagttcgactgcttctcggactt ggggtccgcgccgcccgccggctccgtgagctgcggtggttctggtggtggtt ctggtcagtcccagctcatcaaacccagccgcatgcgcaagtaccccaaccgg cccagcaagacgcccccccacgaacgcccttacgcttgcccagtggagtcctgt gatcgccgcttctccCGCAGCGACAACCTGGTGAGAcacatc cgcatccacacaggccagaagcccttccagtgccgcatctgcatgAGAaact tcagcCGAGAGGATAACTTGCACACTcacatccgcacccac acaggcgaaaagcccttcgcctgcgacatctgtggaagaaagtttgccCGG AGCGATGAACTTGTCCGAcataccaagatccacttgcggcaga aggaccgcccttacgcttgcccagtggagtcctgtgatcgccgcttctccCAA TCAGGGAATCTGACTGAGcacatccgcatccacacaggccag aagcccttccagtgccgcatctgcatgAGAaacttcagcACAAGTGG ACATCTGGTACGCcacatccgcacccacacaggcgaaaagcccttc gcctgcgacatctgtggaagaaagtttgccCAGAATAGTACCTG ACCGAAcataccaagatccacttgcggcagaaggacaag | 208 |
| 49 (coding) | atggagctggaattggatgctggtgaccaagacctgctggccttcctgctagag gaaagtggagatttggggacggcacccgatgaggccgtgagggcccactgg actgggcgctgccggctttctgaggtGccgagcgactgggaagtagatgatttgc | 212 |

(continued)

| CONSTRUCT | SEQUENCE | SEQ ID NO |
|---|---|---|
|  | tgtgctccctgctgagtcccccagcgtcgttgaacattctcagctcctccaacccc tgccttgtccaccatgaccacacctactccctcccacgggaaactgtctctatgga tctagagagtgagagctgtagaaaagaggggacccagatgactccacagcata tggaggagctggcagagcaggagattgctaggctagtactgacagatgaggag aagagtctattggagaaggaggggcttattctgcctgagacacttcctctcactaa gacagaggaacaaattctgaaacgtgtgcggCTCGAACCAGGTGA AAAACCTTACAAATGTCCTGAATGTGGGAAATC ATTCAGTCGCAGCGACAACCTGGTGAGACATCA ACGCACCCATACAGGAGAAAAACCTTATAAATG TCCAGAATGTGGAAAGTCCTTCTCACGAGAGGAT AACTTGCACACTCATCAACGAACACATACTGGTG AAAAACCATACAAGTGTCCCGAATGTGGTAAAA GTTTTAGCCGGAGCGATGAACTTGTCCGACACCA ACGAACCCATACAGGCGAGAAGCCTTACAAATG TCCCGAGTGTGGCAAGAGCTTCTCACAATCAGGG AATCTGACTGAGCATCAACGAACTCATACCGGG GAAAAACCTTACAAGTGTCCAGAGTGTGGGAAG AGCTTTCCACAAGTGGACATCTGGTACGCCACC AGAGGACACATACAGGGGAGAAGCCCTACAAAT GCCCCGAATGCGGTAAAAGTTTCTCTCAGAATAG TACCCTGACCGAACACCAGCGAACACACTGG GAAAAAACGAGTgtgtaCgttgggggtttagagagcCgggtctt gaaatacacagcccagaatatggagcttcagaacaaagtacagcttctggagga acagaatttgtcccttctagatcaactgaggaaactccaggccatggtgattgaga tCtcaaacaaaaccagcagcagcagcacctgcatcttggtcctGctagtctcctt ctgcctcctccttgtacctgctatgtactcctctgacacaaggggggagcctgccag ctgagcatggagtgttgtcccgccagcttcgtgccctccccagtgaggacccta ccagctggagctgcctgccctgcagtcagaagtgccgaaagacagcacacacc agtggttggacggctcagactgtgtactccaggcccctggcaacacttcctgcct gctgcattacatgcctcaggctcccagtgcagagcctcccctggagtggccCtt ccctgacctcttctcagagcctctctgccgaggtcccatcctcccccctgcaggca aatctcacaaggaagggaggatggcttcctactggtagcccctctgtcattttgca ggacagatactcaggc |  |

(continued)

| CONSTRUCT | SEQUENCE | SEQ ID NO |
|---|---|---|
| 50 (coding) | atggagctggaattggatgctggtgaccaagacctgctggccttcctgctagag gaaagtggagatttggggacggcacccgatgaggccgtgagggccccactgg actgggcgctgccgctttctgaggtGccgagcgactgggaagtagatgatttgc tgtgctccctgctgagtcccccagcgtcgttgaacattctcagctcctccaacccc tgccttgtccaccatgaccacacctactccctcccacgggaaactgtctctatgga tctagagagtgagagctgtagaaaagaggggacccagatgactccacagcata tggaggagctggcagagcaggagattgctaggctagtactgacagatgaggag aagagtctattggagaaggaggggcttattctgcctgagacacttcctctcactaa gacagaggaacaaattctgaaacgtgtgcggcgcccttacgcttgcccagtgga gtcctgtgatcgccgcttctccCGCAGCGACAACCTGGTGAG AcacatccgcatccacacaggccagaagcccttccagtgccgcatctgcatgA GAaacttcagcCGAGAGGATAACTTGCACACTcacatccg cacccacacaggcgaaaagcccttcgcctgcgacatctgtggaagaaagtttgc cCGGAGCGATGAACTTGTCCGAcataccaagatccacttgcg gcagaaggaccgcccttacgcttgcccagtggagtcctgtgatcgccgcttctcc CAATCAGGGAATCTGACTGAGcacatccgcatccacacagg ccagaagcccttccagtgccgcatctgcatgAGAaacttcagcACAAGT<br><br>GGACATCTGGTACGCcacatccgcacccacacaggcgaaaagcc cttcgcctgcgacatctgtggaagaaagtttgccCAGAATAGTACCC TGACCGAAcataccaagatccacttgcggcagaaggacgtgtaCgttg ggggtttagagagcCgggtcttgaaatacacagcccagaatatggagcttcag aacaaagtacagcttctggaggaacagaatttgtcccttctagatcaactgagga aactccaggccatggtgattgagatCtcaaacaaaaccagcagcagcagcacc tgcatcttggtcctGctagtctccttctgcctcctccttgtacctgctatgtactcctc tgacacaaggggggagcctgccagctgagcatggagtgttgtcccgccagcttc gtgccctccccagtgaggacccttaccagctggagctgcctgccctgcagtcag aagtgccgaaagacagcacacaccagtggttggacggctcagactgtgtactcc aggcccctggcaacacttcctgcctgctgcattacatgcctcaggctcccagtgc agagcctcccctggagtggccCttccctgacctcttctcagagcctctctgccga ggtcccatcctcccctgcaggcaaatctcacaaggaagggaggatggcttcct actggtagcccctctgtcattttgcaggacagatactcaggc | 216 |

(continued)

| CONSTRUCT | SEQUENCE | SEQ ID NO |
|---|---|---|
| 51 (coding) | atggagctggaattggatgctggtgaccaagacctgctggccttcctgctagag gaaagtggagatttggggacggcacccgatgaggccgtgagggcccccactgg actgggcgctgccgctttctgaggtGccgagcgactgggaagtagatgatttgc tgtgctccctgctgagtcccccagcgtcgttgaacattctcagctcctccaaccccc tgccttgtccaccatgaccacacctactccctcccacgggaaactgtctctatgga tctagagagtgagagctgtagaaaagaggggacccagatgactccacagcata tggaggagctggcagagcaggagattgctaggctagtactgacagatgaggag aagagtctattggagaaggaggggcttattctgcctgagacacttcctctcactaa gacagaggaacaaattctgaaacgtgtgcggcgcccttacgcttgcccagtgga gtcctgtgatcgccgcttctccCGCTCAGACAACCTCGTTCGA cacatccgcatccacacaggccagaagcccttccagtgccgcatctgcatgAG Aaacttcagc CACCGGACTACACTCACGAACcacatccgca cccacacaggcgaaaagcccttcgcctgcgacatctgtggaagaaagtttgcc AGAGAAGACAATCTCCATACTcataccaagatccacttgcg gcagaaggaccgcccttacgcttgcccagtggagtcctgtgatcgccgcttctcc ACCAGCCATTCTCTCACTGAAcacatccgcatccacacagg ccagaagcccttccagtgccgcatctgcatgAGAaacttcagcCAGTCT AGCTCACTGGTGAGGcacatccgcacccacacaggcgaaaagc ccttcgcctgcgacatctgtggaagaaagtttgccAGGGAGGATAAC CTGCATACGcataccaagatccacttgcggcagaaggacgtgtaCgtt gggggtttagagagcCgggtcttgaaatacacagcccagaatatggagcttca gaacaaagtacagcttctggaggaacagaatttgtcccttctagatcaactgagg aaactccaggccatggtgattgagatCtcaaacaaaaccagcagcagcagcac ctgcatcttggtcctGctagtctccttctgcctcctccttgtacctgctatgtactcct ctgacacaaggggggagcctgccagctgagcatggagtgttgtcccgccagctt cgtgccctccccagtgaggacccttaccagctggagctgcctgccctgcagtca gaagtgccgaaagacagcacacaccagtggttggacggctcagactgtgtact ccaggcccctggcaacacttcctgcctgctgcattacatgcctcaggctcccagt gcagagcctcccctggagtggccCttccctgacctcttctcagagcctctctgcc gaggtccatcctccccctgcaggcaaatctcacaaggaagggaggatggcttc ctactggtagcccctctgtcattttgcaggacagatactcaggc | 218 |
| 52 (coding) | atggagctggaattggatgctggtgaccaagacctgctggccttcctgctagag gaaagtggagatttggggacggcacccgatgaggccgtgagggcccccactgg actgggcgctgccgctttctgaggtGccgagcgactgggaagtagatgatttgc tgtgctccctgctgagtcccccagcgtcgttgaacattctcagctcctccaaccccc tgccttgtccaccatgaccacacctactccctcccacgggaaactgtctctatgga | 220 |

(continued)

| CONSTRUCT | SEQUENCE | SEQ ID NO |
|---|---|---|
| | tctagagagtgagagctgtagaaaagaggggacccagatgactccacagcata tggaggagctggcagagcaggagattgctaggctagtactgacagatgaggag aagagtctattggagaaggaggggcttattctgcctgagacacttcctctcactaa gacagaggaacaaattctgaaacgtgtgcggCTCGAACCAGGTGA AAAACCTTACAAATGTCCTGAATGTGGGAAATC ATTCAGTCGCAGCGACAACCTGGTGAGACATCA ACGCACCCATACAGGAGAAAAACCTTATAAATG TCCAGAATGTGGAAAGTCCTTCTCACGAGAGGAT AACTTGCACACTCATCAACGAACACATACTGGTG AAAAACCATACAAGTGTCCCGAATGTGGTAAAA GTTTTAGCCGGAGCGATGAACTTGTCCGACACCA ACGAACCCATACAGGCGAGAAGCCTTACAAATG TCCCGAGTGTGGCAAGAGCTTCTCACAATCAGGG AATCTGACTGAGCATCAACGAACTCATACCGGG GAAAAACCTTACAAGTGTCCAGAGTGTGGGAAG AGCTTTTCCACAAGTGGACATCTGGTACGCCACC AGAGGACACATACAGGGGAGAAGCCCTACAAAT GCCCCGAATGCGGTAAAAGTTTCTCTCAGAATAG TACCCTGACCGAACACCAGCGAACACACTGG GAAAAAACGAGTgtgtaCgttgggggtttagagagcCgggtctt gaaatacacagcccagaatatggagcttcagaacaaagtacagcttctggagga acagaatttgtcccttctagatcaactgaggaaactccaggccatggtgattgaga tatca | |
| 53 (coding) | atggagctggaattggatgctggtgaccaagacctgctggccttcctgctagag gaaagtggagatttggggacggcacccgatgaggccgtgagggcccactgg actgggcgctgccgctttctgaggtGccgagcgactgggaagtagatgatttgc tgtgctccctgctgagtcccccagcgtcgttgaacattctcagctcctccaaccc tgccttgtccaccatgaccacacctactccctcccacgggaaactgtctctatgga tctagagagtgagagctgtagaaaagaggggacccagatgactccacagcata tggaggagctggcagagcaggagattgctaggctagtactgacagatgaggag aagagtctattggagaaggaggggcttattctgcctgagacacttcctctcactaa gacagaggaacaaattctgaaacgtgtgcggcgcccttacgcttgcccagtgga gtcctgtgatcgccgcttctccCGCAGCGACAACCTGGTGAG AcacatccgcatccacacaggccagaagcccttccagtgccgcatctgcatgA GAaacttcagcCGAGAGGATAACTTGCACACTcacatccg cacccacacaggcgaaaagcccttcgcctgcgacatctgtggaagaaagtttgc cCGGAGCGATGAACTTGTCCGAcataccaagatccacttgcg gcagaaggaccgcccttacgcttgcccagtggagtcctgtgatcgccgcttctcc CAATCAGGGAATCTGACTGAGcacatccgcatccacacagg ccagaagcccttccagtgccgcatctgcatgAGAaacttcagcACAAGT GGACATCTGGTACGCcacatccgcacccacacaggcgaaaagcc cttcgcctgcgacatctgtggaagaaagtttgccCAGAATAGTACCC TGACCGAAcataccaagatccacttgcggcagaaggacgtgtaCgttg ggggtttagagagcCgggtcttgaaatacacagcccagaatatggagcttcag aacaaagtacagcttctggaggaacagaatttgtcccttctagatcaactgagga aactccaggccatggtgattgagatCtca | 222 |

**TABLE 8.** Nucleic acid sequences for exemplary MicroRNA and MicroRNA binding sites.

| Description | SEQUENCE | SEQ ID NO |
|---|---|---|
| M1 Binding Site | aaagagaccggttcactgtgacagtaaaagagaccggttcactgtgagaatgaaagag accggttcactgtgatcggaaaagagaccggttcactgtgagcggccttgaaacccagc agacaatgtagctcagtagaaacccagcagacaatgtagctgaatggaaacccagcag acaatgtagcttcggagaaacccagcagacaatgtagct | 7 |
| miR128 Sequence | UCACAGUGAACCGGUCUCUUU | 8 |
| miR128 binding site | AAAGAGACCGGTTCACTGTGA | 9 |
| miR221 sequence | AGCUACAUUGUCUGCUGGGUUUC | 10 |
| miR221 binding site | GAAACCCAGCAGACAATGTAGCT | 11 |
| miR222 sequence | AGCUACAUCUGGCUACUGGGUCU | 12 |
| miR222 binding site | AGACCCAGTAGCCAGATGTAGCT | 13 |
| M2 Binding Site | GAAACCCAGCAGACAATGTAGCTAGACCCAGTAGCC AGATGTAGCTAAAGAGACCGGTTCACTGTGA | 14 |
| M3 Binding Site | GAAACCCAGCAGACAATGTAGCTAGACCCAGTAGCC AGATGTAGCTAAAGAGACCGGTTCACTGTGAGAAAC CCAGCAGACAATGTAGCTAGACCCAGTAGCCAGATG TAGCTAAAGAGACCGGTTCACTGTGA | 15 |

**TABLE 9.** Different types of zinc finger structures and exemplary zinc finger proteins for generating eTFs.

| ZF type name | SEQ ID NO | ZF structure (wherein each x can independently be any residue) | Exemplary proteins that can serve as the protein platform for an eTF or a DNA binding domain of an eTF disclosed herein |
|---|---|---|---|
| Zinc fingers C2H2-type (ZNF) | 136 | C-x-C-x-H-x-H | KLF4, KLF5, EGR3, ZFP637, SLUG, ZNF750, ZNF281, ZBP89, GLIS1, GLIS3 |
| Ring finger proteins (RNF) | 137 | C-x-C-x-C-x-H-xxx-C-x-C-x-C-x-C | MDM2, BRCA1, ZNF179 |
| PHD finger proteins (PHF) | 138 | C-x-C-x-C-x-C-xxx-H-x-C-x-C-x-C | KDM2A, PHF1, ING1 |
| LIM domain containing | 139 | C-x-C-x-H-x-C-x-C-x-C-x-C-x-(C,H,D) | ZNF185, LIMK1, PXN |
| Nuclear hormone receptors (NR) | 140 | C-x-C-x-C-x-C-xxx-C-x-C-x-C-x-C | VDR, ESR1, NR4A1 |
| Zinc fingers CCCH-type (ZC3H) | 141 | C-x-C-x-C-x-H | RC3H1, HELZ, MBNL1, ZFP36, ZFP36L1 |
| Zinc fingers FYVE-type (ZFYVE) | 140 | C-x-C-x-C-x-C-xxx-C-x-C-x-C-x-C | EEA1, HGS, PIKFYVE |
| Zinc fingers CCHC-type (ZCCHC) | 142 | C-x-C-x-H-x-C | CNBP, SF1, LIN28A |

(continued)

| ZF type name | SEQ ID NO | ZF structure (wherein each x can independently be any residue) | Exemplary proteins that can serve as the protein platform for an eTF or a DNA binding domain of an eTF disclosed herein |
|---|---|---|---|
| Zinc fingers DHHC-type (ZDHHC) | 143 | C-x-C-x-H-x-C-xxx-C-x-C-x-H-x-C | ZDHHC2, ZDHHC8, ZDHHC9 |
| Zinc fingers MYND-type (ZMYND) | 144 | C-x-C-x-C-x-C-xxx-C-x-C-x-H-x-C | PDCD2, RUNX1T1, SMYD2, SMYD 1 |
| Zinc fingers RANBP2-type (ZRANB) | 145 | C-x-C-x-C-x-C | YAF2, SHARPIN, EWSR1 |
| Zinc fingers ZZ-type (ZZZ) | 145 | C-x-C-x-C-x-C | HERC2, NBR1, CREBBP |
| Zinc fingers C2HC-type (ZC2HC) | 142 | C-x-C-x-H-x-C | IKBKG, L3MBTL1, ZNF746 |
| GATA zinc-finger domain containing (GATAD) | 145 | C-x-C-x-C-x-C | GATA4, GATA6, MTA1 |
| ZF class homeoboxes and pseudogenes | 136 | C-x-C-x-H-x-H | ADNP, ZEB1, ZHX1 |
| THAP domain containing (THAP) | 141 | C-x-C-x-C-x-H | THAP1, THAP4, THAP11 |
| Zinc fingers CXXC-type (CXXC) | 140 | C-x-C-x-C-x-C-xxx-C-x-C-x-C-x-C | CXXC1, CXXC5, MBD1, DNMT1 |
| Zinc fingers SWIM-type (ZSWIM) | 141 | C-x-C-x-C-x-H | MAP3K1, ZSWIM5, ZSWIM6 |
| Zinc fingers AN1-type (ZFAND) | 146 | C-x-C-x-C-x-C-xxx-C-x-H-x-H-x-C | ZFAND3, ZFAND6, IGHMBP2 |
| Zinc fingers 3CxxC-type (Z3CXXC) | 142 | C-x-C-x-H-x-C | ZAR1, RTP1, RTP4 |
| Zinc fingers CW-type (ZCW) | 145 | C-x-C-x-C-x-C | MORC1, ZCWPW1, KDM1B |
| Zinc fingers GRF-type (ZGRF) | 145 | C-x-C-x-C-x-C | TTF2, NEIL3, TOP3A |
| Zinc fingers MIZ-type (ZMIZ) | 142 | C-x-C-x-H-x-C | PIAS1, PIAS3, PIAS4 |
| Zinc fingers BED-type (ZBED) | 136 | C-x-C-x-H-x-H | ZBED1, ZBED4, ZBED6 |

(continued)

| ZF type name | SEQ ID NO | ZF structure (wherein each x can independently be any residue) | Exemplary proteins that can serve as the protein platform for an eTF or a DNA binding domain of an eTF disclosed herein |
|---|---|---|---|
| Zinc fingers HIT-type (ZNHIT) | 144 | C-x-C-x-C-x-C-xxx-C-x-C-x-H-x-C | ZNHIT3, DDX59, INO80B |
| Zinc fingers MYM-type (ZMYM) | 145 | C-x-C-x-C-x-C | ZMYM2, ZMYM3, ZMYM4 |
| Zinc fingers matrin-type (ZMAT) | 136 | C-x-C-x-H-x-H | ZNF638, ZMAT1, ZMAT3, ZMAT5 |
| Zinc fingers C2H2C-type | 136 | C-x-C-x-H-x-H | MYT1, MYT1L, ST18 |
| Zinc fingers DBF-type (ZDBF) | 136 | C-x-C-x-H-x-H | DBF4, DBF4B, ZDBF2 |
| Zinc fingers PARP-type | 142 | C-x-C-x-H-x-C | LIG3, PARP1 |

**TABLE 10.** Amino acid sequences for exemplary zinc finger DNA binding domains.

| DBD/Target site | SEQUENCE | SEQ ID NO |
|---|---|---|
| eZF | LEPGEKP - [YKCPECGKSFS X HQRTH TGEKP]n - YKCPECGKSFS X HQRTH - TGKKTS, wherein n is an integer from 1-15, and each X is a recognition sequence capable of binding to 3 bp of target sequence | 147 |
| Z1 Target Site | RSDNLVR x REDNLHT x RSDELVR x QSGNLTE x TSGHLVR x QNSTLTE, wherein each x is a linker comprising 1-50 amino acid residues | 148 |
| Z13 Target Site | RSDNLVR x HRTTLTN x REDNLHT x TSHSLTE x QSSSLVR x REDNLHT, wherein each x is a linker comprising 1-50 amino acid residues | 149 |
| Z14 Target Site | DPGALVR x RSDNLVR x QSGDLRR x THLDLIR x TSGNLVR x RSDNLVR, wherein each x is a linker comprising 1-50 amino acid residues | 150 |
| Z15 Target Site | RRDELNV x RSDHLTN x RSDDLVR x RSDNLVR x HRTTLTN x REDNLHT x TSHSLTE x QSSSLVR x REDNLHT, wherein each x is a linker comprising 1-50 amino acid residues | 151 |

**TABLE 11.** Amino acid sequences for exemplary zinc finger recognition sequences disclosed herein.

| SEQUENCE | SEQ ID NO |
|---|---|
| RSDNLVR | 152 |
| REDNLHT | 153 |
| RSDELVR | 154 |
| QSGNLTE | 155 |
| TSGHLVR | 156 |
| QNSTLTE | 157 |
| DPGALVR | 158 |
| HRTTLTN | 159 |
| QSGDLRR | 160 |
| TSHSLTE | 161 |

(continued)

| SEQUENCE | SEQ ID NO |
|----------|-----------|
| THLDLIR | 162 |
| QSSSLVR | 163 |
| TSGNLVR | 164 |
| RRDELNV | 165 |
| RSDDLVR | 166 |
| RSDHLTN | 167 |

**TABLE 12.** Other nucleotide and amino acid sequence disclosed herein.

| Description | SEQUENCE | SEQ ID NO |
|-------------|----------|-----------|
| EGR1 NLS Domain | LIKPSRMRKYPNRPSK | 168 |
| SV40 NLS | PKKKRKV | 169 |
| Nucleoplasmin NLS | KRPAATKKAGQAKKKK | 170 |
| HA Tag | YPYDVPDYA | 171 |
| spA (synthetic polyA) | AATAAAAGATCTTTATTTTCATTAGATCTGTGTGTTGG TTTTTTGTGTGCGGACCGCACGTG | 16 |
| hGH (human growth hormone polyA) | GGGTGGCATCCCTGTGACCCCTCCCCAGTGCCTCTCCT GGCCCTGGAAGTTGCCACTCCAGTGCCCACCAGCCTT GTCCTAATAAAATTAAGTTGCATCATTTTGTCTGACTA GGTGTCCTTCTATAATATTATGGGGTGGAGGGGGGGTG GTATGGAGCAAGGGGCAAGTTGGGAAGACAACCTGT AGGGCCTGCGGGGTCTATTGGGAACCAAGCTGGAGTG CAGTGGCACAATCTTGGCTCACTGCAATCTCCGCCTCC TGGGTTCAAGCGATTCTCCTGCCTCAGCCTCCCGAGTT GTTGGGATTCCAGGCATGCATGACCAGGCTCAGCTAA TTTTTGTTTTTTTGGTAGAGACGGGGTTTCACCATATT GGCCAGGCTGGTCTCCAACTCCTAATCTCAGGTGATCT ACCCACCTTGGCCTCCCAAATTGCTGGGATTACAGGC GTGAACCACTGCTCCCTTCCCTGTCCTT | 17 |
| SCN1A protein | MEQTVLVPPGPDSFNFFTRESLAAIERRIAEEKAKNPKPD KKDDDENGPKPNSDLEAGKNLPFIYGDIPPEMVSEPLED | 172 |

(continued)

| Description | SEQUENCE | SEQ ID NO |
|---|---|---|
| | LDPYYINKKTFIVLNKGKAIFRFSATSALYILTPFNPLRKI AIKILVHSLFSMLIMCTILTNCVFMTMSNPPDWTKNVEY TFTGIYTFESLIKIIARGFCLEDFTFLRDPWNWLDFTVITF AYVTEFVDLGNVSALRTFRVLRALKTISVIPGLKTIVGAL IQSVKKLSDVMILTVFCLSVFALIGLQLFMGNLRNKCIQ WPPTNASLEEHSIEKNITVNYNGTLINETVFEFDWKSYIQ DSRYHYFLEGFLDALLCGNSSDAGQCPEGYMCVKAGRN PNYGYTSFDTFSWAFLSLFRLMTQDFWENLYQLTLRAA GKTYMIFFVLVIFLGSFYLINLILAVVAMAYEEQNQATLE EAEQKEAEFQQMIEQLKKQQEAAQQAATATASEHSREP SAAGRLSDSSSEASKLSSKSAKERRNRRKKRKQKEQSGG EEKDEDEFQKSESEDSIRRKGFRFSIEGNRLTYEKRYSSP HQSLLSIRGSLFSPRRNSRTSLFSFRGRAKDVGSENDFAD DEHSTFEDNESRRDSLFVPRRHGERRNSNLSQTSRSSRM LAVFPANGKMHSTVDCNGVVSLVGGPSVPTSPVGQLLP EVIIDKPATDDNGTTTETEMRKRRSSSFHVSMDFLEDPSQ RQRAMSIASILTNTVEELEESRQKCPPCWYKFSNIFLIWD CSPYWLKVKHVVNLVVMDPFVDLAITICIVLNTLFMAM EHYPMTDHFNNVLTVGNLVFTGIFTAEMFLKIIAMDPYY YFQEGWNIFDGFIVTLSLVELGLANVEGLSVLRSFRLLRV FKLAKSWPTLNMLIKIIGNSVGALGNLTLVLAIIVFIFAVV GMQLFGKSYKDCVCKIASDCQLPRWHMNDFFHSFLIVF RVLCGEWIETMWDCMEVAGQAMCLTVFMMVMVIGNL VVLNLFLALLLSSFSADNLAATDDDNEMNNLQIAVDRM HKGVAYVKRKIYEFIQQSFIRKQKILDEIKPLDDLNNKKD SCMSNHTAEIGKDLDYLKDVNGTTSGIGTGSSVEKYIIDE SDYMSFINNPSLTVTVPIAVGESDFENLNTEDFSSESDLEE SKEKLNESSSSSEGSTVDIGAPVEEQPVVEPEETLEPEACF TEGCVQRFKCCQINVEEGRGKQWWNLRRTCFRIVEHNW FETFIVFMILLSSGALAFEDIYIDQRKTIKTMLEYADKVFT YIFILEMLLKWVAYGYQTYFTNAWCWLDFLIVDVSLVS LTANALGYSELGAIKSLRTLRALRPLRALSRFEGMRVVV NALLGAIPSIMNVLLVCLIFWLIFSIMGVNLFAGKFYHCI NTTTGDRFDIEDVNNHTDCLKLIERNETARWKNVKVNF DNVGFGYLSLLQVATFKGWMDIMYAAVDSRNVELQPK YEESLYMYLYFVIFIIFGSFFTLNLFIGVIIDNFNQQKKKFG GQDIFMTEEQKKYYNAMKKLGSKKPQKPIPRPGNKFQG MVFDFVTRQVFDISIMILICLNMVTMMVETDDQSEYVTT ILSRINLVFIVLFTGECVLKLISLRHYYFTIGWNIFDFVVVI LSIVGMFLAELIEKYFVSPTLFRVIRLARIGRILRLIKGAK GIRTLLFALMMSLPALFNIGLLLFLVMFIYAIFGMSNFAY VKREVGIDDMFNFETFGNSMICLFQITTSAGWDGLLAPIL NSKPPDCDPNKVNPGSSVKGDCGNPSVGIFFFVSYIIISFL VVVNMYIAVILENFSVATEESAEPLSEDDFEMFYEVWEK FDPDATQFMEFEKLSQFAAALEPPLNLPQPNKLQLIAMD LPMVSGDRIHCLDILFAFTKRVLGESGEMDALRIQMEER FMASNPSKVSYQPITTTLKRKQEEVSAVIIQRAYRRHLLK RTVKQASFTYNKNKIKGGANLLIKEDMIIDRINENSITEK TDLTMSTAACPPSYDRVTKPIVEKHEQEGKDEKAKGK | |

(continued)

| Description | SEQUENCE | SEQ ID NO |
|---|---|---|
| dCAS protein | KRNYILGLAIGITSVGYGIIDYETRDVIDAGVRLFKEANV ENNEGRRSKRGARRLKRRRRHRIQRVKKLLFDYNLLTD HSELSGINPYEARVKGLSQKLSEEEFSAALLHLAKRRGV HNVNEVEEDTGNELSTKEQISRNSKALEEKYVAELQLER LKKDGEVRGSINRFKTSDYVKEAKQLLKVQKAYHQLDQ SFIDTYIDLLETRRTYYEGPGEGSPFGWKDIKEWYEMLM GHCTYFPEELRSVKYAYNADLYNALNDLNNLVITRDEN EKLEYYEKFQIIENVFKQKKKPTLKQIAKEILVNEEDIKG YRVTSTGKPEFTNLKVYHDIKDITARKEIIENAELLDQIA KILTIYQSSEDIQEELTNLNSELTQEEIEQISNLKGYTGTH NLSLKAINLILDELWHTNDNQIAIFNRLKLVPKKVDLSQQ KEIPTTLVDDFILSPVVKRSFIQSIKVINAIIKKYGLPNDIIIE LAREKNSKDAQKMINEMQKRNRQTNERIEEIIRTTGKEN AKYLIEKIKLHDMQEGKCLYSLEAIPLEDLLNNPFNYEV DHIIPRSVSFDNSFNNKVLVKQEEASKKGNRTPFQYLSSS DSKISYETFKKHILNLAKGKGRISKTKKEYLLEERDINRF SVQKDFINRNLVDTRYATRGLMNLLRSYFRVNNLDVKV KSINGGFTSFLRRKWKFKKERNKGYKHHAEDALIIANAD FIFKEWKKLDKAKKVMENQMFEEKQAESMPEIETEQEY KEIFITPHQIKHIKDFKDYKYSHRVDKKPNRELINDTLYS TRKDDKGNTLIVNNLNGLYDKDNDKLKKLINKSPEKLL MYHHDPQTYQKLKLIMEQYGDEKNPLYKYYEETGNYL TKYSKKDNGPVIKKIKYYGNKLNAHLDITDDYPNSRNK VVKLSLKPYRFDVYLDNGVYKFVTVKNLDVIKKENYYE VNSKCYEEAKKLKKISNQAEFIASFYNNDLIKINGELYRV IGVNNDLLNRIEVNMIDITYREYLENMNDKRPPRIIKTIAS KTQSIKKYSTDILGNLYEVKSKKHPQIIKKG | 173 |

(continued)

| Description | SEQUENCE | SEQ ID NO |
|---|---|---|
| dCAS9-VP64 construct | MAPKKKRKVGIHGVPAAKRNYILGLAIGITSVGYGIIDYE TRDVIDAGVRLFKEANVENNEGRRSKRGARRLKRRRRH RIQRVKKLLFDYNLLTDHSELSGINPYEARVKGLSQKLSE EEFSAALLHLAKRRGVHNVNEVEEDTGNELSTKEQISRN SKALEEKYVAELQLERLKKDGEVRGSINRFKTSDYVKEA KQLLKVQKAYHQLDQSFIDTYIDLLETRRTYYEGPGEGS PFGWKDIKEWYEMLGHCTYFPEELRSVKYAYNADLY NALNDLNNLVITRDENEKLEYYEKFQIIENVFKQKKKPT LKQIAKEILVNEEDIKGYRVTSTGKPEFTNLKVYHDIKDI TARKEIIENAELLDQIAKILTIYQSSEDIQEELTNLNSELTQ EEIEQISNLKGYTGTHNLSLKAINLILDELWHTNDNQIAIF NRLKLVPKKVDLSQQKEIPTTLVDDFILSPVVKRSFIQSIK VINAIIKKYGLPNDIIIELAREKNSKDAQKMINEMQKRNR QTNERIEEIIRTTGKENAKYLIEKIKLHDMQEGKCLYSLE AIPLEDLLNNPFNYEVDHIIPRSVSFDNSFNNKVLVKQEE ASKKGNRTPFQYLSSSDSKISYETFKKHILNLAKGKGRIS KTKKEYLLEERDINRFSVQKDFINRNLVDTRYATRGLMN LLRSYFRVNNLDVKVKSINGGFTSFLRRKWKFKKERNK GYKHHAEDALIIANADFIFKEWKKLDKAKKVMENQMFE EKQAESMPEIETEQEYKEIFITPHQIKHIKDFKDYKYSHR VDKKPNRELINDTLYSTRKDDKGNTLIVNNLNGLYDKD NDKLKKLINKSPEKLLMYHHDPQTYQKLKLIMEQYGDE<br><br>KNPLYKYYEETGNYLTKYSKKDNGPVIKKIKYYGNKLN AHLDITDDYPNSRNKVVKLSLKPYRFDVYLDNGVYKFV TVKNLDVIKKENYYEVNSKCYEEAKKLKKISNQAEFIAS FYNNDLIKINGELYRVIGVNNDLLNRIEVNMIDITYREYL ENMNDKRPPRIIKTIASKTQSIKKYSTDILGNLYEVKSKK HPQIIKKGKRPAATKKAGQAKKKKGSYPYDVPDYALED ALDDFDLDMLGSDALDDFDLDMLGSDALDDFDLDMLG SDALDDFDLDML | 174 |
| WT EGR3 Protein (human) | MTGKLAEKLPVTMSSLLNQLPDNLYPEEIPSALNLFSGSS DSVVHYNQMATENVMDIGLTNEKPNPELSYSGSFQPAP GNKTVTYLGKFAFDSPSNWCQDNIISLMSAGILGVPPAS GALSTQTSTASMVQPPQGDVEAMYPALPPYSNCGDLYS EPVSFHDPQGNPGLAYSPQDYQSAKPALDSNLFPMIPDY NLYHHPNDMGSIPEHKPFQGMDPIRVNPPPITPLETIKAF KDKQIHPGFGSLPQPPLTLKPIRPRKYPNRPSKTPLHERPH ACPAEGCDRRFSRSDELTRHLRIHTGHKPFQCRICMRSFS RSDHLTTHIRTHTGEKPFACEFCGRKFARSDERKRHAKI HLKQKEKKAEKGGAPSASSAPPVSLAPVVTCA | 175 |

(continued)

| Description | SEQUENCE | SEQ ID NO |
|---|---|---|
| WT EGR1 Protein (human) | MAAAKAEMQLMSPLQISDPFGSFPHSPTMDNYPKLEEM MLLSNGAPQFLGAAGAPEGSGSNSSSSSSGGGGGGGGG SNSSSSSSTFNPQADTGEQPYEHLTAESFPDISLNNEKVL VETSYPSQTTRLPPITYTGRFSLEPAPNSGNTLWPEPLFSL VSGLVSMTNPPASSSSAPSPAASSASASQSPPLSCAVPSN DSSPIYSAAPTFPTPNTDIFPEPQSQAFPGSAGTALQYPPP AYPAAKGGFQVPMIPDYLFPQQQGDLGLGTPDQKPFQG LESRTQQPSLTPLSTIKAFATQSGSQDLKALNTSYQSQLI KPSRMRKYPNRPSKTPPHERPYACPVESCDRRFSRSDEL TRHIRIHTGQKPFQCRICMRNFSRSDHLTTHIRTHTGEKP FACDICGRKFARSDERKRHTKIHLRQKDKKADKSVVASS ATSSLSSYPSPVATSYPSPVTTSYPSPATTSYPSPVPTSFSS PGSSTYPSPVHSGFPSPSVATTYSSVPPAFPAQVSSFPSSA VTNSFSASTGLSDMTATFSPRTIEIC | 176 |
| Linker | GGSGGGSG | 177 |
| Linker | GGSGGGSGGGSGGGSG | 178 |
| Linker | [GGGS]n | 179 |
| Linker | [GGGGS]n | 180 |
| Linker | [GGSG]n | 181 |
| Recognition site for WT EGR1 or EGR3 | GCG(T/G)GGGCG | 182 |
| sgRNA scaffold | GTTTTAGTACTCTGGAAACAGAATCTACTAAAACAAG GCAAAATGCCGTGTTTATCTCGTCAACTTGTTGGCGAG A | 183 |
| Human CREB3 coding sequence | atggagctggaattggatgctggtgaccaagacctgctggccttcctgctagaggaaagt ggagatttggggacggcacccgatgaggccgtgagggcccccactggactgggcgctg ccgctttctgaggtGccgagcgactgggaagtagatgatttgctgtgctccctgctgagtc ccccagcgtcgttgaacattctcagctcctccaacccctgccttgtccaccatgaccacac ctactccctcccacgggaaactgtctctatggatctagagagtgagagctgtagaaaga gggacccagatgactccacagcatatggaggagctggcagagcaggagattgctagg ctagtactgacagatgaggagaagagtctattggagaaggaggggcttattctgcctgag acacttcctctcactaagacagaggaacaaattctgaaacgtgtgcggaggaagattcga aataaaagatctgctcaagagagccgcaggaaaaagaaggtgtaCgttgggggtttaga gagcCgggtcttgaaatacacagcccagaatatggagcttcagaacaaagtacagcttct ggaggaacagaatttgtcccttctagatcaactgaggaaactccaggccatggtgattgag atCtcaaacaaaaccagcagcagcagcacctgcatcttggtcctGctagtctccttctgc ctcctccttgtacctgctatgtactcctctgacacaaggggggagcctgccagctgagcatg gagtgttgtcccgccagcttcgtgccctcccccagtgaggacccttaccagctggagctgc ctgccctgcagtcagaagtgccgaaagacagcacacaccagtggttggacggctcaga ctgtgtactccaggcccctggcaacacttcctgcctgctgcattacatgcctcaggctccca gtgcagagcctcccctggagtggccCttccctgacctcttctcagagcctctctgccgag gtcccatcctccccctgcaggcaaatctcacaaggaagggaggatggcttcctactggta gcccctctgtcattttgcaggacagatactcaggc | 210 |

(continued)

| Description | SEQUENCE | SEQ ID NO |
|---|---|---|
| Human CREB3 AA sequence | MELELDAGDQDLLAFLLEESGDLGTAPDEAVRAPLDWA LPLSEVPSDWEVDDLLCSLLSPPASLNILSSSNPCLVHHD HTYSLPRETVSMDLESESCRKEGTQMTPQHMEELAEQEI ARLVLTDEEKSLLEKEGLILPETLPLTKTEEQILKRVRRKI RNKRSAQESRRKKKVYVGGLESRVLKYTAQNMELQNK VQLLEEQNLSLLDQLRKLQAMVIEISNKTSSSSTCILVLL VSFCLLLVPAMYSSDTRGSLPAEHGVLSRQLRALPSEDP YQLELPALQSEVPKDSTHQWLDGSDCVLQAPGNTSCLL HYMPQAPSAEPPLEWPFPDLFSEPLCRGPILPLQANLTRK GGWLPTGSPSVILQDRYSG | 211 |
| Human CREB3 C-terminal domain with transmembrane region | VYVGGLESRVLKYTAQNMELQNKVQLLEEQNLSLLDQL RKLQAMVIEISNKTSSSSTCILVLLVSFCLLLVPAMYSSD TRGSLPAEHGVLSRQLRALPSEDPYQLELPALQSEVPKD STHQWLDGSDCVLQAPGNTSCLLHYMPQAPSAEPPLEW PFPDLFSEPLCRGPILPLQANLTRKGGWLPTGSPSVILQD RYSG | 225 |
| Human CREB3 C-terminal domain without transmembrane region | VYVGGLESRVLKYTAQNMELQNKVQLLEEQNLSLLDQL RKLQAMVIEIS | 226 |
| CREB3-TRE coding sequence | atggagctggaattggatgctggtgaccaagacctgctggccttcctgctagaggaaagt ggagatttggggacggcacccgatgaggccgtgagggccccactggactgggcgctg ccgctttctgaggtGccgagcgactgggaagtagatgatttgctgtgctccctgctgagtc ccccagcgtcgttgaacattctcagctcctccaaccccctgccttgtccaccatgaccacac ctactccctcccacgggaaactgtctctatggatctagagagtgagagctgtagaaaaga ggggacccagatgactccacagcatatggaggagctggcagagcaggagattgctagg ctagtactgacagatgaggagaagagtctattggagaaggaggggcttattctgcctgag acacttcctctcactaagacagaggaacaaattctgaaacgtgtgcggCTTGAGCC CGGAGAGAAGCCGTACAAGTGCCCTGAGTGCGGCAA GTCTTTTAGCAGAAGAGACGAACTTAATGTCCACCAG CGAACGCATACTGGTGAAAAGCCCTATAAATGTCCTG AATGTGGGAAATCATTCTCCAGCCGCAGAACCTGTAG GGCTCACCAGCGAACACACACCGGCGAAAAACCATA CAAATGTCCAGAATGCGGGAAATCCTTTTCTCAGTCAT CCAACTTGGTGAGACATCAACGCACGCACACTGGAGA | 214 |

(continued)

| Description | SEQUENCE | SEQ ID NO |
|---|---|---|
| | AAAGCCTTACAAATGCCCGGAATGTGGAAAGTCTTTT TCCCAATTGGCCCATTTGCGAGCCCATCAGAGGACTC ACACGGGCGAGAAACCTTACAAATGCCCGGAATGCGG GAAATCTTTTTCAACGAGTGGCAACCTCGTAAGACAC CAAAGAACGCATACAGGCGAAAAGCCATATAAGTGTC CTGAGTGTGGTAAATCATTCTCACACAGGACCACCCT GACAAATCACCAGCGCACGCACACCGGCAAGAAGAC AAGCgtgtaCgttgggggtttagagagcCgggtcttgaaatacacagcccagaatat ggagcttcagaacaaagtacagcttctggaggaacagaatttgtcccttctagatcaactg aggaaactccaggccatggtgattgagatCtcaaacaaaaccagcagcagcagcacct gcatcttggtcctGctagtctccttctgcctcctccttgtacctgctatgtactcctctgacac aaggggggagcctgccagctgagcatggagtgttgtcccgccagcttcgtgccctcccca gtgaggacccttaccagctggagctgcctgccctgcagtcagaagtgccgaaagacagc acacaccagtggttggacggctcagactgtgtactccaggcccctggcaacacttcctgc ctgctgcattacatgcctcaggctcccagtgcagagcctcccctggagtggccCttccct gacctcttctcagagcctctctgccgaggtcccatcctccccctgcaggcaaatctcacaa ggaagggaggatggcttcctactggtagcccctctgtcattttgcaggacagatactcagg c | |
| CREB3-TRE | MELELDAGDQDLLAFLLEESGDLGTAPDEAVRAPLDWA LPLSEVPSDWEVDDLLCSLLSPPASLNILSSSNPCLVHHD HTYSLPRETVSMDLESESCRKEGTQMTPQHMEELAEQEI ARLVLTDEEKSLLEKEGLILPETLPLTKTEEQILKRVRLEP GEKPYKCPECGKSFSRRDELNVHQRTHTGEKPYKCPEC GKSFSSRRTCRAHQRTHTGEKPYKCPECGKSFSQSSNLV RHQRTHTGEKPYKCPECGKSFSQLAHLRAHQRTHTGEK PYKCPECGKSFSTSGNLVRHQRTHTGEKPYKCPECGKSF SHRTTLTNHQRTHTGKKTSVYVGGLESRVLKYTAQNME LQNKVQLLEEQNLSLLDQLRKLQAMVIEISNKTSSSSTCI LVLLVSFCLLLVPAMYSSDTRGSLPAEHGVLSRQLRALP SEDPYQLELPALQSEVPKDSTHQWLDGSDCVLQAPGNTS CLLHYMPQAPSAEPPLEWPFPDLFSEPLCRGPILPLQANL TRKGGWLPTGSPSVILQDRYSG | 215 |

## EXAMPLES

The following examples are included to further describe some aspects of the present disclosure, and should not be used to limit the scope of the invention.

## EXAMPLE 1

## Identification of Target Regions Capable of Upregulating SCN1A Using SCN1A Specific Transcriptional Activators

In order to identify regions of the genome capable of upregulating endogenous SCN1A expression, various engineered transcription factors (either zinc finger nucleases or gRNA/daCas9 constructs) were designed that targeted various regions of the genome as set forth in **TABLEs 4** and **13** above. For gRNA/daCas9 constructs, the gRNA had the same sequence as the target region because the gRNA was designed to target the complementary genomic strand. The dCas9 protein was a dCAS9-VP64 construct (SEQ ID NO: 174).

HEK293 cells were cultured per standard methods, and transfected (FugeneHD, Promega) with 3ug plasmid carrying an engineered transcription factor or control construct per well of a 6-well plate. Cells were transfected with plasmids expressing the constructs indicated in **TABLE 13** below. 48h following transfection, cells were collected and RNA was isolated (Qiagen RNeasy Mini kit), and DNase treated. RNA (3ug) was reverse transcribed using OligoDT primers (Superscript IV, Invitrogen). cDNA samples were analyzed by qPCR using Phusion Polymerase (New England Biolabs) and SYBR Green I: (30s at 98 °C, 40x[10 sec at 98 °C, 15 sec at 66 °C, 15 sec at 72 °C] ). Primers against SCN1A (5'-TGTCTCGGCATTGAGAACATTC-3' (SEQ ID NO: 185); 5'-ATTGGTGGGAGGCCATTGTAT-3' (SEQ ID NO: 186)) were used to quantify levels of endogenous SCN1A transcript, and relative levels of SCN1A expression were determined by the delta-delta Ct method with GAPDH as a reference gene (5'-ACCACAGTCCATGCCATCAC'-3' (SEQ ID NO: 187); 5'-TCCACCACCCTGTTGCTGTA-3' (SEQ ID NO: 188)). Data are presented as fold changes relative to the control condition.

The results are shown in **FIG. 1** and in **TABLE 13** below as fold change of SCN1A transcription relative to control conditions (e.g., EGFP-KASH reporter construct). **TABLE 13** reports values for constructs that led to at least a 1.5 fold increase in transcription relative to the control conditions.

**TABLE 13.** Effect of different genomic target sites and corresponding eTFs had on transcription. CON indicates the zinc finger Construct that was used in the experiment (see **TABLE 1).** For the gRNA constructs, the target site and the gRNA sequence are the same since the gRNAs were designed to target the complementary DNA strand.

| CON | Chr 2 Start Position | Target Sequence | Target Site from FIG. 1 | SEQ ID NO for Target Site | Mean | Ttest |
|---|---|---|---|---|---|---|
| 4 | 166149168 | ctaggtcaagtgtaggag | z1 | 18 | 5.12090848 | 0.0096628 |
| 28 | 166149165 | GGTCAAGTGTAGGAGACA | z8 | 25 | 1.52068773 | 0.62403349 |
| 3 | 166128025 | taggtaccatagagtgag | z13 | 30 | 25.4730028 | 0.14942042 |
| 29 | 166127991 | gaggatactgcagaggtc | z14 | 31 | 8.6766618 | 0.16432794 |
| | 166149176 | aaggctgtctaggtcaagtgt | g9 | 35 | 1.36378425 | 0.18753821 |
| | 166149118 | tgttcctccagattaacactt | g10 | 36 | 1.63040825 | 0.46710683 |
| | 166128002 | gatgaagccgagaggatactg | g11 | 37 | 18.7579211 | 0.13148732 |
| | 166128037 | gctgatttgtattaggtacca | g12 | 38 | 22.4892633 | 0.09291316 |
| | 166177299 | AGAAAGCTGATACAGATACAA | g15 | 39 | 1.7542842 | 0.34519408 |
| | 166178880 | ggtacgggcaaagatttcttg | g17 | 40 | 1.36801947 | 0.48762102 |
| | 166177299 | AGAAAGCTGATACAGATACAA | g19 | 41 | 1.45636874 | 0.44464045 |
| | 166177369 | ACACAATGAGCCACCTACAAG | g20 | 42 | 1.31187425 | 0.42605224 |
| | 166177362 | GTGGCTCATTGTGTGTGTGCC | g22 | 43 | 1.25217773 | 0.26572657 |
| | 166155264 | CATATCCCTGCAGGTTCAGAA | g24 | 44 | 1.75688991 | 0.28984533 |
| | 166155099 | agagagagagagagagagaga | g25 | 45 | 2.05701745 | 0.42409102 |
| | 166155393 | TTCTCAGTTTTGAAATTAAAA | g26 | 46 | 1.64498972 | 0.21705582 |
| | 166155255 | TGGATTCTCTTCTGAACCTGC | g27 | 47 | 2.27026665 | 0.43195546 |
| | 166148361 | TGCTGAGGCAGGACACAGTGT | g29 | 48 | 2.4290169 | 0.30364553 |
| | 166148843 | ATCATCTGTAACCATCAAGGA | g30 | 49 | 2.58328006 | 0.0748197 |
| | 166148565 | TCCTGCCTACTTAGTTTCAAG | g31 | 50 | 1.97097781 | 0.25980859 |
| | 166148953 | ATTACAGTTCTGTCAGCATGC | g32 | 51 | 1.34500323 | 0.32186367 |
| | 166149373 | TGGTCTCATTCTTTTTGTGGG | g33 | 52 | 1.71471378 | 0.32104302 |
| | 166142239 | CGATATTTTCATGGATTCCTT | g34 | 53 | 1.7735976 | 0.21954265 |
| | 166142391 | CTGACACTTACTTTGTCTAAA | g35 | 54 | 1.95513108 | 0.02069095 |

(continued)

| CON | Chr 2 Start Position | Target Sequence | Target Site from FIG. 1 | SEQ ID NO for Target Site | Mean | Ttest |
|---|---|---|---|---|---|---|
| | 166142219 | AAAACTGGAACCGCATTCCCA | g36 | 55 | 2.08698135 | 0.0454403 |
| | 166142396 | ACAAAGTAAGTGTCAGTGTGG | g37 | 56 | 1.30739959 | 0.72725347 |
| | 166142344 | ATAATAGTTGTGTCTTTATAA | g38 | 57 | 1.55783618 | 0.29846459 |
| | 166141162 | TGTACAAGCAGGGCTGCAAAG | g39 | 58 | 1.4663605 | 0.02946062 |
| | 166140590 | GTTAACAAATACACTAAACAC | g40 | 59 | 1.37399196 | 0.33638238 |
| | 166140928 | ttcaacaagctcccaagaagt | g42 | 60 | 1.46929899 | 0.24465271 |
| | 166141090 | ATGTTCAAGGTGCAGAAGGAA | g43 | 61 | 2.04547409 | 0.09880194 |
| | 165990246 | TGTTTGCTCAAACGTGCACCA | g44 | 62 | 2.13402102 | 0.25583999 |
| | 165989684 | AAATAAGACATGAAAACAAGA | g45 | 63 | 1.27016182 | 0.32368695 |
| | 165990193 | AAATATGTACCACAAGAAATG | g46 | 64 | 2.29522738 | 0.41829497 |
| | 165990076 | TATCTGGTTTCTCTCACTGCT | g47 | 65 | 1.44542116 | 0.0947106 |
| | 165989571 | ATTGCAAAGCATAATTTGGAT | g48 | 66 | 1.42246971 | 0.18117243 |

## EXAMPLE 2

### Upregulation of Endogenous SCN1A in HEK293 Cells Using SCN1A Specific Transcription Factors

HEK293 cells were cultured per standard methods and plated into 6-well plates. Cells in each well were transfected (FugeneHD, Promega) with 3ug of a plasmid carrying either a single engineered transcription factor construct, a WT human CREB3 (SEQ ID NO: 211), or an EGFP control construct. The engineered transcription factor constructs tested included: Constructs 1-27 and 46-53 **(TABLE 1)** and a plasmid expressing CREB3-TRE (SEQ ID NO: 215; CREB3 with the bZIP DNA binding domain replaced with the TET promoter-targeted synthetic ZF domain) (each tested separately). 48h following transfection, cells were collected and RNA was isolated (Qiagen RNeasy Mini kit), and DNase treated. RNA (3ug) was reverse transcribed using OligoDT primers (Superscript IV, Invitrogen). cDNA samples were analyzed by qPCR using Phusion Polymerase (New England Biolabs) and SYBR Green I: (30s at 98 °C, 40x[10 sec at 98 °C, 15 sec at 66 °C, 15 sec at 72 °C] ). Primers against SCN1A (5'-TGTCTCGGCATTGAGAACATTC-3' (SEQ ID NO: 185); 5'-ATTGGTGGGAGGCCATTGTAT-3' (SEQ ID NO: 186)) were used to quantify levels of endogenous SCN1A transcript, and relative levels of SCN1A expression were determined by the delta-delta Ct method with GAPDH as a reference gene (5'-ACCACAGTCCATGCCATCAC'3' (SEQ ID NO: 187); 5'-TCCACCACCCTGTTGCTGTA-3' (SEQ ID NO: 188)). Data are presented as fold changes relative to the control condition (see **FIG. 2A, FIG. 2B and FIG. 2C).** The control construct consisted of EGFP expressed under the control of RE 1 (SEQ ID NO: 1). Delivery of engineered transcription factors induced varying degrees of upregulation in endogenous SCN1A transcript with respect to the EGFP condition.

## EXAMPLE 3

### Upregulation of Endogenous SCN1A in GABA Neurons Using SCN1A Specific Transcription Factors

iCell GABA neurons (Cellular Dynamics) were plated in a 6-well plate (~1E6 cells/well) and maintained per manufacturer's recommended protocol. 72 h following plating, recombinant AAV (serotype AAV-DJ) expressing EGFP or an activator Construct 30 in **FIG. 3A** or Construct 25 or Construct 16 in **FIG. 3B)** under the control of a ubiquitous promoter (CBA promoter) was added to the culture media at approximately 2E11 genome copies/well. One week **(FIG. 3A)** or two weeks **(FIG. 3B)** following infection, RNA was isolated from cultured cells (Qiagen RNeasy Mini kit), and DNase treated. Recovered RNA was reverse transcribed using OligoDT primers (Superscript IV, Invitrogen). cDNA samples were analyzed by qPCR using Phusion Polymerase (New England Biolabs) and SYBR Green I: (30s at 98 °C, 40x[10 sec at 98 °C, 15 sec at 66 °C, 15 sec at 72 °C]). Primers against SCN1A (5'-TGTCTCGGCATTGAGAACATTC-3' (SEQ ID NO: 185); 5'-ATTGGTGGGAGGCCATTGTAT-3' (SEQ ID NO: 186)) were used to quantify levels of endogenous SCN1A transcript, and relative levels of SCN1A expression were determined by the delta-delta Ct method with GAPDH as a reference gene (5'-ACCACAGTCCATGCCATCAC'3' (SEQ ID NO: 187); 5'-TCCACCACCCTGTTGCTGTA-3' (SEQ ID NO: 188)). Data are presented as fold changes relative to the control condition (see **FIG. 3A** and **FIG. 3B).** AAV-driven expression of engineered transcription factors produced significant upregulation of endogenous SCN1A transcript in cultured iPS-derived GABA neurons.

**EXAMPLE 4**

**Specific Upregulation of Endogenous SCN1A in GABA Neurons Using an SCN1A Specific Transcription Factor**

iCell GABA neurons (Cellular Dynamics) were plated in a 6-well plate (~1E6 cells/well) and maintained per manufacturer's recommended protocol. 72h following plating, recombinant AAV (serotype AAV-DJ) expressing EGFP or activator (Construct 30), which comprises a zinc finger DBD fused to a VPR TAD driven by a CBA promoter) under the control of a CBA promoter was added to the culture media at approximately 2E11 genome copies/well.

One week following infection, RNA was isolated from cultured cells (Qiagen RNeasy Mini kit), and DNase treated. RNAseq libraries were prepared from the recovered RNA, using the TruSeq Stranded mRNA library kit (Illumina) and sequenced on an Illumina NextSeq (2 x 75 cycle paired end sequencing). Sequencing reads were aligned to human genome (RNASTAR) and differential expression analysis was performed with DESeq2. Data are presented as fold change with respect to control (AAVDJ-CBA-EGFP) samples (see **FIG. 4).** Results are shown in **TABLE 14** and **FIG. 4** illustrates the relative expression of endogenous SCN1A and the 40 nearest neighboring gene transcripts presented as fold changes relative to the control condition. Construct 30, as described in **TABLE 1,** was able to specifically increase expression of the SCN1A gene, or the Nav1.1 protein, as compared to the other genes examined. This indicated the target site recognized by the transcriptional activator of Construct 30 was specific for the SCN1A gene, thus resulting in an increase in SCN1A gene expression in GABA neurons.

**TABLE 14.** Effects on transcription of endogenous SCN1A and the 40 nearest neighbor genes in GABA neurons treated with an SCN1A specific transcription factor (Construct 30).

| Gene Name | Chr 2 Start | Chr 2 End | Chr Strand | Fold Change vs. Control |
|---|---|---|---|---|
| PLA2R1 | 160788518 | 160919121 | - | 0.16367458 |
| ITGB6 | 160956176 | 161128399 | - | 0.20679884 |
| RBMS1 | 161128661 | 161350305 | - | 1.63514667 |
| TANK | 161993418 | 162092732 | + | 0.90946407 |
| PSMD14 | 162164548 | 162268228 | + | 0.92699237 |
| TBR1 | 162272604 | 162282381 | + | 0.53199642 |
| SLC4A10 | 162280842 | 162841792 | + | 1.89407328 |
| DPP4 | 162848750 | 162931052 | - | 2.82345284 |
| FAP | 163027193 | 163101661 | - | 2.26977379 |
| IFIH1 | 163123588 | 163175213 | - | 1.46146481 |
| GCA | 163175349 | 163228105 | + | 2.58702426 |
| FIGN | 164449905 | 164592522 | - | 0.46785861 |
| GRB14 | 165349321 | 165478358 | - | 0.5631965 |
| COBLL1 | 165510133 | 165700189 | - | 0.43199257 |
| SLC38A11 | 165752695 | 165812035 | - | 4.06730119 |
| SCN3A | 165944031 | 166060577 | - | 1.0807866 |
| SCN2A | 166095911 | 166248818 | + | 1.24475196 |
| CSRNP3 | 166326156 | 166545917 | + | 0.82971233 |
| GALNT3 | 166604100 | 166651192 | - | 0.33804418 |
| TTC21B | 166713984 | 166810353 | - | 1.58661143 |
| SCN1A | 166845669 | 166984523 | - | 62.9552975 |

(continued)

| Gene Name | Chr 2 Start | Chr 2 End | Chr Strand | Fold Change vs. Control |
|---|---|---|---|---|
| SCN9A | 167051694 | 167232503 | - | 1.71659087 |
| SCN7A | 167260082 | 167350757 | - | 0.29331967 |
| B3GALT1 | 168675181 | 168730551 | + | 0.64436013 |
| STK39 | 168810529 | 169104651 | - | 1.19821739 |
| CERS6 | 169312371 | 169631644 | + | 0.86828378 |
| NOSTRIN | 169643048 | 169722024 | + | 1.82142718 |
| SPC25 | 169690641 | 169769881 | - | 0.86880697 |
| ABCB11 | 169779447 | 169887832 | - | 3.1441368 |
| DHRS9 | 169921298 | 169952677 | + | 1.10381777 |
| BBS5 | 170335687 | 170382432 | + | 0.65476347 |
| KLHL41 | 170366211 | 170382772 | + | 0.87373377 |
| FASTKD1 | 170386258 | 170430385 | - | 1.02786927 |
| PPIG | 170440849 | 170497916 | + | 1.09866236 |
| CCDC173 | 170501934 | 170550943 | - | 0.67290779 |
| PHOSPHO2 | 170550974 | 170558218 | + | 0.91339152 |
| KLHL23 | 170550997 | 170633499 | + | 0.73926347 |
| SSB | 170648442 | 170668574 | + | 1.00631994 |
| METTL5 | 170666590 | 170681441 | - | 1.21271497 |
| UBR3 | 170683967 | 170940641 | + | 1.21350908 |
| MYO3B | 171034654 | 171511681 | + | 0.52839217 |

## EXAMPLE 5

**Expression of SCN1A From an Expression Cassette *In Vivo***

To test the expression of transcriptional activators of SCN1A *in vivo,* recombinant AAV9 vectors were generated by Vector Biolabs (Malvern, PA). Male C57Bl/6 mice (N=5 per group, 7-8 weeks old) were infused bilaterally with 1.5ul of purified AAV vector into the dorsal hippocampus (AP -2.0 mm, lateral ±1.5, DV -1.4 mm from dura) and ventral hippocampus (AP - 3.1 mm, lateral ±2.8, DV -3.8 mm from dura), for a total of 4 injection sites. AAV was delivered at a rate of 0.3 ul/minute with a 4m rest period following each injection. Four weeks after treatment, mice were euthanized and hippocampal tissue was dissected. For each group, tissue from both the left and right hippocampus tissue was collected pooled for homogenization in most animals (N=4), except for one animal, where only the left hippocampus was collected and homogenized. RNA was isolated from the homogenate (Qiagen RNeasy Mini kit), and DNase treated. RNA (3μg) was reverse transcribed using OligoDT primers (Superscript IV, Invitrogen). cDNA samples were analyzed by qPCR for expression of mouse SCN1A using Phusion Polymerase (New England Biolabs) and SYBR Green I: 30s at 98 °C, 40x [10 sec at 98 °C, 15 sec at 64 °C, 15 sec at 72 °C]. Primers against mouse SCN1A (5'-CAAAAAAGCCACAAAAGCCT-3' (SEQ ID NO: 189); 5'-TTAGCTCCGCAAGAAACATC-3' (SEQ ID NO: 190)) were used to quantify levels of endogenous SCN1A transcript, and relative levels of SCN1A expression *in vivo* were determined by the delta-delta Ct method with GAPDH as a reference gene (5'-ACCACAGTCCATGCCATCAC'3' (SEQ ID NO: 187); 5'-TCCACCACCCTGTTGCTGTA-3' (SEQ ID NO: 188))

FIG. 5A and FIG. 5B illustrate the mean results of five animals, each injected with an AAV9 construct. The eGFP control construct comprised an eGFP reporter transgene. Construct 4 (see TABLE 1) comprised a transcriptional activator that recognized a target sequence comprising SEQ ID NO: 18, as described in TABLE 1 above. FIG. 5A illustrates the relative expression of SCN1A *in vivo.* FIG. 5B illustrates the change in SCN1A expression *in vivo* as a percentage of mean eGFP expression. These results indicated the SCN1A transcriptional activator of expression cassette A resulted in approximately 20%-30% upregulation of SCN1A expression *in vivo.*

Such expression cassettes can be adapted for use in humans to treat Dravet syndrome, epilepsy, seizures, Alzheimer's disease, Parkinson's disease, and/or any other diseases or conditions associated with a deficiency and/or impaired activity of SCN1A.

## EXAMPLE 6

**Hyperthermic Seizure (HTS) Assay in Mouse Models of Dravet Syndrome**

A. Heterozygous Scnla Knockout Mouse Model

Treatment of Dravet syndrome and/or symptoms thereof using the expression cassettes was tested in the Scn1a*tmlKea* mouse line. This mouse line is an established mouse model for Dravet syndrome. Scn1a*tmlKea* mouse lines do not require CRE recombinase. The Scn1a*tmlKea* mouse (available from the Jackson Laboratory; described in Hawkins et al., Scientific Reports, vol. 7: 15327 (2017)) comprises a deletion of the first coding exon of SCN1A. Mice homozygous for the SCN1A knockout allele are characterized by tremors, ataxia, seizures, and die by postnatal day 16. Heterozygous mice on the C57BL/6 background develop spontaneous seizures and a large percentage die within weeks. Such a mouse strain can be used to study safety and efficacy of treatment of epilepsy and Dravet syndrome. See Miller et al., Genes Brain Behav. 2014 Feb;13(2):163-72 for additional information.

To test the efficacy of transcriptional activators in the Scn1a*tmlKea* mouse line, litters of pups produced from male Scn1a +/- crossed with female C57Bl/6J breeding were dosed with AAV vector via bilateral ICV at P1. Mice were dosed with Constructs 31-34 **(TABLE 1)**. Mice were left undisturbed with their dam until weening at P18 and then again left undisturbed until P26-P28 when the hyperthermic seizure (HTS) assay was initiated. Separate litters of dosed P1 mice were weened at P18 and observed for mortality daily. Hyperthermia seizure induction was performed in P26-P28 heterozygous (HET) and WT Scn1a mice in a mixed 129Stac X C57BL/6 background. Prior to the assay mice had a lubricated rectal temperature probe (Ret-4) inserted and connected to a temperature control module (TCAT 2DF, Physitemp) that was connected in series with a heating lamp (HL-1). Mice were then placed into a large glass beaker and briefly allowed to equilibrate to the environment. Following this, body temperature was increased by ~0.5° C every 2 minutes until the onset of the first tonic-clonic seizure accompanied by loss of posture or until 43° C was reached. If a mouse experienced a seizure with loss of posture the experiment was ended and the internal body temperature of the mouse was recorded. If no seizure with loss of posture was detected over the full course of the experiment, that mouse was considered seizure free and the assay concluded. Tissue samples were obtained from the mice at P1 and genotyping of the mice was performed during the course of the experiment using real-time PCR. The genotyping was unblinded after the assay had been completed and the status of the mice as HET or WT was correlated to the data obtained. Data was plotted in a Kaplan-Meier survival curve and significance determined by the Mantel-Cox test. Results are shown in **TABLE 15** and **TABLE 16** and **FIGs. 6A-E.**

**TABLE 15.** Summary of conditions used in Example 6.

| Construct | Dosage (gc/mouse) |
|---|---|
| Construct 31 (FIG. 6A & 6E)) | 6.0E+10 |
| Construct 32 (FIG 6B, 6D, 6E) | 3.1E+11 |
| Construct 33 (FIG. 6C) | 5.8E+10 |
| Construct 34 (FIG. 6D) | 4.9E+ 13 |

**TABLE 16.** Summary of results of hyperthermic seizure assay.

| eTF Construct (FIG.) | # Control Animals (PBS treated) | # Treated Animals | % Seizure Free at 42.6 °C | P Value |
|---|---|---|---|---|
| EGFP reporter | 16 | N/A | 44% | |
| Construct 31 (FIG. 6A & 6E)) | 16 | 18 | 95 | P<0.0001 |
| Construct 32 (FIG 6B, 6D, 6E) | 16 | 21 | 76 | P<0.05 |
| Construct 33 (FIG. 6C) | 16 | 14 | 93 | P<0.001 |
| Construct 34 (FIG. 6D) | 16 | 12 | 83 | P<0.05 |

Additional experiments were conducted in Sen1a*tmlKea* mice as described above to test Constructs 42 and 43 for their effect on seizures in the HTS assay. In these experiments, Construct 42 was dosed at $9 \times 10^{10}$ gc/mouse via bilateral ICV at P1 and Construct 43 was dosed at $6 \times 10^{10}$ gc/mouse via bilateral ICV at P1 or P5. Results are shown in **FIG. 6F** (Construct 42) and **FIG. 6G** (Construct 43). Both constructs showed a significant reduction in seizures as compared to EGFP controls (P < 0.0001 for both Construct 42 and 43).

B. Heterozygous Scn1a^RX Mutant Mouse Model

Treatment of Dravet syndrome and/or symptoms thereof using an expression cassette of the present disclosure was tested in the Scn1a^RX mouse line. This mouse line is an established mouse model for Dravet syndrome. Sen1a^RX mouse lines do not require CRE recombinase. The Scn1a^RX mouse (available from the Jackson Laboratory; described in Ogiwara et al., J. Neuroscience, vol. 27: 5903-5914 (2007)) comprises a loss of function single base nonsense mutation of the in exon 21 of the SCN1A gene (CgG to TgA; R1407X). Heterozygous mice on the C57BL/6 background develop spontaneous seizures and a large percentage die within weeks.

To test the efficacy of transcriptional activators in the Scn1a^RX mouse line, litters of pups produced from male and IVF crossing of Scn1a^RX/+ sperm with female C57Bl/6J ova with embryos implanted into CD-1 dams were dosed with AAV vector (Construct 31) at $5.1 \times 10^{10}$ genome copies (gc)/mouse or PBS control via bilateral ICV at P1. Mice were left undisturbed with their dam until weening at P18 and then again left undisturbed until P26-P28 when the HTS assay was initiated. Separate litters of dosed P1 mice were weened at P18 and observed for mortality daily. Hyperthermia seizure induction was performed in P26-P28 heterozygous (HET) and WT Scnla mice in a C57BL/6 background. Prior to the assay mice had a lubricated rectal temperature probe (Ret-4) inserted and connected to a temperature control module (TCAT 2DF, Physitemp) that was connected in series with a heating lamp (HL-1). Mice were then placed into a large glass beaker and briefly allowed to equilibrate to the environment. Following this, body temperature was increased by ~0.5° C every 2 minutes until the onset of the first tonic-clonic seizure accompanied by loss of posture or until 43° C was reached. If a mouse experienced a seizure with loss of posture the experiment was ended and the internal body temperature of the mouse was recorded. If no seizure with loss of posture was detected over the full course of the experiment, that mouse was considered seizure free and the assay concluded. Tissue samples were obtained from the mice at P1 and genotyping of the mice was performed during the course of the experiment using real-time PCR. The genotyping was unblinded after the assay had been completed and the status of the mice as HET or WT was correlated to the data obtained. None of the WT Scna1 mice tested experienced a seizure. Data for the Construct 31 treated (n=13) and PBS control treated (n=14) HET mice was plotted in a Kaplan-Meier survival curve and significance determined by the Mantel-Cox test. As shown in **FIG. 6H,** Construct 31 treated HET mice show a significant reduction in hyperthermia seizure induction over PBS control treated HET mice (P < 0.01).

**EXAMPLE 7**

**Survival Assay in Mouse Model of Dravet Syndrome**

A. Heterozygous Scn1a Knockout Mouse Model

To test the efficacy of transcriptional activators in the Scn1a^tmlKea mouse line, litters of pups produced from male Scn1a +/- crossed with female C57Bl/6J breeding were dosed with AAV vector via bilateral ICV at P1. Mice were left undisturbed with their dam until weaning. Observation of the health status of Scn1a +/- mice was performed daily following weaning at P18. Mice that were found dead in their home cage of any cause had the date recorded. Data was plotted in a Kaplan-Meier survival curve and significance determined by the Mantel-Cox test.

Results are shown in **TABLE 17** and **FIGs. 7A-D.**

**TABLE 17.** Summary of conditions and results for survival assay.

| SEQ ID | Dosage (gc/mouse) | # Control Animals (PBS treated) | # Treated Animals | % Survival at P100 (*at P83) | P Value |
|---|---|---|---|---|---|
| PBS | N/A | 53 | N/A | 49% | |
| Construct 33 (FIG. 7C & 7D) | 5.8E+ 10 | 53 | 29 | 76% | P<0.05 |
| Construct 31 (FIG. 7B & 7D) | 6.0E+10 | 53 | 34 | 97% | P<0.0001 |

B. Heterozygous Scn1a*RX* Mutant Mouse Model

To test the efficacy of transcriptional activators in the Scn1a*RX* mouse line, litters of pups produced from male and IVF crossing of Scn1a*RX/+* sperm with female C57Bl/6J ova with embryos implanted into CD-1 dams were dosed with AAV vector (Construct 31) at $5.1 \times 10^{10}$ genome copies (gc)/mouse or PBS control via bilateral ICV at P1. Mice were left undisturbed with their dam until weaning. Observation of the health status of Scn1*RX/+* mice was performed daily following weaning at P18. Mice that were found dead in their home cage of any cause had the date recorded. Data for Construct 31 treated (n=27) and control treated (n=18) was plotted in a Kaplan-Meier survival curve and significance determined by the Mantel-Cox test.

As shown in **FIG. 7E,** Construct 31 treated Scn1a*RX/+* mice had increased survival over PBS control treated Scn1a*RX/+* mice (P < 0.0001).

**EXAMPLE 8**

**SCN1A Transcription Levels in Non-Human Primates Following Treatment with AAV Encoding SCN1A Specific Transcription Factor**

The study used male cynomolgus macaques (macaca fascicularis) between ages 2 and 3. Animals were prescreened for cross-reactive antibody to AAV9 prior to enrollment in the study by a cell-based neutralizing antibody assay. AAV9 expressing an SCN1A specific transcription factor (Construct 33) or a control was diluted in PBS and injected intraparenchymally at 1.2E12 gc/animal. Three different stereotaxic coordinates in each hemisphere, six injection sites per animal, were identified for the injections. 10 ul volume was injected per site. Injections in the right hemisphere were symmetrical to those in the left. Two untreated animals were used as a control.

To assess Scn1A mRNA expression, reverse transcription followed by qPCR method was conducted. At necropsy, 28 days post dosing, tissues sections from various regions of the brain (frontal cortex, parietal cortex, temporal cortex, occipital cortex, hippocampus, medulla, cerebellum; 200 mg each) from control and treated animals were collected in RNAlater and then frozen. Briefly, 30 mg of tissue was dissected, RNA extracted (with Qiagen Rneasy Lipid tissue mini kit, catalog # 1023539), converted to cDNA by reverse transcription (using Applied Biosystems high capacity cDNA Reverse Transcription kit, catalog # 4368814) and qPCR performed using primer/probe set for Scn1A and housekeeping gene GAPDH (Applied Biosystems, catalog # Rh02621745-gl FAM).

Primer/probe sets for SCN1A are given below.

**TABLE 18.** Primer Sequences used in Example 8.

| Gene | SEQ ID NO | Sequence (5'-3') | Note |
|---|---|---|---|
| Scn1A | 191 | CCATGGAACTGGCTCGATTTCAC | F-primer |
| | 192 | ATTGGTGGGAGGCCACTGTAT | R-primer |
| | 193 | AGGCCTGAAAACCATTGTGGGAGCCCT | Probe (FAM) |

Gene expression of Scn1A in each test sample was determined by relative quantitation (RQ) using the comparative Ct (ΔCt) method. This method measures Ct difference (ΔCt) between target gene and housekeeping gene, then compares ΔCt values of treatment samples to control samples.

$$\Delta Ct = Ct \text{ average of Target gene} - Ct \text{ average of housekeeping gene}$$

$$\Delta\Delta Ct = \Delta Ct \text{ of treatment sample} - \Delta Ct \text{ control sample}$$

$$\text{Relative expression (treatment sample)} = 2^{-\Delta\Delta Ct}$$

Data is reported as normalized expression of target mRNA in different tissue sections from the brain (see **FIG. 8).** As illustrated in **FIG. 8,** sites in the brain proximal to the intraparenchymal injection sites showed the highest levels of SCN1A transcript expression.

**EXAMPLE 9**

**Selective Transgene Expression in PV Neurons in Non-Human Primates Following Treatment with AAV Having PV Selective Promoter and MicroRNA Binding Site**

The study used six marmosets (Callithrix jacchus) that were prescreened for cross-reactive antibody to AAV9 prior to enrollment in the study. Two monkeys were treated with AAV9 containing an EGFP transgene under the control of the EF 1 alpha promoter, two monkeys were treated with AAV9 containing an EGFP transgene under the control of RE 2 (SEQ ID NO: 2), and two monkeys were treated with AAV9 containing an EGFP transgene under the control of RE 2 (SEQ ID NO: 2) and also containing a microRNA binding site (SEQ ID NO: 7) located between the coding region of EGFP and the polyA tail. The AAV9 vectors were diluted in PBS and the animals were treated with three intracerebral injections (2 uL each) into the hippocampus/entorhinal cortex of each hemisphere for a total of 6 injection sites per animal. The two animals treated with the AAV9 vector containing EF1alpha-EGFP each received a total dose of 5.8E+11 gc/animal, the two animals treated with the AAV9 vector containing RE 2-EGFP each received a total dose of 3.0E+11 gc/animal, and the two animal treated with the AAV9 vector containing RE 2 + m1-EGFP each received a total dose of 2.3E+11 gc/animal.

Immunohistochemistry was used to assess paravalbumin (PV) selective expression. At necropsy, 28 days post dosing, tissues sections from various regions of the brain were collected. Floating Marmoset brain sections (35um) were fixed in 4% paraformaldehyde, blocked with buffer (PBS, 3% BSA, 3% Donkey Serum, 0.3% Triton-X 100, 0.2% Tween-20) and then stained with anti-GFP (Abcam ab290) followed by a secondary antibody conjugated to Alexa-488 (Thermo A21206). This was followed by anti-PV antibody (Swant) and secondary antibody conjugated to Alexa-647 (Thermo A31571) and 4',6-diamidino-2-phenylindole (DAPI). Sections were mounted and imaged using a PerkinElmer Vectra3.

Results are shown in **FIGs. 9A-F** and **10A-L.**

**EXAMPLE 10**

**eTF$^{SCN1A}$ Biodistribution**

The objective of this study was to compare the biodistribution of eTF$^{SCN1A}$ in the central nervous system (CNS) of juvenile cynomolgus macaque monkeys when administered at a dose of 4.8E+13 via unilateral intracerebroventricular (ICV) injection. Each animal was injected with AAV9 containing an expression cassette encoding eTF$^{SCN1A}$ under the control of a GABA selective regulatory element (RE$^{GABA}$-eTF$^{SCN1A}$). The AAV9 particles were formulated in PBS + 0.001% pluronic acid and administered at a dose of 4.8E+13 or 8E+13 vg/animal. A volume of 2 ml of formulated viral particles was administered to each animal. The study design is set forth in **TABLE 19.**

Twenty-four month old cynomolgus macaque monkeys were grouped as indicated in **TABLE 19.** Prior to initiation of the study, blood samples from the animals were tested for levels of neutralizing antibody titer to AAV9 using the NAb titer assay described above. Animals with low or negative results for antibodies were selected for the study. Samples were administered via ICV injection using standard surgical procedures. Thawed dosing material was briefly stored on wet ice and warmed to room temperature just prior to dosing. The animals were anesthetized, prepared for surgery, and mounted in a MRI compatible stereotaxic frame (Kopf). A baseline MRI was performed to establish target coordinates. An incision was made and a single hole was drilled through the skull over the target location. A 3 mL BD syringe attached to a 36" micro-bore extension set was prepared with sample and placed in an infusion pump. The extension line was primed. The dura was opened, and the dosing needle was advanced to a depth of 13.0 to 18.1 mm from the pia. Contrast media injection and fluoroscopy was used to confirm placement of the spinal needle into the right lateral ventricle. The 3.0" 22g Quinke BD spinal huber point needle was filled with contrast to determine placement prior to attaching the primed extension line and syringe. Pump settings were 0.1 mL/minute for 19 to 20 minutes. Buffer was pushed by hand post dose to clear the extension line. The needle remained in place for 1 to 2 minutes post completion of infusion and then the needle was withdrawn. The vehicle and test article were administered once on day 1 and the subjects were maintained for a 27- or 29-day recovery period.

**TABLE 19.** Biodistribution Study Design

| Group | Gender | ID | Dose (VG/animal) |
|---|---|---|---|
| Group 1 (Buffer Control) | M | 21001 | |
| | F | 11501 | |

(continued)

| Group | Gender | ID | Dose (VG/animal) |
|---|---|---|---|
| Group 2 (RE<sup>GABA</sup>-eTF<sup>CN1A</sup>) | M | 2001 | 4.8E+13 |
| | F | 2501 | 4.8E+13 |
| | M | 3001 | 4.8E+13 |
| | M | 3002 | 8E+13 |

Following dosing, animals were routinely monitored throughout the duration of the study and blood samples were periodically withdrawn. eTF$^{SCN1A}$ administration was not associated with any unexpected mortality, clinical findings, or macroscopic observations. AAV9-RE$^{GABA}$-eTF$^{SCN1A}$ treated animals survived until scheduled necropsy at day 28 $\pm$ 2 days. No clinical or behavioral signs, increases in body temperature, or body weight reduction were observed during daily or weekly physical examinations. Transient elevation in liver transaminases (ALT and AST) in AAV9-RE$^{GABA}$-eTF$^{SCN1A}$ treated animals were observed, but were fully resolved by the end of study without immunomodulation, and no concomitant increase in serum bilirubin or alkaline phosphatases was noted. No other measured clinical chemistry endpoint was remarkable. No microscopic observations were reported in the liver histopathology studies. CSF leukocytes were elevated in terminal collection relative to pre-treatment values but comparable between control and AAV9-RE$^{GABA}$-eTF$^{SCN1A}$ treated animals. No AAV9-RE$^{GABA}$-eTF$^{SCN1A}$ associated pleocytosis was observed. Macro-observations and detailed micro-histopathology examination of non-neuronal tissues across all animals were unremarkable. Tissues included major peripheral organs (i.e. heart, lungs, spleen, liver and gonads). Macro-observations and detailed micro-histopathology of neuronal tissues did not show any notable findings. Tissues included brain, spinal cord, and associated dorsal root ganglia (from cervical, thoracic and lumbar region). Studies were conducted by three independent pathologists including one at a specialized neuropathology site.

ICV administration of AAV9 did not prevent post-dose immune response in the serum, as anti-AAV9 capsid neutralizing antibodies were observed four weeks post-dose. However, neutralizing anti-AAV9 antibody levels in the CSF remained unchanged and comparable to pre-dose levels **(TABLE 20).**

**TABLE 20.** AAV9 serum NAb titer

| Subject Number | AAV9 Serum NAb Titer | | | AAV9 CSF NAb Titer | |
|---|---|---|---|---|---|
| | Pre-Screen | At Injection | 4-Weeks Post Injection | At Injection | 4-Weeks Post Injection |
| 21001 | 1:5 | < 1:5 | < 1:5 | < 1:5 | < 1:5 |
| 11501 | < 1:5 | < 1:5 | < 1:5 | < 1:5 | < 1:5 |
| 2001 | < 1:5 | < 1:5 | 1:405 | < 1:5 | 1:5 |
| 2501 | < 1:5 | < 1:5 | 1:135 | < 1:5 | 1:5 |
| 3001 | < 1:5 | < 1:5 | 1:1215 | < 1:5 | < 1:5 |
| 3002 | < 1:5 | < 1:5 | 1:135 | < 1:5 | < 1:5 |

Samples were collected 27-29 days post-dose from major organs (heart ventricles, liver lobes, lung cardiac lobes, kidneys, spleen, pancreas, and cervical lymph nodes) during schedule necroscopy. Punches were collected via eight millimeter punch and further processed as discussed below.

## EXAMPLE 11

### Biodistribution of eTF$^{SCN1A}$ in the Brain

ddPCR was used to measure eTF$^{SCN1A}$ biodistribution in the brain. Samples from various regions of cynomolgus macaque brain tissue (FC: Frontal cortex; PC: parietal cortex; TC: temporal cortex; Hip: hippocampus; Med: medulla; OC: occipital cortex) were measured for vector copy number to assess biodistribution of eTF$^{SCN1A}$ under the control of a GABA selective regulatory element (RE$^{GABA}$-eTF$^{SCN1A}$) when administered in AAV9 by unilateral ICV. Tissue DNA was isolated with DNeasy Blood & Tissues kits (Qiagen). DNA quantity was determined and normalized using UV spectrophotometer. 20 nanograms of tissue DNA was added to a 20 microliter reaction along with ddPCR Super Mix for Probes (no dUTP) (Bio-Rad) and TaqMan primers and probes directed against regions of the eTF$^{SCN1A}$ sequence. Droplets were generated and templates were amplified using automated droplet generator and thermo cycler (Bio-Rad). After the PCR step, the plate was loaded and read by QX2000 Droplet Reader to determine vector copy number in tissues. Monkey *Albumin* (Mf*Alb*) gene served as an internal control for normalizing genomic DNA content and was amplified in the same reaction. Primers and probes for eTF$^{SCN1A}$ and MfAlb are set forth in **TABLE 21.**

**TABLE 21.** Primers and probes for eTF$^{SCN1A}$ and MfAlb

| Primers / probe Name | | Sequence (5'-3') |
|---|---|---|
| eTF$^{SCN1A}$ | eTF$^{SCN1A}$ Forward primer | GAATGTGGGAAATCATTCAGTCGC (SEQ ID NO: 194) |
| | eTF$^{SCN1A}$ Reverse primer | GCAAGTTATCCTCTCGTGAGAAGG (SEQ ID NO: 195) |
| | eTF$^{SCN1A}$ probe | GCGACAACCTGGTGAGACATCAACGCACC (SEQ ID NO: 196) |
| MfAlbumin | MfAlb Forward primer | GCTGTTATCTCTTGTGGGCTGT (SEQ ID NO: 197) |
| | MfAlb Reverse primer | AAACTCATGGGAGCTGCCGGTT (SEQ ID NO: 198) |
| | MfAlb probe | CCACACAAATCTCTCCCTGGCATTG (SEQ ID NO: 199) |

eTF$^{SCN1A}$ was broadly distributed throughout the brain when dosed at 4.8E+13 viral genomes per animal with an average of 1.3-3.5 VG/diploid genome **(FIG. 11).** In addition, when comparing gene transfer throughout the brain of RE$^{GABA}$-eTF$^{SCN1A}$ dosed at 4.8E+13 viral genomes per animal to gene transfer throughout the brain of eGFP dosed via ICV at various doses, an increase in VG/diploid genome was observed with increasing doses. This indicated that gene transfer in the brain occurred in a dose-dependent manner when administered in AAV9 via ICV.

## EXAMPLE 12

### eTF$^{SCN1A}$ Transcription in the Brain

Transcription of eTF$^{SCN1A}$ under the control of a GABA selective regulatory element, RE$^{GABA}$ (RE$^{GABA}$-eTF$^{SCN1A}$), was assessed by measuring eTF$^{SCN1A}$ mRNA using a ddPCR-based gene expression assay. Tissue RNA was isolated with RNeasy Plus Mini kits (Qiagen) or RNeasy Lipid Tissue Mini kits (Qiagen) for brain tissues. RNA quantity was determined and normalized using UV spectrophotometer and RNA quality (RIN) was checked using Bioanalyzer RNA Chip. One microgram of tissue RNA was used for DNase treatment and cDNA synthesis with Super-Script VILO cDNA synthesis kit with ezDNase™ Enzyme kits (Thermo Fisher). 50 micrograms of RNA was converted to cDNA. cDNA was added to a 20 microliter reaction along with ddPCR Super Mix for Probes (no dUTP) (Bio-Rad) and TaqMan primers and probes directed against regions of eTF$^{sCN1A}$ sequence **(TABLE 22).** Droplets were generated and templates were amplified using automated droplet generator and thermo cycler (Bio-Rad). After PCR amplification, the plate was loaded and read by QX2000 Droplet Reader to provide gene expression levels in tissues. The monkey gene *ARFGAP2* (*MfARFGAP2*) (Thermo Fisher Scientific) served as an endogenous control for normalizing gene expression levels and was amplified in the same reaction. Average transcripts for *ARFGAP2* were 1.85E+6/ug RNA **(FIG. 12,** upper boundary). Limit of detection indicated by lower boundary.

eTF$^{SCN1A}$ mRNA was observed throughout the brain in all animals, indicating that the GABA-selective promoter, RE$^{GABA}$, was transcriptionally active in the brain tissue for all AAV9-RE$^{GABA}$-eTF$^{SCN1A}$ treated macaques **(FIG. 12).** FC: Frontal cortex; PC: parietal cortex; TC: temporal cortex; Hip: hippocampus; Med: medulla; OC: occipital cortex.

**TABLE 22.** TaqMan primers and probes directed against regions of eTF$^{SCN1A}$ sequence

| Primers / probe Name | Description | Sequence (5'-3') |
|---|---|---|
| eTF$^{SCN1A}$ | eTF$^{SCN1A}$ Forward primer | GAATGTGGGAAATCATTCAGTCGC (SEQ ID NO: 200) |
| | eTF$^{SCN1A}$ Reverse primer | GCAAGTTATCCTCTCGTGAGAAGG (SEQ ID NO: 201) |
| | eTF$^{SCN1A}$ probe | GCGACAACCTGGTGAGACATCAACGCACC (SEQ ID NO: 202) |
| *MfARFGAP2* | Forward, Reverse Primers, Probe | Thermo Fisher (Cat#: 4448491) |

## EXAMPLE 13

### eTF^SCN1A Biodistribution and Transcription in Peripheral Tissues

Vector copy number was further measured in various organs to evaluate transduction of RE^GABA-eTF^SCN1A in tissues throughout the body when administered in AAV9 by unilateral ICV. Transcript levels of eTF^SCN1A were also measured by ddPCR to assess transcriptional activity eTF^SCN1A under the control of the GABA-selective regulatory element RE^GABA in tissues throughout the body when administered in AAV9 by unilateral ICV. Both methods were performed as generally described above. RE^GABA-eTF^SCN1A transduction and transcription of eTF^SCN1A in the spinal cord (SC) and dorsal root ganglion (DRG) were comparable to levels observed in the brain. With the exception of the liver, RE^GABA-eTF^SCN1A transduction was lower in peripheral tissues outside of the brain **(FIG. 13).** Transduction of RE^GABA-eTF^SCN1A in the liver was higher than in the brain. Transcription of eTF^SCN1A was undetected in peripheral tissues, including the heart, lungs and gonads. However, eTF^SCN1A transcript levels in the liver were comparable to the levels of eTF^SCN1A measured in the brain. Furthermore, eTF^SCN1A transcription in the liver is extremely low when normalized to the number of vector copies present (approximately 1000-fold lower compared to transcription of eTF^SCN1A in the brain). Overall, this demonstrated that transcription of eTF^SCN1A under the control of the GABA-selective regulatory element RE^GABA is restricted to the CNS.

The invention provides the following non-limiting embodiments:

1. An expression cassette comprising a sequence encoding a non-naturally occurring transcription factor which increases expression of the SCN1A gene in a cell, wherein the non-naturally occurring transcription factor comprises a DNA binding domain (DBD) operably linked to at least two transcription activating domains (TAD) in the following manner: TAD1-TAD2-DBD, DBD-TAD3-TAD4, or TAD1-TAD2-DBD-TAD3-TAD4.

2. The expression cassette of embodiment 1, wherein TAD1, TAD2, TAD3 and TAD4 are independently selected from the following: VP16, VP64, Viper, CITED2, CITED4, CREB3 or functional fragments thereof.

3. The expression cassette of embodiment 1 or 2, wherein TAD1 and TAD2 are the same TAD.

4. The expression cassette of embodiment 3, wherein TAD1 and TAD2 are CITED2, or a functional fragment thereof.

5. The expression cassette of embodiment 3, wherein TAD1 and TAD2 are CITED4, or a functional fragment thereof.

6. The expression cassette of any one of embodiments 1-5, wherein TAD3 and TAD4 are the same TAD.

7. The expression cassette of embodiment 6, wherein TAD3 and TAD4 are CITED2, or a functional fragment thereof.

8. The expression cassette of embodiment 6, wherein TAD3 and TAD4 are CITED4, or a functional fragment thereof.

9. The expression cassette of embodiment 1 or 2, wherein TAD1, TAD2, TAD3 and TAD4 are the same TAD.

10. The expression cassette of embodiment 9, wherein TAD1, TAD2, TAD3 and TAD4 are CITED2, or a functional fragment thereof.

11. The expression cassette of embodiment 9, wherein TAD1, TAD2, TAD3 and TAD4 are CITED4, or a functional fragment thereof.

12. The expression cassette of any one of embodiments 1-11, wherein there is no linker between the at least two TAD domains.

13. The expression cassette of any one of embodiments 1-11, wherein there is a linker between the at least two TAD domains.

14. The expression cassette of embodiment 13, wherein the linker is GGSGGGSG (SEQ ID NO: 177) or GGSGGGSGGGGSGGGSG (SEQ ID NO: 178).

15. The expression cassette of any one of embodiments 1-14, wherein the DBD binds to a genomic region having 18-27 nucleotides.

16. The expression cassette of any one of embodiments 1-15, wherein the DBD comprises at least 80% sequence identity to its closest human counterpart.

17. The expression cassette of embodiment 16, wherein the DBD comprises at least 90% sequence identity to its closest human counterpart.

18. The expression cassette of any one of embodiments 1-15, wherein the DBD and the at least two TAD each comprise at least 80% sequence identity to their closest human counterparts.

19. The expression cassette of embodiment 18, wherein the DBD and the at least two TAD each comprise at least 90% sequence identity to their closest human counterparts.

20. The expression cassette of any one of embodiments 1-15, wherein the DBD comprises a guide RNA and a nuclease inactivated Cas protein.

21. The expression cassette of embodiment 20, wherein the nuclease inactivated Cas protein is a nuclease inactivated Cas9.

22. The expression cassette of any one of embodiments 1-19, wherein the DBD comprises a zinc finger domain.

23. The expression cassette of embodiment 22, wherein the DBD comprises six to nine zinc finger domains.

24. The expression cassette of embodiment 23, wherein the DBD comprises six zinc fingers.

25. The expression cassette of embodiment 24, wherein the DBD binds to a genomic region having 18 nucleotides.

26. The expression cassette of embodiment 23, wherein the DBD comprises nine zinc fingers.

27. The expression cassette of embodiment 26, wherein the DBD binds to a genomic region having 27 nucleotides.

28. The expression cassette of any one of embodiments 1-19 or 22-27, wherein the DBD comprises a sequence having at least 95% sequence identity to any of SEQ ID NOs: 148-151.

29. The expression cassette of embodiment 28, wherein the DBD comprises a sequence having any one of SEQ ID NOs: 148-151.

30. The expression cassette of any one of embodiments 1-19 or 22-29, wherein the DBD is derived from human EGR1 or human EGR3.

31. The expression cassette of any one of embodiments 1-19 or 22-30, wherein the DBD comprises a sequence having at least 90% identity to any one of SEQ ID NOs: 77-98.

32. The expression cassette of embodiment 31, wherein the DBD comprises SEQ ID NOs: 77-98.

33. The expression cassette of embodiment 1, wherein the DBD comprises a sequence having at least 90% identity to SEQ ID NO: 92.

34. The expression cassette of embodiment 33, wherein the DBD comprises SEQ ID NO: 92.

35. The expression cassette of embodiment 1, wherein the non-naturally occurring transcription factor comprises a sequence having at least 90% identity to SEQ ID NO: 130 or 131.

36. The expression cassette of embodiment 35, wherein the non-naturally occurring transcription factor comprises SEQ ID NO: 130 or 131.

37. The expression cassette of embodiment 1, comprising a nucleotide sequence having at least 90% identity to any one of SEQ ID NOs: 72 or 73.

38. The expression cassette of embodiment 37, comprising a nucleotide sequence of any one of SEQ ID NOs: 72 or 73.

39. The expression cassette of any one of embodiments 1-38, wherein the expression cassette further comprises a regulatory element that drives expression of the transcription factor at a higher level in PV neurons than in other cell types.

40. The expression cassette of embodiment 39, wherein the regulatory element comprises any one of SEQ ID NOs: 1-4.

41. The expression cassette of embodiment 40, wherein the regulatory element comprises SEQ ID NO: 2 or 3.

42. The expression cassette of any one of embodiments 1-41, wherein the expression cassette further comprises a PV selective microRNA binding site.

43. The expression cassette of embodiment 42, wherein the PV selective microRNA binding site comprises at least 90% identity to any one of SEQ ID NOs: 7, 14 or 15.

44. The expression cassette of embodiment 43, wherein the PV selective microRNA binding site comprises any one of SEQ ID NOs: 7, 14, or 15.

45. The expression cassette of any one of embodiments 1-44, wherein the expression cassette is a part of a viral vector.

46. The expression cassette of embodiment 45, wherein the viral vector is an AAV virus.

47. The expression cassette of embodiment 46, wherein the AAV virus is an AAV9 virus or a scAAV9 virus.

48. The expression cassette of embodiment 45, wherein the viral vector is a Lentivirus.

49. An expression cassette comprising a sequence encoding a non-naturally occurring transcription factor which increases expression of the SCN1A gene in a cell, wherein the non-naturally occurring transcription factor comprises a DNA binding domain operably linked to a transcription activating domain, wherein the DNA binding domain is a zinc finger protein comprising the sequence LEPGEKP - [YKCPECGKSFS X HQRTH TGEKP]n - YKCPECGKSFS X HQRTH - TGKKTS (SEQ ID NO: 147), and wherein there is no HA tag (SEQ ID NO: 171) between the DNA binding domain and the transcription activating domain.

50. The expression cassette of any one of embodiment 49, wherein the transcription activating domain comprises a VP16, VPR or VP64 sequence, or a functional fragment thereof.

51. The expression cassette of embodiment 50, wherein the transcription activating domain comprises VP64.

52. The expression cassette of any one of embodiments 49-51, wherein the DNA binding domain binds to a genomic region having 18-27 nucleotides.

53. The expression cassette of any one of embodiments 49-52, wherein n = 6 to 9.

54. The expression cassette of embodiment 53, wherein n = 6.

55. The expression cassette of embodiment 54, wherein the DNA binding domain binds to a genomic region having 18 nucleotides.

56. The expression cassette of embodiment 53, wherein n = 9.

57. The expression cassette of embodiment 56, wherein the DNA binding domain binds to a genomic region having 27 nucleotides.

58. The expression cassette of any one of embodiments 49-57, wherein the DNA binding domain comprises a sequence having at least 95% sequence identity to any of SEQ ID NOs: 148-151.

59. The expression cassette of embodiment 58, wherein the DNA binding domain comprises a sequence having any one of SEQ ID NOs: 148-151.

60. The expression cassette of any one of embodiments 49-59, wherein the DNA binding domain comprises a sequence having at least 90% identity to any one of SEQ ID NOs: 77-91.

61. The expression cassette of embodiment 60, wherein the DNA binding domain comprises any one of SEQ ID NOs: 77-91.

62. The expression cassette of any one of embodiments 49-61, wherein the expression cassette further comprises a regulatory element that drives expression of the transcription factor at a higher level in PV neurons than in other cell types.

63. The expression cassette of embodiment 62, wherein the regulatory element comprises any one of SEQ ID NOs: 1-4.

64. The expression cassette of embodiment 63, wherein the regulatory element comprises SEQ ID NO: 2 or 3.

65. The expression cassette of embodiment 49, wherein the non-naturally occurring transcription factor comprises a sequence having at least 90% identity to SEQ ID NO: 127.

66. The expression cassette of embodiment 65, wherein the non-naturally occurring transcription factor comprises SEQ ID NO: 127.

67. The expression cassette of embodiment 49, comprising a nucleotide sequence having at least 90% identity to any one of SEQ ID NOs: 70 or 71.

68. The expression cassette of embodiment 67, comprising a nucleotide sequence of any one of SEQ ID NOs: 70 or 71.

69. The expression cassette of any one of embodiments 1-68, wherein the expression cassette further comprises a PV selective microRNA binding site.

70. The expression cassette of embodiment 69, wherein the PV selective microRNA binding site comprises at least 90% identity to any one of SEQ ID NOs: 7, 14 or 15.

71. The expression cassette of embodiment 70, wherein the PV selective microRNA binding site comprises any one of SEQ ID NOs: 7, 14, or 15.

72. The expression cassette of any one of embodiments 49-71, wherein the expression cassette is a part of a viral vector.

73. The expression cassette of embodiment 72, wherein the viral vector is an AAV virus.

74. The expression cassette of embodiment 73, wherein the AAV virus is an AAV9 virus or a scAAV9 virus.

75. The expression cassette of embodiment 72, wherein the viral vector is a Lentivirus.

76. A polynucleotide comprising a PV selective microRNA binding site comprising a sequence having at least 80% sequence identity to SEQ **ID** NO: 14 or 15, wherein the microRNA binding site reduces expression of the transgene in excitatory neurons.

77. The polynucleotide of embodiment 76, wherein the PV selective microRNA binding site comprises SEQ ID NO: 14.

78. The polynucleotide of embodiment 76, wherein the PV selective microRNA binding site comprises SEQ ID NO: 15.

79. An expression cassette comprising the PV selective microRNA binding site of any one of embodiments 76-78 and a promoter and/or enhancer.

80. The expression cassette of embodiment 79, wherein the promoter and/or enhancer is a PV selective regulatory element that drives expression of the transgene at a higher level in parvalbumin (PV) neurons than in other cell types.

81. The expression cassette of embodiment 80, wherein the PV selective regulatory element is operably linked to a transgene.

82. An expression cassette comprising a regulatory element operably linked to a transgene and at least one microRNA binding site, wherein the regulatory element drives expression of the transgene at a higher level in parvalbumin (PV) neurons than in other cell types, wherein the microRNA binding site reduces expression of the transgene in excitatory neurons, and wherein the expression cassette does not comprise SEQ ID NO: 67.

83. The expression cassette of embodiment 82, wherein the microRNA binding site comprises at least one binding site for MIR128 (SEQ ID NO: 9).

84. The expression cassette of embodiment 82 or 83, wherein the microRNA binding site comprises at least one binding site for MIR221 (SEQ ID NO: 11).

85. The expression cassette of any one of embodiments 82-84, wherein the microRNA binding site comprises at least one binding site for MIR222 (SEQ ID NO: 13).

86. The expression cassette of embodiment 82, wherein the microRNA binding site comprises at least one binding site for MIR128 (SEQ ID NO: 9) and at least one binding site for MIR221 (SEQ ID NO: 11).

87. The expression cassette of embodiment 82, wherein the microRNA binding site comprises at least one binding site for MIR128 (SEQ ID NO: 9), at least one binding site for MIR221 (SEQ ID NO: 11), and at least one binding site for MIR222 (SEQ ID NO: 13).

88. The expression cassette of any one of embodiments 82-84, wherein the microRNA binding site comprises a sequence having at least 90% identity to any one of SEQ ID NOs: 7, 14 or 15.

89. The expression cassette of embodiment 88, wherein the microRNA binding site comprises SEQ ID NO: 7.

90. The expression cassette of embodiment 89, wherein the microRNA binding site comprises SEQ ID NO: 14.

91. The expression cassette of embodiment 89, wherein the microRNA binding site comprises SEQ ID NO: 15.

92. The expression cassette of any one of embodiments 81-91, wherein the transgene encodes a polypeptide comprising a non-naturally occurring transcription factor which increases expression of the SCN1A gene in a cell.

93. The expression cassette of embodiment 92, wherein the transcription factor binds to a genomic region having 18-27 nucleotides.

94. The expression cassette of any one of embodiments 92 or 93, wherein the transcription factor comprises a DNA binding domain.

95. The expression cassette of any one of embodiments 92-94, wherein the transcription factor comprises a DNA binding domain and a transcription activating domain.

96. The expression cassette of any one of embodiments 92-95, wherein the DNA binding domain comprises at least 80% sequence identity to its closest human counterpart.

97. The expression cassette of embodiment 96, wherein the DNA binding domain comprises at least 90% sequence identity to its closest human counterpart.

98. The expression cassette of any one of embodiments 92-96, wherein the DNA binding domain and the transcription activating domain both comprise at least 80% sequence identity to their closest human counterparts.

99. The expression cassette of embodiment 98, wherein the DNA binding domain and the transcription activating domain both comprise at least 90% sequence identity to their closest human counterparts.

100. The expression cassette of any one of embodiments 92-95, wherein the DNA binding domain comprises a guide RNA and a nuclease inactivated Cas protein.

101. The expression cassette of embodiment 100, wherein the nuclease inactivated Cas protein is a nuclease inactivated Cas9.

102. The expression cassette of any one of embodiments 92-99, wherein the DNA binding domain comprises a zinc finger domain.

103. The expression cassette of embodiment 102, wherein the DNA binding domain comprises six to nine zinc finger domains.

104. The expression cassette of embodiment 103, wherein the DNA binding domain comprises six zinc fingers.

105. The expression cassette of embodiment 104, wherein the DNA binding domain binds to a genomic region having 18 nucleotides.

106. The expression cassette of embodiment 103, wherein the DNA binding domain comprises nine zinc fingers.

107. The expression cassette of embodiment 106, wherein the DNA binding domain binds to a genomic region having 27 nucleotides.

108. The expression cassette of any one of embodiments 92-99 or 102-107, wherein the DNA binding domain comprises a sequence having at least 95% sequence identity to any of SEQ ID NOs: 148-151.

109. The expression cassette of embodiment 108, wherein the DNA binding domain comprises a sequence having any one of SEQ ID NOs: 148-151.

110. The expression cassette of any one of embodiments 92-99 or 102-109, wherein the DNA binding domain comprises a sequence having at least 90% identity to any one of SEQ ID NOs: 92-98.

111. The expression cassette of embodiment 110, wherein the DNA binding domain comprises any one of SEQ ID NOs: 92-98.

112. The expression cassette of any one of embodiments 92-99 or 102-111, wherein the DNA binding domain is a zinc finger protein comprising the sequence LEPGEKP - [YKCPECGKSFS X HQRTH TGEKP]n - YKCPECGKSFS X HQRTH - TGKKTS (SEQ ID NO: 147).

113. The expression cassette of embodiment 111 or 112, wherein the DNA binding domain comprises a sequence having at least 90% identity to any one of SEQ ID NOs: 77-91.

114. The expression cassette of any one of embodiments 113, wherein the DNA binding domain comprises any one of SEQ ID NOs: 77-91.

115. The expression cassette of any one of embodiments 92-99 or 102-111, wherein the DNA binding domain is derived from human EGR1 or human EGR3.

116. The expression cassette of any one of embodiments 95-99, wherein the transcription activating domain comprises a VP16, VPR, VP64, CITED2, CITED4, or CREB3 sequence, or a functional fragment thereof.

117. The expression cassette of any one of embodiments 92-99, wherein the transcription activating domain comprises a human CITED2, CITED4, or CREB3 sequence, or a functional fragment thereof.

118. The expression cassette of any one of embodiments 81-117, wherein the regulatory element comprises a sequence having any one of SEQ ID NOs: 1-4.

119. The expression cassette of embodiment 118, wherein the regulatory element comprises a sequence having SEQ ID NO: 2 or 3.

120. The expression cassette of embodiment 81 or 82, wherein the non-naturally occurring transcription factor comprises a sequence having at least 90% identity to any one of SEQ ID NOs: 105, 106, and 127-129.

121. The expression cassette of embodiment 120, wherein the non-naturally occurring transcription factor comprises any one of SEQ ID NOs: 105, 106, and 127-129.

122. The expression cassette of embodiments 81, 82 or 120, wherein the transgene comprises a nucleotide sequence having at least 90% identity to any one of SEQ ID NOs: 71, 74, 75, 76 or 184.

123. The expression cassette of embodiment 122, wherein the transgene comprises any one of SEQ ID NO: 71, 74, 75, 76 or 184.

124. The expression cassette of any one of embodiments 81-123, wherein the expression cassette is a part of a viral vector.

125. The expression cassette of embodiment 124, wherein the viral vector is an AAV virus.

126. The expression cassette of embodiment 125, wherein the AAV virus is an AAV9 virus or a scAAV9 virus.

127. The expression cassette of embodiment 124, wherein the viral vector is a Lentivirus.

128. A method for selective expression of a transgene in parvalbumin (PV) neurons of a primate comprising administering to a primate a viral vector comprising a transgene and at least one microRNA binding site, wherein the microRNA binding site reduces expression of the transgene in excitatory neurons.

129. The method of embodiment 128, wherein the viral vector further comprises a regulatory element operably linked to the transgene, wherein the regulatory element drives expression of the transgene at a higher level in parvalbumin (PV) neurons than in other cell types.

130. The method of embodiments 128 or 129, wherein the microRNA binding site comprises at least one binding site for MIR128 (SEQ ID NO: 9).

131. The method of any one of embodiments 128-130, wherein the microRNA binding site comprises at least one binding site for MIR221 (SEQ ID NO: 11).

132. The method of any one of embodiments 128-131, wherein the microRNA binding site comprises at least one binding site for MIR222 (SEQ ID NO: 13).

133. The method of embodiment 128, wherein the microRNA binding site comprises at least one binding site for MIR128 (SEQ ID NO: 9) and at least one binding site for MIR221 (SEQ ID NO: 11).

134. The method of embodiment 128, wherein the microRNA binding site comprises at least one binding site for MIR128 (SEQ ID NO: 9), at least one binding site for MIR221 (SEQ ID NO: 11), and at least one binding site for MIR222 (SEQ ID NO: 13).

135. The method of any one of embodiments 128-134, wherein the microRNA binding site comprises a sequence that is at least 90% identical to any one of SEQ ID NOs: 7, 14 or 15.

136. The method of embodiment 135, wherein the microRNA binding site comprises SEQ ID NO: 7.

137. The method of embodiment 135, wherein the microRNA binding site comprises SEQ ID NO: 14.

138. The method of embodiment 135, wherein the microRNA binding site comprises SEQ ID NO: 15.

139. The method of any one of embodiments 128-138, wherein the transgene comprises a sequence encoding a non-naturally occurring transcription factor which increases expression of the SCN1A gene in a cell.

140. The method of embodiment 139, wherein the transcription factor binds to a genomic region having 18-27 nucleotides.

141. The method of any one of embodiments 139 or 140, wherein the transcription factor comprises a DNA binding domain.

142. The method of any one of embodiments 139-141, wherein the transcription factor comprises a DNA binding domain and a transcription activating domain.

143. The method of any one of embodiments 139-142, wherein the DNA binding domain comprises at least 80% sequence identity to its closest human counterpart.

144. The method of embodiment 143, wherein the DNA binding domain comprises at least 90% sequence identity to its closest human counterpart.

145. The method of any one of embodiments 142 or 143, wherein the DNA binding domain and the transcription activating domain both comprise at least 80% sequence identity to their closest human counterparts.

146. The method of embodiment 145, wherein the DNA binding domain and the transcription activating domain both comprise at least 90% sequence identity to their closest human counterparts.

147. The method of any one of embodiments 139-142, wherein the DNA binding domain comprises a guide RNA and a nuclease inactivated Cas protein.

148. The method of embodiment 147, wherein the nuclease inactivated Cas protein is a nuclease inactivated Cas9.

149. The method of any one of embodiments 139-146, wherein the DNA binding domain comprises a zinc finger domain.

150. The method of embodiment 149, wherein the DNA binding domain comprises six to nine zinc finger domains.

151. The method of embodiment 150, wherein the DNA binding domain comprises six zinc fingers.

152. The method of embodiment 151, wherein the DNA binding domain binds to a genomic region having 18 nucleotides.

153. The method of embodiment 150, wherein the DNA binding domain comprises nine zinc fingers.

154. The method of embodiment 153, wherein the DNA binding domain binds to a genomic region having 27 nucleotides.

155. The method of any one of embodiments 139-146 or 149-154, wherein the DNA binding domain comprises a sequence having at least 95% sequence identity to any of 148-151.

156. The method of embodiment 155, wherein the DNA binding domain comprises a sequence having any one of SEQ ID NOs: 148-151.

157. The method of any one of embodiments 139-146 or 149-156, wherein the DNA binding domain is derived from human EGR1 or human EGR3.

158. The method of any one of embodiments 139-146 or 149-157, wherein the DNA binding domain comprises a sequence having at least 90% identity to any one of SEQ ID NOs: 92-98.

159. The method of embodiment 158, wherein the DNA binding domain comprises any one of SEQ ID NOs: 92-98.

160. The method of any one of embodiments 139-146 or 149-156, wherein the DNA binding domain is a zinc finger protein comprising the sequence LEPGEKP - [YKCPECGKSFS X HQRTH TGEKP]n - YKCPECGKSFS X HQRTH - TGKKTS (SEQ ID NO: 147).

161. The method of any one of embodiments 160, wherein the DNA binding domain comprises a sequence having at least 90% identity to any one of SEQ ID NOs: 77-91.

162. The method of any one of embodiments 161, wherein the DNA binding domain comprises any one of SEQ ID NOs: 77-91.

163. The method of any one of embodiments 142-162, wherein the transcription activating domain comprises a VP16, VPR, VP64, CITED2, CITED4, or CREB3 sequence, or a functional fragment thereof.

164. The method of embodiment 163, wherein the transcription activating domain comprises a human CITED2, CITED4, or CREB3 sequence, or a functional fragment thereof.

165. The method of any one of embodiments 128-164, wherein the regulatory element comprises a sequence having any one of SEQ ID NOs: 1-4.

166. The method of embodiment 165, wherein the regulatory element comprises a sequence having SEQ ID NO: 2 or 3.

167. The method of embodiment 139, wherein the non-naturally occurring transcription factor comprises a sequence having at least 90% identity to any one of SEQ ID NOs: 105, 106, and 127-129.

168. The method of embodiment 167, wherein the non-naturally occurring transcription factor comprises any one of SEQ ID NOs: 105, 106, and 127-129.

169. The method of embodiment 139, wherein the transgene comprises a nucleotide sequence having at least 90% identity to any one of SEQ ID NOs: 71, 74, 75, 76 or 184.

170. The method of embodiment 169, wherein the transgene comprises any one of SEQ ID NOs: 71, 74, 75, 76 or 184.

171. The method of any one of embodiments 128-170, wherein the viral vector is an AAV virus.

172. The method of embodiment 171, wherein the AAV virus is an AAV9 virus or a scAAV9 virus.

173. The method of any one of embodiments 128-170, wherein the viral vector is a Lentivirus.

174. The method of any one of embodiments 128-173, wherein the primate is a human.

175. The method of any one of embodiments 128-173, wherein the primate is a non-human primate.

176. The method of embodiment 175, wherein the non-human primate is an old world monkey, an orangutan, a gorilla, a chimpanzee, a marmoset, a crab-eating macaque, a rhesus macaque or a pig-tailed macaque.

177. An expression cassette comprising a sequence encoding a non-naturally occurring transcription factor which increases expression of the SCN1A gene in a cell, wherein the non-naturally occurring transcription factor comprises a sequence having at least 90% identity to SEQ ID NO: 128 or 129.

178. The expression cassette of embodiment 177, wherein the non-naturally occurring transcription factor comprises SEQ ID NO: 128 or 129.

179. A method of increasing expression of SCN1A in a cell by administering an expression cassette of any one of embodiments 1-127 or 177-178.

180. The method of embodiment 179, wherein the cell is a PV neuron.

181. The method of any one of embodiments 179 or 180, wherein the cell is within a subject.

182. The method of embodiment 181, wherein the subject is a mammal.

183. The method of embodiment 182, wherein the subject is a human.

184. The method of embodiment any one of embodiments 179-183, wherein increasing expression of SCN1A treats a disease, disorder or symptom.

185. The method of embodiment 184, wherein the disorder is a central nervous system disorder.

186. The method of embodiment 185, wherein the disorder is epilepsy associated with SCN1A haploinsufficiency.

187. The method of embodiment 186, wherein the haploinsufficiency is the result of the subject being heterozygous for a loss of function mutation of the SCN1A gene.

188. The method of embodiment 187, wherein the disorder is epilepsy associated with an insertion, deletion, inversion, or substitution in the SCN1A gene.

189. The method of embodiment 188, wherein the disorder is epilepsy associated with a point mutation in the SCN1A gene.

190. The method of any one of embodiments 186-189, wherein the disorder is Dravet Syndrome.

191. The method of any one of embodiments 185-190, wherein a symptom of the central nervous system disorder is neuronal hyperactivity.

192. The method of embodiment 191, wherein treating the central nervous system disorder comprises reducing neuronal hyperactivity.

193. The method of any one of embodiments 185-190, wherein a symptom of the central nervous system disorder is seizures.

194. The method of embodiment 193, wherein treating the central nervous system disorder comprises reducing the frequency of seizures.

195. The method of any one of embodiments 193 or 194, wherein treating the central nervous system disorder comprises reducing the severity of seizures.

196. The method of embodiment 179, wherein the cell is a neuronal cell.

197. The method of embodiment 196, wherein the neuronal cell is selected from the group consisting of unipolar, bipolar, multipolar, or pseudounipolar neurons.

198. The method of embodiment 196, wherein the cell is GABAergic neuron.

199. The method of embodiment 179, wherein the cell is a non-neuronal cell.

200. The method of embodiment 199, wherein the cell is a glial cell.

201. The method of embodiment 200, wherein the glial cell is selected from the group consisting of astrocytes, oligodendrocytes, ependymal cells, Schwann cells, and satellite cells.

202. A method of increasing expression of SCN1A in the CNS by administering an expression cassette of any one of embodiments 1-127 or 177-178.

203. The method of any one of embodiments 202, wherein the increased expression of SCN1A occurs in the brain.

204. The method of embodiment 203, wherein the increased expression of SCN1A occurs in the frontal cortex, parietal cortex, temporal cortex, hippocampus, medulla, and/or occipital cortex.

205. The method of any one of embodiments 202, wherein the increased expression of SCN1A occurs in the spine.

206. The method of embodiment 205, wherein the increased expression of SCN1A occurs in the spinal cord and/or dorsal root ganglion.

**Claims**

1. A polynucleotide comprising a nucleic acid sequence encoding an engineered transcription factor (eTF) comprising a DNA binding domain and a transcription activating domain, wherein the DNA binding domain comprises SEQ ID NO: 77, wherein the transcription activating domain comprises a VP64, VPR, CITED2, CITED4, or CREB3 sequence, wherein there is no HA tag (SEQ ID NO: 171) between the DNA binding domain and the transcription activating domain, and wherein the eTF increases expression of a SCN1A gene in a cell.

2. An expression cassette comprising the polynucleotide of claim 1, and a regulatory element that drives expression of the eTF encoded by the polynucleotide.

3. The expression cassette of claim 2, wherein the regulatory element drives expression of the eTF at a higher level in parvalbumin (PV) neurons than in other cell types, optionally wherein the regulatory element comprises any one of SEQ ID NOS: 1-4.

4. The expression cassette of claim 2 or 3, further comprising one or more microRNA binding sites for one or more microRNAs that promote parvalbumin (PV) neuron selective expression.

5. The expression cassette of claim 4, wherein the one or more microRNAs that promote PV neuron selective expression are miR-128, miR-221, or miR-222, optionally wherein the PV selective microRNA binding site comprises SEQ ID NO: 7, 14, or 15.

6. The expression cassette of any one of claims 2 to 5, where the expression cassette further comprises a polyA sequence positioned downstream of the polynucleotide encoding the eTF, optionally wherein the polyA sequence comprises an artificial polyA sequence, a bovine growth hormone polyA sequence, an SV40 early polyA sequence, an SV40 late polyA sequence, a rabbit beta globin polyA sequence, an HSV thymidine kinase polyA sequence, a protamine gene polyA sequence, an adenovirus 5 Elb polyA sequence, a growth hormone polyA sequence, or a PBGD polyA sequence.

7. A viral vector comprising (i) the polynucleotide of claim 1, or (ii) the expression cassette of any one of claims 2 to 6.

8. The viral vector of claim 7, wherein the viral vector is an adeno-associated virus (AAV) vector, optionally wherein the AAV vector comprises 5' and 3' inverted terminal repeat (ITR) sequences from an AAV serotype 2, optionally wherein the AAV vector is a self-complementary AAV (scAAV) vector.

9. A viral vector particle comprising the viral vector of claim 7 or 8, optionally wherein the capsid is derived from AAV1, AAV2, AAV3, AAV4, AAV5, AAV6, AAV7, AAV8, AAV9, AAVI10, AAV11, AAV12, AAV13, rh10, avian AAV, bovine AAV, canine AAV, equine AAV, primate AAV, non-primate AAV, or ovine AAV, or hybrids thereof.

10. The viral particle of claim 9, wherein the viral vector comprises the expression cassette of claim 5 flanked by 5' and 3' ITR sequences from an AAV serotype 2 and the capsid is an AAV9 serotype capsid.

11. A pharmaceutical composition comprising the expression cassette of any one of claims 2 to 6, the viral vector of claim 7 or 8, or the viral particle of claim 9 or 10, and one or more pharmaceutically acceptable excipients.

12. The expression cassette of any one of claims 2 to 6, the viral vector of claim 7 or 8, the viral particle of claim 9 or 10, or the pharmaceutical composition of claim 11 for use in therapy of a subject.

13. The expression cassette, viral vector, viral particle, or pharmaceutical composition for use according to claim 12, wherein the subject has a central nervous system disorder, optionally wherein:

    (i) the disorder is epilepsy associated with SCN1A haploinsufficiency;
    (ii) a symptom of the central nervous system disorder is neuronal hyperactivity; and/or
    (iii) a symptom of the central nervous system disorder is seizures.

14. The expression cassette, viral vector, viral particle, or pharmaceutical composition for use according to claim 13, wherein the epilepsy associated with SCN1A haploinsufficiency is Dravet Syndrome.

**FIG. 1**

**Different target sites**

**FIG. 2A**

**FIG. 2B**

**FIG. 2C**

**FIG. 3A**

**FIG. 3B**

**FIG. 4**

**FIG. 5A**

**FIG. 5B**

**FIG. 6A**

**FIG. 6B**

**FIG. 6C**

**FIG. 6D**

**FIG. 6E**

**FIG. 6F**

**FIG. 6G**

**FIG. 6H**

**FIG. 7A**

**FIG. 7B**

**FIG. 7C**

**FIG. 7D**

**FIG. 7E**

**FIG. 8**

## FIG. 9

## FIG. 10

FIG. 11

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62854238
- US 62857727
- US 63008569
- US 6479626 B
- US 6903185 B
- US 7153949 B
- WO 2018213786 A
- WO 2018187363 A
- WO 2018126116 A
- WO 2007069666 A
- WO 2008118820 A

- WO 0138362 A
- WO 0185938 A
- WO 03076601 A
- WO 2005042728 A
- WO 2006108846 A
- WO 2008129058 A
- WO 2008142124 A
- US 6566118 B
- US 6989264 B
- US 8137948 B
- WO 2010148143 A

### Non-patent literature cited in the description

- **KARLIN** ; **ALTSCHUL**. *Proc. Natl. Acad. Sci. USA*, 1990, vol. 87, 2264-2268
- **ALTSCHUL et al.** *J. Mol. Biol.*, 1990, vol. 215, 403-410
- **KARLIN** ; **ALTSCHUL**. *Proc. Natl. Acad. Sci. USA*, 1993, vol. 90, 5873-5877
- **ALTSCHUL et al.** *Nucleic Acids Res.*, 1997, vol. 25, 3389-3402
- **WOOTTON** ; **FEDERHEN**. *Computers and Chemistry*, 1993, vol. 17, 149-163
- **LIU Q et al.** Design of polydactyl zinc-finger proteins for unique addressing within complex genomes. *Proc Natl Acad Sci USA.*, 1997, vol. 94 (11), 5525-30
- **WRIGHT DA et al.** Standardized reagents and protocols for engineering zinc finger nucleases by modular assembly. *Nat Protoc . Nat Protoc.*, 2006, vol. 1 (3), 1637-52
- **CA GERSBACH** ; **T GAJ**. Synthetic Zinc Finger Proteins: The Advent of Targeted Gene Regulation and Genome Modification Technologies. *Am Chem Soc*, 2014, vol. 47, 2309-2318
- Flexible algorithm for identifying specific Cas9 targets in genomes. **AACH et al.** BioRxiv. Cold Spring Harbor Labs, 2014
- **BAE et al.** Cas-OFFinder: a fast and versatile algorithm that searches for potential off-target sites of Cas9 RNA-guided endonucleases.. *Bioinformatics.*, 2014, vol. 30 (10), 1473-1475
- **DOENCH, J. G. et al.** Optimized sgRNA design to maximize activity and minimize off-target effects of CRISPR-Cas9. *Nat Biotech*, 2016, vol. 34, 184-191

- **GRATZ et al.** Highly specific and efficient CRISPR-Cas9-catalyzed homology-directed repair in Drosophila.. *Genetics*, 2014, vol. 196 (4), 961-971
- **HEIGWER et al.** E-CRISP: fast CRISPR target site identification.. *Nat Methods.*, 2014, vol. 11 (2), 122-123
- **MA et al.** A guide RNA sequence design platform for the CRISPR/Cas9 system for model organism genomes.. *Biomed Res Int.*, 2013
- **MONTAGUE et al.** CHOPCHOP: a CRISPR/Cas9 and TALEN web tool for genome editing. *Nucleic Acids Res.*, 2014, vol. 42 (W1), W401-W407
- **LIU et al.** CRISPR-ERA: a comprehensive design tool for CRISPR-mediated gene editing, repression and activation. *Bioinformatics*, 2015, vol. 31 (22), 3676-3678
- **RAN et al.** In vivo genome editing using Staphylococcus aureus Cas9.. *Nature*, 2015, vol. 520 (7546), 186-191
- **WU et al.** Target specificity of the CRISPR-Cas9 system. *Quant Biol.*, 2015, vol. 2 (2), 59-70
- **XIAO et al.** CasOT: a genome-wide Cas9/gRNA off-target searching tool.. *Bioinformatics*, 2014, vol. 30 (8), 1180-1182
- **ZETSCHE et al.** Cpf1 is a single RNA-guided endonuclease of a Class 2 CRISPR-Cas System.. *Cell*, 2015, vol. 163 (3), 759-771
- **T. CERMAK et al.** *Nucleic Acids Research*, 2011, vol. 39 (12), e82
- **F. ZHANG F et al.** *Nature Biotechnology*, 2011, vol. 29 (2), 149-53
- **R. MORBITZER et al.** *Nucleic Acids Research*, 2011, vol. 39 (13), 5790-9

- **T. LI et al.** *Nucleic Acids Research*, 2011, vol. 39 (14), 6315-25
- **R. GEISSLER et al.** *PLOS One.*, 2011, vol. 6 (5), e19509
- **E. WEBER et al.** *PLOS One.*, 2011, vol. 6 (5), e19722
- **D. HOOVER D**. *Methods in Molecular Biology.*, 2012, vol. 852, 215-23
- **C. DINGWALL et al.** *J. Cell Biol.*, 1988, vol. 107, 841-9
- **J.P. MAKKERH et al.** *Curr Biol.*, 1996, vol. 6, 1025-7
- **M. RAY et al.** *Bioconjug. Chem.*, 2015, vol. 26, 1004-7
- **BLOUIN V et al.** *J Gene Med.*, 2004, vol. 6, S223-S228
- **GRIMM D. et al.** *Hum. Gene Ther.*, 2003, vol. 7, 839-850
- **URABE et al.** *Hum. Gene Ther.*, 2002, vol. 13, 1935-1943
- Series: Methods in Molecular Biology. Humana Press (Springer), 2011, vol. 737
- **M. GIACCA.** Gene Therapy.. Springer-Verlag, 2010
- Viral Vectors for Gene Transfer: Current Status of Gene Therapeutics. **HEILBRONN R.** ; **WEGER S.** Drug Delivery, Handbook of Experimental Pharmacology. Springer-Verlag, 2010, vol. 197, 143-170
- Adeno-Associated Virus: Methods and Protocols. Humana Press (Springer), 2011
- Recent developments in adeno-associated virus technology. **BUNNING H. et al.** J. Gene Med.. 2008, vol. 10, 717-733
- Adenovirus: Methods and Protocols.. Humana Press (Springer), 2014
- **BUNNING H et al.** *J Gene Med*, 2008, vol. 10, 717-733
- **M. HOCQUEMILLER et al.** *Hum Gene Ther*, 2016, vol. 27 (7), 478-496
- **CONWAY, J E et al.** *J. Virology*, 1997, vol. 71 (11), 8780-8789
- **AYUSO E et al.** *Curr. Gene Ther.*, 2010, vol. 10, 423-436
- **XIAO et al.** *Journal of Virology*, 1998, vol. 72, 2224-2232
- **LOCK M et al.** *Hum. Gene Ther.*, 2010, vol. 21, 1273-1285
- **M. LOCK et al.** *Hum Gene Ther Methods.*, April 2014, vol. 25 (2), 115-25
- **HAWKINS et al.** *Scientific Reports*, 2017, vol. 7, 15327
- **MILLER et al.** *Genes Brain Behav.*, February 2014, vol. 13 (2), 163-72
- **OGIWARA et al.** *J. Neuroscience*, 2007, vol. 27, 5903-5914